(19) Europäisches Patentamt
European Patent Office
Office européen des brevets

(11) EP 1 888 491 B1

(12) # EUROPÄISCHE PATENTSCHRIFT

(45) Veröffentlichungstag und Bekanntmachung des Hinweises auf die Patenterteilung:
**01.03.2017 Patentblatt 2017/09**

(21) Anmeldenummer: **06724806.2**

(22) Anmeldetag: **11.05.2006**

(51) Int Cl.:
***C07C 9/16*** *(2006.01)* ***A61Q 17/04*** *(2006.01)*
***C07C 9/22*** *(2006.01)* ***A61K 47/06*** *(2006.01)*
***C07C 5/03*** *(2006.01)* ***A61Q 19/10*** *(2006.01)*
***A61K 8/31*** *(2006.01)* ***A61Q 15/00*** *(2006.01)*
***A61Q 5/00*** *(2006.01)* ***C10L 1/04*** *(2006.01)*
***A61Q 1/02*** *(2006.01)* ***A61Q 1/04*** *(2006.01)*
***A61Q 1/12*** *(2006.01)* ***A61Q 5/02*** *(2006.01)*
***A61Q 5/06*** *(2006.01)* ***A61Q 5/10*** *(2006.01)*
***A61Q 5/12*** *(2006.01)* ***A61Q 9/02*** *(2006.01)*
***A61Q 11/00*** *(2006.01)* ***A61Q 19/00*** *(2006.01)*

(86) Internationale Anmeldenummer:
**PCT/EP2006/004461**

(87) Internationale Veröffentlichungsnummer:
**WO 2006/120003 (16.11.2006 Gazette 2006/46)**

(54) **ISOALKANGEMISCH, DESSEN HERSTELLUNG UND VERWENDUNG**

ISOALKANE MIXTURE, ITS PREPARATION AND USE

MELANGE D'ISOALCANES, SA FABRICATION ET SON UTILISATION

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **12.05.2005 DE 102005022021**
**03.04.2006 EP 06007065**

(43) Veröffentlichungstag der Anmeldung:
**20.02.2008 Patentblatt 2008/08**

(73) Patentinhaber: **BASF SE**
**67056 Ludwigshafen am Rhein (DE)**

(72) Erfinder:
- **LANGE, Arno**
  **67098 Bad Dürkheim (DE)**
- **ULONSKA, Armin**
  **67150 Niederkirchen (DE)**
- **WENDEL, Volker**
  **60529 Frankfurt (DE)**

(74) Vertreter: **BASF IP Association**
**BASF SE**
**ZRX-C6**
**67056 Ludwigshafen (DE)**

(56) Entgegenhaltungen:

| | |
|---|---|
| **WO-A-2004/091555** | **FR-A- 2 792 328** |
| **GB-A- 1 286 571** | **US-A- 4 225 743** |
| **US-A- 4 334 113** | **US-A- 4 520 008** |
| **US-A- 5 525 344** | **US-A1- 2001 051 686** |

- **A. ANSALDI: "Aliphatic Branched Chain Hydrocarbons" COSMETICS AND TOILETRIES MANUFACTURE WORLDWIDE, [Online] 2004, Seiten 2-6, XP002394736 Gefunden im Internet: URL:http://www.ctmw.com/pdfarticles/prespe rsearticle.pdf> [gefunden am 2006-08-08] in der Anmeldung erwähnt**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 888 491 B1

## Beschreibung

Die vorliegende Erfindung betrifft kosmetische oder pharmazeutische Mittel, die ein Isoalkangemisch enthalten und Verwendungen dieses kosmetischen oder pharmazeutischen Mittels.

Kohlenwasserstoffgemische, die kurzkettigen Olefine, z. B. mit 2 bis 6 Kohlenstoffatomen enthalten, sind im großtechnischen Maßstab erhältlich. So fällt z. B. bei der Aufarbeitung von Erdöl durch Steamcracken oder Fluidized Catalyst Cracking (FCC) ein als $C_4$-Schnitt bezeichnetes Kohlenwasserstoffgemisch mit einem hohen Gesamtolefingehalt an, wobei es sich im Wesentlichen um Olefine mit 4 Kohlenstoffatomen handelt. Solche $C_4$-Schnitte, d. h. Gemische aus isomeren Butenen und Butanen, eignen sich, gegebenenfalls nach einer vorherigen Abtrennung des Isobutens und Hydrierung des enthaltenen Butadiens, sehr gut zur Herstellung von Oligomeren, insbesondere von Octenen und Dodecenen.

Eine große Bedeutung haben die aus Olefingemischen mit überwiegend linearen Ausgangsolefinen erhältlichen im Wesentlichen linearen Oligomerengemische erlangt. Sie eignen sich z. B. als Dieselkraftstoffkomponente sowie als Zwischenprodukte zur Herstellung funktionalisierter, überwiegend linearer Kohlenwasserstoffe. So erhält man durch Hydroformylierung und anschließende Hydrierung der Olefinoligomere die entsprechenden Alkohole, die unter anderem als Ausgangsstoffe für Detergenzien und als Weichmacher verwendet werden. Für viele Einsatzbereiche, z. B. als Weichmacheralkohole, spielt der Verzweigungsgrad der Olefine eine entscheidende Rolle. Der Verzweigungsgrad wird dabei beispielsweise durch den ISO-Index beschrieben, der die mittlere Zahl der Methylverzweigungen der jeweiligen Olefinfraktion angibt. So tragen z. B. bei einer $C_8$-Fraktion die n-Octene mit 0, Methylheptene mit 1 und Dimethylhexene mit 2 zum ISO-Index der Fraktion bei. Je niedriger der ISO-Index ist, umso größer ist die Linearität der Moleküle in der jeweiligen Fraktion.

Es ist bekannt, zur Herstellung wenig verzweigter, ebenfalls olefinisch ungesättigter Oligomere aus niederen Olefinen Katalysatoren einzusetzen, die als aktive Komponente Metalle und ganz überwiegend Nickel enthalten. Heterogene Katalysatoren weisen dabei gegenüber homogenen den Vorteil auf, dass eine Abtrennung des Katalysators vom Reaktoraustrag entfällt. So ist z. B. aus der DE-A-43 39 713 (= WO 95/14647) ist ein Verfahren zum Oligomerisieren von unverzweigten $C_2$-bis $C_6$-Olefinen an einem Festbettkatalysator bei erhöhtem Druck und erhöhter Temperatur bekannt, wobei der Katalysator als wesentliche aktive Bestandteile 10 bis 70 Gew.-% Nickeloxid, 5 bis 30 Gew.-% Titandioxid und/oder Zirkondioxid, 0 bis 20 Gew.-% Aluminiumoxid und als Rest Siliciumoxid enthält. Weitere Oligomerisierungskatalysatoren und -verfahren sind z. B. in der WO 99/25668, WO 00/59849, WO 00/53546, WO 01/72670 und EP-A-1 457 475 beschrieben.

A. Ansaldi beschreibt in Cosmetics and Toiletries Manufacture Worldwide, S. 128 - 133 hochverzweigte Isoparaffine, die von Presperse Inc. unter der Bezeichnung Permethyl kommerziell vertrieben werden.

Neben Wasser zählen so genannte Ölkörper zu den wichtigsten Rohstoffen bei der Formulierung kosmetischer und pharmazeutischer Mittel. Dabei kommt eine breite Vielfalt von hydrophoben Komponenten sowohl als Grund- und Hilfsstoffe, wie auch als Wirkstoffe zum Einsatz. Zu diesen Komponenten zählen ganz allgemein natürliche Fette und Öle, Erdölprodukte, wie Paraffine, Silikonöle, Fettalkohole, Fettsäuren, etc. Schwierigkeiten bereitet dabei oft die Bereitstellung von Produkten mit einem komplexen Eigenschaftsprofil. So besteht ein Bedarf an Ölkörpern für kosmetische und pharmazeutische Mittel, die z. B. über sehr gute dermatologische Verträglichkeit verfügen, dem Haar und der Haut einen angenehmen Griff verleihen, eine gute Konditionierwirkung aufweisen (z. B. befähigt sind Anfassgefühl, Glanz und Erscheinungsform, Trocken- und Nasskämmbarkeit, etc. zu verbessern), mit einer Vielzahl anderer Wirk- und Hilfsstoffe verträglich sind und eine Einstellung der rheologischen Eigenschaften der Mittel in weitem Bereich (z. B. von flüssig bis gelförmig) ermöglichen. Zudem wünschen viele Verbraucher zunehmend Produkte, die nur einen dezenten Geruch aufweisen oder völlig frei von Parfümstoffen sind.

Die DE 10 2004 018 753 A1 beschreibt ein Verfahren zur Herstellung eines $C_{16}$-Alkangemischs, bei dem ein Buten-haltiger $C_4$-Kohlenwasserstoffstrom in Gegenwart eines Nickel-haltigen Katalysators oligomerisiert wird, dann die $C_{16}$-Olefinfraktion abgetrennt und die abgetrennte $C_{16}$-Fraktion hydriert wird. Beschrieben sind weiterhin $C_{16}$-Alkangemische, die einen Anteil von $C_{16}$-Alkanen von $\geq$ 95 Massen-% aufweisen und deren Verwendung. Dabei ist ganz allgemein, ohne Angaben zur Formulierung oder Stützung durch ein Ausführungsbeispiel, auch ein Einsatz in der Kosmetik erwähnt.

Die DE-OS 2360306 beschreibt ein Öl für kosmetische Zwecke, welches durch:

1. Polymerisieren von Isobuten oder eines isobutenhaltigen $C_4$-Olefingemischs in Gegenwart eines Friedel-Crafts-Katalysators,
2. Destillieren,
3. Hydrieren und
4. Wasserdampfdestillation zum Desodorieren

erhältlich ist.

Die WO 2004/091555 beschreibt ein kosmetisches Mittel, dass wenigstens ein verzweigtes $\alpha$-Olefin oder ein Hydrierungsprodukt davon enthält. Dabei weist das $\alpha$-Olefin wenigstens eine $C_2$- oder längerkettige Alkylverzweigung auf und ist durch Oligomerisierung bestimmter linearer oder verzweigter $\alpha$-Olefine in Gegenwart eines sauren Katalysators erhältlich. Nachteilig an diesen Produkten ist ihr hoher Verzweigungsgrad, ihr Gehalt an tert.-Butylgruppen und ihre Uneinheitlichkeit, so dass das damit erzielte Eigenschaftsprofil für einen Einsatz in kosmetischen und pharmazeutischen Formulierungen noch verbesserungswürdig ist. So weisen die eingesetzten Oligomere beispielsweise einen deutlichen Eigengeruch auf, der an Terpene erinnert.

Die US 2001/0051686 A1 beschreibt inverse Wasser-in-Öl-Latices und deren Verwendung als Verdicker für kosmetische Produkte, wobei die Ölphase ausgewählt ist unter Weißölen, Squalan, hydriertem Polyisobuten, Isohexadecan oder Isododecan.

Die US 4,334,113 beschreibt synthetische Kohlenwasserstoffoligomere, die durch Oligomerisierung von Olefinen in Gegenwart eines Wolframhexafluorid-Katalysators erhältlich sind. Die erhaltenen Produkte weisen etwa 20 bis 60 Kohlenstoffatome pro Molekül auf.

Die US 4,225,743 lehrt die Dimerisierung von Butenen, die einen hohen Anteil an Isobuten von 5 bis 55 Gew.-% enthalten. Demgegenüber wird zur Herstellung der erfindungsgemäßen Isoalkangemische ein $C_4$-Kohlenwasserstoffgemisch eingesetzt, das weniger als 3 Gew.-% Isobuten enthält. Der beanspruchte Gegenstand ist somit auch neu gegenüber D3.

Der vorliegenden Erfindung liegt die Aufgabe zu Grunde, gesättigte Kohlenwasserstoffe zur Verfügung zu stellen, die sich in vorteilhafter Weise als Ölkörper für kosmetische und pharmazeutische Mittel eignen. Sie sollen sich insbesondere zur Herstellung von Produkten eignen, die im Wesentlichen keinen auf den Ölkörper zurückzuführenden Eigengeruch aufweisen.

Überraschenderweise wurde nun gefunden, dass diese Aufgabe durch ein Isoalkangemisch mit einem mittleren Verzweigungsgrad gelöst wird.

Ein erster Gegenstand der Erfindung ist ein kosmetisches oder pharmazeutisches Mittel, enthaltend

A) wenigstens ein Isoalkangemisch, dessen 1 H-NMR-Spektrum im Bereich einer chemischen Verschiebung $\delta$ von 0,6 bis 1,0 ppm, bezogen auf Tetramethylsilan, ein Flächenintegral von 25 bis 70 %, bezogen auf die Gesamtintegralfläche, aufweist, das wenigstens 70 Gew.-% Alkane mit 8 bis 20 Kohlenstoffatomen enthält, erhalten durch ein Verfahren, bei dem man

a) ein $C_4$-Kohlenwasserstoffgemisch bereitstellt, das weniger als 3 Gew.-% Isobuten enthält,

b) das Kohlenwasserstoffeinsatzmaterial einer Oligomerisierung an einem Übergangsmetall-haltigen Katalysator unterwirft,

c) das in Schritt b) erhaltene Oligomerisierungsprodukt vollständig hydriert,

B) wenigstens einen kosmetisch oder pharmazeutisch akzeptablen Wirk- oder Effektstoff,

C) gegebenenfalls wenigstens einen weiteren, von B) verschiedenen kosmetisch oder pharmazeutisch akzeptablen Hilfsstoff.

Ein weiterer Gegenstand der Erfindung ist ein haarkosmetisches Mittel, enthaltend

a) 0,05 bis 20 Gew.-% wenigstens eines Haarpolymers,

b) 20 bis 99,95 Gew.-% eines Trägers, enthaltend wenigstens ein Isoalkangemisch, wie in einem der Ansprüche 1 bis 14 definiert,

c) 0 bis 50 Gew.-% wenigstens eines Treibgases,

d) 0 bis 5 Gew.-% wenigstens eines Emulgators,

e) 0 bis 3 Gew.-% wenigstens eines Verdickers, sowie

f) bis zu 25 Gew.-% weitere Bestandteile.

Ein weiterer Gegenstand der Erfindung ist die Verwendung eines kosmetischen oder pharmazeutischen Mittels,

wie in einem der Ansprüche 1 bis 14 definiert, als oder in der hydrophoben Komponente einer homogenphasigen oder heterogenphasigen kosmetischen oder pharmazeutischen Zusammensetzung.

Ein weiterer Gegenstand der Erfindung ist die Verwendung eines kosmetischen oder pharmazeutischen Mittels wie in einem der Ansprüche 1 bis 14 definiert, in Mitteln zur Pflege und zum Schutz der Haut, Hautreinigungsmitteln, Zubereitungen für die dekorative Kosmetik, in Haarbehandlungsmitteln, zur Feuchthaltung der Haut, zur Konditionierung von Haut oder Haaren, zur Modifizierung der rheologischen Eigenschaften, zur Verbesserung der Hautverträglichkeit und/oder zur Erzeugung einer besseren Benetzung.

Bevorzugt sind Isoalkangemische, deren $^1$H-NMR-Spektrum im Bereich einer chemischen Verschiebung $\delta$ von 0,6 bis 1,0 ppm ein Flächenintegral von 30 bis 60 %, bevorzugt von 35 bis 55 %, bezogen auf die Gesamtintegralfläche, aufweist.

Bevorzugt weisen die eingesetzten Isoalkangemische keine olefinischen Doppelbindungen auf. Geeignete Isoalkangemische weisen im $^1$H-NMR-Spektrum im Bereich einer chemischen Verschiebung $\delta$ von 4 bis 6 ppm keine Signale auf, die auf Olefinprotonen zurückgehen.

Des Weiteren bevorzugt weist das eingesetzte Isoalkangemisch im $^1$H-NMR-Spektrum im Bereich einer chemischen Verschiebung $\delta$ von 0,5 bis 3 ppm (d. h. im Bereich der aliphatischen Protonen) ein Flächenintegral von bis zu 95 %, besonders bevorzugt von bis zu 98 %, bezogen auf die Gesamtintegralfläche, auf.

Die eingesetzten Isoalkangemische weisen im Wesentlichen keine tert.-Butylgruppen ($-C(CH_3)_3$) auf. Der Anteil an endständigen tert.-Butylgruppen beträgt vorzugsweise höchstens 20 %, besonders bevorzugt höchstens 10 %, insbesondere höchstens 5 % und speziell höchstens 2 %.

Vorzugsweise sind die eingesetzten Isoalkane einheitlich aufgebaut. So wiesen sie, bezogen auf die längste durchgängige Kohlenstoffkette, im Wesentlichen oder ausschließlich Methylverzweigungen auf. Der Anteil an Seitenketten mit Alkylgruppen, die 2 oder mehr als zwei Kohlenstoffatome aufweisen, beträgt weniger als 20 %, bevorzugt höchstens 10%, besonders bevorzugt höchstens 5 %, insbesondere höchstens 1 %, bezogen auf die Gesamtzahl an Verzweigungsstellen. Vorzugsweise enthalten die Isoalkangemische wenigstens 70 Gew.-%, bevorzugt wenigstens 85 Gew.-%, insbesondere wenigstens 95 Gew.-%, Alkane mit 8 bis 20 Kohlenstoffatomen.

Vorzugsweise enthalten die Isoalkangemische wenigstens 70 Gew.-%, bevorzugt wenigstens 80 Gew.-%, insbesondere wenigstens 90 Gew.-% (wie z. B. wenigstens 94 Gew.-%), Alkane mit 12 bis 20 Kohlenstoffatomen.

Das eingesetzte Alkangemisch enthält vorzugsweise wenigstens 70 Gew.-%, bevorzugt wenigstens 85 Gew.-%, insbesondere wenigstens 95 Gew.-%, Alkane mit einer geraden Kohlenstoffatomanzahl. Eine spezielle Ausführung ist ein Isoalkangemisch, das im Wesentlichen aus Alkanen mit 8 oder 12 oder 16 Kohlenstoffatomen besteht.

Die eingesetzten (und nach dem im Folgenden beschriebenen Verfahren erhältlichen) Isoalkangemische weisen vorzugsweise eine oligomere Verteilung, d. h. eine Uneinheitlichkeit bezüglich der Kohlenstoffatomanzahl (und somit des Molekulargewichts) der enthaltenden Alkane auf. Bevorzugt enthalten die eingesetzten Isoalkangemische, bezogen auf ihr Gesamtgewicht, weniger als 95 Gew.-%, besonders bevorzugt höchstens 90 Gew.-%, Alkane desselben Molekulargewichts. Solche uneinheitlichen Isoalkangemische können besonders vorteilhafte anwendungstechnische Eigenschaften, speziell für einen Einsatz in kosmetischen Mitteln, aufweisen. Dazu zählen speziell die rheologischen Eigenschaften der erfindungsgemäßen Isoalkangemische, wie das Spreitverhalten. So liegen die relativen Spreitwerte der erfindungsgemäßen Isoalkangemische (bezogen auf Paraffinum perliquidum als Standard) bei wenigstens 130 %, besonders bevorzugt bei wenigstens 140 %, insbesondere bei wenigstens 150 %. Sie übertreffen somit die aus dem Stand der Technik üblicherweise eingesetzten Ölkörper und insbesondere Isohexadecan ($C_{16}$-Isoalkan, und Gemische mit sehr hohem $C_{16}$-Isoalkangehalt, z. B. gemäß DE 10 2004 018 753). Die erfindungsgemäß eingesetzten Isoalkangemische weisen vorzugsweise eine Viskosität, bestimmt nach Brookfield, im Bereich von 2 bis 10 mPas, besonders bevorzugt im Bereich von 4 bis 8 mPas, auf. Die kinematische Viskosität liegt vorzugsweise im Bereich von 5 bis 25 cSt, besonders bevorzugt im Bereich von 10 bis 20 cSt.

Die eingesetzten Isoalkangemische eignen sich weiterhin besonders vorteilhaft zur Herstellung stabiler Emulsionen, insbesondere stabiler kosmetischer Emulsionen.

Die eingesetzten Isoalkangemische weisen vorzugsweise eine Dichte im Bereich von 0,7 bis 0,82 g/cm$^3$, besonders bevorzugt im Bereich von 0,75 bis 0,8 g/cm$^3$, auf.

Die eingesetzten Isoalkangemische weisen vorzugsweise einen Brechungsindex im Bereich von 1,4 bis 1,5 auf.

Die eingesetzten Isoalkangemische weisen vorzugsweise einen Verzweigungsgrad V im Bereich von 0,1 bis 0,35, besonders bevorzugt 0,12 bis 0,3, insbesondere 0,15 bis 0,27 und speziell 0,17 bis 0,23 auf.

Im Rahmen der vorliegenden Erfindung ist der Verzweigungsgrad V molekulargewichtsunabhängig definiert als Zahl der Verwezigungen pro Kohlenstoffatom (V = Anzahl der Verzweigungen/Anzahl der Kohlenstoffatome, z. B. n-Octan: 0/8 = 0, Methylheptan: 1/8 = 0,125, Dimethylhexan: 2/8 = 0,25, Squalan: 6/30 = 0,2.

Bei den eingesetzten Isoalkangemischen handelt es sich vorzugsweise nicht um Reinsubstanzen und/oder Naturprodukte. Insbesondere bestehen die eingesetzten Isoalkangemische nicht aus Squalan (2,6,10,15,19,23-Hexamethyltetracosan) und enthalten es insbesondere auch nicht. Die erfindungsgemäß eingesetzten Isoalkangemische eignen sich jedoch in vorteilhafter Weise als Substitut für Squalan. Squalan ist ein hochwertiges natives Öl, das z. B.

aus Haifischleber gewonnen wird. Als Naturprodukt ist es nur begrenzt verfügbar und aus Gründen des Artenschutzes besteht ein starker Bedarf nach einem geeigneten Ersatzstoff. Obwohl sich die eingesetzten Isoalkangemische ausweislich ihres $^1$H-NMR-Spektrums strukturell deutlich von Squalan unterscheiden, verfügen sie über einen ähnlichen Verzweigungsgrad und eignen sich in vorteilhafter Weise als Ersatzstoff.

Geeignete Isoalkangemische werden erhalten durch ein Verfahren, bei dem man

a) ein $C_4$-Kohlenwasserstoffgemisch bereitstellt, das weniger als 3 Gew.-% Isobuten enthält

b) das Kohlenwasserstoffeinsatzmaterial einer Oligomerisierung an einem Übergangsmetall-haltigen Katalysator unterwirft,

c) das in Schritt b) erhaltene Oligomerisierungsprodukt vollständig hydriert.

Schritt a)

Vorzugsweise wird in Schritt a) ein technisch zur Verfügung stehendes olefinhaltiges Kohlenwasserstoffgemisch eingesetzt.

Bevorzugte großtechnisch zur Verfügung stehende Olefingemische resultieren aus der Kohlenwasserstoff-Spaltung bei der Erdölverarbeitung, beispielsweise durch Katcracken, wie Fluid Catalytic Cracking (FCC), Thermocracken oder Hydrocracken mit anschließender Dehydrierung. Ein bevorzugtes technisches Olefingemisch ist der $C_4$-Schnitt. $C_4$-Schnitte sind beispielsweise durch Fluid Catalytic Cracking oder Steamcracken von Gasöl bzw. durch Steamcracken von Naphtha erhältlich. Je nach Zusammensetzung des $C_4$-Schnitts unterscheidet man den Gesamt-$C_4$-Schnitt (Roh-$C_4$-Schnitt), das nach der Abtrennung von 1,3-Butadien erhaltene so genannte Raffinat I sowie das nach der Isobutenabtrennung erhaltene Raffinat II. Für den Einsatz in Schritt a) geeignete olefinhaltige Kohlenwasserstoffgemische mit 4 Kohlenstoffatomen lassen sich weiterhin durch katalytische Dehydrierung geeigneter großtechnisch zur Verfügung stehender Paraffingemische erhalten. So gelingt beispielsweise die Herstellung von $C_4$-Olefin-Gemischen aus Flüssiggasen (liquified petroleum gas, LPG) und verflüssigbaren Erdgasen (liquified natural gas, LNG). Letztere umfassen neben der LPG-Fraktion auch zusätzlich größere Mengen höhermolekularer Kohlenwasserstoffe (leichtes Naphtha) und eignen sich somit auch zur Herstellung von $C_5$- und $C_6$-Olefin-Gemischen. Die Herstellung von olefinhaltigen Kohlenwasserstoffgemischen, die Monoolefine mit 4 bis 6 Kohlenstoffatomen enthalten, aus LPG- oder LNG-Strömen gelingt nach üblichen, dem Fachmann bekannten Verfahren, die neben der Dehydrierung in der Regel noch einen oder mehrere Aufarbeitungsschritte umfassen. Dazu zählt beispielsweise die Abtrennung wenigstens eines Teils der in den zuvor genannten Olefin-Einsatzgemischen enthaltenen gesättigten Kohlenwasserstoffe. Diese können beispielsweise erneut zur Herstellung von Olefin-Einsatzmaterialien durch Crackung und/oder Dehydrierung eingesetzt werden. Die in Schritt a) eingesetzten Olefine können jedoch auch einen Anteil gesättigter Kohlenwasserstoffe enthalten, die sich gegenüber den Oligomerisierungsbedingungen inert verhalten. Der Anteil dieser gesättigten Komponenten beträgt im Allgemeinen höchstens 60 Gew.-%, bevorzugt höchstens 40 Gew.-%, besonders bevorzugt höchstens 20 Gew.-%, bezogen auf die Gesamtmenge der in dem Kohlenwasserstoff-Einsatzmaterial enthaltenen Olefine und gesättigten Kohlenwasserstoffe.

Vorzugsweise wird in Schritt a) ein Kohlenwasserstoffgemisch bereitgestellt, das 20 bis 100 Gew.-% $C_4$-Olefine, 0 bis 80 Gew.-% $C_5$-Olefine, 0 bis 60 Gew.-% $C_6$-Olefine und 0 bis 10 Gew.-% von den zuvor genannten Olefinen verschiedene Olefine, jeweils bezogen auf den Gesamtolefingehalt, enthält.

Vorzugsweise wird in Schritt a) ein Kohlenwasserstoffgemisch bereitgestellt, das einen Gehalt an linearen Monoolefinen von mindestens 80 Gew.-%, besonders bevorzugt mindestens 90 Gew.-% und insbesondere mindestens 95 Gew.-%, bezogen auf den Gesamtolefingehalt, aufweist. Dabei sind die linearen Monoolefine ausgewählt unter 1-Buten, 2-Buten, 1-Penten, 2-Penten, 1-Hexen, 2-Hexen, 3-Hexen und Mischungen davon. Zur Einstellung des gewünschten Verzweigungsgrades des Isoalkangemischs kann es von Vorteil sein, wenn das in Schritt a) eingesetzte Kohlenwasserstoffgemisch bis zu 20 Gew.-%, bevorzugt bis zu 5 Gew.-%, insbesondere bis zu 3 Gew.-% verzweigte Olefine, bezogen auf den Gesamtolefingehalt, enthält.

Besonders bevorzugt wird in Schritt a) ein $C_4$-Kohlenwasserstoffgemisch bereitgestellt.

Der Butengehalt, bezogen auf 1-Buten, 2-Buten und Isobuten, des in Schritt a) bereitgestellten $C_4$-Kohlenwasserstoffgemischs beträgt vorzugsweise 10 bis 100 Gew.-%, besonders bevorzugt 50 bis 99 Gew.-%, und insbesondere 70 bis 95 Gew.-%, bezogen auf den Gesamtolefingehalt. Vorzugsweise liegt das Verhältnis von 1-Buten zu 2-Buten in einem Bereich von 20 : 1 bis 1 : 2, insbesondere bei etwa 10 : 1 bis 1 : 1. Erfindungsgemäß enthält das in Schritt a) eingesetzte $C_4$-Kohlenwasserstoffgemisch weniger als 3 Gew.-% Isobuten.

Die Bereitstellung der olefinhaltigen Kohlenwasserstoffe in Schritt a) kann eine Abtrennung von verzweigten Olefinen umfassen. Geeignet sind übliche, aus dem Stand der Technik bekannte Abtrennverfahren, die auf unterschiedlichen physikalischen Eigenschaften von linearen und verzweigten Olefinen bzw. auf unterschiedlichen Reaktivitäten,

die selektive Umsetzungen ermöglichen, beruhen. So kann beispielsweise Isobuten von $C_4$-Olefingemischen, wie Raffinat I, durch eine der folgenden Methoden abgetrennt werden:

- Molsiebtrennung,
- fraktionierte Destillation,
- reversible Hydratisierung zu tert.-Butanol,
- sauer katalysierte Alkohol-Addition an einen tertiären Ether, z. B. Methanol-Addition zu Methyl-tert.-butylether (MTBE),
- irreversible katalysierte Oligomerisierung zu Di- und Tri-Isobuten,
- irreversible Polymerisation zu Polyisobuten,

Derartige Verfahren sind in K. Weissermel, H.-J. Arpe, Industrielle organische Chemie, 4. Auflage, S. 76-81, VCH-Verlagsgesellschaft Weinheim, 1994 beschrieben, worauf hier in vollem Umfang Bezug genommen wird.

Vorzugsweise wird in Schritt a) ein Raffinat II bereitgestellt.

Ein zum Einsatz in dem erfindungsgemäßen Verfahren geeignetes Raffinat II hat beispielsweise die folgende Zusammensetzung:

0,5 bis 5 Gew.-% Isobutan,
5 bis 20 Gew.-% n-Butan,
20 bis 40 Gew.-% trans-2-Buten,
10 bis 20 Gew.-% cis-2-Buten,
25 bis 55 Gew.-% 1-Buten,
0,5 bis 5 Gew.-% Isobuten

sowie Spurengase, wie 1,3-Butadien, Propen, Propan, Cyclopropan, Propadien, Methylcyclopropan, Vinylacetylen, Pentene, Pentane etc. im Bereich von jeweils maximal 1 Gew.-%.

Ein geeignetes Raffinat II weist folgende typische Zusammensetzung auf:

| | |
|---|---|
| i-,n-Butan | 26 Gew.-% |
| i-Buten | 1 Gew.-% |
| 1-Buten | 26 Gew.-% |
| trans-2-Buten | 31 Gew.-% |
| cis-2-Buten | 16 Gew.-% |

Sind Diolefine oder Alkine in dem olefinreichen Kohlenwasserstoffgemisch vorhanden, so können diese vor der Oligomerisierung auf vorzugsweise weniger als 10 Gew.-ppm aus demselben entfernt werden. Sie werden bevorzugt durch selektive Hydrierung, z. B. gemäß EP-81 041 und DE-15 68 542 entfernt, besonders bevorzugt durch eine selektive Hydrierung bis auf einen Restgehalt von unter 5 Gew.-ppm, insbesondere 1 Gew.-ppm.

Aus dem olefinreichen Kohlenwasserstoffgemisch werden zweckmäßigerweise außerdem sauerstoffhaltige Verbindungen, wie Alkohole, Aldehyde, Ketone oder Ether, weitgehend entfernt. Hierzu kann das olefinreiche Kohlenwasserstoffgemisch mit Vorteil über ein Adsorptionsmittel, wie z. B. ein Molekularsieb, insbesondere eines mit einem Porendurchmesser von > 4 Å bis 5 Å, geleitet werden. Die Konzentration an sauerstoffhaltigen, schwefelhaltigen, stickstoffhaltigen und halogenhaltigen Verbindungen im olefinreichen Kohlenwasserstoffgemisch beträgt vorzugsweise weniger als 1 Gew.-ppm, insbesondere weniger als 0,5 Gew.-ppm.

Schritt b)

Im Rahmen der vorliegenden Erfindung umfasst der Begriff "Oligomere" Dimere, Trimere, Tetramere und höhere Produkte aus der Aufbaureaktion der eingesetzten Olefine. Vorzugsweise sind die in Schritt b) erhaltenen Oligomere ausgewählt unter Dimeren, Trimeren und Tetrameren. Die Oligomere sind ihrerseits olefinisch ungesättigt. Durch geeignete Wahl des zur Oligomerisierung eingesetzten Kohlenwasserstoffeinsatzmaterials und des Oligomerisierungskatalysators, wie im Folgenden beschrieben, können die angestrebten Isoalkane erhalten werden.

Zur Oligomerisierung in Schritt b) kann ein Reaktionssystem eingesetzt werden, das einen oder mehrere, gleiche oder verschiedene Reaktoren umfasst. Im einfachsten Fall wird zur Oligomerisierung in Schritt b) ein einzelner Reaktor eingesetzt. Es können jedoch auch mehrere Reaktoren eingesetzt werden, die jeweils gleiche oder verschiedene Vermischungscharakteristiken aufweisen. Die einzelnen Reaktoren können gewünschtenfalls durch Einbauten ein- oder mehrfach unterteilt sein. Bilden zwei oder mehrere Reaktoren das Reaktionssystem, so können diese untereinander beliebig verschaltet sein, z. B. parallel oder in Reihe. In einer geeigneten Ausführung wird z.B. ein Reaktionssystem eingesetzt, das aus zwei in Reihe geschalteten Reaktoren besteht.

Geeignete druckfeste Reaktionsapparaturen für die Oligomerisierung sind dem Fachmann bekannt. Dazu zählen die allgemein üblichen Reaktoren für Gas-fest- und Gas-flüssig-Reaktionen, wie z. B. Rohrreaktoren, Rührkessel, Gasumlaufreaktoren, Blasensäulen etc., die gegebenenfalls durch Einbauten unterteilt sein können. Vorzugsweise werden Rohrbündelreaktoren oder Schachtöfen eingesetzt. Wird zur Oligomerisierung ein heterogener Katalysator eingesetzt, so kann dieser in einem einzigen oder in mehreren Katalysator-Festbetten angeordnet sein. Dabei ist es möglich, in verschiedenen Reaktionszonen unterschiedliche Katalysatoren einzusetzen. Bevorzugt ist jedoch der Einsatz des gleichen Katalysators in allen Reaktionszonen.

Die Temperatur bei der Oligomerisierungsreaktion liegt im Allgemeinen in einem Bereich von etwa 20 bis 280 °C, bevorzugt von 25 bis 200 °C, insbesondere von 30 bis 140 °C. Der Druck bei der Oligomerisierung liegt im Allgemeinen in einem Bereich von etwa 1 bis 300 bar, vorzugsweise von 5 bis 100 bar und insbesondere von 20 bis 70 bar. Umfasst das Reaktionssystem mehr als einen Reaktor, so können diese gleiche oder verschiedene Temperaturen und gleiche oder verschiedene Drücke aufweisen. So kann beispielsweise im zweiten Reaktor einer Reaktorkaskade eine höhere Temperatur und/oder ein höherer Druck als im ersten Reaktor eingestellt werden, z. B. um einen möglichst vollständigen Umsatz zu erzielen.

In einer speziellen Ausführung werden die zur Oligomerisierung eingesetzten Temperatur und Druckwerte so gewählt, dass das olefinhaltige Einsatzmaterial flüssig oder im überkritischen Zustand vorliegt.

Die Umsetzung in Schritt b) wird vorzugsweise adiabatisch durchgeführt. Dieser Begriff wird im Rahmen der vorliegenden Erfindung im technischen und nicht im physikochemischen Sinne verstanden. So verläuft die Oligomerisierungsreaktion in der Regel exotherm, so dass das Reaktionsgemisch beim Strömen durch das Reaktionssystem, beispielsweise ein Katalysatorbett, eine Temperaturerhöhung erfährt. Unter adiabatischer Reaktionsführung wird eine Vorgehensweise verstanden, bei der die in einer exothermen Reaktion freiwerdende Wärmemenge von der Reaktionsmischung im Reaktor aufgenommen und keine Kühlung durch Kühlvorrichtungen angewandt wird. Somit wird die Reaktionswärme mit dem Reaktionsgemisch aus dem Reaktor abgeführt, abgesehen von einem Restanteil, der durch natürliche Wärmeleitung und Wärmeabstrahlung vom Reaktor an die Umgebung abgegeben wird.

Zur Oligomerisierung in Schritt b) wird ein Übergangsmetall-haltiger Katalysator eingesetzt. Bevorzugt handelt es sich dabei um heterogene Katalysatoren. Bevorzugte Katalysatoren, die bekanntermaßen eine geringe Oligomeren-Verzweigung Nickel enthaltende Katalysatoren für die Umsetzung in Schritt a), die bekanntermaßen eine geringe Oligomeren-Verzweigung bewirken, sind dem Fachmann allgemein bekannt. Dazu zählen die in Catalysis Today, 6, 329 (1990), insbesondere Seiten 336-338, sowie die in der DE-A-43 39 713 (= WO-A 95/14647) und der DE-A-199 57 173 beschriebenen Katalysatoren, auf die hiermit ausdrücklich Bezug genommen wird. Ein geeignetes Oligomerisierungsverfahren, bei dem der zur Oligomerisierung eingesetzten Feed-Strom aufgeteilt und mindestens zwei bei unterschiedlichen Temperaturen betriebenen Reaktionszonen zugeführt wird ist in der EP-A-1 457 475 beschrieben, auf die ebenfalls Bezug genommen wird.

Die eingesetzten heterogenen Nickel enthaltenden Katalysatoren können unterschiedliche Strukturen aufweisen. Geeignet sind prinzipiell Vollkatalysatoren sowie geträgerte Katalysatoren. Letztere werden bevorzugt eingesetzt. Die Trägermaterialien können z. B. Kieselsäure, Tonerde, Aluminosilicate, Aluminosilicate mit Schichtstrukturen und Zeolithe, wie Mordenit, Faujasit, Zeolith X, Zeolith-Y und ZSM-5, Zirkoniumoxid, das mit Säuren behandelt ist, oder sulfatiertes Titandioxid sein. Besonders geeignet sind Fällungskatalysatoren, die durch Mischen wässriger Lösungen von Nickelsalzen und Silicaten, z. B. Natriumsilicat mit Nickelnitrat, und gegebenenfalls Aluminiumsalzen, wie Aluminiumnitrat, und Calcinieren erhältlich sind. Weiterhin sind Katalysatoren verwendbar, die durch Einlagerung von $Ni^{2+}$-Ionen durch Ionenaustausch in natürliche oder synthetische Schichtsilicate, wie Montmorillonite, erhalten werden. Geeignete Katalysatoren können auch durch Imprägnieren von Kieselsäure, Tonerde oder Alumosilicaten mit wässrigen Lösungen löslicher Nickelsalze, wie Nickelnitrat, Nickelsulfat oder Nickelchlorid, und anschließende Calcinierung erhalten werden.

Nickeloxid enthaltende Katalysatoren sind bevorzugt. Besonders bevorzugt sind Katalysatoren, die im Wesentlichen aus NiO, $SiO_2$, $TiO_2$ und/oder $ZrO_2$ sowie gegebenenfalls $Al_2O_3$ bestehen. Am meisten bevorzugt ist ein Katalysator, der als wesentliche aktive Bestandteile 10 bis 70 Gew.-% Nickeloxid, 5 bis 30 Gew.-% Titandioxid und/oder Zirkondioxid, 0 bis 20 Gew.-% Aluminiumoxid und als Rest Siliciumdioxid enthält. Ein solcher Katalysator ist durch Fällung der Katalysatormasse bei pH 5 bis 9 durch Zugabe einer Nickelnitrat enthaltenden wässrigen Lösung zu einer Alkaliwasserglaslösung, die Titandioxid und/oder Zirkondioxid enthält, Filtrieren, Trocknen und Tempern bei 350 bis 650 °C erhältlich. Zur Herstellung dieser Katalysatoren wird im Einzelnen auf die DE-43 39 713 verwiesen. Auf die Offenbarung dieser Druckschrift und den darin zitierten Stand der Technik wird vollinhaltlich Bezug genommen.

In einer weiteren Ausführungsform wird als Katalysator in Schritt b) ein Nickelkatalysator gemäß der DE-A-199 57 173 eingesetzt. Dabei handelt es sich im Wesentlichen um Aluminiumoxid, das mit einer Nickelverbindung und einer Schwefelverbindung beaufschlagt wurde. Vorzugsweise liegt im fertigen Katalysator ein molares Verhältnis von Schwefel zu Nickel im Bereich von 0,25 : 1 bis 0,38 : 1 vor. Der Katalysator liegt vorzugsweise in stückiger Form, z. B. in Form von Tabletten, z. B. mit einem Durchmesser von 2 bis 6 mm und einer Höhe von 3 bis 5 mm, Ringen mit z. B. 5 bis 7 mm Außendurchmesser, 2 bis 5 mm Höhe und 2 bis 3 mm Lochdurchmesser, oder Strängen unterschiedlicher Länge eines Durchmessers von z. B. 1,5 bis 5 mm, vor. Derartige Formen werden auf an sich bekannte Weise durch Tablettierung oder Extrusion, meist unter Verwendung eines Tablettierhilfsmittels, wie Graphit oder Stearinsäure, erhalten.

Schritt c)

Im Schritt c) können als Hydrierungskatalysatoren in der Regel alle Katalysatoren des Standes der Technik eingesetzt werden, welche die Hydrierung von Olefinen zu den entsprechenden Alkanen katalysieren. Die Katalysatoren können sowohl in heterogener Phase als auch als Homogenkatalysatoren eingesetzt werden. Vorzugsweise enthalten die Hydrierungskatalysatoren wenigstens ein Metall der Gruppe VIII.

Besonders geeignete Metalle der Gruppe VIII sind ausgewählt unter Ruthenium, Cobalt, Rhodium, Nickel, Palladium und Platin.

Die Metalle können auch als Gemische eingesetzt werden. Außerdem können die Katalysatoren neben den Metallen der Gruppe VIII auch geringe Mengen weiterer Metalle, beispielsweise Metalle der Gruppe VIIa, insbesondere Rhenium, oder Metalle der Gruppe Ib, d. h. Kupfer, Silber oder Gold, enthalten. Besonders bevorzugte Metalle der Gruppe VIII sind Ruthenium, Nickel, Palladium und Platin, insbesondere Platin, Nickel und Palladium, und stärker bevorzugt Palladium und Nickel. Speziell enthält der Katalysator Palladium als katalytisch aktive Spezies.

Wird ein Heterogenkatalysator eingesetzt, so liegt dieser geeigneter Weise in feinverteilter Form vor. Die feinverteilte Form wird beispielsweise folgendermaßen erreicht:

- Schwarzkatalysator: Das Metall wird kurz vor der Verwendung als Katalysator aus der Lösung eines seiner Salze reduktiv abgeschieden.

- Adams-Katalysator: Die Metalloxide, insbesondere die Oxide von Platin und Palladium, werden in situ durch den zur Hydrierung eingesetzten Wasserstoff reduziert.

- Skelett- oder Raney-Katalysator: Der Katalysator wird als "Metallschwamm" aus einer binären Legierung des Metalls (insbesondere Nickel oder Cobalt) mit Aluminium oder Silicium durch Herauslösen eines Partners mit Säure oder Lauge hergestellt. Reste des ursprünglichen Legierungspartners wirken oft synergistisch.

- Trägerkatalysator: Schwarzkatalysatoren lassen sich auch auf der Oberfläche einer Trägersubstanz niederschlagen. Geeignete Träger und Trägermaterialien sind nachfolgend beschrieben.

Solche Heterogenkatalysatoren sind in allgemeiner Form beispielsweise im Organikum, 17. Auflage, VEB Deutscher Verlag der Wissenschaften, Berlin, 1988, S. 288 beschrieben. Außerdem werden heterogene Hydrierungskatalysatoren, die für die Reduktion von Olefinen zu Alkanen geeignet sind, in folgenden Schriften näher beschrieben:

Die US 3,597,489, US 2,898,387 und GB 799,396 beschreiben die Hydrierung von Benzol zu Cyclohexan an Nickel- und Platinkatalysatoren in der Gas- oder Flüssigphase. GB 1,155,539 beschreibt die Verwendung eines mit Rhenium dotierten Nickelkatalysators zur Hydrierung von Benzol. Die US 3,202,723 beschreibt die Hydrierung von Benzol mit Raney-Nickel. Ruthenium-haltige Suspensionskatalysatoren, die mit Palladium, Platin oder Rhodium dotiert sind, werden in der SU 319582 zur Hydrierung von Benzol zu Cyclohexan eingesetzt. Aluminiumoxid-geträgerte Katalysatoren werden in der US 3,917,540 und US 3,244,644 beschrieben. Auf die in diesen Schriften beschriebenen Hydrierungskatalysatoren wird in vollem Umfang Bezug genommen.

Je nach Ausgestaltung des Hydrierverfahrens kann das Trägermaterial verschiedene Gestalten aufweisen. Wird die Hydrierung in Sumpffahrweise durchgeführt, so wird das Trägermaterial in der Regel in Form eines feinteiligen Pulvers eingesetzt. Wird der Katalysator hingegen in Form eines Festbettkatalysators eingesetzt, so setzt man z. B. Formkörper als Trägermaterial ein. Solche Formkörper können in Gestalt von Kugeln, Tabletten, Zylindern, Hohlzylindern, Raschigringen, Strängen, Sattelkörpern, Sternen, Spiralen usw. mit einer Größe (Abmessung der längsten Ausdehnung) von etwa 1 bis 30 mm vorliegen. Außerdem können die Träger in Form von Monolithen, wie sie z. B. in der DE-A-19642770 beschrieben sind, vorliegen. Des Weiteren können die Träger in Form von Drähten, Blechen, Gittern, Netzen, Geweben und dergleichen eingesetzt werden.

Die Träger können aus metallischem oder nichtmetallischem, porösem oder nichtporösem Material bestehen.

Geeignete metallische Materialien sind beispielsweise hochlegierte Edelstähle. Geeignete nichtmetallische Materialien sind beispielsweise mineralische Werkstoffe, z. B. natürliche und synthetische Mineralien, Gläser oder Keramiken, Kunststoffe, z. B. künstliche oder natürliche Polymere, oder eine Kombination aus beiden.

Bevorzugte Trägermaterialien sind Kohle, insbesondere Aktivkohle, Siliciumdioxid, insbesondere amorphes Siliciumdioxid, Aluminiumoxid, und außerdem die Sulfate und Carbonate der Erdalkalimetalle, Calciumcarbonat, Calciumsulfat, Magnesiumcarbonat, Magnesiumsulfat, Bariumcarbonat und Bariumsulfat.

Der Katalysator kann durch übliche Verfahren auf den Träger aufgebracht werden, z. B. durch Tränken, Benetzen oder Besprühen des Trägers mit einer Lösung, die den Katalysator oder eine geeignete Vorstufe davon enthält.

Geeignete Träger und Verfahren zum Aufbringen des Katalysators auf diese sind beispielsweise in der DE-A-10128242 beschrieben, auf die hiermit in vollem Umfang Bezug genommen wird.

Auch homogene Hydrierungskatalysatoren können in das Verfahren eingesetzt werden. Beispiele hierfür sind die Nickelkatalysatoren, die in der EP-A-0668257 beschrieben sind. Nachteilig bei einer Verwendung von Homogenkatalysatoren ist jedoch ihre Herstellungskosten und auch die Tatsache, dass sie in der Regel nicht regenerierbar sind.

Daher werden im Verfahren vorzugsweise heterogene Hydrierungskatalysatoren eingesetzt.

Besonders bevorzugt enthalten die im Verfahren eingesetzten Heterogenkatalysatoren wenigstens ein Metall der VIII. Nebengruppe, das ausgewählt ist unter Ruthenium, Nickel, Cobalt, Palladium und Platin, und das gegebenenfalls mit einem weiteren Übergangsmetall, insbesondere mit einem der Nebengruppe VIIa, Ib oder IId und insbesondere mit Rhenium dotiert ist.

Besonders bevorzugt wird das Metall in geträgerter Form oder als Metallschwamm eingesetzt. Beispiele für geträgerte Katalysatoren sind insbesondere Palladium, Nickel oder Ruthenium auf Kohle, insbesondere Aktivkohle, Siliciumdioxid, insbesondere auf amorphem Siliciumdioxid, Bariumcarbonat, Calciumcarbonat, Magnesiumcarbonat oder Aluminiumoxid, wobei die Träger in den oben beschriebenen Formen vorliegen können. Bevorzugte Trägerformen sind die oben beschriebenen Formkörper.

Die metallischen Katalysatoren können auch in Form ihrer Oxide, insbesondere Palladiumoxid, Platinoxid oder Nickeloxid, eingesetzt werden, die dann unter den Hydrierbedingungen zu den entsprechenden Metallen reduziert werden.

Als Metallschwamm wird insbesondere Raney-Nickel eingesetzt.

Speziell verwendet man im Verfahren Palladium auf Trägermaterialien, wie Aktivkohle, als Hydrierungskatalysator.

Die einzusetzende Katalysatormenge hängt unter anderem vom jeweiligen katalytisch aktiven Metall und von dessen Einsatzform ab und kann vom Fachmann im Einzelfall bestimmt werden. So wird beispielsweise ein Nickel- oder Cobalt-haltiger Hydrierungskatalysator in einer Menge von vorzugsweise 0,5 bis 70 Gew.-%, besonders bevorzugt von 1 bis 20 Gew.-% und insbesondere von 2 bis 10 Gew.-%, bezogen auf das Gewicht des zu hydrierenden Oligomerisierungsprodukts, eingesetzt. Die angegebene Katalysatormenge bezieht sich dabei auf die Menge an Aktivmetall, d. h. auf die katalytisch wirksame Komponente des Katalysators. Bei Verwendung von Edelmetallkatalysatoren, die beispielsweise Platin oder Palladium enthalten, gelten um etwa den Faktor 10 kleinere Werte.

Die Hydrierung erfolgt bei einer Temperatur von vorzugsweise 20 bis 250 °C, besonders bevorzugt von 50 bis 240 °C und insbesondere von 150 bis 220 °C.

Der Reaktionsdruck der Hydrierreaktion liegt vorzugsweise im Bereich von 1 bis 300 bar, besonders bevorzugt von 50 bis 250 bar und insbesondere von 150 bis 230 bar.

Sowohl Reaktionsdruck als auch Reaktionstemperatur hängen unter anderem von der Aktivität und Menge des eingesetzten Hydrierkatalysators ab und können im Einzelfall vom Fachmann bestimmt werden.

Gewünschtenfalls kann das Oligomerisierungsprodukt zur Erzielung einer möglichst vollständigen Hydrierung mehrfach hydriert ("nachhydriert") werden. Dabei wird, sobald kein Wasserstoffverbbrauch mehr festgestellt werden kann, weiterer Wasserstoff aufgepresst. Vorzugsweise wird vor dem Aufpressen von Wasserstoff zunächst frischer Katalysator zugesetzt.

Die Hydrierung kann in einem geeigneten Lösungsmittel oder vorzugsweise in Substanz vorzugsweise. Geeignete Lösungsmittel sind solche, die unter den Reaktionsbedingungen inert sind, d. h. weder mit dem Edukt oder Produkt reagieren noch selbst verändert werden, und die sich problemlos von den erhaltenen Isoalkanen abtrennen lassen. Zu den geeigneten Lösungsmitteln gehören offenkettige und cyclische Ether, wie Diethylether, Methyl-tert.-Butylether, Tetrahydrofuran oder 1,4-Dioxan und Alkohole, insbesondere $C_1$-$C_3$-Alkanole, wie Methanol, Ethanol, n-Propanol oder Isopropanol. Geeignet sind auch Gemische der vorstehend genannten Lösungsmittel.

Der für die Hydrierung erforderliche Wasserstoff kann sowohl in Reinform als auch in Form von Wasserstoff-haltigen Gasgemischen eingesetzt werden. Letztere dürfen jedoch keine schädlichen Mengen an Katalysatorgiften, wie CO, enthalten. Beispiele für geeignete Wasserstoff-haltige Gasgemische sind solche aus dem Reforming-Verfahren. Vorzugsweise wird jedoch Wasserstoff in Reinform eingesetzt.

Die Hydrierung kann sowohl kontinuierlich als auch diskontinuierlich ausgestaltet sein.

Die Durchführung der Hydrierung erfolgt in der Regel so, dass man das Oligomerisierungsprodukt, gegebenenfalls in einem Lösungsmittel, vorlegt. Diese Reaktionslösung wird anschließend vorzugsweise zunächst mit dem Hydrierungskatalysator versetzt, bevor dann die Wasserstoffeinleitung beginnt. In Abhängigkeit vom verwendeten Hydrierungskatalysator erfolgt die Hydrierung bei erhöhter Temperatur und/oder bei erhöhtem Druck. Für die Reaktionsführung unter Druck können die üblichen, aus dem Stand der Technik bekannten Druckgefäße, wie Autoklaven, Rührautoklaven und Druckreaktoren, verwendet werden. Wird nicht bei Wasserstoff-Überdruck gearbeitet, so kommen die üblichen Reaktionsvorrichtungen des Standes der Technik in Betracht, die für Normaldruck geeignet sind. Beispiele hierfür sind übliche Rührkessel, die vorzugsweise mit einer Siedekühlung, geeigneten Mischern, Einleitungsvorrichtungen, gegebenenfalls Wärmetauscherelementen und Inertisierungsvorrichtungen ausgerüstet sind. Bei der kontinuierlichen Reaktionsführung kann die Hydrierung unter Normaldruck in hierfür üblichen Reaktionskesseln, Rohrreaktoren, Festbettreaktoren und dergleichen durchgeführt werden.

Nach beendeter Hydrierung werden in der Regel der Katalysator und das Lösungsmittel entfernt. Der heterogene Katalysator wird vorzugsweise durch Filtration oder durch Sedimentation und Entfernung der oberen, produkthaltigen Phase abgetrennt. Auch andere Abtrennungsverfahren zur Entfernung von Feststoffen aus Lösungen, wie beispielsweise Zentrifugieren, sind zur Entfernung des heterogenen Katalysators geeignet. Die Entfernung homogener Katalysatoren erfolgt durch übliche Verfahren zur Trennung von gleichphasigen Gemischen, beispielsweise durch chromatografische Methoden. Gegebenenfalls kann es je nach Katalysatortyp erforderlich sein, diesen vor der Entfernung zu desaktivieren. Dies kann durch übliche Verfahren erfolgen, beispielsweise durch Waschen der Reaktionslösung mit protischen Lösungsmitteln, z. B. mit Wasser oder mit $C_1$-$C_3$-Alkanolen, wie Methanol, Ethanol, Propanol oder Isopropanol, die erforderlichenfalls basisch oder sauer eingestellt sind.

Die Entfernung des Lösungsmittels erfolgt durch übliche Verfahren, beispielsweise destillativ, insbesondere unter verringertem Druck.

In einer speziellen Ausführung des Verfahrens wird das in Schritt b) erhaltene Oligomerisierungsprodukt und/oder das in Schritt c) erhaltene Hydrierungsprodukt einer Auftrennung unterzieht. Dabei wird zumindest eine an einem bestimmten Oligomeren, bzw. hydrierten Oligomeren angereicherte Fraktion erhalten. So kann beispielsweise ein $C_4$-Kohlenwasserstoffgemisch in Schritt b) einer Oligomerisierung unterzogen und anschließend, vor der Hydrierung, einer Auftrennung unterworfen werden, wobei wenigstens eine an $C_8$-, $C_{12}$- oder $C_{16}$-Oligomeren angereicherte Fraktion erhalten wird. Diese Oligomeren-angereicherte(n) Fraktion(en) wird/werden dann zur Hydrierung eingesetzt. Desgleichen kann ein Oligomerengemisch aus Schritt b) zunächst ohne Auftrennung hydriert und anschließend das Produktgemisch der Hydrierung einer Auftrennung unterworfen werden, wobei beispielsweise bei Einsatz eines $C_4$-Kohlenwasserstoffgemischs zur Oligomerisierung wenigstens eine an $C_8$-, $C_{12}$- oder $C_{16}$-Isoalkanen angereicherte Fraktion erhalten wird. Die an einem der vorgenannten Alkane angereicherten Fraktionen enthalten in einer bevorzugten Ausführung, wie eingangs ausgeführt, weitere davon verschiedene Alkane. Dabei handelt es sich vorzugsweise im Wesentlichen um Alkane mit höherem Molekulargewicht als das angereicherte Alkan. Bevorzugt wird dann beispielsweise eine an $C_{16}$-Isoalkanen angereicherte Fraktion erhalten, die im Wesentlichen keine $C_8$-und/oder $C_{12}$-Isoalkane enthält, die aber $C_{20}$-Isoalkane und gegebenenfalls höhere Homologe enthält.

Zur Auftrennung kann das Reaktionsgemisch der Oligomerisierung oder der Hydrierung einem oder mehreren Trennschritten unterworfen werden. Geeignete Trennvorrichtungen sind die üblichen, dem Fachmann bekannten Apparaturen. Dazu zählen z. B. Destillationskolonnen, z. B. Bodenkolonnen, die gewünschtenfalls mit Glocken, Siebplatten, Siebböden, Ventilen, Seitenabzügen, etc. ausgerüstet sein können, Verdampfer, wie Dünnschichtverdampfer, Fallfilmverdampfer, Wischblattverdampfer, Sambay-Verdampfer, etc. und Kombinationen davon. Bevorzugt erfolgt die Isolierung der Olefinfraktion durch ein- oder mehrstufige fraktionierte Destillation.

Die Isoalkangemische eignen sich besonders vorteilhaft für einen Einsatz in kosmetischen und pharmazeutischen Mitteln. Sie sind im Allgemeinen geruchslos und weisen insbesondere keine vom Verbraucher von kosmetischen und pharmazeutischen Formulierungen als "fremdartig" oder "chemisch" empfundene Geruchskomponenten, z. B. einen terpenartigen Geruch, auf, der den Einsatz von intensiv riechenden oder von größeren Mengen Parfümölen zur Maskierung erfordert.

Obwohl die eingesetzten Isoalkangemische als solche keinen Eigengeruch aufweisen, kann es von Vorteil sein, das in Schritt c) erhaltene hydrierte Oligomerisierungsprodukt wenigstens einem Aufarbeitungsschritt zur Entfernung unerwünschter Komponenten zu unterziehen. Dazu zählen z. B. die optischen und/oder die olfaktorischen Eigenschaften u. U. noch beeinträchtigenden Komponenten. Vorzugsweise wird das in Schritt c) erhaltene hydrierte Oligomerisierungsprodukt einer Aufarbeitung durch Inkontaktbringen mit wenigstens einem Adsorptionsmittel unterzogen. Geeignete Adsorptionsmittel sind z. B. in Ullmann's Encyclopedia of Industrial Chemistry, 6th Edition, 2000 Electronic Release, "Adsorption", 3. Absorbents und den zugehörigen Tabellen 1 und 2 beschrieben, worauf hier in vollem Umfang Bezug genommen wird.

Die Adsorptionsmittel sind vorzugsweise unter Siliciumdioxiden, Kieselgur, natürlichen und synthetischen Aluminiumsilicaten, Aluminiumoxiden, Aluminiumoxid-haltigen Feststoffen, Aluminiumphosphaten, von Aluminiumphosphaten verschiedenen Phosphaten, Titandioxiden, Zirkoniumdioxiden, Kohlenstoff-haltigen Adsorbentien, organischen Absorberharzen und Mischungen davon ausgewählt.

Geeignete Adsorptionsmittel sind z. B. aktive Aluminiumoxide. Ihre Herstellung erfolgt z. B. ausgehend von Aluminiumhydroxid, welches durch übliche Fällungsverfahren aus Aluminiumsalzlösungen erhältlich ist. Für das erfindungsgemäße Verfahren geeignete aktive Aluminiumoxide sind auch ausgehend von Aluminiumhydroxid-Gelen erhältlich. Zur Herstellung solcher Gele kann z. B. gefälltes Aluminiumhydroxid nach üblichen Aufarbeitungsschritten, wie Filtern, Waschen und Trocknen, aktiviert und anschließend gegebenenfalls vermahlen oder agglomeriert werden. Gewünschtenfalls kann man das resultierende Aluminiumoxid anschließend noch einem Formgebungsverfahren, wie Extrusion, Granulation, Tablettierung etc. unterwerfen. Als Adsorptionsmittel eignen sich vorzugsweise die Selexsorb TM-Typen der Firma Alcoa.

Geeignete Adsorptionsmittel sind weiterhin Aluminiumoxid-haltige Feststoffe. Hierzu zählen z. B. die so genannten Tonerden, die als Hauptbestandteil ebenfalls Aluminiumoxide aufweisen. Geeignet sind weiterhin natürliche und synthetische Aluminiumsilicate. Dazu zählen Schichtsilicate, wie Tonmineralien, und besonders bevorzugt Gerüstsilicate, speziell Zeolithe.

Des Weiteren geeignete Adsorptionsmittel sind Aluminiumphosphate.

Weiterhin geeignete Adsorptionsmittel sind Siliciumdioxide, die z. B. durch Entwässerung und Aktivierung von Kieselgelen erhältlich sind. Ein weiteres Verfahren zur Herstellung von Siliciumdioxid ist die Flammhydrolyse von Siliciumtetrachlorid, wobei durch geeignete Variationen der Reaktionsparameter, wie z. B. der stöchiometrischen Zusammensetzung des Eduktgemischs und der Temperatur, die gewünschten Oberflächeneigenschaften des resultierenden Siliciumdioxids in weiten Bereichen variiert werden können.

Weiterhin geeignete Adsorptionsmittel sind Kieselgure, die ebenfalls als Hauptbestandteil Siliciumdioxide aufweisen. Dazu zählt z. B. die aus Kieselsedimenten gewonnene Diatomeenerde.

Weiterhin geeignete Adsorptionsmittel sind Titandioxide und Zirkoniumdioxide, wie sie z. B. in Römpp, Chemie Lexikon, 9. Aufl. (Paperback), Bd. 6, S. 4629f. und S. 5156f. und der dort zitierten Literatur beschrieben sind. Hierauf wird hier in vollem Umfang Bezug genommen.

Weiterhin geeignete Adsorptionsmittel sind Phosphate, insbesondere kondensierte Phosphate, wie z. B. Schmelz- oder Glühphosphate, die eine große aktive Oberfläche aufweisen. Geeignete Phosphate sind z. B. in Römpp, Chemie Lexikon, 9. Aufl. (Paperback), Bd. 4, S. 3376f. und der dort zitierten Literatur beschrieben. Hierauf wird hier in vollem Umfang Bezug genommen.

Weiterhin geeignete Adsorptionsmittel sind kohlenstoffhaltige Adsorbentien, bevorzugt Aktivkohle. Unter Aktivkohle versteht man hierbei im Allgemeinen Kohlenstoff mit poröser Struktur und hoher innerer Oberfläche. Zur Herstellung von Aktivkohle werden pflanzliche, tierische und/oder mineralische kohlenstoffhaltige Rohstoffe, z. B. mit Dehydratisierungsmitteln, wie Zinkchlorid oder Phosphorsäure, erhitzt oder durch trockene Destillation verkohlt und anschließend oxidativ aktiviert. Dazu kann man z. B. das verkohlte Material bei erhöhten Temperaturen von etwa 700 bis 1000 °C mit Wasserdampf, Kohlendioxid und/oder Gemischen davon behandeln.

Möglich ist auch ein Einsatz von Ionenaustauschern und/oder Adsorberharzen.

Die Adsorptionsmittel weisen im Allgemeinen eine spezifische Oberfläche, bestimmt nach BET, im Bereich von etwa 10 bis 2000 $m^2/g$, insbesondere im Bereich von 10 bis 1500 $m^2/g$ und speziell im Bereich von 20 bis 600 $m^2/g$, auf.

Die eingesetzten Isoalkangemische sind des Weiteren in der Regel farblos oder weisen nur eine geringe Eigenfarbe auf. Vorzugsweise weisen die erfindungsgemäßen und nach dem erfindungsgemäßen Verfahren erhältlichen Isoalkangemische eine Hazen- oder APHA-Farbzahl (bestimmt nach DIN 6271) von höchstens 6, besonders bevorzugt von höchstens 5, auf. Je nach Molekulargewicht sind die Isoalkane von flüssiger bis ölartigen Konsistenz. Für einen Einsatz in kosmetischen und pharmazeutischen Mitteln werden vorzugsweise Isoalkangemische eingesetzt, die Isoalkane mit 8 bis 28, bevorzugt 8 bis 20 Kohlenstoffatomen aufweisen. Diese Isoalkangemische sind unter Normalbedingungen (25 °C, 1013 mbar) flüssig.

Isoalkangemische die eine hohe Einheitlichkeit bezüglich des Molekulargewichts der enthaltenen Isoalkane aufweisen, werden in kosmetischen oder pharmazeutischen Mitteln verwendet. Dabei handelt es sich bevorzugt um Dimere, Trimere oder Tetramere. Diese Isoalkangemische weisen dann vorzugsweise wenigstens 70 Gew.-%, besonders bevorzugt wenigstens 85 Gew.-% und insbesondere wenigstens 95 % Isoalkane gleicher Kohlenstoffatomanzahl auf.

Die eingesetzten Isoalkane zeichnen sich gegenüber den aus dem Stand der Technik bekannten Ölkörpern für kosmetische und pharmazeutische Mittel durch vergleichbare oder bessere anwendungstechnische Eigenschaften aus. So besitzen sie eine ausgezeichnete Hautverträglichkeit, führen nicht zu Irritationen und eignen sich dazu, die reizende Wirkung anderer Inhaltsstoffe abzumildern oder die dermale Applikation von Wirkstoffen zu verbessern. Sie lassen sich mit einer Vielzahl von kosmetischen Wirk- und Hilfsstoffen formulieren. Dabei weisen sie in der Regel eine geringere Flüchtigkeit auf als entsprechende Siliconöle gleichen Molekulargewichts. Ihre Flammpunkte sind ausreichend hoch, um bei der Herstellung und Anwendung strengen sicherheitstechnischen Standards zu genügen. So weist beispielsweise ein erfindungsgemäßes $C_{16}$-Isoalkangemisch in der Regel einen Flammpunkt von wenigstens 100 °C, wie z. B. von 105 °C, auf. Des Weiteren verfügen die erfindungsgemäßen Isoalkangemische über ausgezeichnete sensorische Eigenschaften. Sie hinterlassen ein gutes Hautgefühl, ziehen gut auf keratinische Oberflächen auf und verleihen damit behandeltem Haar eine gute Kämmbarkeit. Sie eignen sich somit in besonders vorteilhafter Weise zum teilweisen oder vollständigen Ersatz von anderen hydrophoben Komponenten kosmetischer und pharmazeutischer Mittel, insbesondere von Siliconölen und Mineralölen.

Neben den oben beschriebenen Isoalkangemischen eignen sich für den Einsatz in kosmetischen oder pharmazeutischen Mitteln auch Isoalkangemische, die einen sehr hohen Anteil an Alkanen desselben Molekulargewichts aufweisen. Dazu zählen Isoalkangemische, die größer gleich 95 Gew.-%, bevorzugt wenigstens 96 Gew.-%, insbesondere wenigstens 97 Gew.-% Alkane desselben Molekulargewichts aufweisen. Bevorzugt handelt es sich um $C_{16}$-Isoalkangemische (im Folgenden als "hoch $C_{16}$-haltige Isoalkangemische" bezeichnet). Bevorzugt enthalten die kosmetischen oder pharmazeutischen Mittel dann ein $C_{16}$-Alkane aufweisendes Gemisch, das dadurch gekennzeichnet ist, dass das Gemisch eine Zusammensetzung aufweist, bei der die enthaltenen Moleküle im Mittel weniger als 1,0 quartäre C-Atome je Molekül enthalten, wobei das Gemisch einen Anteil an $C_{16}$-Alkanen von größer gleich 95 Massen-% und wobei das Gemisch einen Anteil von weniger als 5 Massen-% an n-Hexadecan aufweist.

Derartige $C_{16}$-Isoalkane aufweisende Gemische sind erhältlich durch ein Verfahren, wobei

a) ein Buten-haltiger $C_4$-Kohlenwasserstoffstrom, der weniger als 5 Massen-%, bezogen auf die Summe aller Butene, an Isobuten aufweist, in Gegenwart eines Nickel-haltigen Katalysators oligomerisiert wird,

b) dass aus dem Reaktionsgemisch eine $C_{16}$-Olefinfraktion abgetrennt wird, und

c) dass die $C_{16}$-Fraktion hydriert wird.

Geeignete $C_{16}$-Isoalkangemische und ein Verfahren zu ihrer Herstellung sind in der DE 10 2004 018 753 A1 beschrieben, auf die hier in vollem Umfang Bezug genommen wird. In der DE 10 2004 018 753 A1 werden die Kohlenwasserstoffgemische als "$C_{16}$-"Alkan"gemische bezeichnet, obwohl sie auch nur einen sehr geringen Anteil an n-Hexadecan aufweisen. Im Rahmen der vorliegenden Anmeldung wurde die gleichbedeutende Bezeichnung "Iso"alkangemisch verwendet, um Alkangemische zu bezeichnen, die verzweigte Alkane enthalten.

Die nach dem zuerst beschriebenen Verfahren erhältlichen Isoalkangemische, weisen aber vorteilhafte Eigenschaften auf.

Insbesondere übertreffen sie hinsichtlich der rheologischen Eigenschaften und speziell beim Spreitverhalten alle aus dem Stand der

Technik bekannten Ölkörper, auch die der DE 10 2004 018 753 A1, die ansonsten gute anwendungstechnische Eigenschaften aufweisen.

Die eingesetzten Isoalkangemische und die hoch $C_{16}$-haltige Isoalkangemische eignen sich sowohl zur Herstellung homogenphasiger hydrophober Zusammensetzungen als auch zur Formulierung von heterogenphasigen Zusammensetzungen, die zusätzlich wenigstens eine wasserlösliche (hydrophile) flüssige oder feste Verbindung umfassen. "Homogenphasige Zusammensetzungen" weisen unabhängig von der Anzahl ihrer Bestandteile nur eine einzige Phase auf. "Heterogenphasige Zusammensetzungen" sind disperse Systeme von zwei oder mehreren miteinander nicht mischbaren Komponenten. Dazu zählen fest/flüssig-, flüssig/flüssig- und fest/flüssig/flüssig-Zusammensetzungen, wie Dispersionen und Emulsionen, z. B. O/W- und W/O-Formulierungen, die wenigstens ein erfindungsgemäßes Isoalkangemisch als Öl- bzw. Fettkomponente und Wasser als nicht mischbare Phasen aufweisen.

Die eingesetzten Isoalkangemische und die hoch $C_{16}$-haltige Isoalkangemische eignen sich auch in vorteilhafter Weise zur Herstellung von kosmetischen Zusammensetzungen und pharmazeutischen Zusammensetzungen in Form von opaken bis klaren Gelen.

Unter Wirkstoffen für Kosmetika und Arzneimittel werden im Allgemeinen Substanzen verstanden, die bereits in geringer Konzentration eine Wirkung entfalten, z. B. eine kosmetische Wirkung auf Haut und/oder Haaren bzw. eine pharmakologische Wirkung in einem Organismus. Effektstoffe sind Stoffe, die Lebewesen oder unbelebten Substraten eine bestimmte Eigenschaft verleihen, beispielsweise Farbpigmente für Schminken.

Die erfindungsgemäßen kosmetischen oder pharmazeutischen Mittel enthalten die Komponente A) vorzugsweise in einem Anteil von 0,01 bis 99,9 Gew.-%, besonders bevorzugt 0,1 bis 75 Gew.-%, insbesondere 1 bis 50 Gew.-%, bezogen auf das Gesamtgewicht des Mittels.

Vorzugsweise liegen die erfindungsgemäßen Mittel in Form einer Salbe, Creme, Emulsion, Suspension, Lotion, Milch, Paste, eines Gels, Schaums oder Sprays vor.

Sofern nicht bereits die Isoalkane A) als Träger zur Herstellung der Mittel dienen, weisen diese vorzugsweise eine Trägerkomponente C) auf, die ausgewählt ist unter Wasser, hydrophilen Komponenten, hydrophoben Komponenten und Mischungen davon.

Geeignete hydrophile Träger C) sind z. B. ein-, zwei- oder mehrwertige Alkohole mit vorzugsweise 1 bis 8 Kohlenstoffatomen, wie Ethanol, n-Propanol, Isopropanol, Propylenglycol, Glycerin, Sorbit, etc.

Geeignete hydrophobe Träger C) sind vorzugsweise ausgewählt unter

i) von Komponente A) verschiedenen Ölen, Fetten, Wachsen,

ii) Estern von $C_6$-$C_{30}$-Monocarbonsäuren mit ein-, zwei- oder dreiwertigen Alkoholen,

iii) von Komponente A) verschiedenen gesättigten acyclischen und cyclischen Kohlenwasserstoffen,

iv) Fettsäuren,

v) Fettalkoholen,

vi) Treibgasen,

und Mischungen davon.

Die erfindungsgemäßen Mittel weisen z. B. eine Öl- bzw. Fettkomponente C) auf, die ausgewählt ist unter: Kohlenwasserstoffen geringer Polarität, wie Mineralölen; linearen gesättigten Kohlenwasserstoffen, vorzugsweise mit mehr als 8 C-Atomen, wie Tetradecan, Hexadecan, Octadecan etc.; cyclischen Kohlenwasserstoffen, wie Decahydronaphthalin; von A) verschiedenen verzweigten Kohlenwasserstoffen; tierischen und pflanzlichen Ölen; Wachsen; Wachsestern; Vaselin; Estern, bevorzugt Estern von Fettsäuren, wie z. B. die Ester von $C_1$-$C_{24}$-Monoalkoholen mit $C_1$-$C_{22}$-Monocarbonsäuren, wie Isopropylisostearat, n-Propylmyristat, iso-Propylmyristat, n-Propylpalmitat, iso-Propylpalmitat, Hexacosanylpalmitat, Octacosanylpalmitat, Triacontanylpalmitat, Dotriacontanylpalmitat, Tetratriacontanylpalmitat, Hexancosanylstearat, Octacosanylstearat, Triacontanylstearat, Dotriacontanylstearat, Tetratriacontanylstearat; Salicylaten, wie $C_1$-$C_{10}$-Salicylaten, z. B. Octylsalicylat; Benzoatestern, wie $C_{10}$-$C_{15}$-Alkylbenzoaten, Benzylbenzoat; anderen kosmetischen Estern, wie Fettsäuretriglyceriden, Propylenglykolmonolaurat, Polyethylenglykolmonolaurat, $C_{10}$-$C_{15}$-Alkyllactaten, etc. und Mischungen davon.

Geeignete Siliconöle C) sind z. B. lineare Polydimethylsiloxane, Poly(methylphenylsiloxane), cyclische Siloxane und Mischungen davon. Das zahlenmittlere Molekulargewicht der Polydimethylsiloxane und Poly(methylphenylsiloxane) liegt vorzugsweise in einem Bereich von etwa 1000 bis 150000 g/mol. Bevorzugte cyclische Siloxane weisen 4- bis 8-gliedrige Ringe auf. Geeignete cyclische Siloxane sind z. B. unter der Bezeichnung Cyclomethicon kommerziell erhältlich.

Bevorzugte Öl- bzw. Fettkomponenten C) sind ausgewählt unter Paraffin und Paraffinölen; Vaselin; natürlichen Fetten und Ölen, wie Castoröl, Sojaöl, Erdnussöl, Olivenöl, Sonnenblumenöl, Sesamöl, Avocadoöl, Kakaobutter, Mandelöl, Pfirsichkernöl, Ricinus-öl, Lebertran, Schweineschmalz, Walrat, Spermacetöl, Spermöl, Weizenkeimöl, Macadamianussöl, Nachtkerzenöl, Jojobaöl; Fettalkoholen, wie Laurylalkohol, Myristylalkohol, Cetylalkohol, Stearylalkohol, Oleylalkohol, Cetylalkohol; Fettsäuren, wie Myristinsäure, Stearinsäure, Palmitinsäure, Ölsäure, Linolsäure, Linolensäure und davon verschiedenen gesättigten, ungesättigten und substituierten Fettsäuren; Wachsen, wie Bienenwachs, Carnaubawachs, Candilillawachs, Walrat sowie Mischungen der zuvor genannten Öl- bzw. Fettkomponenten.

Geeignete kosmetisch und pharmazeutisch verträgliche Öl- bzw. Fettkomponenten C) sind in Karl-Heinz Schrader, Grundlagen und Rezepturen der Kosmetika, 2. Auflage, Verlag Hüthig, Heidelberg, S. 319 - 355 beschrieben, worauf hier Bezug genommen wird.

Die Komponenten B) und C) werden nach dem gewünschten Einsatzbereich der Zusammensetzung ausgewählt. Neben einsatzbereichtypischen Komponenten (z. B. bestimmten pharmazeutischen Wirkstoffen) sind sie ausgewählt unter Trägern, Exzipienten, Emulgatoren, Tensiden, Konservierungsmitteln, Duftstoffen, Parfümölen, Verdickern, Polymeren, Gelbildnern, Farbstoffen, Pigmenten, Lichtschutzmitteln, Konsistenzgebern, Antioxidantien, Entschäumern, Antistatika, Harzen, Lösemitteln, Lösungsvermittlern, Neutralisierungsmitteln, Stabilisatoren, Sterilantien, Treibmitteln, Trocknungsmitteln, Trübungsmitteln, kosmetisch aktiven Wirkstoffen, Haarpolymeren, Haar- und Hautconditionern, Pfropfpolymeren, wasserlöslichen oder dispergierbaren silikonhaltigen Polymeren, Bleichmitteln, Pflegemitteln, Färbemitteln, Tönungsmitteln, Bräunungsmitteln, Feuchthaltemitteln, Rückfettern, Collagen, Eiweisshydrolysaten, Lipiden, Emollienzien und Weichmachern.

Die erfindungsgemäßen kosmetischen oder pharmazeutischen Mittel können als kosmetischen und/oder pharmazeutischen Wirkstoff B) (wie gegebenenfalls auch als Hilfsstoff C)) wenigstens ein kosmetisch oder pharmazeutisch akzeptables Polymer enthalten. Dazu zählen ganz allgemein anionische, kationische, amphotere und neutrale Polymere.

Beispiele für anionische Polymere sind Homo- und Copolymerisate von Acrylsäure und Methacrylsäure oder deren Salze, Copolymere von Acrylsäure und Acrylamid und deren Salze; Natriumsalze von Polyhydroxycarbonsäuren, wasserlösliche oder wasserdispergierbare Polyester, Polyurethane, z. B. Luviset PUR® der Fa. BASF, und Polyharnstoffe. Besonders geeignete Polymere sind Copolymere aus t-Butylacrylat, Ethylacrylat, Methacrylsäure (z. B. Luvimer® 100P), Copolymere aus Ethylacrylat und Methacrylsäure (z. B. Luviumer® MAE), Copolymere aus N-tert.-Butylacrylamid, Ethylacrylat, Acrylsäure (Ultrahold® 8, strong), Copolymere aus Vinylacetat, Crotonsäure und gegebenenfalls weitere

Vinylester (z. B. Luviset®-Marken), Maleinsäureanhydridcopolymere, gegebenenfalls mit Alkohol umgesetzt, anionische Polysiloxane, z. B. carboxyfunktionelle, t-Butylacrylat, Methacrylsäure (z. B. Luviskol® VBM), Copolymere von Acrylsäure und Methacrylsäure mit hydrophoben Monomeren, wie z. B. $C_4$-$C_{30}$-Alkylester der Meth(acrylsäure), $C_4$-$C_{30}$-Alkylvinylester, $C_4$-$C_{30}$-Alkylvinylether und Hyaluronsäure. Beispiele für anionische Polymere sind weiterhin Vinylacetat/Crotonsäure-Copolymere, wie sie beispielsweise unter den Bezeichnungen Resyn® (National Starch) und Gafset® (GAF) im Handel sind und Vinylpyrrolidon/Vinylacrylat-Copolymere, erhältlich beispielsweise unter dem Warenzeichen Luviflex® (BASF). Weitere geeignete Polymere sind das unter der Bezeichnung Luviflex® VBM-35 (BASF) erhältliche Vinylpyrrolidon/Acrylat-Terpolymer und Natriumsulfonat-haltige Polyamide oder Natriumsulfonat-haltige Polyester. Weiterhin geeignet sind Vinylpyrrolidon/Ethylmethacrylat/Methacrylsäure-Copolymere wie sie von der Fa. Stepan unter den Bezeichnungen Stepanhold-Extra und -R1 vertrieben werden und die Carboset®-Marken der Fa. BF Goodrich.

Geeignete kationische Polymere sind z. B. kationische Polymere mit der Bezeichnung Polyquaternium nach INCI, z. B. Copolymere aus Vinylpyrrolidon/N-Vinylimidazoliumsalzen (Luviquat® FC, Luviquat® HM, Luviquat® MS, Luviquat® Care), Copolymere aus N-Vinylpyrrolidon/Dimethylaminoethylmethacrylat, quaternisiert mit Diethylsulfat (Luviquat® PQ 11), Copolymere aus N-Vinylcaprolactam/N-Vinylpyrrolidon/N-Vinylimidazoliumsalzen (Luviquat® Hold); kationische Cellulosederivate (Polyquaternium-4 und -10), Acrylamidocopolymere (Polyquaternium-7) und Chitosan. Geeignete kationische (quaternisierte) Polymere sind auch Merquat® (Polymer auf Basis von Dimethyldiallylammoniumchlorid), Gafquat® (quaternäre Polymere, die durch Reaktion von Polyvinylpyrrolidon mit quaternären Ammoniumverbindungen entstehen), Polymer JR (Hydroxyethylcellulose mit kationischen Gruppen) und kationische Polymere auf pflanzlicher Basis, z. B. Guarpolymere, wie die Jaguar®-Marken der Fa. Rhodia.

Weitere geeignete Polymere sind auch neutrale Polymere, wie Polyvinylpyrrolidone, Copolymere aus N-Vinylpyrrolidon und Vinylacetat und/oder Vinylpropionat, Polysiloxane, Polyvinylcaprolactam und andere Copolymere mit N-Vinylpyrrolidon, Polyethylenimine und deren Salze, Polyvinylamine und deren Salze, Cellulosederivate, Polyasparaginsäuresalze und Derivate. Dazu zählt beispielsweise Luviflex® Swing (teilverseiftes Copolymerisat von Polyvinylacetat und Polyethylenglykol, Fa. BASF).

Geeignete Polymere sind auch nichtionische, wasserlösliche bzw. wasserdispergierbare Polymere oder Oligomere, wie Polyvinylcaprolactam, z. B. Luviskol® Plus (BASF), oder Polyvinylpyrrolidon und deren Copolymere, insbesondere mit Vinylestern, wie Vinylacetat, z. B. Luviskol® VA 37 (BASF); Polyamide, z. B. auf Basis von Itaconsäure und aliphatischen Diaminen, wie sie z. B. in der DE-A-43 33 238 beschrieben sind.

Geeignete Polymere sind auch amphotere oder zwitterionische Polymere, wie die unter den Bezeichnungen Amphomer® (National Starch) erhältlichen Octylacrylamid/Methyl-methacrylat/tert.-Butylaminoethylmethacrylat/2-Hydroxypropylmethacrylat-Copolymere sowie zwitterionische Polymere, wie sie beispielsweise in den deutschen Patentanmeldungen DE 39 29 973, DE 21 50 557, DE 28 17 369 und DE 37 08 451 offenbart sind. Acrylamidopropyltrimethylammoniumchlorid/Acrylsäure- bzw. - Methacrylsäure-Copolymerisate und deren Alkali- und Ammoniumsalze sind bevorzugte zwitterionische Polymere. Weiterhin geeignete zwitterionische Polymere sind Methacroylethylbetain/Methacrylat-Copolymere, die unter der Bezeichnung Amersette® (AMERCHOL) im Handel erhältlich sind, und Copolymere aus Hydroxyethylmethacrylat, Methylmethacrylat, N,N-Dimethylaminoethylmethacrylat und Acrylsäure (Jordapon®).

Geeignete Polymere sind auch nichtionische, siloxanhaltige, wasserlösliche oder -dispergierbare Polymere, z. B. Polyethersiloxane, wie Tegopren® (Fa. Goldschmidt) oder Belsil® (Fa. Wacker).

Geeignete Verdickungsmittel sind vernetzte Polyacrylsäuren und deren Derivate, Polysaccharide und deren Derivate, wie Xanthan-gum, Agar-Agar, Alginate oder Tylosen, Cellulosederivate, z. B. Carboxymethylcellulose oder Hydroxycarboxymethylcellulose, Fettalkohole, Monoglyceride und Fettsäuren, Polyvinylalkohol und Polyvinylpyrrolidon.

Geeignete kosmetisch und/oder dermatologisch aktive Wirkstoffe sind z. B. färbende Wirkstoffe, Haut- und Haarpigmentierungsmittel, Tönungsmittel, Bräunungsmittel, Bleichmittel, Keratin-härtende Stoffe, antimikrobielle Wirkstoffe, Lichtfilterwirkstoffe, Repellentwirkstoffe, hyperemisierend wirkende Stoffe, keratolytisch und keratoplastisch wirkende Stoffe, Antischuppenwirkstoffe, Antiphlogistika, keratinisierend wirkende Stoffe, antioxidativ bzw. als Radikalfänger aktive Wirkstoffe, hautbefeuchtende oder -feuchthaltende Stoffe, rückfettende Wirkstoffe, desodorierende Wirkstoffe, sebostatische Wirkstoffe, Pflanzenextrakte, antierythimatös oder antiallergisch aktive Wirkstoffe und Mischungen davon.

Künstlich hautbräunende Wirkstoffe, die geeignet sind, die Haut ohne natürliche oder künstliche Bestrahlung mit UV-Strahlen zu bräunen, sind z. B. Dihydroxyaceton, Alloxan und Walnussschalenextrakt. Geeignete Keratin-härtende Stoffe sind in der Regel Wirkstoffe, wie sie auch in Antitranspirantien eingesetzt werden, wie z. B. Kaliumaluminiumsulfat, Aluminiumhydroxychlorid, Aluminiumlactat, etc. Antimikrobielle Wirkstoffe werden eingesetzt, um Mikroorganismen zu zerstören bzw. ihr Wachstum zu hemmen und dienen somit sowohl als Konservierungsmittel als auch als desodorierend wirkender Stoff, welcher die Entstehung oder die Intensität von Körpergeruch vermindert. Dazu zählen z. B. übliche, dem Fachmann bekannte Konservierungsmittel, wie p-Hydroxybenzoesäureester, Imidazolidinyl-Harnstoff, Formaldehyd, Sorbinsäure, Benzoesäure, Salicylsäure, etc. Derartige desodorierend wirkende Stoffe sind z. B. Zinkricinoleat, Triclosan, Undecylensäurealkylolamide, Citronensäuretriethylester, Chlorhexidin etc. Geeignete Repellentwirkstoffe sind Verbindungen, die in der Lage sind, bestimmte Tiere, insbesondere Insekten, vom Menschen abzuhalten oder zu vertreiben. Dazu gehört z. B. 2-Ethyl-1,3-hexandiol, N,N-Diethyl-m-toluamid etc. Geeignete hyperemisierend

wirkende Stoffe, welche die Durchblutung der Haut anregen, sind z. B. ätherische Öle, wie Latschenkiefer, Lavendel, Rosmarin, Wacholderbeer, Rosskastanienextrakt, Birkenblätterextrakt, Heublumenextrakt, Ethylacetat, Campher, Menthol, Pfefferminzöl, Rosmarinextrakt, Eukalyptusöl, etc. Geeignete keratolytisch und keratoplastisch wirkende Stoffe sind z. B. Salicylsäure, Kalziumthioglykolat, Thioglykolsäure und ihre Salze, Schwefel, etc. Geeignete Antischuppen-Wirkstoffe sind z. B. Schwefel, Schwefelpolyethylenglykolsorbitanmonooleat, Schwefelricinolpolyethoxylat, Zinkpyrithion, Aluminiumpyrithion, etc. Geeignete Antiphlogistika, die Hautreizungen entgegenwirken, sind z. B. Allantoin, Bisabolol, Dragosantol, Kamillenextrakt, Panthenol, etc.

Geeignete Lichtfilterwirkstoffe sind Stoffe, die UV-Strahlen in einen UV-B- und/oder UV/A-Bereich absorbieren. Dazu zählen Lichtschutzpigmente, z. B. feindisperse Metalloxide und -salze. Dazu zählen beispielsweise Titandioxid, Talkum, Zinkoxid, Bariumsulfat, Zinkstearat, etc.

Vorteilhafte Breitbandfilter, UV-A- oder UV-B-Filtersubstanzen sind beispielsweise Vertreter der folgenden Verbindungsklassen:

Bis-Resorcinyltriazinderivate mit der folgenden Struktur:

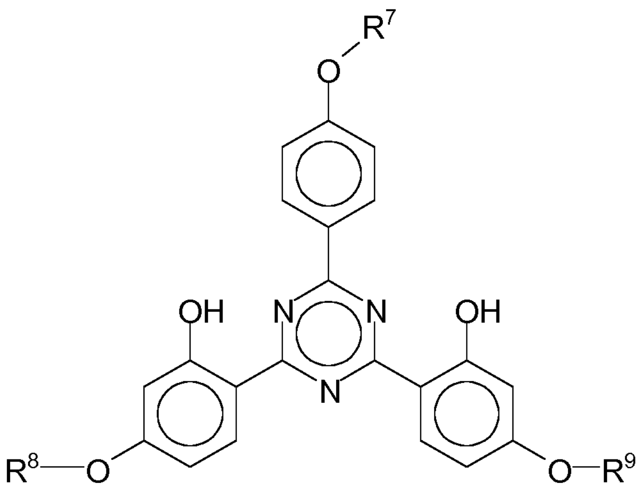

wobei $R^7$, $R^8$ und $R^9$ unabhängig voneinander gewählt werden aus der Gruppe der verzweigten und unverzweigten Alkylgruppen mit 1 bis 10 Kohlenstoffatomen bzw. ein einzelnes Wasserstoffatom darstellen. Insbesondere bevorzugt sind das 2,4-Bis-{[4-(2-Ethyl-hexyloxy)-2-hydroxy]-phenyl}-6-(4-methoxyphenyl)-1,3,5-triazin (INCI: Aniso Triazin), welches unter der Handelsbezeichnung Tinosorb® S bei der CIBA-Chemikalien GmbH erhältlich ist.

Auch andere UV-Filtersubstanzen, welche das Strukturmotiv

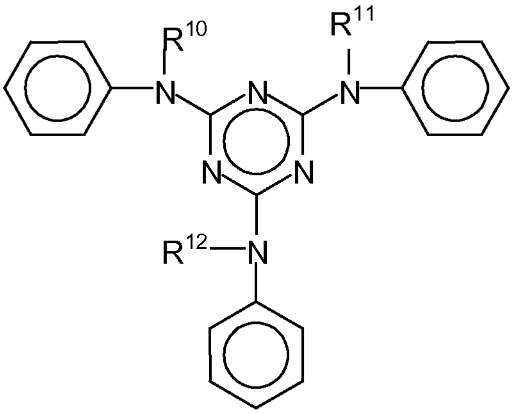

aufweisen, sind vorteilhafte UV-Filtersubstanzen im Sinne der vorliegenden Erfindung, beispielsweise die in der Europäischen Offenlegungsschrift EP 570 838 A1 beschriebenen s-Triazinderivate, deren chemische Struktur durch die

generische Formel

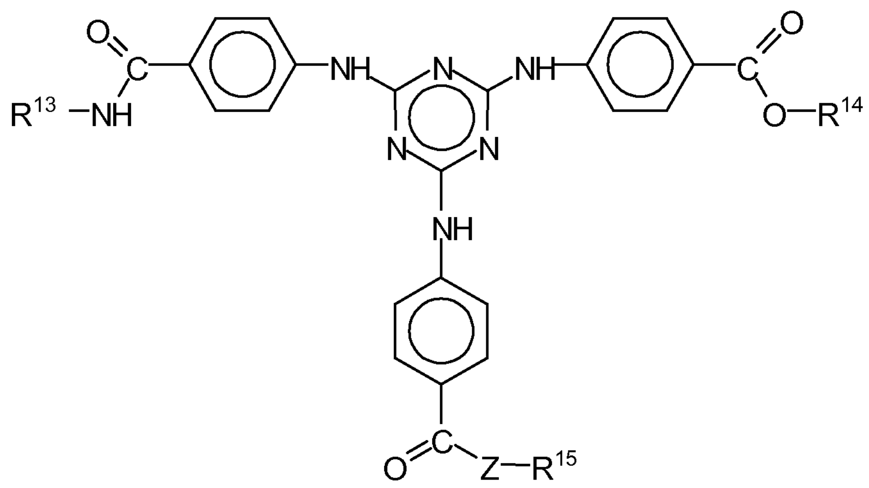

wiedergegeben wird, wobei

$R^{13}$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$-Alkylgruppen, darstellt,

Z ein Sauerstoffatom oder eine NH-Gruppe darstellt,

$R^{14}$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$-Alkylgruppen, oder ein Wasserstoffatom, ein Alkalimetallatom, eine Ammoniumgruppe oder eine Gruppe der Formel

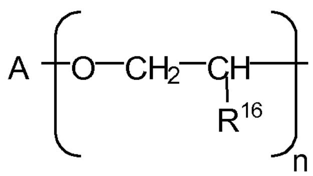

bedeutet, in welcher

A einen verzweigten oder unverzweigten $C_1$-$C_{18}$-Alkylrest, einen $C_5$-$C_{12}$-Cycloalkyl- oder Arylrest darstellt, gegebenenfalls substituiert mit einer oder mehreren $C_1$-$C_4$-Alkylgruppen,

$R^{16}$ ein Wasserstoffatom oder eine Methylgruppe darstellt,

n eine Zahl von 1 bis 10 darstellt,

$R^{15}$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$-Alkylgruppen, darstellt, wenn X die NH-Gruppe darstellt, und einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$-Alkylgruppen, oder ein Wasserstoffatom, ein Alkalimetallatom, eine Ammoniumgruppe oder eine Gruppe der Formel

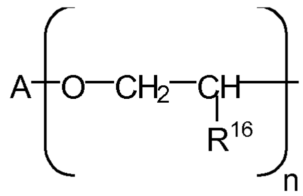

bedeutet, in welcher

- A einen verzweigten oder unverzweigten $C_1$-$C_{18}$-Alkylrest, einen $C_5$-$C_{12}$-Cycloalkyl- oder Arylrest darstellt, gegebenenfalls substituiert mit einer oder mehreren $C_1$-$C_4$-Alkylgruppen,
- $R^{16}$ ein Wasserstoffatom oder eine Methylgruppe darstellt,
- n eine Zahl von 1 bis 10 darstellt, wenn X ein Sauerstoffatom darstellt.

Besonders bevorzugte UV-Filtersubstanz im Sinne der vorliegenden Erfindung ist ferner ein unsymmetrisch substituiertes s-Triazin, dessen chemische Struktur durch die Formel

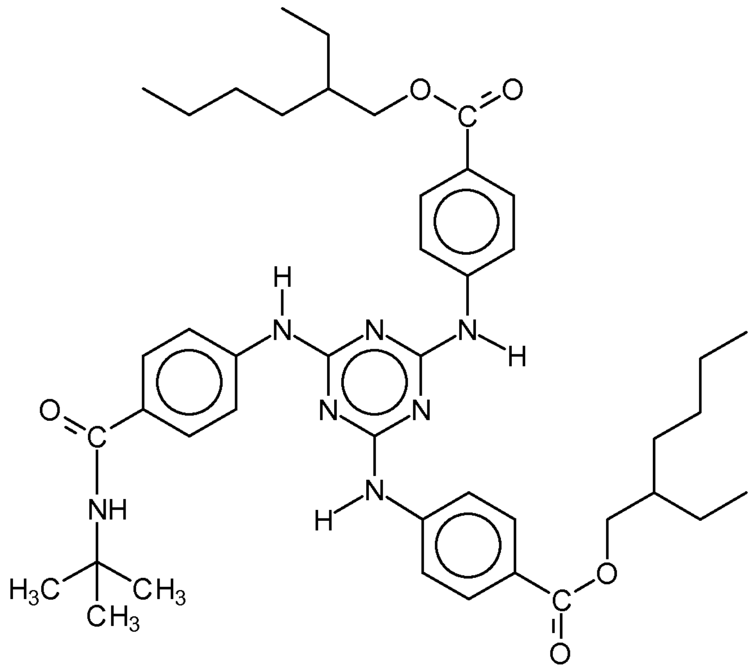

wiedergegeben wird, welches im Folgenden auch als Dioctylbutylamidotriazon (INCI: Diethylhexylbutamidotriazone) bezeichnet wird und unter der Handelsbezeichnung UVASORB® HEB bei Sigma 3V erhältlich ist.

Vorteilhaft im Sinne der vorliegenden Erfindung ist auch ein symmetrisch substituiertes s-Triazin, das 4,4',4"-(1,3,5-Triazin-2,4,6-triyltriimino)-tris-benzoesäure-tris(2-ethyl-hexylester), synonym: 2,4,6-Tris-[anilino-(p-carbo-2'-ethyl-1'-hexyloxy)]-1,3,5-triazin (INCI: Ethylhexyl Triazone), welches von der BASF Aktiengesellschaft unter der Warenbezeichnung UVINUL® T 150 vertrieben wird.

Auch in der Europäischen Offenlegungsschrift 775 698 werden bevorzugt einzusetzende Bis-Resorcinyltriazinderivate beschrieben, deren chemische Struktur durch die generische Formel

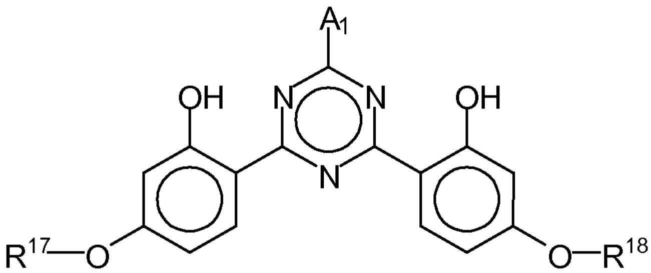

wiedergegeben wird, wobei $R^{17}$ und $R^{18}$ u. a. $C_3$-$C_{18}$-Alkyl oder $C_2$-$C_{18}$-Alkenyl und $A_1$ einen aromatischen Rest repräsentieren.

Vorteilhaft im Sinne der vorliegenden Erfindung sind ferner das 2,4-Bis-{[4-(3-sulfonato)-2-hydroxy-propyloxy)-2-hydroxy]-phenyl}-6-(4-methoxy-phenyl)-1,3,5-triazin Natriumsalz, das 2,4-Bis-{[4-(3-(2-Propyloxy)-2-hydroxy-propyloxy)-2-hydroxy]-phenyl}-6-(4-methoxyphenyl)-1,3,5-triazin, das 2,4-Bis-{[4-(2-ethyl-hexyloxy)-2-hydroxy]-phenyl}-6-[4-(2-methoxyethyl-carboxyl)-phenylamino]-1,3,5-triazin, das 2,4-Bis-{[4-(3-(2-propyloxy)-2-hydroxy-propyloxy)-2-hydroxy]-phenyl}-6-[4-(2-ethyl-carboxyl)-phenylamino]-1,3,5-triazin, das 2,4-Bis-{[4-(2-ethyl-hexyloxy)-2-hydroxy]-phenyl}-6-(1-methyl-pyrrol-2-yl)-1,3,5-triazin, das 2,4-Bis-{[4-tris(trimethylsiloxy-silylpropyloxy)-2-hydroxy]-phenyl}-6-(4-methoxyphenyl)-1,3,5-triazin, das 2,4-Bis-{[4-(2"-methylpropenyloxy)-2-hydroxy]-phenyl}-6-(4-methoxyphenyl)-1,3,5-triazin und das 2,4-Bis-{[4-(1',1',1',3',5',5',5'-Heptamethylsiloxy-2"-methyl-propyloxy)-2-hydroxy]-phenyl}-6-(4-methoxy-phenyl)-1,3,5-triazin.

Vorteilhafte öllösliche UV-B- und/oder Breitband-Filtersubstanzen sind z. B.:

3-Benzylidencampher-Derivate, vorzugsweise 3-(4-Methylbenzyliden)campher, 3-Benzylidencampher;
4-Aminobenzoesäure-Derivate, vorzugsweise
4-(Dimethylamino)-benzoesäure(2-ethylhexyl)ester,
4-(Dimethylamino)benzoesäureamylester,

Derivate des Benzophenons, vorzugsweise 2-Hydroxy-4-methoxybenzophenon (unter der Handelsbezeichnung Uvinul® M40 von der Fa. BASF erhältlich) 2-Hydroxy-4-methoxy-4'-methylbenzophenon, 2,2'-Dihydroxy-4-methoxybenzophenon, 2,2',4,4'-Tetrahydroxybenzophenon (unter der Handelsbezeichnung Uvinul® D 50 von der Fa. BASF erhältlich).

Besonders vorteilhafte bei Raumtemperatur flüssige UV-Filtersubstanzen im Sinne der vorliegenden Erfindung sind Homomenthylsalicylat, 2-Ethylhexyl-2-cyano-3,3-diphenylacrylat, 2-Ethylhexyl-2-hydroxybenzoat und Ester der Zimtsäure, vorzugsweise 4-Methoxyzimtsäure(2-ethylhexyl)ester und 4-Methoxyzimtsäureisopentylester.

Homomethylsalicylat (INCI: Homosalate) zeichnet sich durch die folgende Struktur aus:

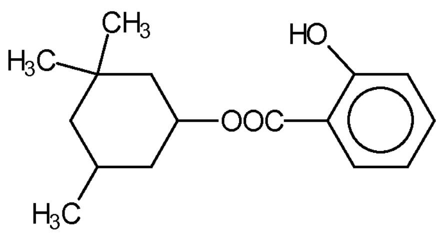

2-Ethylhexyl-2-cyano-3,3-diphenylacrylat (INCI: Octocrylene) ist von BASF unter der Bezeichnung Uvinul® N 539T erhältlich und zeichnet sich durch folgende Struktur aus:

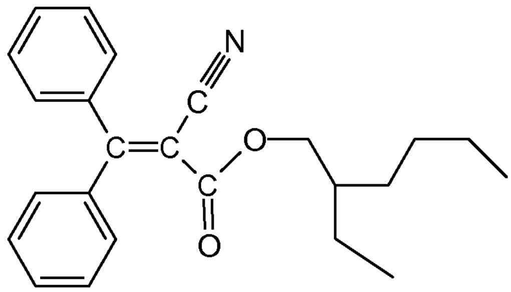

2-Ethylhexyl-2-hydroxybenzoat (2-Ethylhexylsalicylat, Octylsalicylat, INCI: Ethylhexyl Salicylate) ist beispielsweise bei Haarmann & Reimer unter der Handelsbezeichnung Neo Heliopan® OS erhältlich und zeichnet sich durch die folgende Struktur aus:

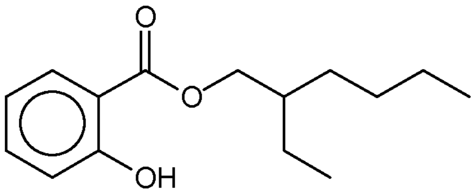

4-Methoxyzimtsäure(2-ethylhexyl)ester (2-Ethylhexyl-4-methoxycinnamat, INCI: Ethylhexyl Methoxycinnamate) ist beispielsweise bei Fa. BASF unter der Handelsbezeichnung Uvinul® MC 80 erhältlich und zeichnet sich durch die folgende Struktur aus:

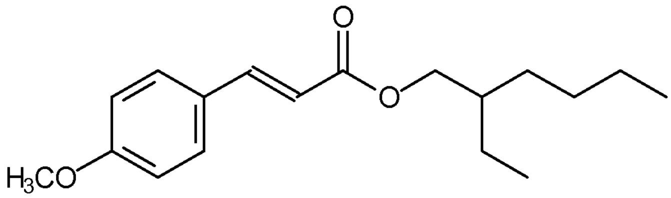

4-Methoxyzimtsäureisopentylester (Isopentyl-4-methoxycinnamat, INCI: Isoamyl p-Methoxycinnamate) ist beispielsweise bei Haarmann & Reimer unter der Handelsbezeichnung Neo Heliopan® E 1000 erhältlich und zeichnet sich durch die folgende Struktur aus:

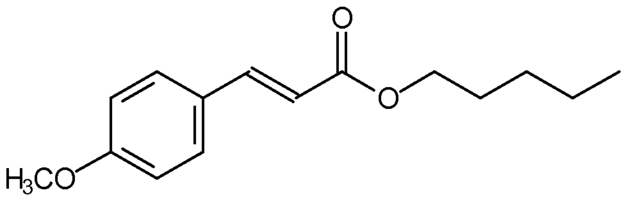

Vorteilhafte Dibenzoylmethanderivate im Sinne der vorliegenden Erfindung sind, insbesondere das 4-(tert.-Butyl)-4'-methoxydibenzoylmethan (CAS-Nr. 70356-09-1), welches von BASF unter der Marke Uvinul® BMBM und von Merck unter der Handelsbezeichnung Eusolex® 9020 verkauft wird zeichnet sich durch folgende Struktur aus:

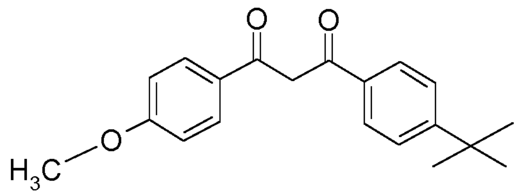

Ein weiteres vorteilhaftes Dibenzoylmethanderivat ist das 4-Isopropyl-Dibenzoylmethan (CAS-Nr. 63250-25-9), welches von Merck unter dem Namen Eusolex® 8020 verkauft wird. Das Eusolex 8020 zeichnet sich durch folgende Struktur aus:

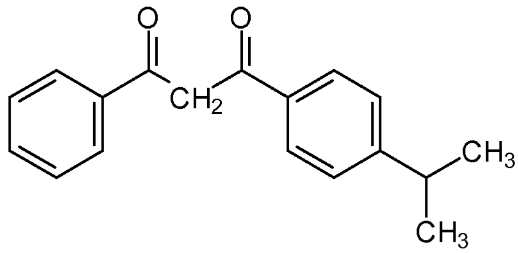

Benzotriazole zeichnen sich durch die folgende Strukturformel aus:

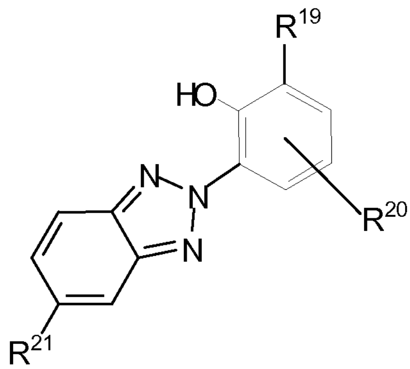

,

worin

$R^{19}$ und $R^{20}$ unabhängig voneinander lineare oder verzweigte, gesättigte oder ungesättigte, substituierte (z. B. mit einem Phenylrest substituierte) oder unsubstituierte Alkylreste mit 1 bis 18 Kohlenstoffatomen stehen.

Vorteilhaftes Benzotriazol im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]-propyl]-phenol (CAS-Nr.: 155633-54-8) mit der INCI-Bezeichnung Drometrizole Trisiloxane, welches von Fa. Chimex unter der Marke Mexoryl® XL verkauft wird und durch die folgende chemische Strukturformel

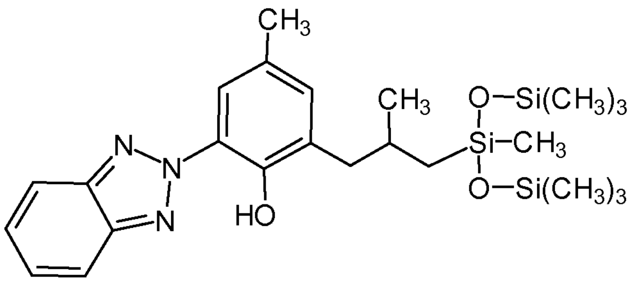

gekennzeichnet ist.

Weitere vorteilhafte Benzotriazole im Sinne der vorliegenden Erfindung sind [2,4'-Dihydroxy-3-(2H-benzotriazol-2-yl)-5-(1,1,3,3-tetramethylbutyl)-2'-n-octoxy-5'-benzoyl]diphenylmethan, 2,2' Methylen-bis-[6-(2H-benzotriazol-2-yl)-4-(methyl)phenol], 2,2'-Methylen-bis-[6-(2H-benzotriazol-2-yl)-4-(1,1,3,3-tetramethylbutyl)phenol], 2-(2'-Hydroxy-5'-octylphenyl)-benzotriazol, 2-(2'-Hydroxy-3',5'-di-t-amylphenyl)benzotriazol und 2-(2'-Hydroxy-5'-methylphenyl)benzotriazol.

Ein weiterer im Sinne der vorliegenden Erfindung vorteilhafter UV-Filter ist die in EP-A-0 916 335 beschriebene Diphenylbutadienverbindung der folgenden Formel.

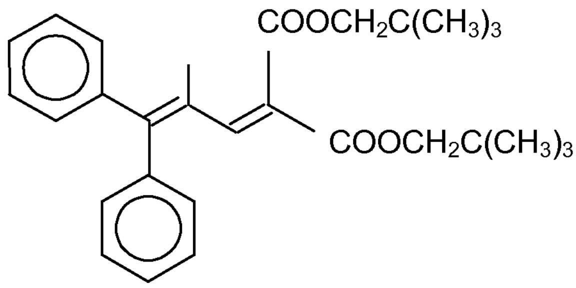

Ein weiterer im Sinne der vorliegenden Erfindung vorteilhafter UV-A-Filter ist der in EP-A-0 895 776 beschriebene 2-(4-Ethoxy-anilinomethylen)-propandicarbonsäure-diethylester der folgenden Formel.

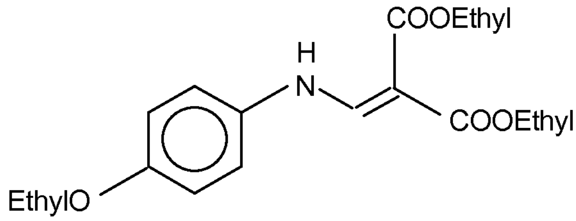

Vorteilhaft im Sinne der vorliegenden Erfindung ist ebenfalls ein aminosubstituiertes Hydroxybenzophenon der folgenden Formel:

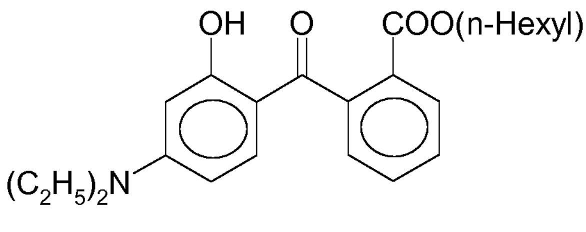

welche von der BASF Aktiengesellschaft als UV-A Filter unter der Warenbezeichnung UVINUL® A Plus vertrieben wird.

Nach einer bevorzugten Ausführungsform handelt es sich bei den erfindungsgemäßen Mitteln um ein Hautreinigungsmittel.

Bevorzugte Hautreinigungsmittel sind Seifen von flüssiger bis gelförmiger Konsistenz, wie Transparentseifen, Luxusseifen, Deoseifen, Cremeseifen, Babyseifen, Hautschutzseifen, Abrasivseifen und Syndets, pasteuse Seifen, Schmierseifen und Waschpasten, flüssige Wasch-, Dusch- und Badepräparate, wie Waschlotionen, Duschbäder und -gele, Schaumbäder, Ölbäder und Scrub-Präparate, Rasierschäume, -lotionen und -cremes.

Nach einer weiteren bevorzugten Ausführungsform handelt es sich bei den erfindungsgemäßen Mitteln um kosmetische Mittel zur Pflege und zum Schutz der Haut, Nagelpflegemittel oder Zubereitungen für die dekorative Kosmetik.

Geeignete hautkosmetische Mittel sind z. B. Gesichtswässer, Gesichtsmasken, Deodorantien und andere kosmetische Lotionen. Mittel für die Verwendung in der dekorativen Kosmetik umfassen beispielsweise Abdeckstifte, Theaterfarben, Mascara und Lidschatten, Lippenstifte, Lipgloss, Kajalstifte, Eyelinern, Rouges, Puder und Augenbrauenstifte.

Außerdem können die Isoalkangemische A) verwendet werden in Nose-Strips zur Porenreinigung, in Antiaknemitteln, Repellents, Rasiermitteln, Haarentfernungsmitteln, Intimpflegemitteln, Fußpflegemitteln sowie in der Babypflege.

Bei den erfindungsgemäßen Hautpflegemitteln handelt es sich insbesondere um W/O- oder O/W-Hautcremes, Tag- und Nachtcremes, Augencremes, Gesichtscremes, Antifaltencremes, Feuchthaltecremes, Bleichcremes, Vitamincremes, Hautlotionen, Pflegelotionen und Feuchthaltelotionen.

Hautkosmetische und dermatologische Mittel auf Basis der zuvor beschriebenen Isoalkangemische A) zeigen vorteilhafte Wirkungen. Die Polymere können unter anderem zur Feuchthaltung und Konditionierung der Haut und zur Verbesserung des Hautgefühls beitragen. Durch Zusatz der erfindungsgemäßen Polymere kann in bestimmten Formulierungen eine erhebliche Verbesserung der Hautverträglichkeit erreicht werden.

Hautkosmetische und dermatologische Mittel enthalten vorzugsweise wenigstens ein Isoalkangemisch A) in einem Anteil von etwa 0,001 bis 30 Gew.-%, vorzugsweise 0,01 bis 20 Gew.-%, ganz besonders bevorzugt 0,1 bis 12 Gew.-%, bezogen auf das Gesamtgewicht des Mittels.

Besonders Lichtschutzmittel auf Basis der Isoalkangemische A) besitzen die Eigenschaft, die Verweilzeit der UV-absorbierenden Inhaltsstoffe im Vergleich zu gängigen Hilfsmitteln wie Polyvinylpyrrolidon zu erhöhen.

Je nach Anwendungsgebiet können die erfindungsgemäßen Mittel in einer zur Hautpflege geeigneten Form, wie z. B. als Creme, Schaum, Gel, Stift, Mousse, Milch, Spray (Pumpspray oder treibmittelhaltiger Spray) oder Lotion appliziert werden.

Die hautkosmetischen Zubereitungen können neben den Isoalkangemischen A) und geeigneten Trägern noch weitere in der Hautkosmetik übliche Wirkstoffe und Hilfsstoffe, wie zuvor beschrieben, enthalten. Dazu zählen vorzugsweise Emulgatoren, Konservierungsmittel, Parfümöle, kosmetische Wirkstoffe wie Phytantriol, Vitamin A, E und C, Retinol, Bisabolol, Panthenol, Lichtschutzmittel, Bleichmittel, Färbemittel, Tönungsmittel, Bräunungsmittel, Collagen, Eiweißhydrolysate, Stabilisatoren, pH-Wert-Regulatoren, Farbstoffe, Salze, Verdicker, Gelbildner, Konsistenzgeber, Silicone, Feuchthaltemittel, Rückfetter und weitere übliche Additive.

Bevorzugte zusätzliche Öl- und Fettkomponenten der hautkosmetischen und dermatologischen Mittel sind die zuvor genannten mineralischen und synthetischen Öle, wie z. B. Paraffine, Siliconöle und aliphatische Kohlenwasserstoffe mit mehr als 8 Kohlenstoffatomen, tierische und pflanzliche Öle, wie z. B. Sonnenblumenöl, Kokosöl, Avocadoöl, Olivenöl, Lanolin, oder Wachse, Fettsäuren, Fettsäureester, wie z. B. Triglyceride von $C_6$-$C_{30}$-Fettsäuren, Wachsester, wie z. B. Jojobaöl, Fettalkohole, Vaseline, hydriertes Lanolin und acetyliertes Lanolin sowie Mischungen davon.

Man kann die erfindungsgemäßen Isoalkangemische und hoch $C_{16}$-haltige Isoalkangemische auch mit herkömmlichen Ölkörpern, wie zuvor beschrieben, abmischen, falls spezielle Eigenschaften eingestellt werden sollen.

Die erfindungsgemäß eingesetzten Isoalkangemische und hoch $C_{16}$-haltige Isoalkangemische eignen sich vorteilhaft zur Verbesserung des Anfassgefühls, des Spreitverhaltens, der Wasserresistenz und/oder der Bindung von Wirk- und Hilfsstoffen, wie Pigmenten. Zusätzlich können die kosmetischen Zubereitungen konditionierende Substanzen auf Basis von Siliconverbindungen enthalten. Geeignete Siliconverbindungen sind beispielsweise Polyalkylsiloxane, Polyarylsiloxane, Polyarylalkylsiloxane, Polyethersiloxane oder Siliconharze.

Die Herstellung der kosmetischen oder dermatologischen Zubereitungen erfolgt nach üblichen, dem Fachmann bekannten Verfahren.

Bevorzugt liegen die kosmetischen und dermatologischen Mittel in Form von Emulsionen insbesondere als Wasser-in-Öl-(W/O)- oder Öl-in-Wasser(O/W)-Emulsionen vor. Es ist aber auch möglich, andere Formulierungsarten zu wählen, beispielsweise Hydrodispersionen, Gele, Öle, Oleogele, multiple Emulsionen, beispielsweise in Form von W/O/W- oder O/W/O-Emulsionen, wasserfreie Salben bzw. Salbengrundlagen, usw.

Die Herstellung von Emulsionen erfolgt nach bekannten Methoden. Die Emulsionen enthalten neben wenigstens einem Isoalkangemisch A) und Wasser gegebenenfalls weitere übliche Bestandteile, wie Fettalkohole, Fettsäureester und insbesondere Fettsäuretriglyceride, Fettsäuren, Lanolin und Derivate davon, natürliche oder synthetische Öle oder Wachse und Emulgatoren in Anwesenheit von Wasser. Die Auswahl der Emulsionstyp-spezifischen Zusätze und die Herstellung geeigneter Emulsionen ist beispielsweise beschrieben in Schrader, Grundlagen und Rezepturen der Kosmetika, Hüthig Buch Verlag, Heidelberg, 2. Auflage, 1989, dritter Teil, worauf hiermit ausdrücklich Bezug genommen wird.

Eine geeignete Emulsion, z. B. für eine Hautcreme etc., enthält im Allgemeinen eine wässrige Phase, die mittels eines geeigneten Emulgatorsystems in einer Öl- oder Fettphase emulgiert ist. Zur Bereitstellung der hydrophoben Phase wird ein Isoalkangemisch A) sowie gegebenenfalls weitere Ölkörper eingesetzt.

Bevorzugte Fettkomponenten, welche in der Fettphase der Emulsionen zusätzlich enthalten sein können, sind: Kohlenwasserstofföle, wie Paraffinöl, Purcellinöl, Perhydrosqualen und Lösungen mikrokristalliner Wachse in diesen Ölen; tierische oder pflanzliche Öle, wie Süßmandelöl, Avocadoöl, Calophylumöl, Lanolin und Derivate davon, Ricinusöl, Sesamöl, Olivenöl, Jojobaöl, Karité-Öl, Hoplostethus-Öl; mineralische Öle, deren Destillationsbeginn unter Atmosphärendruck bei ca. 250 °C und deren Destillationsendpunkt bei 410 °C liegt, wie z. B. Vaselinöl; Ester gesättigter oder ungesättigter Fettsäuren, wie Alkylmyristate, z. B. i-Propyl-, Butyl- oder Cetylmyristat, Hexadecylstearat, Ethyl- oder i-Propylpalmitat, Octan- oder Decansäuretriglyceride und Cetylricinoleat.

Die Fettphase kann auch in anderen Ölen lösliche Siliconöle, wie Dimethylpolysiloxan, Methylphenylpolysiloxan

und das Siliconglykol-Copolymer, Fettsäuren und Fettalkohole enthalten.

Neben den Isoalkangemischen A) können auch Wachse verwendet werden, wie z. B. Carnaubawachs, Candilillawachs, Bienenwachs, mikrokristallines Wachs, Ozokeritwachs und Ca-, Mg- und Al-Oleate, -Myristate, -Linoleate und -Stearate.

Weiterhin kann eine erfindungsgemäße Emulsion als O/W-Emulsion vorliegen. Eine derartige Emulsion enthält üblicherweise eine Ölphase, Emulgatoren, die die Ölphase in der Wasserphase stabilisieren, und eine wässrige Phase, die üblicherweise verdickt vorliegt. Als Emulgatoren kommen vorzugsweise O/W-Emulgatoren, wie Polyglycerinester, Sorbitanester oder teilveresterte Glyceride, in Betracht.

Nach einer weiteren bevorzugten Ausführungsform handelt es sich bei den erfindungsgemäßen Mitteln um ein Duschgel, eine Shampoo-Formulierung oder ein Badepräparat.

Solche Formulierungen enthalten wenigstens ein Isoalkangemisch A) sowie üblicherweise anionische Tenside als Basistenside und amphotere und/oder nichtionische Tenside als Cotenside. Weitere geeignete Wirkstoffe und/oder Hilfsstoffe sind im Allgemeinen ausgewählt unter Lipiden, Parfümölen, Farbstoffen, organischen Säuren, Konservierungsstoffen und Antioxidantien sowie Verdickern/Gelbildnern, Hautkonditioniermitteln und Feuchthaltemitteln.

Diese Formulierungen enthalten vorzugsweise 2 bis 50 Gew.-%, bevorzugt 5 bis 40 Gew.-%, besonders bevorzugt 8 bis 30 Gew.% Tenside, bezogen auf das Gesamtgewicht der Formulierung.

In den Wasch-, Dusch- und Badepräparaten können alle in Körperreinigungsmitteln üblicherweise eingesetzte anionische, neutrale, amphotere oder kationische Tenside verwendet werden.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisothionate, Alkylphosphate, Alkyletherphosphate, Alkylethercarboxylate, Alpha-Olefinsulfonate, insbesondere die Alkali- und Erdalkalimetallsalze, z. B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid- oder Propylenoxideinheiten, bevorzugt 1 bis 3 Ethylenoxideinheiten im Molekül aufweisen.

Dazu zählen z. B. Natriumlaurylsulfat, Ammoniumlaurylsulfat, Natriumlaurylethersulfat, Ammoniumlaurylethersulfat, Natriumlaurylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind z. B. Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol. Ferner sind Alkylaminoxide, Mono- oder Dialkylalkanolamide, Fettsäureester von Polyethylenglykolen, ethoxylierte Fettsäureamide, Alkylpolyglycoside oder Sorbitanetherester geeignet.

Außerdem können die Wasch-, Dusch- und Badepräparate übliche kationische Tenside enthalten, wie z. B. quaternäre Ammoniumverbindungen, beispielsweise Cetyltrimethylammoniumchlorid.

Weiterhin können die Duschgel-/Shampoo-Formulierungen Verdicker, wie z. B. Kochsalz, PEG-55, Propylenglykol-Oleat, PEG-120-Methylglucosedioleat und andere, sowie Konservierungsmittel, weitere Wirk- und Hilfsstoffe und Wasser enthalten.

Nach einer weiteren bevorzugten Ausführungsform handelt es sich bei den erfindungsgemäßen Mitteln um ein Haarbehandlungsmittel.

Erfindungsgemäße Haarbehandlungsmittel enthalten vorzugsweise wenigstens ein Isoalkangemisch A) in einer Menge im Bereich von etwa 0,1 bis 30 Gew.-%, bevorzugt 0,5 bis 20 Gew.-%, bezogen auf das Gesamtgewicht des Mittels.

Vorzugsweise liegen die erfindungsgemäßen Haarbehandlungsmittel in Form eines Schaumfestigers, Haarmousses, Haargels, Shampoos, Haarsprays, Haarschaums, Spitzenfluids, Egalisierungsmittels für Dauerwellen, Haarfärbe- und -bleichmittels oder "Hot-Oil-Treatments" vor. Je nach Anwendungsgebiet können die haarkosmetischen Zubereitungen als (Aerosol-)Spray, (Aerosol-)Schaum, Gel, Gelspray, Creme, Lotion oder Wachs appliziert werden. Haarsprays umfassen dabei sowohl Aerosolsprays als auch Pumpsprays ohne Treibgas. Haarschäume umfassen sowohl Aerosolschäume wie auch Pumpschäume ohne Treibgas. Haarsprays und Haarschäume umfassen vorzugsweise überwiegend oder ausschließlich wasserlösliche oder wasserdispergierbare Komponenten. Sind die in den erfindungsgemäßen Haarsprays und Haarschäumen eingesetzten Verbindungen wasserdispergierbar, können sie in Form von wässrigen Mikrodispersionen mit Teilchendurchmessern von üblicherweise 1 bis 350 nm, bevorzugt 1 bis 250 nm, zur Anwendung gebracht werden. Die Feststoffgehalte dieser Präparate liegen dabei üblicherweise in einem Bereich von etwa 0,5 bis 20 Gew.-%. Diese Mikrodispersionen benötigen in der Regel keine Emulgatoren oder Tenside zu ihrer Stabilisierung.

Die erfindungsgemäßen haarkosmetischen Formulierungen enthalten in einer bevorzugten Ausführungsform

a) 0,05 bis 20 Gew.-% wenigstens eines Haarpolymers,

b) 20 bis 99,95 Gew.-% Trägermaterial auf Basis eines erfindungsgemäßen Isoalkangemischs oder eines hoch $C_{16}$-haltigen Isoalkangemischs sowie Wasser und/oder Alkohol,

c) 0 bis 50 Gew.-% wenigstens eines Treibgases,

d) 0 bis 5 Gew.-% wenigstens eines Emulgators,

e) 0 bis 3 Gew.-% wenigstens eines Verdickers, sowie

f) bis zu 25 Gew.-% weitere Bestandteile.

Unter Alkohol sind alle in der Kosmetik üblichen Alkohole zu verstehen, z. B. Ethanol, Isopropanol, n-Propanol.

Unter weiteren Bestandteilen sind die in der Kosmetik üblichen Zusätze zu verstehen, beispielsweise Treibmittel, Entschäumer, grenzflächenaktive Verbindungen, d. h. Tenside, Emulgatoren, Schaumbildner und Solubilisatoren. Die eingesetzten grenzflächenaktiven Verbindungen können anionisch, kationisch, amphoter oder neutral sein. Weitere übliche Bestandteile können ferner sein z. B. Konservierungsmittel, Parfümöle, Trübungsmittel, Wirkstoffe, UV-Filter, Pflegestoffe wie Panthenol, Collagen, Vitamine, Eiweißhydrolysate, Alpha- und Beta-Hydroxycarbonsäuren, Stabilisatoren, pH-Wert-Regulatoren, Farbstoffe, Viskositätsregulierer, Gelbildner, Salze, Feuchthaltemittel, Rückfetter, Komplexbildner und weitere übliche Additive.

Geeignete Haarpolymere sind alle in der Kosmetik bekannten Styling- und Conditionerpolymere. Als herkömmliche Haarkosmetik-Polymere eignen sich beispielsweise die zuvor genannten kationischen, anionischen, neutralen, nichtionischen und amphoteren Polymere, auf die hier Bezug genommen wird.

Zur Einstellung bestimmter Eigenschaften können die Zubereitungen zusätzlich auch konditionierende Substanzen auf Basis von Silikonverbindungen enthalten. Geeignete Silikonverbindungen sind beispielsweise Polyalkylsiloxane, Polyarylsiloxane, Polyarylalkylsiloxane, Polyethersiloxane, Silikonharze oder Dimethicon Copolyole (CTFA) und aminofunktionelle Silikonverbindungen wie Amodimethicone (CTFA).

Die erfindungsgemäßen Polymerisate eignen sich insbesondere als Festigungsmittel in Haarstyling-Zubereitungen, insbesondere Haarsprays (Aerosolsprays und Pumpsprays ohne Treibgas) und Haarschäume (Aerosolschäume und Pumpschäume ohne Treibgas).

In einer bevorzugten Ausführungsform enthalten Spray-Zubereitungen

a) 0,1 bis 10 Gew.-% wenigstens eines Haarpolymers,
b) 20 bis 99,9 Gew.-% Wasser und/oder Alkohol,
c) 0 bis 70 Gew.-% wenigstens eines Treibmittel,
d) 0 bis 20 Gew.-% weitere Bestandteile,
e) 0,01 bis 10 Gew.-% erfindungsgemäßes Isoalkangemisch oder hoch $C_{16}$-haltiges Isoalkangemisch.

Treibmittel sind die für Haarsprays oder Aerosolschäume üblich verwendeten Treibmittel. Bevorzugt sind Gemische aus Propan/Butan, Pentan, Dimethylether, 1,1-Difluorethan (HFC-152 a), Kohlendioxid, Stickstoff oder Druckluft.

Eine erfindungsgemäß bevorzugte Formulierung für Aerosolhaarschäume enthält

a) 0,1 bis 10 Gew.-% wenigstens eines Haarpolymers,
b) 55 bis 99,8 Gew.-% Wasser und/oder Alkohol,
c) 5 bis 20 Gew.-% eines Treibmittel,
d) 0,1 bis 5 Gew.-% eines Emulgators,
e) 0 bis 10 Gew.-% weitere Bestandteile,
f) 0,01 bis 10 Gew.-% erfindungsgemäßes Isoalkangemisch oder hoch $C_{16}$-haltiges Isoalkangemisch.

Als Emulgatoren können alle in Haarschäumen üblicherweise eingesetzten Emulgatoren verwendet werden. Geeignete Emulgatoren können nichtionisch, kationisch bzw. anionisch oder amphoter sein.

Beispiele für nichtionische Emulgatoren (INCI-Nomenklatur) sind Laurethe, z. B. Laureth-4; Cetethe, z. B. Cetheth-1, Polyethylenglykolcetylether; Ceteareth, z. B. Cetheareth-25, Polyglykolfettsäureglyceride, hydroxyliertes Lecithin, Lactylester von Fettsäuren, Alkylpolyglycoside.

Beispiele für kationische Emulgatoren sind Cetyldimethyl-2-hydroxyethylammonium-dihydrogenphosphat, Cetyltrimoniumchlorid, Cetyltrimmoniumbromid, Cocotrimoniummethylsulfat, Quaternium-1 bis x (INCI).

Anionische Emulgatoren können beispielsweise ausgewählt werden aus der Gruppe der Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisothionate, Alkylphosphate, Alkyletherphosphate, Alkylethercarboxylate, Alpha-Olefinsulfonate, insbesondere die Alkali- und Erdalkalimetallsalze, z. B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.

Eine erfindungsgemäß für Styling-Gele geeignete Zubereitung kann beispielsweise wie folgt zusammengesetzt sein:

a) 0,1 bis 10 Gew.-% wenigstens eines Haarpolymers,
b) 80 bis 99,85 Gew.-% Wasser und/oder Alkohol,
c) 0 bis 3 Gew.-%, bevorzugt 0,05 bis 2 Gew.-%, eines erfindungsgemäßen Isoalkangemischs oder hoch $C_{16}$-haltigen Isoalkangemischs,
d) 0 bis 20 Gew.-% weitere Bestandteile.

Bei der Herstellung von Gelen auf Basis der erfindungsgemäßen Isoalkangemische A) oder auf Basis hoch $C_{16}$-haltiger Isoalkangemische können erforderlichenfalls zusätzlich übliche Gelbildner eingesetzt werden, beispielsweise, um spezielle rheologische oder andere anwendungstechnische Eigenschaften der Gele einzustellen. Als Gelbildner können alle in der Kosmetik üblichen Gelbildner eingesetzt werden.

Hierzu zählen leicht vernetzte Polyacrylsäure, beispielsweise Carbomer (INCI), Cellulosederivate, z. B. Hydroxypropylcellulose, Hydroxyethylcellulose, kationisch modifizierte Cellulosen, Polysaccharide, z. B. Xanthangummi, Capryl/Caprin-Triglycerid, Natriumacrylat-Copolymere, Polyquaternium-32 (und) Paraffinum Liquidum (INCI), Natriumacrylat-Copolymere (und) Paraffinum Liquidum (und) PPG-1 Trideceth-6, Acrylamidopropyltrimoniumchlorid/Acrylamid-Copolymere, Steareth-10 Allylether Acrylat-Copolymere, Polyquaternium-37 (und) Paraffinum Liquidum (und) PPG-1 Trideceth-6, Polyquaternium 37 (und) Propylenglycoldicapratdicaprylat (und) PPG-1 Trideceth-6, Polyquaternium-7, Polyquaternium-44. Als Gelbildner geeignete vernetzte Homopolymere der Acrylsäure sind beispielsweise kommerziell unter dem Namen Carbopol® von der Firma BF GOODRICH erhältlich. Bevorzugt sind auch hydrophob modifizierte vernetzte Polyacrylat Polymere, wie Carbopol® Ultrez 21 von der Firma Noveon. Weitere Beispiele für als Gelbildner geeignete anionische Polymere sind Copolymere von Acrylsäure und Acrylamid und deren Salze; Acrylatcopolymere, wie Luvigel® EM; Natriumsalze von Polyhydroxycarbonsäuren, wasserlösliche oder wasserdispergierbare Polyester, Polyurethane und Polyharnstoffe. Besonders geeignete Polymere sind Copolymere aus (Meth)acrylsäure und Polyetheracrylaten, wobei die Polyetherkette mit einem $C_8$-$C_{30}$-Alkylrest terminiert ist. Dazu zählen z. B. Acrylat/Beheneth-25-methacrylat-Copolymere, die unter der Bezeichnung Aculyn® von der Firma Rohm und Haas erhältlich sind.

Die erfindungsgemäßen Isoalkangemische A) können in kosmetischen Zubereitungen als Konditioniermittel eingesetzt werden.

Die erfindungsgemäßen Isoalkangemische A) können bevorzugt in Shampooformulierungen als Festigungs- und/oder Konditioniermittel eingesetzt werden. Bevorzugte Shampooformulierungen enthalten

a) 0,05 bis 10 Gew.-% wenigstens eines erfindungsgemäßen Isoalkangemischs oder hoch $C_{16}$-haltigen Isoalkangemischs,
b) 25 bis 94,95 Gew.-% Wasser,
c) 5 bis 50 Gew.-% Tenside,
c) 0 bis 5 Gew.-% eines weiteren Konditioniermittels,
d) 0 bis 10 Gew.-% weitere kosmetische Bestandteile.

In den Shampooformulierungen können alle in Shampoos üblicherweise eingesetzte anionische, neutrale, amphotere oder kationische Tenside verwendet werden.

Geeignete anionische Tenside sind beispielsweise Alkylsulfate, Alkylethersulfate,

Alkylsulfonate, Alkylarylsulfonate, Alkylsuccinate, Alkylsulfosuccinate, N-Alkoylsarkosinate, Acyltaurate, Acylisothionate, Alkylphosphate, Alkyletherphosphate, Alkylethercarboxylate, Alpha-Olefinsulfonate, insbesondere die Alkali- und Erdalkalimetallsalze, z. B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.

Geeignet sind zum Beispiel Natriumlaurylsulfat, Ammoniumlaurylsulfat, Natriumlaurylethersulfat, Ammoniumlaurylethersulfat, Natriumlaurylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind zum Beispiel Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol. Ferner sind Alkylaminoxide, Mono- oder Dialkylalkanolamide, Fettsäureester von Polyethylenglykolen, Alkylpolyglykoside oder Sorbitanetherester geeignet.

Außerdem können die Shampooformulierungen übliche kationische Tenside enthalten, wie z. B. quaternäre Ammoniumverbindungen, beispielsweise Cetyltrimethylammoniumchlorid.

In den Shampooformulierungen können zur Erzielung bestimmter Effekte übliche Konditioniermittel in Kombination mit den Isoalkangemischen A) eingesetzt werden. Hierzu zählen beispielsweise die zuvor genannten kationischen Polymere mit der Bezeichnung Polyquaternium nach INCI, insbesondere Copolymere aus Vinylpyrrolidon/ N-Vinylimidazoliumsalzen (Luviquat® FC, Luviquat® HM, Luviquat® MS, Luviquat® Care), Copolymere aus N-Vinylpyrrolidon/Dimethylaminoethylmethacrylat, quaternisiert mit Diethylsulfat (Luviquat® PQ 11), Copolymere aus N-Vinylcaprolactam/ N-Vinylpyrrolidon/N-Vinylimidazoliumsalzen (Luviquat® Hold); kationische Cellulosederivate (Polyquaternium-4 und -10), Acrylamidcopolymere (Polyquaternium-7). Ferner können Eiweißhydrolysate verwendet werden, sowie konditionierende Substanzen auf Basis von Silikonverbindungen, beispielsweise Polyalkylsiloxane, Polyarylsiloxane, Polyarylalkylsiloxane, Polyethersiloxane oder Silikonharze. Weitere geeignete Silikonverbindungen sind Dimethicon Copolyole (CTFA) und aminofunktionelle Silikonverbindungen wie Amodimethicone (CTFA). Ferner können kationische Guarderivate wie Guarhydroxypropyltrimoniumchlorid (INCI) verwendet werden.

Die eingesetzten Isoalkangemische und die hoch $C_{16}$-haltigen Isoalkangemische eignen sich besonders vorteilhaft zur Herstellung von und/oder gemeinsamen Verwendung mit Copolymerisaten Ca), die erhältlich sind durch Copolymerisation von

(A) mindestens einem ethylenisch ungesättigten Dicarbonsäureanhydrid, abgeleitet von mindestens einer Dicarbonsäure mit 4 bis 8 C-Atomen,

(B) mindestens einem Oligomer, das sich von wenigstens einem verzweigten oder unverzweigten $C_3$-$C_{10}$-Alken ableitet, wobei das Oligomer ein mittleres Molekulargewicht $M_n$ im Bereich von 300 bis 5000 g/mol, bevorzugt bis zu 1200 g/mol aufweist oder durch Oligomerisierung von mindestens 3 Äquivalenten $C_3$-$C_{10}$-Alken erhältlich ist,

(C) optional mindestens einem $\alpha$-Olefin mit bis zu 24, bevorzugt mit bis zu 16 C-Atomen,

(D) optional mindestens einem weiteren von (A), (B) und (C) verschiedenen ethylenisch ungesättigten Comonomer, gegebenenfalls Umsetzung mit

(E) mindestens einer Verbindung der allgemeinen Formel Ia, Ib, Ic oder Id

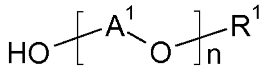

Ia

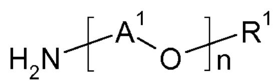

Ib

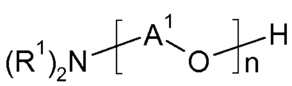

Ic

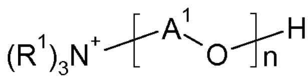

Id

wobei

A¹ $C_2$-$C_{20}$-Alkylen, gleich oder verschieden,
R¹ $C_1$-$C_{30}$-Alkyl, linear oder verzweigt, Phenyl oder Wasserstoff,
n eine ganze Zahl von 1 bis 200 und

gegebenenfalls anschließendes Kontaktieren mit Wasser.

Gegebenenfalls können die Copolymerisate Ca) im Gemisch mit wenigstens einem zusätzlichen Oligomer Z), wie zuvor als Komponente B) definiert, vorliegen.

Als Oligomere B) eignen sich die eingesetzten Isoalkangemische. Als Oligomere B) kommen auch davon

verschiedene Oligomere in Betracht, insbesondere Oligomere des Propylens oder unverzweigter oder vorzugsweise verzweigter $C_4$-$C_{10}$-Olefine, wobei mindestens ein Oligomer ein mittleres Molekulargewicht $M_n$ im Bereich von 300 bis 5000 g/mol, bevorzugt bis zu 1200 g/mol aufweist oder durch Oligomerisierung von mindestens 3 Äquivalenten $C_3$-$C_{10}$-Alken erhältlich ist. Als Komponente B) eignen sich auch Gemische der erfindungsgemäßen Isoalkangemische mit davon verschiedenen Oligomeren. Auch als zusätzliche Oligomerkomponente Z) eignen sich die Isoalkangemische. Als Oligomere Z) kommen des Weiteren auch davon verschiedene Oligomere in Betracht, insbesondere Oligomere des Propylens oder unverzweigter oder vorzugsweise verzweigter $C_4$-$C_{10}$-Olefine, wobei mindestens ein Oligomer ein mittleres Molekulargewicht $M_n$ im Bereich von 300 bis 5000 g/mol, bevorzugt bis zu 1200 g/mol aufweist oder durch Oligomerisierung von mindestens 3 Äquivalenten $C_3$-$C_{10}$-Alken erhältlich ist. Als Komponente Z) eignen sich auch Gemische der Isoalkangemische mit davon verschiedenen Oligomeren.

Erfindungsgemäß umfasst die Komponente B) und/oder das Oligomer Z) wenigstens ein Isoalkangemisch, wie zuvor definiert, oder besteht aus einem solchen Isoalkangemisch.

Formulierungen der Copolymerisate Ca) auf Basis der Isoalkangemische eignen sich insbesondere für kosmetische Mittel zur Behandlung keratinischer Oberflächen.

Bezüglich geeigneter Formulierungskomponenten dieser kosmetischen Mittel wird auf die vorherigen Ausführungen Bezug genommen. Geeignete Copolymerisate Ca) sind im Folgenden beschrieben.

Die Copolymerisate Ca) oder deren Gemische mit wenigstens einem weiteren Oligomer Z) sind in den erfindungsgemäßen Mitteln in einer Menge von 0,1 bis 15 Gew.-%, besonders bevorzugt 1 bis 10, insbesondere 2 bis 6 Gew.-%, bezogen auf das Gewicht des Mittels, enthalten. Liegt das Copolymerisat Ca) gemischt mit einer Oligomerkomponente Z) vor, so beträgt das Gewichtsverhältnis von Oligomerkomponente Z) zu Copolymerisat Ca) vorzugsweise 1:10 bis 3:1, besonders bevorzugt 1:5 bis 2:1 und ganz besonders bevorzugt 1:2 bis 1,5:1.

Das Copolymerisat Ca) ist erhältlich durch vorzugsweise radikalische Copolymerisation von

A) mindestens einem ethylenisch ungesättigten Dicarbonsäureanhydrid, abgeleitet von mindestens einer Dicarbonsäure mit 4 bis 8 C-Atomen, beispielsweise Maleinsäureanhydrid, Itaconsäureanhydrid, Citraconsäureanhydrid, Methylenmalonsäureanydrid, bevorzugt Itaconsäureanhydrid und Maleinsäureanhydrid und ganz besonders bevorzugt Maleinsäureanhydrid;

B) mindestens einem Oligomeren von verzweigtem oder unverzweigtem $C_3$-$C_{10}$-Alken, wobei mindestens ein Oligomer ein mittleres Molekulargewicht $M_n$ im Bereich von 300 bis 5000 g/mol, bevorzugt bis zu 1200 g/mol aufweist oder durch Oligomerisierung von mindestens 3 Äquivalenten $C_3$-$C_{10}$-Alken erhältlich ist,

C) optional mindestens einem $\alpha$-Olefin mit bis zu 24, bevorzugt mit bis zu 16 C-Atomen,

D) optional mindestens einem weiteren von A), B) und C) verschiedenen ethylenisch ungesättigten Comonomer, gegebenenfalls Umsetzung mit

E) mindestens einer Verbindung der allgemeinen Formel Ia, Ib, Ic oder Id

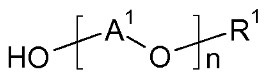

Ia

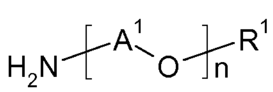

Ib

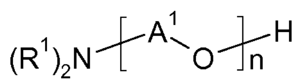

Ic

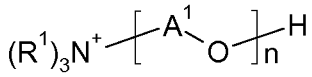

Id

wobei

- $A^1$ $C_2$-$C_{20}$-Alkylen, gleich oder verschieden,
- $R^1$ $C_1$-$C_{30}$-Alkyl, linear oder verzweigt, Phenyl oder Wasserstoff,
- n eine ganze Zahl von 1 bis 200,

wobei die Carboxylgruppen des Copolymerisats a) zumindest partiell verestert oder amidiert sein können, und gegebenenfalls anschließendem Kontaktieren mit Wasser.

Als Oligomere B) und/oder Z) eignen sich, wie zuvor ausgeführt, die erfindungsgemäßen Isoalkangemische und die hoch $C_{16}$-haltigen Isoalkangemische, davon verschiedene Oligomere sowie Gemische der erfindungsgemäßen Isoalkangemische mit davon verschiedenen Oligomeren.

Beispielhaft für von den Isoalkangemischen verschiedene Oligomere seien Oligomere von Propylen, Isobuten, 1-Penten, 2-Methylbuten-1, 1-Hexen, 2-Methylpenten-1, 2-Methylhexen-1, 2,4-Dimethyl-1-hexen, Diisobuten (Gemisch aus 2,4,4-Trimethyl-1-penten und 2,4,4-Trimethyl-2-penten), 2-Ethylpenten-1, 2-Ethylhexen-1 und 2-Propylhepten-1, 1-Okten, 1-Decen und 1-Dodecen genannt, ganz besonders bevorzugt sind Oligomere von Isobuten, Diisobuten und 1-Dodecen. Diese Oligomere B) bzw. Z) weisen eine ethylenisch ungesättigte Gruppe auf, die in Form einer Vinyl-, Vinyliden- oder Alkylvinylidengruppe vorliegen kann.

Auch Co-Oligomere der vorstehend genannten Olefine untereinander oder mit bis zu 20 Gew.-%, bezogen auf B) bzw. Z), Vinylaromaten wie Styrol und $\alpha$-Methylstyrol, $C_1$-$C_4$-Alkylstyrol wie beispielsweise 2-, 3- und 4-Methylstyrol sowie 4-tert.-Butylstyrol kommen in Frage.

Besonders bevorzugte Oligomere B) bzw. Z) sind Oligopropylene und Oligoisobutene mit einem mittleren Molekulargewicht $M_n$ bis zu 1200 g/mol, bevorzugt im Bereich von 300 bis 1000 g/mol, besonders bevorzugt von mindestens 400 g/mol, ganz besonders bevorzugt von mindestens 500 g/mol, beispielsweise bestimmt mittels Gelpermeationschromatographie (GPC).

In einer Ausführungsform der vorliegenden Erfindung weisen Oligomere B) bzw. Z) eine Polydispersität $M_w/M_n$ im Bereich von 1,1 bis 10, bevorzugt bis 5 und besonders bevorzugt von 1,5 bis 1,8 auf.

In einer Ausführungsform der vorliegenden Erfindung weisen Oligomere B) bzw. Z) eine bimodale Molekulargewichtsverteilung auf mit einem Maximum von $M_n$ im Bereich von 500 bis 1200 g/mol und einem lokalen Maximum von $M_n$ im Bereich von 2000 bis 5000 g/mol.

Oligomer B) kann gleich oder verschieden sein von gegebenenfalls zusätzlich eingesetzten Oligomeren Z). In einer Ausführungsform der vorliegenden Erfindung sind Oligomer B) und zusätzliches Oligomer Z) gleich.

Als zusätzliche Oligomere Z) sind bevorzugt Oligomere von $C_4$-Olefinen geeignet. In einer Ausführungsform der Erfindung sind die Oligomere Z) hydrierte Oligomere von $C_4$-Olefinen. Besonders bevorzugt als Oligomere Z) sind auch, gegebenenfalls hydrierte, Oligomere aus 3, 4, 5, 6, 7 oder 8 $C_4$-Olefin-Molekülen.

Comonomer C)

Als Comonomer C) eingesetzte $\alpha$-Olefine mit bis zu 16 C-Atomen sind gewählt aus Propylen, 1-Buten, Isobuten, 1-Penten, 4-Methylbut-1-en, 1-Hexen, Diisobuten (Gemisch aus 2,4,4-Trimethyl-1-penten und 2,4,4-Trimethyl-2-penten), 1-Hepten, 1-Octen, 1-Decen, 1-Dodecen, 1-Tetradecen und 1-Hexadecen; besonders bevorzugt sind Isobuten, Diisobuten und 1-Dodecen.

Man kann zur Herstellung von erfindungsgemäß verwendetem Copolymerisat Ca) die Komponenten A), B) und gegebenenfalls C) miteinander copolymerisieren. Man kann auch zur Herstellung von erfindungsgemäßem Copolymerisat Ca) die Komponenten A), B) und gegebenenfalls C) miteinander copolymerisieren und gegebenenfalls mit E) umsetzen oder A), B) und gegebenenfalls C) und gegebenenfalls ein weiteres Comonomer D) miteinander copolymerisieren, oder man kann A) und B) und gegebenenfalls C) und gegebenenfalls ein weiteres Comonomer D) miteinander copolymerisieren und gegebenenfalls mit E) umsetzen.

Wünscht man ein Copolymerisat Ca) einzusetzen, dessen Carboxylgruppen zumindest partiell verestert oder amidiert sind, so wählt man als Verbindung E) mindestens eine Verbindung der allgemeinen Formel Ia bis Id, vorzugsweise Ia,

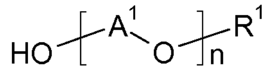

Ia

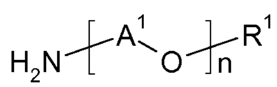

Ib

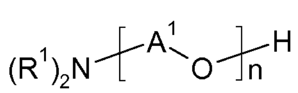

Ic

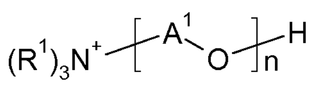

Id

wobei die Variablen wie folgt definiert sind:

$A^1$ $C_2$-$C_{20}$-Alkylen, beispielsweise $-(CH_2)_2-$, $-CH_2-CH(CH_3)-$, $-(CH_2)_3-$, $-CH_2-CH(C_2H_5)-$, $-(CH_2)_4-$, $-(CH_2)_5-$, $-(CH_2)_6-$, vorzugsweise $C_2$-$C_4$-Alkylen; insbesondere $-(CH_2)_2-$, $-CH_2-CH(CH_3)-$ und $-CH_2-CH(C_2H_5)-$;

$R^1$ Phenyl,
Wasserstoff
oder vorzugsweise $C_1$-$C_{30}$-Alkyl, linear oder verzweigt, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, n-Octyl, n-Nonyl, n-Decyl, n-Dodecyl, n-Hexadecyl, n-Octadecyl, n-Eicosyl; besonders bevorzugt $C_1$-$C_4$-Alkyl wie Methyl, Ethyl, n-Propyl, iso-Propyl, n-Butyl, iso-Butyl, sec.-Butyl und tert.-Butyl, ganz besonders bevorzugt Methyl.

n eine ganze Zahl im Bereich von 1 bis 200, bevorzugt 4 bis 20.

Die Gruppen $A^1$ können natürlich nur dann verschieden sein, wenn n eine Zahl größer 1 ist oder wenn man verschiedene Verbindungen der allgemeinen Formel I a bis I d einsetzt.

Besondere Beispiele für Verbindungen der allgemeinen Formel Ia sind

- Methylendgruppenverschlossene Polyethylenglykole der Formel $HO-(CH_2CH_2O)_m-CH_3$ mit m = 1 bis 200, vorzugsweise 4 bis 100, besonders bevorzugt 4 bis 50;

- Methylendgruppenverschlossene Blockcopolymere aus Ethylenoxid, Propylenoxid und/oder Butylenoxid mit einem Molekulargewicht $M_n$ von 300 bis 5000 g/mol;

- Methylendgruppenverschlossene statistische Copolymere aus Ethylenoxid, Propylenoxid und/oder Butylenoxid mit einem Molekulargewicht $M_n$ von 300 bis 5000 g/mol;

- Alkoxylierte $C_2$-$C_{30}$-Alkohole, insbesondere Fettalkoholalkoxylate, Oxoalkoholalkoxylate oder Guerbet-Alkoholalkoxylate, wobei die Alkoxylierung mit Ethylenoxid, Propylenoxid und/oder Butylenoxid durchgeführt werden kann, Beispiele sind;

- $C_{13}$-$C_{15}$-Oxoalkoholethoxylate mit 3 bis 30 Ethylenoxideinheiten;

- $C_{13}$-Oxoalkoholethoxylate mit 3 bis 30 Ethylenoxideinheiten;

- $C_{12}C_{14}$-Fettalkoholethoxylate mit 3 bis 30 Ethylenoxideinheiten;

- $C_{10}$-Oxoalkoholethoxylate mit 3 bis 30 Ethylenoxideinheiten;

- $C_{10}$-Guerbetalkoholethoxylate mit 3 bis 30 Ethylenoxideinheiten;

- $C_9$-$C_{11}$-Oxoalkoholalkoxylate mit 2 bis 20 Ethylenoxideinheiten, 2 bis 20 Propylenoxideinheiten und/oder 1 bis 5 Butylenoxideinheiten;

- $C_{13}$-$C_{15}$-Oxoalkoholalkoxylate mit 2 bis 20 Ethylenoxideinheiten, 2 bis 20 Propylenoxideinheiten und/oder 1 bis 5 Butylenoxideinheiten;

- $C_4$-$C_{20}$-Alkoholethoxylate mit 2 bis 20 Ethylenoxideinheiten.

Bevorzugte Beispiele für Verbindungen der Formel Ib sind Methylendgruppenverschlossene Polyethylenglykolamine der Formel $H_2N-(CH_2CH_2O)_m-CH_3$ mit m = 1 bis 200, vorzugsweise 4 bis 100, besonders bevorzugt 4 bis 50.

Wünscht man mit Verbindung Id umzusetzen, so kann man Verbindung Ic mit Alkylierungsagenzien wie beispielsweise Halogeniden oder Sulfaten der Formel $R^1$-Y mit Y gewählt aus Cl, Br und I oder $(R^1)_2SO_4$ umsetzen. Je nach Verwendung des oder der Alkylierungsagenzien erhält man Verbindung Id mit Y, $SO_4^{2-}$ oder $R^1$-$SO_4^-$ als Gegenion.

In einer Ausführungsform der vorliegenden Erfindung setzt man Mischungen von verschiedenen Komponenten E), beispielsweise der Formel Ia ein. Insbesondere kann man solche Mischungen an Verbindungen der Formel Ia einsetzen, in denen - bezogen jeweils auf die Mischung - mindestens 95 mol-%, bevorzugt mindestens 98 mol-% bis maximal 99,8 mol-% $R^1$ für $C_1$-$C_{30}$-Alkyl steht und mindestens 0,2 mol-% und maximal 5 mol-%, bevorzugt maximal 2 mol-% für Wasserstoff.

In einer Ausführungsform der vorliegenden Erfindung kontaktiert man zur Herstellung des erfindungsgemäß eingesetzten Copolymerisats Ca) die Reaktionsmischung nach der vorzugsweise radikalischen Copolymerisation und gegebenenfalls der Umsetzung mit E) mit Wasser, wobei das Wasser noch Brønsted-Säure oder bevorzugt Brønsted-Base enthalten kann. Beispiele für Brønsted-Säuren sind Schwefelsäure, Salzsäure, Weinsäure und Zitronensäure. Beispiele für Brønsted-Base sind Alkalimetallhydroxid wie beispielsweise NaOH und KOH, Alkalimetallcarbonat wie beispielsweise $Na_2CO_3$ und $K_2CO_3$, Alkalimetallhydrogencarbonat wie beispielsweise $NaHCO_3$ und $KHCO_3$, Ammoniak, Amine wie beispielsweise Trimethylamin, Triethylamin, Diethylamin, Ethanolamin, N,N-Diethanolamin, N,N,N-Triethanolamin, N-Methylethanolamin.

In einer anderen Ausführungsform der vorliegenden Erfindung kann man bereits während der vorzugsweise radikalischen Copolymerisation mit Wasser kontaktieren.

Monomere D)

Das oder die Monomere D), das bzw. die man optional zur Herstellung von erfindungsgemäß verwendeten Copolymerisat Ca) verwenden kann, sind von A), B) und C) verschieden. Als bevorzugte Monomere D) sind zu nennen:

$C_3$-$C_8$-Carbonsäuren bzw. Carbonsäurederivaten der allgemeinen Formel II

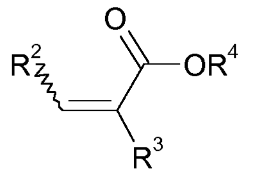

II

Carbonsäureamide der Formel III,

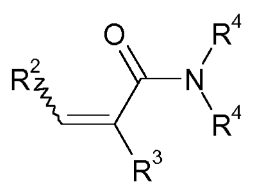

III

nicht-cyclische Amide der allgemeinen Formel IV a und cyclische Amide der allgemeinen Formel IVb

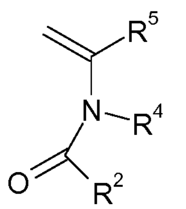

IV a

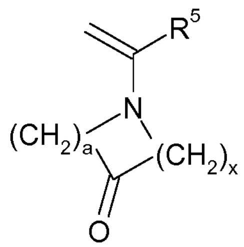

IV b

$C_1$-$C_{20}$-Alkylvinylether wie Methylvinylether, Ethylvinylether, n-Propylvinylether, iso-Propylvinylether, n-Butylvinylether, iso-Butylvinylether, 2-Ethylhexylvinylether oder n-Octadecylvinylether;

N-Vinylderivate von stickstoffhaltigen aromatischen Verbindungen, bevorzugt N-Vinylimidazol, 2-Methyl-1-vinylimidazol, N-Vinyloxazolidon, N-Vinyltriazol, 2-Vinylpyridin, 4-Vinylpyridin, 4-Vinylpyridin-N-oxid, N-Vinylimidazolin, N-Vinyl-2-methylimidazolin,

a,β-ungesättigte Nitrile wie beispielsweise Acrylnitril, Methacrylnitril;

alkoxylierte ungesättigte Ether der allgemeinen Formel V,

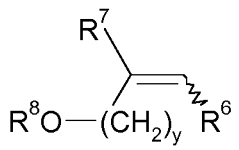

V

Ester und Amide der allgemeinen Formel VI,

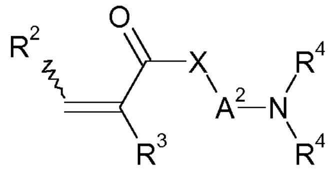

VI

ungesättigte Ester der allgemeinen Formel VII

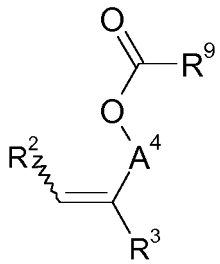

VII

vinylaromatische Verbindungen der allgemeinen Formel VIII

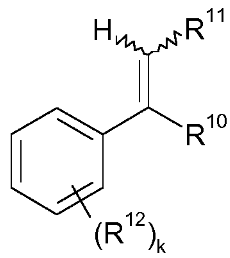

VIII

Phosphat-, phosphonat-, sulfat-, und sulfonathaltige Comonomere wie beispielsweise [2-{(Meth)acryloyloxy}-ethyl]-phosphat, 2-(Meth)acrylamido-2-methyl-1-propansulfonsäure;

$\alpha$-Olefine, linear oder verzweigt, mit 18 bis 40 Kohlenstoffatomen, bevorzugt mit bis 24 Kohlenstoffatomen, beispielsweise 1-Oktadecen, 1-Eicosen, $\alpha$-$C_{22}H_{44}$, $\alpha$-$C_{24}H_{48}$ und Gemische der vorstehend genannten $\alpha$-Olefine.

Dabei sind die Variablen wie folgt definiert:

$R^2$, $R^3$ gleich oder verschieden und gewählt aus unverzweigten oder verzweigten $C_1$-$C_5$-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, besonders bevorzugt $C_1$-$C_4$-Alkyl wie Methyl, Ethyl, n-Propyl, iso-Propyl, n-Butyl, iso-Butyl, sec.-Butyl und tert.-Butyl;

und insbesondere Wasserstoff;

$R^4$ gleich oder verschieden und $C_1$-$C_{22}$-Alkyl, verzweigt oder unverzweigt, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, n-Octyl, n-Nonyl, n-Decyl, n-Dodecyl, n-Eicosyl; besonders bevorzugt $C_1$-$C_4$-Alkyl wie Methyl, Ethyl, n-Propyl, iso-Propyl, n-Butyl, iso-Butyl, sec.-Butyl und tert.-Butyl;

oder besonders bevorzugt Wasserstoff;

$R^5$ Wasserstoff oder Methyl;

x eine ganze Zahl im Bereich von 2 bis 6, vorzugsweise 3 bis 5;

y eine ganze Zahl, ausgewählt aus 0 oder 1, vorzugsweise 1;

a eine ganze Zahl im Bereich von 0 bis 6, vorzugsweise im Bereich von 0 bis 2;

$R^6$, $R^7$ gleich oder verschieden und gewählt aus Wasserstoff, unverzweigten oder verzweigten $C_1$-$C_{10}$-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, n-Octyl, n-Nonyl, n-Decyl, bevorzugt $C_1$-$C_4$-Alkyl wie Methyl, Ethyl, n-Propyl, iso-Propyl, n-Butyl, iso-Butyl, sec.-Butyl und tert.-Butyl, ganz besonders bevorzugt Methyl;

X Sauerstoff oder N-$R^4$;

$R^8$ LA$^3$-O]$_n$-$R^4$;

$R^9$ gewählt aus unverzweigten oder verzweigten $C_1$-$C_{20}$-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, n-Octyl, n-Nonyl, n-Decyl, n-Dodecyl, n-Tetradecyl, n-Hexadecyl, n-Octadecyl, n-Eicosyl; bevorzugt $C_1$-$C_{14}$-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, n-Octyl, n-Nonyl, n-Decyl, n-Dodecyl, n-Tetradecyl,

und insbesondere Wasserstoff oder Methyl;

$R^{10}$, $R^{11}$ unabhängig voneinander jeweils Wasserstoff, Methyl oder Ethyl, bevorzugt sind $R^{10}$ und $R^{11}$ jeweils Wasserstoff;

$R^{12}$ Methyl oder Ethyl;

k eine ganze Zahl im Bereich von 0 bis 2 bedeutet, bevorzugt gilt k = 0;

$A^2$, $A^3$ gleich oder verschieden und $C_2$-$C_{20}$-Alkylen, beispielsweise $-(CH_2)_2-$, $-CH_2-CH(CH_3)-$, $-(CH_2)_3-$, $-CH_2-CH(C_2H_5)-$, $-(CH_2)_4-$, $-(CH_2)_5-$, $-(CH_2)_6-$, vorzugsweise $C_2$-$C_4$-Alkylen; insbesondere $-(CH_2)_2-$, $-CH_2-CH(CH_3)-$ und - $(CH_2)_3-$;

$A^4$ $C_1$-$C_{20}$-Alkylen, beispielsweise $-CH_2-$, $-CH(CH_3)$., $-CH(C_6H_5)-$, $-C(CH_3)_2-$, $-(CH_2)_2-$, $-CH_2-CH(CH_3)-$, $-(CH_2)_3-$, $-CH_2-CH(C_2H_5)-$, $-(CH_2)_4-$, $-(CH_2)_5-$, $-(CH_2)_6-$, vorzugsweise $C_2$-$C_4$-Alkylen; insbesondere $-(CH_2)_2-$, $-CH_2-CH(CH_3)-$ und $-(CH_2)_3-$, oder insbesondere eine Einfachbindung.

Die übrigen Variablen sind wie oben stehend definiert.

Beispielhaft ausgewählte Verbindungen der Formel III sind (Meth)Acrylamide wie Acrylamid, N-Methylacrylamid, N,N-Dimethylacrylamid, N-Ethylacrylamid, N-Propylacrylamid, N-tert.-Butylacrylamid, N-tert.-Octylacrylamid, N-Undecylacrylamid oder die entsprechenden Methacrylamide.

Beispielhaft ausgewählte Verbindungen der Formel IV a sind N-Vinylcarbonsäureamide wie N-Vinylformamid, N-Vinyl-N-methylfomamid, N-Vinylacetamid oder N-Vinyl-N-methylacetamid; Beispielhaft ausgewählte Vertreter für Verbindungen der Formel IV b sind N-Vinylpyrrolidon, N-Vinyl-4-piperidon und N-Vinyl-ε-caprolactam.

Beispielhaft ausgewählte Verbindungen der Formel VI sind (Meth)acrylsäureester und -amide wie N,N-Dialkylaminoalkyl(meth)acrylate oder N,N-Dialkylaminoalkyl(meth)acrylamide; Beispiele sind N,N-Dimethylaminoethylacrylat, N,N-Dimethylaminoethylmethacrylat, N,N-Diethylaminoethylacrylat, N,N-Diethylaminoethylmethacrylat, N,N-Dimethylaminopropylacrylat, N,N-Dimethylaminopropylmethacrylat, N,N-Diethylaminopropylacrylat, N,N-Diethylaminopropylmethacrylat, 2-(N,N-Dimethylamino)ethylacrylamid, 2-(N,N-Dimethylamino)ethylmethacrylamid, 2-(N,N-Diethylamino)ethylacrylamid, 2-N,N-Diethylamino)ethylmethacrylamid, 3-(N,N-Dimethylamino)propylacrylamid und 3-(N,N-Dimethylamino)propylmethacrylamid.

Beispielhaft ausgewählte Verbindungen der Formel VII sind Vinylacetat, Allylacetat, Vinylpropionat, Vinylbutyrat, Vinyl-2-ethylhexanoat oder Vinyllaurat.

Beispielhaft ausgewählte vinylaromatische Verbindungen der allgemeinen Formel VIII sind α-Methylstyrol, para-Methylstyrol und insbesondere Styrol.

Ganz besonders bevorzugt wird als Comonomer D) eingesetzt: Acrylsäure, 1-Octadecen, Methacrylsäure, Methylacrylat, Methylmethacrylat, Acrylamid, Vinyl-n-butylether, Vinyl-iso-butylether, Styrol, N-Vinylformamid, N-Vinylpyrrolidon, 1-Vinylimidazol und 4-Vinylpyridin.

Die Copolymerisate Ca) können betreffend A), B), gegebenenfalls C) und gegebenenfalls D) Blockcopolymerisate, alternierende Copolymerisate oder statistische Copolymerisate sein, wobei alternierende Copolymerisate bevorzugt sind.

In einer Ausführungsform der vorliegenden Erfindung liegen die Anhydridgruppen von Copolymerisat Ca) nach der Polymerisation vollständig oder partiell hydrolysiert und gegebenenfalls neutralisiert vor.

In einer Ausführungsform der vorliegenden Erfindung liegen die Anhydridgruppen von Copolymerisat Ca) nach der Copolymerisation als Anhydridgruppen vor.

In einer Ausführungsform der vorliegenden Erfindung betragen die Molverhältnisse von in erfindungsgemäß verwendetem Copolymerisat Ca)

A) im Bereich von 5 bis 60 mol-%, bevorzugt 10 bis 55 mol-%,

B) im Bereich von 1 bis 95 mol-%, bevorzugt 5 bis 70 mol-%,

C) im Bereich von 0 bis 60 mol-%, bevorzugt 10 bis 55 mol-%,

D) 0 bis 70 mol-%, bevorzugt 1 bis 50 mol-%, jeweils bezogen auf Copolymerisat, wobei die Summe aus A), B), C) und D) 100 mol-% ergibt, und

E) im Bereich von 0 bis 50 mol-%, bevorzugt 1 bis 30 mol-%, besonders bevorzugt 2 bis 20 mol-%, bezogen auf alle Carboxylgruppen des Copolymerisats.

In einer Ausführungsform wählt man ein Gewichtsverhältnis von zusätzlichen Oligomeren Z) zu Copolymerisat Ca) im Bereich von 0,1:1 bis 100:1, bevorzugt von 0,5:1 bis 10:1.

In einer anderen Ausführungsform wählt man ein Gewichtsverhältnis von zusätzlichen Oligomeren Z) zu Copolymerisat Ca) im Bereich von 1:1 bis 100:1, bevorzugt von 10:1 bis 50:1.

Die erfindungsgemäß verwendeten Copolymerisate Ca) sowie deren Mischungen Oligomeren Z) sind beschrieben in den deutschen Patentanmeldungen mit den Aktenzeichen DE 10353557.8, DE 10355402.5 und DE 10345094.7, auf die hier in vollem Umfang Bezug genommen wird.

In einer Ausführungsform der vorliegenden Erfindung haben die erfindungsgemäß verwendeten Copolymerisate Ca) von A), B) und gegebenenfalls C) und D) eine mittlere Molmasse $M_w$ im Bereich von 1000 g/mol bis 50000 g/mol, bevorzugt 1500 g/mol bis 25000 g/mol, bestimmt beispielsweise durch Gelpermeationschromatographie mit Dimethylacetamid als Lösemittel und Polymethylmethacrylat als Standard.

Erfindungsgemäß verwendete Copolymerisate Ca) von A), B) und gegebenenfalls C) und D) und E) können betreffend A), B) und gegebenenfalls C) und D) Blockcopolymerisate, alternierende Copolymerisate oder statistische Copolymerisate sein, wobei alternierende Copolymerisate bevorzugt sind.

Die Polydispersität $M_w/M_n$ der Copolmyerisate Ca) liegt im Allgemeinen im Bereich von 1,1 bis 20, bevorzugt von 2 bis 10.

Vorzugsweise haben die Copolymerisate Ca) K-Werte nach Fikentscher im Bereich von 5 bis 100, vorzugsweise 8 bis 30 (gemessen nach H. Fikentscher bei 25 °C in Cyclohexanon und einer Polymerkonzentration von 2 Gew.-%).

Zur Herstellung von geeigneten Copolymerisaten Ca) geht man aus von A), B) und gegebenenfalls C) und D), die man vorzugsweise radikalisch miteinander copolymerisiert und gegebenenfalls mit E) umsetzt. Die Umsetzung mit E) kann vor, während und nach der Copolymerisation erfolgen. Während oder vorzugsweise nach der Copolymerisation kann man mit Wasser kontaktieren. Man kann aber zur Herstellung der Copolymerisate Ca) auch auf das Kontaktieren mit Wasser verzichten.

Wünscht man eine Umsetzung von Copolymerisat aus A), B) und gegebenenfalls C) und D) mit E) oder eine radikalische Copolymerisation in Gegenwart von E), dann berechnet man gesamte Menge an E) vorzugsweise so, dass man von einer vollständigen Umsetzung von E) ausgeht und bis 50 mol-%, bevorzugt 1 bis 30 mol-%, besonders bevorzugt 2 bis 20 mol-% E), bezogen auf alle Carboxylgruppen des Copolymerisats, einsetzt. Unter dem Begriff "alle im Polymerisat enthaltene Carboxylgruppen" sind im Rahmen der vorliegenden Erfindung diejenigen Carboxylgruppen aus einpolymerisierten Comonomeren A) und gegebenenfalls D) zu verstehen, die als Anhydrid, als $C_1$-$C_4$-Alkylester

oder als Carbonsäure vorliegen.

Man startet die radikalische Copolymerisation vorteilhaft durch die üblichen, dafür bekannten Initiatoren, beispielsweise Peroxide oder Hydroperoxide.

Man kann die Copolymerisation in Anwesenheit oder in Abwesenheit von Lösungsmitteln und Fällungsmitteln durchführen. Als Lösemittel für die radikalische Copolymerisation kommen polare, gegenüber Säureanhydrid inerte Lösemittel in Betracht wie z. B. Aceton, Tetrahydrofuran und Dioxan. Als Fällungsmittel eignen sich beispielsweise Toluol, ortho-Xylol, meta-Xylol und aliphatische Kohlenwasserstoffe.

In einer bevorzugten Ausführungsform arbeitet man ohne Lösungsmittel oder in Gegenwart von nur geringen Mengen an Lösungsmittel, d. h. 0,1 bis maximal 10 Gew.-%, bezogen auf sie Gesamtmasse an Comonomeren A), B) und gegebenenfalls C) und D). Als Lösungsmittel sind unter den Bedingungen der Copolymerisation und der Veresterung beziehungsweise Amidbildung inerte Stoffe zu verstehen, insbesondere aliphatische und aromatische Kohlenwasserstoffe wie beispielsweise Cyclohexan, n-Heptan, Isododekan, Benzol, Toluol, Ethylbenzol, Xylol als Isomerengemisch, meta-Xylol, ortho-Xylol. Arbeitet man bei der Umsetzung mit E) ohne sauren Katalysator oder verzichtet man auf die Umsetzung mit E), so kann man die radikalische Copolymerisation und gegebenenfalls Umsetzung mit E) auch in Lösungsmitteln, gewählt aus Ketonen wie beispielsweise Aceton, Methylethylketon, oder cyclischen oder nicht-cyclischen Ethern wie beispielsweise Tetrahydrofuran oder Di-n-Butylether durchführen.

Die Copolymerisation und gegebenenfalls die Umsetzung mit E) übt man vorzugsweise unter Ausschluss von Sauerstoff aus, beispielsweise in einer Stickstoff- oder Argonatmosphäre, vorzugsweise in einem Stickstoffstrom.

Vorzugsweise liegt die Temperatur für die Copolymerisation von A), B) und gegebenenfalls C) und D) im Bereich von 80 bis 300 °C, bevorzugt 90 bis 200 °C.

Der Druck liegt beispielsweise im Bereich von 1 bis 15 bar, bevorzugt 1 bis 10 bar.

Man kann Regler einsetzen, beispielsweise $C_1$-$C_4$-Aldehyde, Ameisensäure und organische SH-Gruppen enthaltende Verbindungen, wie 2-Mercaptoethanol, 2-Mercaptopropanol, Mercaptoessigsäure, tert.-Butylmercaptan, n-Dodecylmercaptan. Polymerisationsregler werden im Allgemeinen in Mengen von 0,1 bis 10 Gew.-%, bezogen auf die Gesamtmasse der eingesetzten Comonomeren eingesetzt. Bevorzugt arbeitet man ohne Einsatz von Reglern.

Man kann während der Copolymerisation einen oder mehrere Polymerisationsinhibitoren in geringen Mengen zugeben, beispielsweise Hydrochinonmonomethylether. Polymerisationsinhibitor kann man vorteilhaft mit B) und gegebenenfalls C) und D) dosieren. Geeignete Mengen an Polymerisationsinhibitor sind 0,01 bis 1 Gew.-%, vorzugsweise 0,05 bis 0,5 Gew.-%, berechnet auf die Masse aller Comonomeren. Die Zugabe von Polymerisationsinhibitor ist insbesondere dann bevorzugt, wenn man die Copolymerisation bei Temperaturen über 80 °C durchführt.

Nach Beendigung der Zugabe von A), B) und gegebenenfalls C) und D), gegebenenfalls E) sowie gegebenenfalls Initiator kann man nachreagieren lassen.

Man kann die Umsetzung mit E) in Abwesenheit oder auch Anwesenheit von Katalysatoren durchführen, insbesondere sauren Katalysatoren wie z. B. Schwefelsäure, Methansulfonsäure, p-Toluolsulfonsäure, n-Dodecylbenzolsulfonsäure, Salzsäure oder sauren Ionenaustauschern.

In einer weiteren Variante des beschriebenen Verfahrens führt man die Umsetzung mit E) in Anwesenheit eines Schleppmittels durch, das mit bei der Reaktion gegebenenfalls entstehendem Wasser ein Azeotrop bildet.

Im Allgemeinen regiert unter den Bedingungen der oben beschriebenen Schritte E) vollständig oder zu einem gewissen Prozentsatz mit den Carboxylgruppen der Anhydride A) und gegebenenfalls den Carboxylgruppen aus D). Im Allgemeinen bleiben weniger als 40 mol-% als nicht umgesetztes E) zurück.

Es ist möglich, durch an sich bekannte Methoden wie beispielsweise Extraktion nicht umgesetztes E) von nach dem erfindungsgemäßen Herstellverfahren erhältlichen Copolymerisat abzutrennen.

In einer Ausführungsform kann man auf den weiteren Schritt der Abtrennung von nicht abreagiertem E) von den hergestellten Copolymerisaten verzichten. In dieser Ausführungsform setzt man Copolymerisate zusammen mit einem gewissen Prozentsatz an nicht abreagiertem E) zur Behandlung von faserigen Substraten ein.

Durch die oben beschriebene Copolymerisation von A), B) und gegebenenfalls C) und D) erhält man Copolymerisate. Die anfallenden Copolymerisate kann man einer Reinigung nach konventionellen Methoden unterziehen, beispielsweise Umfällen oder extraktiver Entfernung nicht-umgesetzter Monomere. Wenn ein Lösemittel oder Fällungsmittel eingesetzt wurde, so ist es möglich, dieses nach beendeter Copolymerisation zu entfernen, beispielsweise durch Abdestillieren.

Im Rahmen der vorliegenden Erfindung kann man wie oben beschrieben hergestelltes Copolymerisat mit Wasser kontaktieren, und zwar berechnet man die Menge an zugesetztem Wasser so, dass man erfindungsgemäße Dispersion erhält, die einen Wassergehalt im Bereich von 30 bis 99,5 Gew.-%, bezogen auf die Gesamtmasse an Hilfsmittel aufweisen.

In einer Ausführungsform versetzt man nach der radikalischen Copolymerisation und gegebenenfalls der Umsetzung mit E) mit Wasser, wobei das Wasser noch Brønsted-Säure oder bevorzugt Brønsted-Base enthalten kann. Beispiele für Brønsted-Säuren sind Schwefelsäure, Salzsäure, Weinsäure und Zitronensäure. Beispiele für Brønsted-Base sind Alkalimetallhydroxid wie beispielsweise NaOH und KOH, Alkalimetallcarbonat wie beispielsweise $Na_2CO_3$

und $K_2CO_3$, Alkalimetallhydrogencarbonat wie beispielsweise $NaHCO_3$ und $KHCO_3$, Ammoniak, Amine wie beispielsweise Trimethylamin, Triethylamin, Diethylamin, Ethanolamin, N,N-Diethanolamin, N,N,N-Triethanolamin, N-Methylethanolamin. Die Konzentration an Brønsted-Säure oder bevorzugt Brønsted-Base beträgt im Allgemeinen 1 bis 20 Gew.-%, bezogen auf die Summe aus Wasser und Brønsted-Säure bzw. Wasser und Brønsted-Base.

Man kann bereits während der radikalischen Copolymerisation Wasser zusetzen, vorzugsweise setzt man jedoch erst gegen Ende der radikalischen Copolymerisation Wasser zu. Hat man die radikalische Copolymerisation und die Umsetzung mit E) in Gegenwart von Lösungsmitteln durchgeführt, so ist es bevorzugt, zunächst Lösungsmittel zu entfernen, beispielsweise durch Abdestillieren und erst danach mit Wasser zu kontaktieren.

Durch das Kontaktieren mit Wasser, das gegebenenfalls Brønsted-Säure oder bevorzugt Brønsted-Base enthalten kann, können die im Copolymerisat Ca) vorhandenen Carbonsäureanhydridgruppen partiell oder vollständig hydrolysiert werden. Nach dem Kontaktieren mit Wasser, das gegebenenfalls Brønsted-Säure oder bevorzugt Brønsted-Base enthalten kann, kann man bei Temperaturen im Bereich von 20 bis 120 °C, bevorzugt bis 100 °C nachreagieren lassen, und zwar für einen Zeitraum von 10 Minuten bis 48 Stunden.

Die zu verwendenden Isoalkangemische A) eignen sich ebenso in pharmazeutischen Zubereitungen jeder Art, z. B. als Ölkörper, zur Modifizierung der rheologischen Eigenschaften sowie als Solubilisator.

Ein weiterer Gegenstand der Erfindung ist daher ein pharmazeutisches Mittel, enthaltend

A) wenigstens ein Isoalkangemisch, wie zuvor definiert,

B) wenigstens einen pharmazeutisch akzeptablen Wirkstoff und

C) gegebenenfalls wenigstens einen weiteren, von B) verschiedenen pharmazeutisch akzeptablen Wirk- oder Hilfsstoff.

In einer weiteren geeigneten Ausführungsform enthalten die pharmazeutischen Mittel als Komponente A) wenigstens ein hoch $C_{16}$-haltiges Isoalkangemisch, wie zuvor definiert.

Die Formulierungsgrundlage der erfindungsgemäßen pharmazeutischen Mittel enthält bevorzugt pharmazeutisch akzeptable Hilfsstoffe. Pharmazeutisch akzeptabel sind die im Bereich der Pharmazie, der Lebensmitteltechnologie und angrenzenden Gebieten bekanntermaßen verwendbaren Hilfsstoffe, insbesondere die in einschlägigen Arzneibüchern (z. B. DAB Ph. Eur. BP NF) gelisteten sowie andere Hilfsstoffe, deren Eigenschaften einer physiologischen Anwendung nicht entgegenstehen.

Geeignete Hilfsstoffe können sein: Gleitmittel, Netzmittel, emulgierende und suspendierende Mittel, konservierende Mittel, Antioxidantien, Antireizstoffe, Chelatbildner, Emulsionsstabilisatoren, Filmbildner, Gelbildner, Geruchsmaskierungsmittel, Harze, Hydrokolloide, Lösemittel, Lösungsvermittler, Neutralisierungsmittel, Permeationsbeschleuniger, Pigmente, quaternäre Ammoniumverbindungen, Rückfettungs- und Überfettungsmittel, Salben-, Creme- oder Öl-Grundstoffe, Siliconderivate, Stabilisatoren, Sterilantien, Treibmittel, Trocknungsmittel, Trübungsmittel, Verdickungsmittel, Wachse, Weichmacher, Weißöle. Eine diesbezügliche Ausgestaltung beruht auf fachmännischem Wissen, wie sie beispielsweise in Fiedler, H. P. Lexikon der Hilfsstoffe für Pharmazie, Kosmetik und angrenzende Gebiete, 4. Aufl., Aulendorf: ECV-Editio-Kantor-Verlag, 1996, dargestellt sind.

Zur Herstellung von erfindungsgemäßen pharmazeutischen Mitteln können die Wirkstoffe mit einem geeigneten Hilfsstoff (Exzipient) vermischt oder verdünnt werden. Exzipienten können feste, halb feste oder flüssige Materialien sein, die als Vehikel, Träger oder Medium für den Wirkstoff dienen können. Die Zumischung weiterer Hilfsstoffe erfolgt gewünschtenfalls in der dem Fachmann bekannten Weise. Insbesondere handelt es sich dabei um wässrige Lösungen bzw. Solubilisate zur oralen oder zur parenteralen Applikation. Des Weiteren eignen sich die erfindungsgemäß zu verwendenden Copolymere auch zum Einsatz in oralen Darreichungsformen wie Tabletten, Kapseln, Pulvern, Lösungen. Hier können Sie den Arzneistoff mit einer erhöhten Bioverfügbarkeit zur Verfügung stellen. Bei der parenteralen Applikation können neben Solubilisaten auch Emulsionen, beispielsweise Fettemulsionen eingesetzt werden. Auch für diesen Zweck eignen sich die erfindungsgemäßen Isoalkangemische A) um einen z. B. in wässrigen Medien schwerlöslichen Arzneistoff zu verarbeiten.

Pharmazeutische Formulierungen der oben genannten Art können durch Verarbeiten der erfindungsgemäß zu verwendenden Copolymere A) mit pharmazeutischen Wirkstoffen nach herkömmlichen Methoden und unter Einsatz bekannter und neuer Wirkstoffe erhalten werden.

Das erfindungsgemäße Mittel kann zusätzlich pharmazeutische Hilfsstoffe und/oder Verdünnungsmittel enthalten. Als Hilfsstoffe werden Cosolventien, Stabilisatoren, Konservierungsmittel besonders aufgeführt.

Die verwendeten pharmazeutischen Wirkstoffe sind in Wasser lösliche oder unlösliche bzw. wenig lösliche Substanzen. Gemäß DAB 9 (Deutsches Arzneimittelbuch) erfolgt die Einstufung der Löslichkeit pharmazeutischer Wirkstoffe wie folgt: wenig löslich (löslich in 30 bis 100 Teilen Lösungsmittel); schwer löslich (löslich in 100 bis 1000 Teilen Lösungsmittel); praktisch unlöslich (löslich in mehr als 10000 Teilen Lösungsmittel). Die Wirkstoffe können dabei aus

jedem Indikationsbereich kommen.

Besonders bevorzugt sind von den oben genannten pharmazeutischen Mitteln solche, bei denen es sich um parenteral applizierbare Formulierungen handelt.

Der Gehalt an Isoalkangemisch A) in den pharmazeutischen Mitteln liegt, abhängig vom Wirkstoff, im Bereich von 0,01 bis 50 Gew.-%, bevorzugt 0,1 bis 40 Gew.-%, besonders bevorzugt 1 bis 30 Gew.-%, bezogen auf das Gesamtgewicht des Mittels. Zur Herstellung der erfindungsgemäßen pharmazeutischen Mittel eignen sich prinzipiell alle pharmazeutischen Wirkstoffe und Pro-Drugs. Hierzu zählen Benzodiazepine, Antihypertensiva, Vitamine, Cytostatika - insbesondere Taxol, Anästhetika, Neuroleptika, Antidepressiva, Antibiotika, Antimykotika, Fungizide, Chemotherapeutika, Urologika, Thrombozytenaggregationshemmer, Sulfonamide, Spasmolytika, Hormone, Immunglobuline, Sera, Schilddrüsentherapeutika, Psychopharmaka, Parkinsonmittel und andere Antihyperkinetika, Ophthalmika, Neuropathiepräparate, Calciumstoffwechselregulatoren, Muskelrelaxantia, Narkosemittel, Lipidsenker, Lebertherapeutika, Koronarmittel, Kardiaka, Immuntherapeutika, regulatorische Peptide und ihre Hemmstoffe, Hypnotika, Sedativa, Gynäkologika, Gichtmittel, Fibrinolytika, Enzympräparate und Transportproteine, Enzyminhibitoren, Emetika, Durchblutungsfördernde Mittel, Diuretika, Diagnostika, Corticoide, Cholinergika, Gallenwegstherapeutika, Antiasthmatika, Broncholytika, Betarezeptorenblocker, Calciumantagonisten, ACE-Hemmer, Arteriosklerosemittel, Antiphlogistika, Antikoagulantia, Antihypotonika, Antihypoglykämika, Antihypertonika, Antifibrinolytika, Antiepileptika, Antiemetika, Antidota, Antidiabetika, Antiarrhythmika, Antianämika, Antiallergika, Anthelmintika, Analgetika, Analeptika, Aldosteronantagonisten, Abmagerungsmittel. Beispiele für geeignete pharmazeutische Wirkstoffe sind die insbesondere die in den Absätzen 0105 bis 0131 der US 2003/0157170 genannten Wirkstoffe.

Die nach dem oben beschriebenen Verfahren erhältlichen Isoalkangemische sind frei von Eigengerüchen und weisen ein äußerst weites Flüssigintervall auf (zumindest von -70 bis +200 °C). Sie eignen sich daher in besonders vorteilhafter Weise als Wärmeträgerflüssigkeit, z. B. für Solarthermie-Anlagen, Erdwärmeanlagen, technisch eingesetzte Wärmetauscher etc. Sie eignen sich weiterhin als Lösungsmittel für Kraftstoffadditive und Tieftemperaturkraftstoff (z. B. als oder in jet fuel(s)).

Das Isoalkangemisch eignet sich auch als Stoßdämpferöl. Dabei kann es rein oder im Gemisch mit wenigstens einem weiteren Öl, z. B. einem Mineralöl und/oder synthetischen Öl (z. B. Polyalphaolefin), eingesetzt werden. Ebenso können weitere Komponenten wie Viskositäts-Verbesserer, Pour-Point Depressants oder Antioxidantien zugesetzt werden.

Die Erfindung wird anhand der folgenden, nicht einschränkenden Beispiele näher erläutert.

## Beispiele

### I. Herstellungsbeispiele:

### Beispiel 1: Herstellung eines Isoalkengemischs

Man stellte ein Oligomerisat gemäß Beispiel 3 aus WO 95/14647 ausgehend von dem darin beschriebenen $C_4$-Kohlenwasserstoffgemisch unter Verwendung des in Beispiel 1 aus WO 95/14647 beschriebenen Katalysators her. 2 kg dieses Oligomerisats unterzog man einer Fraktionierung an einer 80 cm Füllkörperkolonne (Maschendrahtwendel) mit einem Rücklaufverhältnis 1:5. Man erhielt:

| Fraktion | Siedeintervall [°C] | Druck [mbar] | Menge [g] | Bezeichnung |
|---|---|---|---|---|
| 1a | 100-130 | 950 | 1450 | Buten-Dimer |
| 1b | 100-120 | 100 | 350 | Buten-Trimer |
| 1c | Sumpf | -- | 180 | Buten-Tetramer |

Gemäß der GC-Analyse bestand Fraktion 1 c aus 7 % $C_{12}$-Oligomerisierungsprodukt, 70 % $C_{16}$-Oligomerisierungsprodukt, 17 % $C_{20}$-Oligomerisierungsprodukt sowie höheren Homologen.

### Beispiel 2: Hydrierung von Fraktion 1c

In einen 9-l-Rührdruckbehälter gab man 6 l eines Buten-Tetrameren 1c aus Beispiel 1 und 50 g Pd/Aktivkohle (10 % Pd). Es wurde zunächst bis maximal 50 bar (5 MPa) Wasserstoff aufgepresst, so dass die Temperatur nicht über 50 °C stieg. Danach wurde bei 50 °C der Wasserstoffdruck auf 200 bar (20 MPa) erhöht und 2 Stunden nachhydriert. Nach beendeter Hydrierung wurde der Katalysator zunächst über einen Faltenfilter und dann über eine kurze Säule mit

$Al_2O_3$ abgetrennt. Man erhielt 4,4 kg einer klaren, hellen, geruchlosen Flüssigkeit.
$^1$H-NMR (16 Scans, 400 MHz, 10 % in $CDCl_3$): keine Signale im Bereich δ 7,0 - 2,5 ppm nachweisbar; 48 % des Integrals im Bereich δ 0,6 - 1 ppm.

Beispiel 3: Hydrierung von Fraktion 1a

In einen 3,5-I-Rührdruckbehälter gab man 2 I eines Buten-Dimeren 1a aus Beispiel 1 und 20 g Pd/Aktivkohle (10 % Pd). Es wurde zunächst bis maximal 20 bar (2 MPa) Wasserstoff aufgepresst, so dass die Temperatur nicht über 50 °C stieg. Danach wurde bei 50 °C der Wasserstoffdruck auf 200 bar (20 MPa) erhöht und 2 Stunden nachhydriert. Nach beendeter Hydrierung wurde der Katalysator zunächst über einen Faltenfilter und dann über eine kurze Säule mit $Al_2O_3$ abgetrennt. Man erhielt 1,2 kg einer klaren, hellen, geruchlosen Flüssigkeit.
$^1$H-NMR (16 Scans, 400 MHz, 10 % in $CDCl_3$): keine Signale im Bereich δ 7,0 - 2,5 ppm nachweisbar; 53 % des Integrals im Bereich δ 0,6 - 1 ppm.

Beispiel 4: Hydrierung von Fraktion 1b

In einen 3,5-I-Rührdruckbehälter gab man 2 I eines Buten-Trimeren 1 b aus Beispiel 1 und 20 g Pd/Aktivkohle (10 % Pd). Es wurde zunächst bis maximal 20 bar (2 MPa) Wasserstoff aufgepresst, so dass die Temperatur nicht über 50 °C stieg. Danach wurde bei 50 °C der Wasserstoffdruck auf 200 bar (20 MPa) erhöht und 2 Stunden nachhydriert. Nach beendeter Hydrierung wurde der Katalysator zunächst über einen Faltenfilter und dann über eine kurze Säule mit $Al_2O_3$ abgetrennt. Man erhielt 1,2 kg einer klaren, hellen, geruchlosen Flüssigkeit. 1,2 kg einer klaren, hellen, geruchlosen Flüssigkeit.
$^1$H-NMR (16 Scans, 400 MHz, 10 % in $CDCl_3$): keine Signale im Bereich δ 7,0 - 2,5 ppm nachweisbar; 51 % des Integrals im Bereich δ 0,6 - 1 ppm.

Beispiel 5 (Vergleichsbeispiel, in Anlehnung an Beispiel 5 aus WO 2004/091555)

In einem 3-I-Rührdruckbehälter legte man 800 g i-Buten und 200 g 1-Buten vor sowie 100 g Lewatit® SPC 112. (Im Gegensatz zur WO 2004/091555 überführte man das Ionenaustauscherharz zuvor mit 10 % $H_2SO_4$ in die aktive $H^+$-Form, wusch mit $H_2O$ gründlich und trocknete. Ohne diese Katalysatoraktivierung erzielte man keinen Umsatz.). Man rührte 3 Stunden bei 100 °C, wobei ein Maximaldruck von 20 bar (2 MPa) erreicht wurde. Der Reaktorinhalt wurde ausgetragen, vom Katalysator getrennt und am Rotationsverdampfer bei 40 °C / 100 mbar (0,02 MPa) entgast. Man erhielt 815 g einer wasserhellen, dünnflüssigen, Flüssigkeit mit olefinischem Geruch.
$^1$H-NMR (16 Scans, 400 MHz, 10 % in $CDCl_3$): 7,7 % des Integrals im Olefinbereich δ 5,5 - 4,5 ppm
In einen 3-I-Rührdruckbehälter gab man 770 g des obigen Olefingemischs und 5 g Pd/Aktivkohle (10 % Pd). Es wurde zunächst bis maximal 20 bar (2 MPa) Wasserstoff aufgepresst, so dass die Temperatur nicht über 50 °C steigt. Danach wird bei 100 °C der Wasserstoffdruck auf 200 bar (20 MPa) erhöht und insgesamt 12 Stunden hydriert. Nach beendeter Hydrierung wurde der Katalysator zunächst über einen Faltenfilter und dann über eine kurze Säule mit $Al_2O_3$ abgetrennt. Man erhielt 670 g einer klaren, hellen, Flüssigkeit mit leichtem Terpengeruch.
$^1$H-NMR (16 Scans, 400 MHz, 10 % in $CDCl_3$): keine Signale im Bereich δ 7,0 - 2,5 ppm nachweisbar; 83 % des Integrals im Bereich δ 0,6 - 1 ppm.
In Tabelle 1 sind der Verzweigungsgrad V (= Zahl der Verzweigungen / C-Atom) sowie das Integral im Bereich δ 0,6 -1 ppm der Isoalkangemische aus den Beispielen 2, 3, 4 und 5 angegeben. Squalan dient zur Kontrolle.

Tabelle 1

| Beispiel | Isoalkangemisch | Integral 0,6 - 1 ppm | Verzweigungen/C |
|---|---|---|---|
| 2 | Hydriertes Buten-Tetramer | 48% | 0,21 |
| 3 | Hydriertes Buten-Dimer | 53% | 0,15 |
| 4 | Hydriertes Buten-Trimer | 51 % | 0,2 |
| 5 (Vergleich) | Beispiel 5 aus WO 2004/091555 | 83% | 0,38 |
| Kontrolle Squalan | | 40% | 0,2 |

II. Spreitverhalten

Die im Folgenden angegebenen Spreitwerte wurden bestimmt nach dem folgenden Verfahren: Ein Stück Filterpapier (Filterpapier Typ 1243/90, weiß, Bogen 500 x 500 mm, Hersteller: Pörringer, ca. 200 x 200 mm) wird auf ein Uhrglas bzw. eine Petrischale frei schwebend gelegt und mit 10 µl des zu vermessenen Lipids mittig versetzt. Nach 10 Minuten wird die lipidbenetzte Fläche markiert, ausgeschnitten und ausgewogen. Das gleiche Verfahren erfolgt mit dem internen Standard (Paraffinum perliquidum). Aus den ermittelten Werten wird der relative Spreitwert nach folgender Formel berechnet:

relativer Spreitwert = (Fläche Messsubstanz x 100) / Fläche Paraffinum perliquidum

Tabelle 2

| Öl (Tradenames) | Relativer Spreitwert [%] | INCI/chemische Bezeichnung/ Hersteller |
|---|---|---|
| Abil 350 | 55 | Dimethicone |
| Panalane L14E | 82 | Hydrogenated Polyisobuten |
| Polysynlan | 97 | Hydrogenated Polyisobuten |
| DC 245 fluid | 109 | Cyclopentasiloxane |
| Finsolv TN | 109 | $C_{12-15}$ Alkylbenzoate |
| Fitoderm | 111 | Squalane |
| IPP | 111 | Isopropylpalmitate |
| IPM | 113 | Isopropylmyristate |
| Cetiol CC | 113 | Dicaprylyl Carbonate |
| Creasil ISO 20 | 122 | Hydrogenated Polyisobuten |
| Sophim MC30 | 127 | Hydrogenated Polyisobuten |
| Permethyl 101A | 134 | Isohexadecane |
| Isohexadecane | 152 | Isohexadecane |
| erfindungsgemäßes Isoalkangemisch aus Beispiel 2 | 156 | |

III. Anwendungsbeispiele

Anwendungsbeispiele 1-3: Hautcreme

Anwendungsbeispiel 1:

Gemäß folgender Rezeptur wurde eine Wasser/Öl-Cremeemulsion (Hautcreme A) hergestellt:

| CTFA-Name | | Gew.-% |
|---|---|---|
| Cremophor® A6 (BASF AG) | Ceteareth-6 (Stearylalkohol-Ethoxilat) und Stearylalkohol | 2,0 |
| Cremophor® A25 (BASF AG) | Ceteareth-25 (Fettalkohol-Ethoxilat) | 2,0 |
| Lanette® O | Cetearylalkohol | 2,0 |
| Imwitor® 960K | Glycerylstearat SE | 3,0 |
| Isoalkangemisch aus Beispiel 2 | | 4,0 |

(fortgesetzt)

| CTFA-Name | | Gew.-% |
|---|---|---|
| Luvitol® EHO (BASF AG) | Cetearyloctanoat (2-Ethylhexansäurecetylstearylester) | 3,0 |
| ABIL® 350 (Fa. Goldschmidt) | Dimethicon (Polydimethylsiloxan) | 1,0 |
| Amerchol® L101 (Amerchol Edison) | Mineralöl und Lanolinalkohol | 3,0 |
| Veegum Ultra | Magnesiumaluminiumsilikat | 0,5 |
| 1,2-Propylenglykol | Propylenglykol | 5,0 |
| Abiol | Imidazolindinyl-Harnstoff | 0,3 |
| Phenoxyethanol | | 0,5 |
| D-Panthenol USP | | 1,0 |
| Polymer Vinylpyrrolidon/Stearylmethacrylat 70/30 Gew.-% (K-Wert 50; 1 % in Isopropanol) | | 0,5 |
| Wasser | | ad 100 |

Anwendungsbeispiel 2 und Anwendungsbeispiel 3: Man wiederholte Anwendungsbeispiel 1, setzte aber anstelle des Isoalkangemischs aus Beispiel 2 das Isoalkangemisch aus Beispiel 3 beziehungsweise aus Beispiel 4 ein.

Anwendungsbeispiele 4-6: Feuchthalteformulierung

Anwendungsbeispiel 4:

| CTFA-Name | | Gew.-% |
|---|---|---|
| Cremophor® A6 (BASF AG) | Ceteareth-6 (Stearylalkohol-Ethoxilat) und Stearylalkohol | 2,0 |
| Cremophor® A25 (BASF AG) | Ceteareth-25 (Fettalkohol-Ethoxilat) | 2,0 |
| Isoalkangemisch aus Beispiel 2 | | 10 |
| Lanette® O | Cetearylalkohol | 2,0 |
| Stearinsäure | | 3,0 |
| Nip-Nip- (Nipa Laboratories Ltd., USA) | Methyl- und Propyl-4-hydroxybenzoat (7:3) | 0,5 |
| Abiol | Imidazolindinyl-Harnstoff | 0,5 |
| Polymer Vinylpyrrolidon/Stearylmethacrylat 80/20 Gew.-% (K-Wert 25; 1 % in Isopropanol) | | 3,0 |
| Wasser | | ad 100 |

Zur Herstellung der Formulierung wurden beide Phasen auf 80 °C erhitzt, Phase a) und b) (Phase b = Wasser) eingerührt, homogenisiert und kaltgerührt, und anschließend mit 10%iger wässriger NaOH-Lösung auf pH 6 eingestellt.
Anwendungsbeispiel 5 und Anwendungsbeispiel 6: Man wiederholte Anwendungsbeispiel 4, setzte aber anstelle des Isoalkangemischs aus Beispiel 2 das Isoalkangemisch aus Beispiel 3 beziehungsweise aus Beispiel 4 ein.

Anwendungsbeispiele 7-9: O/W-Creme zur Hautfeuchthaltung

Anwendungsbeispiel 7:

| Zusatz | Gew.-% |
|---|---|
| Glycerinmonostearat | 2,0 |
| Cetylalkohol | 3,0 |

(fortgesetzt)

| Zusatz | Gew.-% |
|---|---|
| Isoalkangemisch aus Beispiel 2 | 15,0 |
| Vaseline | 3,0 |
| Caprylcaprinsäuretriglycerid | 4,0 |
| Octyldodecanol | 2,0 |
| Hydriertes Kokosfett | 2,0 |
| Cetylphosphat | 0,4 |
| Polymer Vinylpyrrolidon/Acrylsäure/Stearylmethacrylat 60/5/35 Gew.-% (K-Wert 41; 1 % in Isopropanol) | 3,0 |
| Glycerin | 3,0 |
| Natriumhydroxid | q.s. |
| Parfümöl | q.s. |
| Konservierungsmittel | q.s. |
| Wasser | ad 100 |

Anwendungsbeispiel 8 und Anwendungsbeispiel 9: Man wiederholte Anwendungsbeispiel 7, setzte aber anstelle des Isoalkangemischs aus Beispiel 2 das Isoalkangemisch aus Beispiel 3 beziehungsweise aus Beispiel 4 ein.

Anwendungsbeispiele 10-12: O/W-Lotion

Anwendungsbeispiel 10:

| Zusatz | Gew.-% |
|---|---|
| Stearinsäure | 1,5 |
| Sorbitanmonostearat | 1,0 |
| Sorbitanmonooleat | 1,0 |
| Isoalkangemisch aus Beispiel 2 | 7,0 |
| Cetylalkohol | 1,0 |
| Polydimethylsiloxan | 1,5 |
| Glycerin | 3,0 |
| Polymer Vinylpyrrolidon/Acrylsäure/Stearylmethacrylat 50/10/40 Gew.-% (K-Wert 36; 1 % in Isopropanol) | 0,5 |
| Parfümöl | q.s. |
| Konservierungsmittel | q.s. |
| Wasser | ad 100 |

Anwendungsbeispiel 11 und Anwendungsbeispiel 12: Man wiederholte Anwendungsbeispiel 10, setzte aber anstelle des Isoalkangemischs aus Beispiel 2 das Isoalkangemisch aus Beispiel 3 beziehungsweise aus Beispiel 4 ein.

Anwendungsbeispiele 13-15: W/O-Lotion

Anwendungsbeispiel 13:

| Zusatz | Gew.-% |
|---|---|
| PEG-7-hydriertes Ricinusöl | 4,0 |
| Wollwachsalkohol | 1,5 |
| Bienenwachs | 3,0 |
| Triglycerid, flüssig | 5,0 |
| Vaseline | 9,0 |
| Ozokerit | 4,0 |
| Isoalkangemisch aus Beispiel 2 | 4,0 |
| Glycerin | 2,0 |
| Polymer Vinylpyrrolidon/Acrylsäure/Stearylmethacrylat 80/10/10 Gew.-% (K-Wert 45; 1 % in Isopropanol) | 10,0 |
| Magnesiumsulfat*7$H_2O$ | 0,7 |
| Parfümöl | q.s. |
| Konservierungsmittel | q.s. |
| Wasser | ad 100 |

Anwendungsbeispiel 14 und Anwendungsbeispiel 15: Man wiederholte Anwendungsbeispiel 13, setzte aber anstelle des Isoalkangemischs aus Beispiel 2 das Isoalkangemisch aus Beispiel 3 beziehungsweise aus Beispiel 4 ein.

Anwendungsbeispiel 16-18: Hydrogel zur Hautpflege

Anwendungsbeispiel 16:

| Zusatz | Gew.-% |
|---|---|
| Polymer Vinylpyrrolidon/Acrylsäure/Stearylmethacrylat 50/20/30 Gew.-% (K-Wert 36; 1 % in Isopropanol) | 20,0 |
| Sorbit | 2,0 |
| Isoalkangemisch aus Beispiel 2 | 3,0 |
| Polyethylenglycol 400 | 5,0 |
| Ethanol | 1,0 |
| Parfümöl | q.s. |
| Konservierungsmittel | q.s. |
| Wasser | ad 100 |

Anwendungsbeispiel 17 und Anwendungsbeispiel 18: Man wiederholte Anwendungsbeispiel 16, setzte aber anstelle des Isoalkangemischs aus Beispiel 2 das Isoalkangemisch aus Beispiel 3 beziehungsweise aus Beispiel 4 ein.

Anwendungsbeispiele 19-21: Flüssige Seife

Anwendungsbeispiel 19:

| Zusatz | Gew.-% |
|---|---|
| Kokosfettsäure, Kaliumsalz | 15 |
| Kaliumoleat | 3 |
| Isoalkangemisch aus Beispiel 2 | 5 |
| Polymer Vinylpyrrolidon/Stearylmethacrylat 70/30 Gew.-% (K-Wert 47; 1 % in Isopropanol) | 2 |
| Glycerinstearat | 1 |
| Ethylenglycoldistearat | 2 |
| Spezifische Zusätze, Komplexierungsmittel, Duftstoffe, Wasser | ad 100 |

Anwendungsbeispiel 20 und Anwendungsbeispiel 21: Man wiederholte Anwendungsbeispiel 19, setzte aber anstelle des Isoalkangemischs aus Beispiel 2 das Isoalkangemisch aus Beispiel 3 beziehungsweise aus Beispiel 4 ein.

Anwendungsbeispiele 22-24: W/O-Sonnenschutzemulsion

Anwendungsbeispiel 22:

| Zusatz | Gew.-% |
|---|---|
| Magnesiumstearat | 0,5 |
| Aluminiumstearat | 0,5 |
| Hydriertes Ricinusöl, 7 EO | 6,0 |
| Polymer Vinylpyrrolidon/Stearylmethacrylat 70/30 Gew.-% (K-Wert 47; 1 % in Isopropanol) | 5,0 |
| Isoalkangemisch aus Beispiel 2 | 1,0 |
| 2-Ethylhexansäurecetylstearylester | 10,0 |
| 2-Hydroxy-4-methoxybenzophenon | 2,0 |
| 4-Bis(polyethoxy)aminobenzoesäure-polyethoxyethylester | 5,0 |
| $\alpha$-Bisabolol | 0,2 |
| 1,2-Propylenglykol | 3,0 |
| Parfümöl | q.s. |
| Konservierungsmittel | q.s. |
| Wasser | ad 100 |

Anwendungsbeispiel 23 und Anwendungsbeispiel 24: Man wiederholte Anwendungsbeispiel 22, setzte aber anstelle des Isoalkangemischs aus Beispiel 2 das Isoalkangemisch aus Beispiel 3 beziehungsweise aus Beispiel 4 ein.

Anwendungsbeispiele 25-27: Sonnenschutzöl

Anwendungsbeispiel 25:

| Zusatz | Gew.-% |
|---|---|
| Polymer Vinylpyrrolidon/Stearylmethacrylat 70/30 Gew.-% (K-Wert 45; 1 % in Isopropanol) | 10,0 |
| 3-(4'-Methyl)-benzylidenbornan-2-on | 2,5 |
| 2-Hydroxy-4-methoxybenzophenon | 5,5 |
| Caprylsäure/Caprinsäure-Triglycerid | 10,0 |
| Dibutyladipat | 22,5 |
| Isopropylmyristat | 7,5 |
| Isoalkangemisch aus Beispiel 2 | 42,0 |

Anwendungsbeispiel 26 und Anwendungsbeispiel 27: Man wiederholte Anwendungsbeispiel 25, setzte aber anstelle des Isoalkangemischs aus Beispiel 2 das Isoalkangemisch aus Beispiel 3 beziehungsweise aus Beispiel 4 ein.

Anwendungsbeispiele 28-57: PIT - Emulsionen

Anwendungsbeispiele 28-32:

| Zusatz | Anbsp. 28 | Anbsp. 29 | Anbsp. 30 | Anbsp. 31 | Anbsp. 32 |
|---|---|---|---|---|---|
| Glycerinmonostearat selbstemulgierend | 0,50 | | 3,00 | 2,00 | 4,00 |
| Polyoxyethylen(12)cetylstearylether | | 5,00 | | 1,00 | 1,50 |
| Polyoxyethylen(20)cetylstearylether | | | | 2,00 | |
| Polyoxyethylen(30)cetylstearylether | 5,00 | | 1,00 | | |
| Stearylalkohol | | | 3,00 | | 0,50 |
| Cetylalkohol | 2,50 | 1,00 | | 1,50 | |
| 2-Ethylhexylmethoxycinnamat | | | | 5,00 | 8,00 |
| 2,4-Bis-(4-(2-ethyl-hexyloxy-)2-hydroxyl)-phenyl)-6-(4-methoxyphenyl)-(1,3,5)-triazin | | 1,50 | | 2,00 | 2,50 |
| Butyldimethoxydibenzoylmethan | | | 2,00 | | |
| Diethylhexylbutamidotriazon | 1,00 | 2,00 | | 2,00 | |
| Ethylhexyltriazon | 4,00 | | 3,00 | 4,00 | |
| 4-Methylbenzylidencampher | | 4,00 | | | 2,00 |
| Octocrylen | | 4,00 | | | 2,50 |
| Phenylen-1,4-bis-(mononatrium, 2-benzimidazyl-5,7)-disulfonsäure | | | 0,50 | | 1,50 |
| Phenylbenzimidazolsulfonsäure | 0,50 | | | 3,00 | |
| $C_{12-15}$-Alkylbenzoat | | 2,50 | | | 5,00 |
| Titandioxid | 0,50 | 1,00 | | 3,00 | 2,00 |
| Zinkoxid | 2,00 | | 3,00 | 0,50 | 1,00 |
| Dicaprylylether | | | 3,50 | | |
| Butylenglycol-Dicaprylat/-Dicaprat | 5,00 | | | 6,00 | |
| Dicaprylylcarbonat | | | 6,00 | | 2,00 |

(fortgesetzt)

| Zusatz | Anbsp. 28 | Anbsp. 29 | Anbsp. 30 | Anbsp. 31 | Anbsp. 32 |
|---|---|---|---|---|---|
| Dimethiconpolydimethylsiloxan | | 0,50 | 1,00 | | |
| Phenylmethylpolysiloxan | 2,00 | | | 0,50 | 0,50 |
| Shea-Butter (Sheabutter) | | 2,00 | | | 0,50 |
| PVP Hexadecencopolymer | 0,50 | | | 0,50 | 1,00 |
| Glycerin | 3,00 | 7,50 | 5,00 | 7,50 | 2,50 |
| Tocopherolacetat | 0,50 | | 0,25 | | 1,00 |
| Isoalkangemisch nach Beispiel 2 | 0,2 | 1,1 | 0,3 | 0,8 | 0,5 |
| Alpha-Glucosylrutin | 0,10 | | 0,20 | | |
| Konservierungsmittel | q.s. | q.s. | q.s. | q.s. | q.s. |
| Ethanol | 3,00 | 2,00 | 1,50 | | 1,00 |
| Parfüm | q.s. | q.s. | q.s. | q.s. | q.s. |
| Wasser | ad 100 | ad 100 | ad 100 | ad 100 | ad 100 |
| Anbsp. Anwendungsbeispiel | | | | | |

Anwendungsbeispiele 33-37 beziehungsweise Anwendungsbeispiele 38-42: Man wiederholte Anwendungsbeispiele 28-32, setzte aber anstelle des Isoalkangemischs aus Beispiel 2 das Isoalkangemisch aus Beispiel 3 beziehungsweise aus Beispiel 4 ein.

Anwendungsbeispiele 43-47:

| Zusatz | Anbsp. 43 | Anbsp. 44 | Anbsp. 45 | Anbsp. 46 | Anbsp. 47 |
|---|---|---|---|---|---|
| Glycerinmonostearat selbstemulgierend | 0,50 | | 3,00 | 2,00 | 4,00 |
| Polyoxyethylen(12)cetylstearylether | | 5,00 | | 1,00 | 1,50 |
| Polyoxyethylen(20)cetylstearylether | | | | 2,00 | |
| Polyoxyethylen(30)cetylstearylether | 5,00 | | 1,00 | | |
| Stearylalkohol | | | 3,00 | | 0,50 |
| Cetylalkohol | 2,50 | 1,00 | | 1,50 | |
| 2-Ethylhexylmethoxycinnamat | | | | 5,00 | 8,00 |
| 2,4-Bis-(4-(2-ethyl-hexyloxy-)2-hydroxyl)-phenyl)-6-(4-methoxyphenyl)-(1,3,5)-triazin | | 1,50 | | 2,00 | 2,50 |
| Butyldimethoxydibenzoylmethan | | | 2,00 | | |
| Dimethicodiethylbenzalmalonat | | 6,50 | | | |
| Diethylhexylbutamidotriazon | 1,00 | 2,00 | | 2,00 | |
| Ethylhexyltriazon | 4,00 | | 3,00 | 4,00 | |
| 2-(4'-(Diethylamino)-2'-hydroxybenzoyl)-benzoesäurehexylester | 1,50 | 4,00 | 3,50 | 5,00 | 2,00 |
| Octocrylen | | 4,00 | | | 2,50 |
| Phenylen-1,4-bis-(mononatrium) 2-benzimidazyl-5,7-disulfonsäure | | | 0,50 | | 1,50 |

(fortgesetzt)

| Zusatz | Anbsp. 43 | Anbsp. 44 | Anbsp. 45 | Anbsp. 46 | Anbsp. 47 |
|---|---|---|---|---|---|
| Phenylbenzimidazolsulfonsäure | 0,50 | | | 3,00 | |
| $C_{12-15}$-Alkylbenzoat | | 2,50 | | | 5,00 |
| Dicaprylylether | | | 3,50 | | |
| Butylenglycol-Dicaprylat/-Dicaprat | 5,00 | | | 6,00 | |
| Dicaprylylcarbonat | | | 6,00 | | 2,00 |
| Dimethiconpolydimethylsiloxan | | 0,50 | 1,00 | | |
| Phenylmethylpolysiloxan | 2,00 | | | 0,50 | 0,50 |
| Shea-Butter (Sheabutter) | | 2,00 | | | 0,50 |
| PVP Hexadecencopolymer | 0,50 | | | 0,50 | 1,00 |
| Glycerin | 3,00 | 7,50 | 5,00 | 7,50 | 2,50 |
| Tocopherolacetat | 0,50 | | 0,25 | | 1,00 |
| Isoalkangemisch nach Beispiel 2 | 0,10 | 1,00 | 0,20 | 0,50 | 1,50 |
| Diethylhexyl-2,6-naphthalat | | | 2,00 | | |
| Alpha-Glucosylrutin | 0,10 | | 0,20 | | |
| DMDM Hydantoin | | 0,25 | | 0,60 | 0,45 |
| Paraben | 0,15 | | 0,50 | 0,30 | |
| Konkaben LMB ® | 0,20 | | 0,40 | | |
| Trinatrium EDTA | | 0,80 | | | 1,00 |
| Phenoxyethanol | 0,30 | | | 0,20 | 0,50 |
| Ethanol | 3,00 | 2,00 | 1,50 | | 1,00 |
| Parfüm | q.s. | q.s. | q.s. | q.s. | q.s. |
| Wasser | ad 100 | ad 100 | ad 100 | ad 100 | ad 100 |
| Anbsp. Anwendungsbeispiel | | | | | |

Anwendungsbeispiele 48-52 beziehungsweise Anwendungsbeispiele 53-57: Man wiederholte Anwendungsbeispiele 43-47, setzte aber anstelle des Isoalkangemischs aus Beispiel 2 das Isoalkangemisch aus Beispiel 3 beziehungsweise aus Beispiel 4 ein.

Anwendungsbeispiele 58-117: O/W-Emulsionen

Anwendungsbeispiele 58-62:

| Zusatz | Anbsp. 58 | Anbsp. 59 | Anbsp. 60 | Anbsp. 61 | Anbsp. 62 |
|---|---|---|---|---|---|
| Glycerylstearatcitrat | | | 2,00 | | 2,00 |
| Glycerylsterat selbstemulgierend | 4,00 | 3,00 | | | |
| PEG-40-Stearat | 1,00 | | | | |
| Polyglyceryl-3-Methylglucose-Distearat | | | | 3,00 | |
| Sorbitanstearat | | | | | 2,00 |
| Stearinsäure | | 1,00 | | | |

(fortgesetzt)

| Zusatz | Anbsp. 58 | Anbsp. 59 | Anbsp. 60 | Anbsp. 61 | Anbsp. 62 |
|---|---|---|---|---|---|
| Stearylalkohol | | | 5,00 | | |
| Cetylalkohol | 3,00 | 2,00 | | 3,00 | |
| Cetylstearylalkohol | | | | | 2,00 |
| Caprylic-/Capric-Triglycerid | 5,00 | 3,00 | 4,00 | 3,00 | 3,00 |
| Octyldodecanol | | | 2,00 | | 2,00 |
| Dicaprylylether | | 4,00 | | 2,00 | 1,00 |
| Paraffinum liquidum | 5,00 | 2,00 | | 3,00 | |
| Titandioxid | | | 1,00 | | |
| Octocrylen | | | 3,50 | | |
| Butyldimethoxydibenzoylmethan | | | 0,50 | | |
| Isoalkangemisch nach Beispiel 2 | 0,10 | 0,20 | 0,70 | 0,15 | 1,00 |
| Tocopherol | 0,10 | | | | 0,20 |
| Biotin | | | 0,05 | | |
| Ethylendiamintetraessigsäure Trinatrium | 0,1 | | 0,10 | 0,1 | |
| Konservierungsmittel | q.s. | q.s. | q.s. | q.s. | q.s. |
| Polyacrylsäure | 3,00 | 0,1 | | 0,1 | 0,1 |
| Natronlauge 45 % | q.s | q.s. | q.s. | q.s. | q.s. |
| Glycerin | 5,00 | 3,00 | 4,00 | 3,00 | 3,00 |
| Butylenglycol | | 3,00 | | | |
| Parfüm | q.s. | q.s. | q.s. | q.s. | q.s. |
| Wasser | ad 100 | ad 100 | ad 100 | ad 100 | ad 100 |
| Anbsp. Anwendungsbeispiel | | | | | |

Anwendungsbeispiele 63-67 beziehungsweise Anwendungsbeispiele 68-72: Man wiederholte Anwendungsbeispiele 58-62, setzte aber anstelle des Isoalkangemischs aus Beispiel 2 das Isoalkangemisch aus Beispiel 3 beziehungsweise aus Beispiel 4 ein.

Anwendungsbeispiele 73-77:

| Zusatz | Anbsp. 73 | Anbsp. 74 | Anbsp. 75 | Anbsp. 76 | Anbsp. 77 |
|---|---|---|---|---|---|
| Glycerylstearatcitrat | | 2,00 | 2,00 | | |
| Glycerylsterat selbstemulgierend | 5,00 | | | | |
| Stearinsäure | | | | 2,50 | 3,50 |
| Stearylalkohol | 2,00 | | | | |
| Cetylalkohol | | | | 3,00 | 4,50 |
| Cetylstearylalkohol | | 3,00 | 1,00 | | 0,50 |
| $C_{12-15}$-Alkylbenzoat | | 2,00 | 3,00 | | |
| Caprylic-/Capric-Triglycerid | 2,00 | | | | |

(fortgesetzt)

| Zusatz | Anbsp. 73 | Anbsp. 74 | Anbsp. 75 | Anbsp. 76 | Anbsp. 77 |
|---|---|---|---|---|---|
| Octyldodecanol | 2,00 | 2,00 | | 4,00 | 6,00 |
| Dicaprylylether | | | | | |
| Paraffinum liquidum | | 4,00 | 2,00 | | |
| Cyclisches Dimethylpolysiloxan | | | | 0,50 | 2,00 |
| Dimethiconpolydimethylsiloxan | 2,00 | | | | |
| Titandioxid | 2,00 | | | | |
| 4-Methylbenzylidencampher | 1,00 | | | | |
| Ethylhexyltriazon | | | | | 2,00 |
| Butyldimethoxydibenzoylmethan | 0,50 | | | | 0,50 |
| Isoalkangemisch nach Beispiel 2 | 0,30 | 0,10 | 1,00 | 0,50 | 0,10 |
| Tocopherol | | | | | 0,10 |
| Ethylendiamintetraessigsäure Trinatrium | | | 0,20 | | 0,20 |
| Konservierungsmittel | q.s. | q.s. | q.s. | q.s. | q.s. |
| Xanthangummi | | | 0,20 | | |
| Polyacrylsäure | 0,15 | 0,1 | | 0,05 | 0,05 |
| Natronlauge 45 % | q.s. | q.s. | q.s. | q.s. | q.s. |
| Glycerin | 3,00 | | 3,00 | 5,00 | 3,00 |
| Butylenglycol | | 3,00 | | | |
| Ethanol | | 3,00 | | 3,00 | |
| Parfüm | q.s. | q.s. | q.s. | q.s. | q.s. |
| Wasser | ad 100 | ad 100 | ad 100 | ad 100 | ad 100 |
| Anbsp. Anwendungsbeispiel | | | | | |

Anwendungsbeispiele 78-82 beziehungsweise Anwendungsbeispiele 83-87: Man wiederholte Anwendungsbeispiele 73-77, setzte aber anstelle des Isoalkangemischs aus Beispiel 2 das Isoalkangemisch aus Beispiel 3 beziehungsweise aus Beispiel 4 ein.

Anwendungsbeispiele 88-92:

| Zusatz | Anbsp. 88 | Anbsp. 89 | Anbsp. 90 | Anbsp. 91 | Anbsp. 92 |
|---|---|---|---|---|---|
| Glycerylstearatcitrat | | | 2,00 | | 2,00 |
| Glycerylsterat selbstemulgierend | 4,00 | 3,00 | | | |
| PEG-40-Stearat | 1,00 | | | | |
| Polyglyceryl-3-Methylglucose-Distearat | | | | 3,00 | |
| Natriumcetearylsulfat | 0,50 | | | | 1,00 |
| Sorbitanstearat | | | | | 2,00 |
| Stearinsäure | | 1,00 | | | |
| Stearylalkohol | | | 5,00 | | |

(fortgesetzt)

| Zusatz | Anbsp. 88 | Anbsp. 89 | Anbsp. 90 | Anbsp. 91 | Anbsp. 92 |
|---|---|---|---|---|---|
| Cetylalkohol | 3,00 | 2,00 | | 3,00 | |
| Cetylstearylalkohol | | | | | 2,00 |
| Caprylic-/Capric-Triglycerid | 5,00 | 3,00 | 4,00 | 3,00 | 3,00 |
| Octyldodecanol | | | 2,00 | | 2,00 |
| Dicaprylylether | | 4,00 | | 2,00 | 1,00 |
| Paraffinum liquidum | 5,00 | 2,00 | | 3,00 | |
| Zinkoxid | 1,00 | | | | 2,00 |
| Titandioxid | | | 1,00 | | |
| 2-(4'-(Diethylamino)-2'-hydroxybenzoyl)-benzoesäurehexylester | 0,50 | 2,50 | 3,00 | 1,50 | 5,50 |
| 2-Ethylhexylmethoxycinnamat | | 5,50 | | | |
| 2,4-Bis-(4-(2-ethyl-hexyloxy-)2-hydroxyl)-phenyl)-6-(4-methoxyphenyl)-(1,3,5)-triazin | 3,00 | | 1,50 | 0,80 | |
| Diethylhexylbutamidotriazon | | 2,00 | | | 1,00 |
| Ethylhexyltriazon | 1,80 | | | | |
| Phenylen-1,4-bis-(mononatrium, 2-benzimidazyl-5,7-disulfonsäure) | 0,50 | | | | 0,50 |
| Phenylbenzimidazolsulfonsäure | | 0,50 | | 2,00 | |
| Octocrylen | | | 3,50 | | |
| Butyldimethoxydibenzoylmethan | | | 0,50 | | |
| Isoalkangemisch nach Beispiel 2 | 0,10 | 0,20 | 0,70 | 0,15 | 1,00 |
| Tocopherol | 0,10 | | | | 0,20 |
| Diethylhexy1-2,6-naphthalat | | | 3,50 | | |
| Biotin | | | 0,05 | | |
| Ethylendiamintetraessigsäure Trinatrium | 0,1 | | 0,10 | 0,1 | |
| Konservierungsmittel | q.s. | q.s. | q.s. | q.s. | q.s. |
| Polyacrylsäure | 3,00 | 0,1 | | 0,1 | 0,1 |
| Natronlauge 45 % | q.s | q.s. | q.s. | q.s. | q.s. |
| Glycerin | 5,00 | 3,00 | 4,00 | 3,00 | 3,00 |
| Butylenglycol | | 3,00 | | | |
| Parfüm | q.s. | q.s. | q.s. | q.s. | q.s. |
| Wasser | ad 100 | ad 100 | ad 100 | ad 100 | ad 100 |
| Anbsp. Anwendungsbeispiel | | | | | |

Anwendungsbeispiele 93-97 beziehungsweise Anwendungsbeispiele 98-102: Man wiederholte Anwendungsbeispiele 88-92, setzte aber anstelle des Isoalkangemischs aus Beispiel 2 das Isoalkangemisch aus Beispiel 3 beziehungsweise aus Beispiel 4 ein.

Anwendungsbeispiele 103-107:

| Zusatz | Anbsp. 103 | Anbsp. 104 | Anbsp. 105 | Anbsp. 106 | Anbsp. 107 |
|---|---|---|---|---|---|
| Glycerylstearatcitrat | | 2,00 | 2,00 | | |
| Glycerylsterat selbstemulgierend | 5,00 | | | | |
| Stearinsäure | | | | 2,50 | 3,50 |
| Stearylalkohol | 2,00 | | | | |
| Cetylalkohol | | | | 3,00 | 4,50 |
| Cetylstearylalkohol | | 3,00 | 1,00 | | 0,50 |
| $C_{12-15}$-Alkylbenzoat | | 2,00 | 3,00 | | |
| Caprylic-/Capric-Triglycerid | 2,00 | | | | |
| Octyldodecanol | 2,00 | 2,00 | | 4,00 | 6,00 |
| Dicaprylylether | | | | | |
| Paraffinum liquidum | | 4,00 | 2,00 | | |
| Cyclisches Dimethylpolysiloxan | | | | 0,50 | 2,00 |
| Dimethiconpolydimethylsiloxan | 2,00 | | | | |
| Titandioxid | 2,00 | | | | |
| 4-Methylbenzylidencampher | 1,00 | | | | |
| Ethylhexyltriazon | | 3,00 | | | 2,00 |
| Butyldimethoxydibenzoylmethan | 0,50 | | | | 0,50 |
| 2-(4'-(Diethylamino)-2'-hydroxybenzoyl)-benzoesäurehexylester | 0,50 | 1,50 | 5,00 | 3,30 | 4,00 |
| Isoalkangemisch nach Beispiel 2 | 0,30 | 0,10 | 1,00 | 0,50 | 0,10 |
| 2-Ethylhexylmethoxycinnamat | 1,50 | 4,00 | | 2,50 | |
| 2,4-Bis-(4-(2-ethyl-hexyloxy-)2-hydroxyl)-phenyl)-6-(4-methoxyphenyl)-(1,3,5)-triazin | 0,80 | | 1,50 | 2,50 | |
| Dimethicodiethylbenzalmalonat | | | 6,00 | | |
| Diethylhexylbutamidotriazon | 1,00 | 3,00 | | 2,00 | |
| Octocrylen | 4,00 | | 5,00 | | 3,50 |
| Phenylen-1,4-bis-(mononatrium, 2-benzimidazyl-5,7-disulfonsäure) | 0,50 | | 1,00 | | |
| Phenylbenzimidazolsulfonsäure | | 2,00 | | 1,50 | 0,50 |
| Tocopherol | | | | | 0,10 |
| Ethylendiamintetraessigsäure Trinatrium | | | 0,20 | | 0,20 |
| Konservierungsmittel | q.s. | q.s. | q.s. | q.s. | q.s. |
| Xanthangummi | | | 0,20 | | |
| Polyacrylsäure | 0,15 | 0,1 | | 0,05 | 0,05 |
| Natronlauge 45 % | q.s. | q.s. | q.s. | q.s. | q.s. |
| Glycerin | 3,00 | | 3,00 | 5,00 | 3,00 |

(fortgesetzt)

| Zusatz | Anbsp. 103 | Anbsp. 104 | Anbsp. 105 | Anbsp. 106 | Anbsp. 107 |
|---|---|---|---|---|---|
| Butylenglycol | | 3,00 | | | |
| Ethanol | | 3,00 | | 3,00 | |
| Parfüm | q.s. | q.s. | q.s. | q.s. | q.s. |
| Wasser | ad 100 | ad 100 | ad 100 | ad 100 | ad 100 |
| Anbsp. Anwendungsbeispiel | | | | | |

Anwendungsbeispiele 108-112 beziehungsweise Anwendungsbeispiele 113-117: Man wiederholte Anwendungsbeispiele 103-107, setzte aber anstelle des Isoalkangemischs aus Beispiel 2 das Isoalkangemisch aus Beispiel 3 beziehungsweise aus Beispiel 4 ein.

Anwendungsbeispiele 118-159: W/O-Emulsionen

Anwendungsbeispiele 118-122:

| Zusatz | Anbsp. 118 | Anbsp. 119 | Anbsp. 120 | Anbsp. 121 | Anbsp. 122 |
|---|---|---|---|---|---|
| Cetyldimethiconcopolyol | | 2,50 | | 4,00 | |
| Polyglyceryl-2-dipolyhydroxystearat | 5,00 | | | | 4,50 |
| PEG-30-dipolyhydroxystearat | | | 5,00 | | |
| 2-Ethylhexyl Methoxycinnamat | | 8,00 | | 5,00 | 4,00 |
| 2,4-Bis-(4-(2-ethyl-hexyloxy-)2-hydroxyl)-phenyl)-6-(4-methoxyphenyl)-(1,3,5)-triazin | 2,00 | 2,50 | | 2,00 | 2,50 |
| Butyl-dimethoxydibenzoylmethan | | | 2,00 | 1,00 | |
| Diethylhexylbutamidotriazon | 3,00 | 1,00 | | | 3,00 |
| Ethylhexyltriazon | | | 3,00 | 4,00 | |
| 4-Methylbenzylidencampher | | 2,00 | | 4,00 | 2,00 |
| Octocrylen | 7,00 | 2,50 | 4,00 | | 2,50 |
| Diethylhexylbutamidotriazon | 1,00 | | | 2,00 | |
| Phenylen-1,4-bis-(mononatrium, 2-benzimidazyl-5,7-disulfonsäure) | 1,00 | 2,00 | 0,50 | | |
| Phenylbenzimidazolsulfonsäure | 0,50 | | | 3,00 | 2,00 |
| Titandioxid | | 2,00 | 1,50 | | 3,00 |
| Zinkoxid | 3,00 | 1,00 | 2,00 | 0,50 | |
| Paraffinum Liquidum | | | 10,0 | | 8,00 |
| $C_{12-15}$-Alkylbenzoat | | | | 9,00 | |
| Dicaprylylether | 10,00 | | | | 7,00 |
| Butylen-Glycol-Dicaprylat/-Dicaprat | | | 2,00 | 8,00 | 4,00 |
| Dicaprylylcarbonat | 5,00 | | 6,00 | | |
| Dimethiconpolydimethylsiloxan | | 4,00 | 1,00 | 5,00 | |
| Phenylmethylpolysiloxan | 2,00 | 25,00 | | | 2,00 |

(fortgesetzt)

| Zusatz | Anbsp. 118 | Anbsp. 119 | Anbsp. 120 | Anbsp. 121 | Anbsp. 122 |
|---|---|---|---|---|---|
| Shea Butter | | | 3,00 | | |
| PVP Hexadecencopolymer | 0,50 | | | 0,50 | 1,00 |
| Octoxyglycerin | | 0,30 | 1,00 | | 0,50 |
| Glycerin | 3,00 | 7,50 | | 7,50 | 2,50 |
| Glycinsoja | | 1,00 | 1,50 | | |
| Magnesiumsulfat | 1,00 | 0,50 | | 0,50 | |
| Magnesiumchlorid | | | 1,00 | | 0,70 |
| Tocopherolacetat | 0,50 | | 0,25 | | 1,00 |
| Isoalkangemisch nach Beispiel 2 | 0,2 | 0,3 | 0,6 | 1 | 1,5 |
| Konservierungsmittel | q.s. | q.s. | q.s. | q.s. | q.s. |
| Ethanol | 3,00 | | 1,50 | | 1,00 |
| Parfüm | q.s. | q.s. | q.s. | q.s. | q.s. |
| Wasser | ad 100 | ad 100 | ad 100 | ad 100 | ad 100 |
| Anbsp. Anwendungsbeispiel | | | | | |

Anwendungsbeispiele 123-127 beziehungsweise Anwendungsbeispiele 128-132: Man wiederholte Anwendungsbeispiele 118-122, setzte aber anstelle des Isoalkangemischs aus Beispiel 2 das Isoalkangemisch aus Beispiel 3 beziehungsweise aus Beispiel 4 ein.

Anwendungsbeispiele 133-134:

| Zusatz | Anbsp. 133 | Anbsp. 134 |
|---|---|---|
| Polyglyceryl-2-dipolyhydroxystearat | 4,00 | 5,00 |
| Lanolinalkohol | 0,50 | 1,50 |
| Isohexadecan | 1,00 | 2,00 |
| Myristyl-Myristat | 0,50 | 1,50 |
| Vaseline | 1,00 | 2,00 |
| Butyldimethoxydibenzoylmethan | 0,50 | 1,50 |
| 4-Methylbenzylidencampher | 1,00 | 3,00 |
| Butylen-Glycol-Dicaprylat/-Dicaprat | 4,00 | 5,00 |
| Shea Butter | | 0,50 |
| Butylenglycol | | 6,00 |
| Octoxyglycerin | | 3,00 |
| Glycerin | 5,00 | |
| Tocopherolacetat | 0,50 | 1,00 |
| Isoalkangemisch nach Beispiel.2 | 0,50 | 0,20 |
| Trisodium EDTA | 0,20 | 0,20 |
| Konservierungsmittel | q.s. | q.s. |

(fortgesetzt)

| Zusatz | Anbsp. 133 | Anbsp. 134 |
|---|---|---|
| Ethanol | | 3,00 |
| Parfüm | q.s. | q.s. |
| Wasser | ad 100 | ad 100 |
| Anbsp. Anwendungsbeispiel | | |

Anwendungsbeispiele 135-136 beziehungsweise Anwendungsbeispiele 137-138: Man wiederholte Anwendungsbeispiele 133-134, setzte aber anstelle des Isoalkangemischs aus Beispiel 2 das Isoalkangemisch aus Beispiel 3 beziehungsweise aus Beispiel 4 ein.

Anwendungsbeispiele 139-143:

| Zusatz | Anbsp. 139 | Anbsp. 140 | Anbsp. 141 | Anbsp. 142 | Anbsp. 143 |
|---|---|---|---|---|---|
| Cetyldimethiconcopolyol | | 2,50 | | 4,00 | |
| Polyglyceryl-2-dipolyhydroxystearat | 5,00 | | | | 4,50 |
| PEG-30-dipolyhydroxystearat | | | 5,00 | | |
| Ethylhexylmethoxyzinnamat | | 8,00 | | 5,00 | 4,00 |
| 2,4-Bis-(4-(2-ethyl-hexyloxy-)2-hydroxyl)-phenyl)-6-(4-methoxyphenyl)-(1,3,5)-triazin | 2,00 | 2,50 | | 2,00 | 2,50 |
| Butyldimethoxydibenzoylmethan | | | 2,00 | 1,00. | |
| Diethylhexylbutamidotriazon | 3,00 | 1,00 | | | 3,00 |
| Ethylhexyltriazon | | | 3,00 | 4,00 | |
| 4-Methylbenzylidencampher | | 2,00 | | | |
| 2-(4'-(Diethylamino)-2'-hydroxybenzoyl)-benzoesäurehexylester | 0,50 | 2,50 | 4,50 | 3,00 | 1,80 |
| Octocrylen | 7,00 | 2,50 | 4,00 | | 2,50 |
| Phenylen-1,4-bis-(mononatrium, 2-benzimidazyl-5,7-disulfonsäure) | 1,00 | 2,00 | 0,50 | | |
| Phenylbenzimidazolsulfonsäure | 0,50 | | | 3,00 | 2,00 |
| Dimethicodiethylbenzalmalonat | | | 5,50 | | |
| Titandioxid | | 2,00 | 1,50 | | 3,00 |
| Zinkoxid | 3,00 | 1,00 | 2,00 | 0,50 | |
| Paraffinum liquidum | | | 10,0 | | 8,00 |
| $C_{12-15}$-Alkylbenzoat | | | | 9,00 | |
| Dicaprylylether | 10,00 | | | | 7,00 |
| Butylen-Glycol-Dicaprylat/-Dicaprat | | | 2,00 | 8,00 | 4,00 |
| Dicaprylylcarbonat | 5,00 | | 6,00 | | |
| Dimethiconpolydimethylsiloxan | | 4,00 | 1,00 | 5,00 | |
| Phenylmethylpolysiloxan | 2,00 | 25,00 | | | 2,00 |
| Shea Butter | | | 3,00 | | |

(fortgesetzt)

| Zusatz | Anbsp. 139 | Anbsp. 140 | Anbsp. 141 | Anbsp. 142 | Anbsp. 143 |
|---|---|---|---|---|---|
| Diethylhexyl-2,6-naphthalat | | | 6,50 | | |
| PVP Hexadecencopolymer | 0,50 | | | 0,50 | 1,00 |
| Octoxyglycerin | | 0,30 | 1,00 | | 0,50 |
| Glycerin | 3,00 | 7,50 | | 7,50 | 2,50 |
| Glycinsoja | | 1,00 | 1,50 | | |
| Magnesiumsulfat | 1,00 | 0,50 | | 0,50 | |
| Magnesiumchlorid | | | 1,00 | | 0,70 |
| Tocopherolacetat | 0,50 | | 0,25 | | 1,00 |
| Isoalkangemisch nach Beispiel 2 | 0,10 | 0,20 | 0,50 | 0,90 | 1,00 |
| DMDM Hydantoin | | 0,25 | | 0,60 | 0,45 |
| Parabene | 0,15 | | 0,50 | 0,30 | |
| Konkaben LMB ® | 0,20 | | 0,40 | | |
| Trinatrium EDTA | | 0,80 | | | 1,00 |
| Phenoxyethanol | 0,30 | | | 0,20 | 0,50 |
| Ethanol | 3,00 | 2,00 | 1,50 | | 1,00 |
| Parfüm | q.s. | q.s. | q.s. | q.s. | q.s. |
| Wasser | ad 100 | ad 100 | ad 100 | ad 100 | ad 100 |
| Anbsp. Anwendungsbeispiel | | | | | |

Anwendungsbeispiele 144-148 beziehungsweise Anwendungsbeispiele 149-153: Man wiederholte Anwendungsbeispiele 139-143, setzte aber anstelle des Isoalkangemischs aus Beispiel 2 das Isoalkangemisch aus Beispiel 3 beziehungsweise aus Beispiel 4 ein.

Anwendungsbeispiele 154-155:

| Zusatz | Anbsp. 154 | Anbsp. 155 |
|---|---|---|
| Polyglyceryl-2-dipolyhydroxystearat | 4,00 | 5,00 |
| Lanolinalkohol | 0,50 | 1,50 |
| Isohexadecan | 1,00 | 2,00 |
| Myristyl-Myristat | 0,50 | 1,50 |
| Vaseline | 1,00 | 2,00 |
| Butyldimethoxydibenzoylmethan | 0,50 | 1,50 |
| Diethylhexylbutamidotriazon | 1,50 | 0,50 |
| Butylen-Glycol-Dicaprylat/-Dicaprat | 4,00 | 5,00 |
| 2-(4'-(Diethylamino)-2'-hydroxybenzoyl)-benzoesäurehexylester | 2,50 | 4,50 |
| Shea Butter | | 0,50 |
| Butylenglycol | | 6,00 |
| Octoxyglycerin | | 3,00 |

(fortgesetzt)

| Zusatz | Anbsp. 154 | Anbsp. 155 |
|---|---|---|
| Glycerin | 5,00 | |
| Tocopherolacetat | 0,50 | 1,00 |
| Isoalkangemisch nach Beispiel 2 | 0,10 | 0,70 |
| Trisodium EDTA | 0,20 | 0,20 |
| Konservierungsmittel | q.s. | q.s. |
| Ethanol | | 3,00 |
| Parfüm | q.s. | q.s. |
| Wasser | ad 100 | ad 100 |
| Anbsp. Anwendungsbeispiel | | |

Anwendungsbeispiele 156-157 beziehungsweise Anwendungsbeispiele 158-159: Man wiederholte Anwendungsbeispiele 154-155, setzte aber anstelle des Isoalkangemischs aus Beispiel 2 das Isoalkangemisch aus Beispiel 3 beziehungsweise aus Beispiel 4 ein.

Anwendungsbeispiele 160-189: Hydrodispersionen

Anwendungsbeispiele 160-164:

| Zusatz | Anbsp. 160 | Anbsp. 161 | Anbsp. 162 | Anbsp. 163 | Anbsp. 164 |
|---|---|---|---|---|---|
| Polyoxyethylen(20)cetylstearylether | 1,00 | | | 0,5 | |
| Cetylalkohol | | | 1,00 | | |
| Natriumpolyacrylat | | 0,20 | | 0,30 | |
| Acrylat/$C_{10-30}$-Alkylacrylat-Crosspolymer | 0,50 | | 0,40 | 0,10 | 0,10 |
| Xanthangummi | | 0,30 | 0,15 | | 0,50 |
| 2-Ethylhexylmethoxycinnamat | | | | 5,00 | 8,00 |
| 2,4-Bis-(4-(2-ethyl-hexyloxy-)2-hydroxyl)-phenyl)-6-(4-methoxyphenyl)-(1,3,5)-triazin | | 1,50 | | 2,00 | 2,50 |
| Butyldimethoxydibenzoylmethan | 1,00 | | 2,00 | | |
| Diethylhexylbutamidotriazon | | 2,00 | | 2,00 | 1,00 |
| Ethylhexyltriazon | 4,00 | | 3,00 | 4,00 | |
| 4-Methylbenzylidencampher | 4,00 | 4,00 | | | 2,00 |
| Octocrylen | | 4,00 | 4,00 | | 2,50 |
| Phenylen-1,4-bis-(mononatrium, 2-benzimidazyl-5,7-disulfonsäure | 1,00 | | 0,50 | | 2,00 |
| Phenylbenzimidazolsulfonsäure | 0,50 | | | 3,00 | |
| Titandioxid | 0,50 | | 2,00 | 3,00 | 1,00 |
| Zinkoxid | 0,50 | 1,00 | 3,00 | | 2,00 |
| $C_{12-15}$-Alkylbenzoat | 2,00 | 2,50 | | | |
| Dicaprylylether | | 4,00 | | | |

(fortgesetzt)

| Zusatz | Anbsp. 160 | Anbsp. 161 | Anbsp. 162 | Anbsp. 163 | Anbsp. 164 |
|---|---|---|---|---|---|
| Butylenglycol-Dicaprylat/-Dicaprat | 4,00 | | 2,00 | 6,00 | |
| Dicaprylylcarbonat | | 2,00 | 6,00 | | |
| Dimethiconpolydimethylsiloxan | | 0,50 | 1,00 | | |
| Phenylmethylpolysiloxan | 2,00 | | | 0,50 | 2,00 |
| Shea Butter | | 2,00 | | | |
| PVP Hexadecencopolymer | 0,50 | | | 0,50 | 1,00 |
| Octoxyglycerin | | | 1,00 | | 0,50 |
| Glycerin | 3,00 | 7,50 | | 7,50 | 2,50 |
| Glycinsoja | | | 1,50 | | |
| Tocopherolacetat | 0,50 | | 0,25 | | 1,00 |
| Isoalkangemisch nach Beispiel 2 | 0,4 | 0,2 | 0,6 | 0,5 | 1 |
| Konservierungsmittel | q.s. | q.s. | q.s. | q.s. | q.s. |
| Ethanol | 3,00 | 2,00 | 1,50 | | 1,00 |
| Parfüm | q.s. | q.s. | q.s. | q.s. | q.s. |
| Wasser | ad 100 | ad 100 | ad 100 | ad 100 | ad 100 |
| Anbsp. Anwendungsbeispiel | | | | | |

Anwendungsbeispiele 165-169 beziehungsweise Anwendungsbeispiele 170-174: Man wiederholte Anwendungsbeispiele 160-164, setzte aber anstelle des Isoalkangemischs aus Beispiel 2 das Isoalkangemisch aus Beispiel 3 beziehungsweise aus Beispiel 4 ein.

Anwendungsbeispiele 175-179:

| Zusatz | Anbsp. 175 | Anbsp. 176 | Anbsp. 177 | Anbsp. 178 | Anbsp. 179 |
|---|---|---|---|---|---|
| Polyoxyethylen(20)cetylstearylether | 1,00 | | | 0,5 | |
| Cetylalkohol | | | 1,00 | | |
| Natriumpolyacrylat | | 0,20 | | 0,30 | |
| Acrylat/$C_{10-30}$-Alkylacrylat-Crosspolymer | 0,50 | | 0,40 | 0,10 | 0,10 |
| Xanthangummi | | 0,30 | 0,15 | | 0,50 |
| 2-Ethylhexylmethoxycinnamat | | | | 5,00 | 8,00 |
| 2,4-Bis-(4-(2-ethyl-hexyloxy-)2-hydroxyl)-phenyl)-6-(4-methoxyphenyl)-(1,3,5)-triazin | | 1,50 | 2,00 | | 2,50 |
| Dimethicodiethylbenzalmalonat | | 3,50 | | | |
| Butyldimethoxydibenzoylmethan | 1,00 | | 2,00 | | |
| Diethylhexylbutamidotriazon | | 2,00 | | 2,00 | 1,00 |
| Ethylhexyltriazon | 4,00 | | 3,00 | 4,00 | |
| 4-Methylbenzylidencampher | | | | | 2,00 |

(fortgesetzt)

| Zusatz | Anbsp. 175 | Anbsp. 176 | Anbsp. 177 | Anbsp. 178 | Anbsp. 179 |
|---|---|---|---|---|---|
| 2-(4'-(Diethylamino)-2'-hydroxybenzoyl)-benzoesäurehexylester | 2,00 | 1,40 | 0,50 | 4,60 | 5,20 |
| Octocrylen | | 4,00 | 4,00 | | 2,50 |
| Phenylen-1,4-bis-(mononatrium, 2-benzimidazyl-5,7-disulfonsäure) | 1,00 | | 0,50 | | 2,00 |
| Phenylbenzimidazolsulfonsäure | 0,50 | | | 3,00 | |
| Titandioxid | 0,50 | | 2,00 | 3,00 | 1,00 |
| Zinkoxid | 0,50 | 1,00 | 3,00 | | 2,00 |
| $C_{12-15}$-Alkylbenzoat | 2,00 | 2,50 | | | |
| Diethylhexyl-2,6-naphthalat | 4,00 | | | | |
| Dicaprylylether | | 4,00 | | | |
| Butylenglycol-Dicaprylat/-Dicaprat | 4,00 | | 2,00 | 6,00 | |
| Dicaprylylcarbonat | | 2,00 | 6,00 | | |
| Dimethiconpolydimethylsiloxan | | 0,50 | 1,00 | | |
| Phenylmethylpolysiloxan | 2,00 | | | 0,50 | 2,00 |
| Shea Butter | | 2,00 | | | |
| PVP Hexadecencopolymer | 0,50 | | | 0,50 | 1,00 |
| Octoxyglycerin | | | 1,00 | | 0,50 |
| Glycerin | 3,00 | 7,50 | | 7,50 | 2,50 |
| Glycinsoja | | | 1,50 | | |
| Tocopherolacetat | 0,50 | | 0,25 | | 1,00 |
| Isoalkangemisch nach Beispiel 2 | 0,3 | 0,10 | 0,50 | 1,00 | 0,20 |
| DMDM Hydantoin | | 0,25 | | 0,60 | 0,45 |
| Parabene | 0,15. | | 0,50 | 0,30 | |
| Konkaben LMB ® | 0,10 | | 0,30 | | |
| Trinatrium EDTA | | | 0,70 | | 1,00 |
| Phenoxyethanol | | 0,40 | | 0,20 | 0,50 |
| Ethanol | 3,00 | 2,00 | 1,50 | | 1,00 |
| Parfüm | q.s. | q.s. | q.s. | q.s. | q.s. |
| Wasser | ad 100 | ad 100 | ad 100 | ad 100 | ad 100 |
| Anbsp. Anwendungsbeispiel | | | | | |

Anwendungsbeispiele 180-184 beziehungsweise Anwendungsbeispiele 185-189: Man wiederholte Anwendungsbeispiele 175-179, setzte aber anstelle des Isoalkangemischs aus Beispiel 2 das Isoalkangemisch aus Beispiel 3 beziehungsweise aus Beispiel 4 ein.

Anwendungsbeispiele 191-196: Gelcreme

Anwendungsbeispiel 191:

| Zusatz | Gew.-% |
|---|---|
| Acrylat/$C_{10-30}$-Alkylacrylat-Crosspolymer | 0,40 |
| Polyacrylsäure | 0,20 |
| Xanthangummi | 0,10 |
| Cetearylalkohol | 3,00 |
| $C_{12-15}$-Alkylbenzoat | 4,00 |
| Caprylic/Caprictriglycerid | 3,00 |
| Ethylhexylmethoxycinnamat | 5,00 |
| Bis-Ethylhexyloxyphenol Methoxyphenyltriazin | 1,00 |
| Ethylhexyltriazon | 2,00 |
| Cyclisches Dimethylpolysiloxan | 5,00 |
| Dimethiconpolydimethylsiloxan | 1,00 |
| Isoalkangemisch nach Beispiel 2 | 1,00 |
| Glycerin | 3,00 |
| Natriumhydroxid | q.s. |
| Konservierungsmittel | q.s. |
| Parfüm | q.s. |
| Wasser | ad 100,0 |
| pH-Wert eingestellt auf 6,0 | |

Anwendungsbeispiel 192 und Anwendungsbeispiele 193: Man wiederholte Anwendungsbeispiel 191, setzte aber anstelle des Isoalkangemischs aus Beispiel 2 das Isoalkangemisch aus Beispiel 3 beziehungsweise aus Beispiel 4 ein.

Anwendungsbeispiel 194:

| Zusatz | Gew.-% |
|---|---|
| Acrylat/$C_{10-30}$-Alkylacrylat-Crosspolymer | 0,40 |
| Polyacrylsäure | 0,20 |
| Xanthangummi | 0,10 |
| Cetearylalkohol | 3,00 |
| $C_{12-15}$-Alkylbenzoat | 4,00 |
| Caprylic/Caprictriglycerid | 3,00 |
| Cyclisches Dimethylpolysiloxan | 5,00 |
| Dimeticonpolydimethylsiloxan | 1,00 |
| 2-(4'-(Diethylamino)-2'-hydroxybenzoyl)-benzoesäurehexylester | 1,20 |
| Isoalkangemisch nach Beispiel 2 | 1,00 |
| Ethylhexyltriazon | 2,00 |
| 2,4-Bis-(4-(2-ethylhexyloxy-)2-hydroxyl)-phenyl)-6-(4-methoxyphenyl)-(1,3,5)-triazin | 1,50 |
| Glycerin | 3,00 |

(fortgesetzt)

| Zusatz | Gew.-% |
|---|---|
| Natriumhydroxid | q.s. |
| Konservierungsmittel | q.s. |
| Parfüm | q.s. |
| Wasser | ad 100,0 |
| pH-Wert eingestellt auf 6,0 | |

Anwendungsbeispiel 195 und Anwendungsbeispiele 196: Man wiederholte Anwendungsbeispiel 194, setzte aber anstelle des Isoalkangemischs aus Beispiel 2 das Isoalkangemisch aus Beispiel 3 beziehungsweise aus Beispiel 4 ein.

Anwendungsbeispiele 197-199: W/O-Creme

Anwendungsbeispiel 197

| Zusatz | Gew.-% |
|---|---|
| Polyglyceryl-3-Diisostearate | 3,50 |
| Glycerin | 3,00 |
| Polyglyceryl-2-Dipolyhydroxystearate | 3,50 |
| Isoalkangemisch nach Beispiel 2 | 1 |
| Konservierungsmittel | q.s. |
| Ethylhexylmethoxycinnamat | 5,00 |
| Bis-Ethylhexyloxyphenolmethoxyphenyltriazin | 0,80 |
| Octocrylen | 3,00 |
| Parfüm | q.s. |
| Wasser | ad 100,0 |
| Magnesiumsulfat | 0,6 |
| Isopropylstearat | 2,0 |
| Caprylylether | 8,0 |
| Cetearylisononanoat | 6,0 |

Anwendungsbeispiel 198 und Anwendungsbeispiele 199: Man wiederholte Anwendungsbeispiel 197, setzte aber anstelle des Isoalkangemischs aus Beispiel 2 das Isoalkangemisch aus Beispiel 3 beziehungsweise aus Beispiel 4 ein.

Anwendungsbeispiele 200-202: W/O/W-Creme

Anwendungsbeispiel 200:

| Zusatz | Gew.-% |
|---|---|
| Glycerylstearat | 3,00 |

(fortgesetzt)

| Zusatz | Gew.-% |
|---|---|
| PEG-100-Stearat | 0,75 |
| Behenylalkohol | 2,00 |
| Caprylic-/Capric-Triglycerid | 8,0 |
| Octyldodecanol | 5,00 |
| $C_{12-15}$-Alkyjbenzoat | 3,00 |
| Isoalkangemisch nach Beispiel 2 | 1 |
| Ethylhexylmethoxycinnamat | 5,00 |
| Bis-Ethylhexyloxyphenol Methoxyphenyltriazin | 1,80 |
| Ethylhexyltriazon | 1,50 |
| Magnesiumsulfat ($MgSO_4$) | 0,80 |
| Ethylendiamintetraessigsäure | 0,10 |
| Konservierungsmittel | q.s. |
| Parfüm | q.s. |
| Wasser | ad 100,0 |
| pH-Wert eingestellt auf 6,0 | |

Anwendungsbeispiel 201 und Anwendungsbeispiele 202: Man wiederholte Anwendungsbeispiel 200 setzte aber anstelle des Isoalkangemischs aus Beispiel 2 das Isoalkangemisch aus Beispiel 3 beziehungsweise aus Beispiel 4 ein.

Anwendungsbeispiele 203-220: Conditioner-Shampoo mit Perlglanz

Anwendungsbeispiele 203-205:

| Zusatz | Anbsp.203 | Anbsp. 204 | Anbsp. 205 |
|---|---|---|---|
| Polyquaternium-10 | 0,5 | 0,5 | 0,5 |
| Natriumlaurethsulfat | 9,0 | 9,0 | 9,0 |
| Cocoamidopropylbetain | 2,5 | 2,5 | 2,5 |
| Benzophenon-3 | 1,5 | 0,5 | 1,00 |
| Perlglanzmittel | 2,0 | 2,0 | 2,0 |
| Isoalkangemisch nach Beispiel 2 | 0,1 | 0,15 | 0,05 |
| Dinatrium EDTA | 0,1 | 0,2 | 0,15 |
| Konvervierungsmittel, Parfüm, Verdicker, pH-Einstellung und Lösungsvermittler | q.s. | q.s. | q.s. |
| Wasser, VES (vollentsalzt) | ad 100,0 | ad 100,0 | ad 100,0 |
| Der pH-Wert wird auf 6 eingestellt. | | | |
| Anbsp. Anwendungsbeispiel | | | |

Anwendungsbeispiele 206-208 beziehungsweise Anwendungsbeispiele 209-211: Man wiederholte Anwendungsbeispiele 203-205, setzte aber anstelle des Isoalkangemischs aus Beispiel 2 das Isoalkangemisch aus Beispiel

3 beziehungsweise aus Beispiel 4 ein.

Anwendungsbeispiele 212-214:

| Zusatz | Anbsp. 212 | Anbsp. 213 | Anbsp. 214 |
|---|---|---|---|
| Polyquarternium-10 | 0,50 | 0,50 | 0,40 |
| Natriumlaurethsulfat | 9,00 | 8,50 | 8,90 |
| Cocoamidopropylbetain | 2,50 | 2,60 | 3,00 |
| Benzophenon-4 | 0,50 | 0,50 | 1,00 |
| Isoalkangemisch aus Beispiel 2 | 10,50 | 3,00 | 1,00 |
| Perglanzmittel | 2,00 | 2,50 | |
| Dinatrium EDTA | 0,10 | 0,15 | 0,05 |
| Konservierungsmittel, Parfüm, Verdicker, pH-Einstellung und Lösungsvermittler | q.s. | q.s. | q.s. |
| Wasser, (vollentsalzt) | ad 100 | ad 100 | ad 100 |
| pH auf 6,0 eingestellt | | | |
| Anbsp. Anwendungsbeispiel | | | |

Anwendungsbeispiele 215-217 beziehungsweise Anwendungsbeispiele 218-220: Man wiederholte Anwendungsbeispiele 212-214, setzte aber anstelle des Isoalkangemischs aus Beispiel 2 das Isoalkangemisch aus Beispiel 3 beziehungsweise aus Beispiel 4 ein.

Anwendungsbeispiele 221-253: Klares Conditioner-Shampoo

Anwendungsbeispiele 221-223:

| Zusatz | Anbsp.221 | Anbsp. 222 | Anbsp. 223 |
|---|---|---|---|
| Polyquaternium-10 | 0,5 | 0,5 | 0,5 |
| Natriumlaurethsulfat | 9,0 | 9,0 | 9,0 |
| Benzophenon 3 | 1,00 | 1,50 | 0,50 |
| Cocoamidopropylbetain | 2,5 | 2,5 | 2,5 |
| Isoalkangemisch nach Beispiel 2 | 0,05 | 0,15 | 0,1 |
| Iminodibernsteinsäure, Na-Salz | 0,2 | 0,3 | 0,8 |
| Konservierungsmittel, Parfüm, Verdicker, pH-Einstellung und Lösungsvermittler | q.s. | q.s. | q.s. |
| Wasser, VES (vollentsalzt) | ad 100,0 | ad 100,0 | ad 100,0 |
| Der pH-Wert wird auf 6 eingestellt | | | |
| Anbsp. Anwendungsbeispiel | | | |

Anwendungsbeispiele 224-226 beziehungsweise Anwendungsbeispiele 227-229: Man wiederholte Anwendungsbeispiele 221-223, setzte aber anstelle des Isoalkangemischs aus Beispiel 2 das Isoalkangemisch aus Beispiel 3 beziehungsweise aus Beispiel 4 ein.

Anwendungsbeispiele 230-234:

| Zusatz | Anbsp.230 | Anbsp.231 | Anbsp. 232 | Anbsp.233 | Anbsp. 234 |
|---|---|---|---|---|---|
| Amphotensid GB 2009 | 10,00 | 15,00 | 20,00 | 12,00 | 17,00 |
| Plantacare 2000 | 5,00 | 6,00 | 7,00 | 8,00 | 4,00 |
| Tego Betain L7 | 15,00 | 12,00 | 10,00 | 18,00 | 20,00 |
| Luviquat FC 550 | 0,30 | 0,20 | 0,20 | 0,20 | 0,30 |
| Parfüm | 0,10 | 0,10 | 0,10 | 0,10 | 0,10 |
| Isoalkangemisch aus Beispiel 2 | 2,00 | 4,00 | 7,00 | 1,90 | 6,00 |
| Cremophor PS 20 | 5,00 | 1,00 | 1,00 | 7,00 | 5,00 |
| Konservierungsmittel | 0,10 | 0,10 | 0,10 | 0,10 | 0,10 |
| Rewopal LA 3 | 2,00 | 1,00 | 0,50 | 2,00 | 2,00 |
| Zitronensäure | 0,20 | 0,20 | 0,20 | 0,20 | 0,20 |
| Stepan PEG 600 DS | 3,00 | 2,00 | 2,00 | 3,00 | 2,50 |
| Wasser dem. | ad 100 | ad 100 | ad 100 | ad 100 | ad 100 |
| Anbsp. Anwendungsbeispiel | | | | | |

Anwendungsbeispiele 235-239 beziehungsweise Anwendungsbeispiele 240-244: Man wiederholte Anwendungsbeispiele 230-234, setzte aber anstelle des Isoalkangemischs aus Beispiel 2 das Isoalkangemisch aus Beispiel 3 beziehungsweise aus Beispiel 4 ein.

Anwendungsbeispiele 245-247:

| Zusatz | Anbsp. 245 | Anbsp. 246 | Anbsp. 247 |
|---|---|---|---|
| Polyquarternium-10 | 0,50 | 0,50 | 0,50 |
| Natriumlaurethsulfat | 9,00 | 8,50 | 9,50 |
| Isoalkangemisch aus Beispiel 2 | 4,00 | 3,00 | 9,00 |
| Benzophenon-3 | 1,00 | 1,50 | 0,50 |
| Imidobernsteinsäure, Na-Salz | 0,20 | 0,20 | 0,80 |
| Konservierungsmittel, Parfüm, Verdicker, pH-Einstellung und Lösungsvermittler | q.s. | q.s. | q.s. |
| Wasser, (vollentsalzt) | ad 100 | ad 100 | ad 100 |
| pH auf 6,0 eingestellt | | | |
| Anbsp. Anwendungsbeispiel | | | |

Anwendungsbeispiele 248-250 beziehungsweise Anwendungsbeispiele 251-253: Man wiederholte Anwendungsbeispiele 245-247, setzte aber anstelle des Isoalkangemischs aus Beispiel 2 das Isoalkangemisch aus Beispiel 3 beziehungsweise aus Beispiel 4 ein.

Anwendungsbeispiele 254-271: Klares Light-Shampoo mit Volumeneffekt

Anwendungsbeispiele 254-256:

| Zusatz | Anbsp. 254 | Ânbsp. 255 | Anbsp. 256 |
|---|---|---|---|
| Natriumlaurethsulfat | 10,0 | 10,0 | 10,0 |
| Ethylhexylmethoxycinnamat | 2 | 2 | 2 |
| Cocoamidopropylbetain | 2,5 | 2,5 | 2,5 |
| Isoalkangemisch nach Beispiel 2 | 0,05 | 0,1 | 0,01 |
| Dinatrium EDTA | 0,2 | 0,15 | 0,7 |
| Konvervierungsmittel, Parfüm, Verdicker, pH-Einstellung und Lösungsvermittler | q.s. | q.s. | q.s. |
| Wasser, VES (vollentsalzt) | ad 100,0 | ad 100,0 | ad 100,0 |
| Der pH-Wert wird auf 5,5 eingestellt. | | | |
| Anbsp. Anwendungsbeispiel | | | |

Anwendungsbeispiele 257-259 beziehungsweise Anwendungsbeispiele 260-262: Man wiederholte Anwendungsbeispiele 254-256, setzte aber anstelle des Isoalkangemischs aus Beispiel 2 das Isoalkangemisch aus Beispiel 3 beziehungsweise aus Beispiel 4 ein.

Anwendungsbeispiele 263-265:

| Zusatz | Anbsp.263 | Anbsp. 264 | Anbsp. 265 |
|---|---|---|---|
| Natriumlaurethsulfat | 10,00 | 10,50 | 11,00 |
| Ethylhexylmethoxycinnamat | 2,00 | 1,50 | 2,30 |
| Isoalkangemisch aus Beispiel 2 | 9,00 | 7,00 | 1,00 |
| Cocoamidopropylbetain | 2,50 | 2,60 | 2,20 |
| Dinatrium EDTA | 0,01 | 0,10 | 0,01 |
| Konservierungsmittel, Parfüm, Verdicker, pH-Einstellung und Lösungsvermittler | q.s. | q.s. | q.s. |
| Wasser, (vollentsalzt) | ad 100 | ad 100 | ad 100 |
| pH auf 5,5 eingestellt | | | |
| Anbsp. Anwendungsbeispiel | | | |

Anwendungsbeispiele 266-268 beziehungsweise Anwendungsbeispiele 269-271: Man wiederholte Anwendungsbeispiele 263-265, setzte aber anstelle des Isoalkangemischs aus Beispiel 2 das Isoalkangemisch aus Beispiel 3 beziehungsweise aus Beispiel 4 ein.

Anwendungsbeispiele 272-286: Klares Shampoo

Anwendungsbeispiele 272-276:

| Zusatz | Anbsp. 272 | Anbsp. 273 | Anbsp. 274 | Anbsp. 275 | Anbsp. 276 |
|---|---|---|---|---|---|
| Texapon N 70 | 13,00 | 15,00 | 10,50 | 12,50 | 10,00 |
| Dehyton PK 45 | 7,50 | 7,00 | 5,00 | 5,50 | 10,00 |
| Cetiol HE | 2,00 | 2,50 | 3,50 | 5,00 | 2,30 |
| Parfüm | 0,10 | 0,10 | 0,10 | 0,10 | 0,10 |
| Isoalkangemisch aus Beispiel 2 | 2,00 | 4,00 | 6,00 | 3,50 | 7,00 |
| D-Panthenol USP | 1,00 | 1,50 | 1,80 | 1,70 | 1,40 |
| Konservierungsmittel | 0,10 | 0,10 | 0,10 | 0,10 | 0,10 |
| Zitronensäure | 0,10 | 0,10 | 0,10 | 0,10 | 0,10 |
| Luviquat Ultra Care | 1,50 | 1,00 | 1,50 | 1,20 | 1,10 |
| Natriumchlorid | 1,50 | 1,40 | 1,40 | 1,30 | 1,50 |
| Wasser dem. | ad 100 | ad 100 | ad 100 | ad 100 | ad 100 |
| Anbsp. Anwendungsbeispiel | | | | | |

Anwendungsbeispiele 277-281 beziehungsweise Anwendungsbeispiele 282-286: Man wiederholte Anwendungsbeispiele 254-256, setzte aber anstelle des Isoalkangemischs aus Beispiel 2 das Isoalkangemisch aus Beispiel 3 beziehungsweise aus Beispiel 4 ein.

Anwendungsbeispiele 287-301: Shampoo

Anwendungsbeispiele 287-291:

| Zusatz | Anbsp. 287 | Anbsp. 288 | Anbsp. 289 | Anbsp. 290 | Anbsp. 291 |
|---|---|---|---|---|---|
| Texapon NSO | 35,00 | 40,00 | 30,00 | 45,00 | 27,00 |
| Plantacare 2000 | 5,00 | 5,50 | 4,90 | 3,50 | 7,00 |
| Tego Betain L7 | 10,00 | 5,00 | 12,50 | 7,50 | 15,00 |
| Parfüm | 0,10 | 0,10 | 0,10 | 0,10 | 0,10 |
| Isoalkangemisch aus Beispiel 2 | 3,50 | 3,50 | 10,5 | 10,00 | 20,00 |
| D-Panthenol USP | 0,50 | 1,00 | 0,80 | 1,50 | 0,50 |
| Konservierungsmittel | 0,10 | 0,10 | 0,10 | 0,10 | 0,10 |
| Zitronensäure | 0,10 | 0,10 | 0,10 | 0,10 | 0,10 |
| Rewopal LA 3 | 0,50 | 2,00 | 0,50 | 0,50 | 2,00 |
| Natriumchlorid | 1,50 | 1,50 | 1,50 | 1,50 | 1,50 |
| Wasser dem. | ad 100 | ad 100 | ad 100 | ad 100 | ad 100 |
| Anbsp. Anwendungsbeispiel | | | | | |

Anwendungsbeispiele 292-296 beziehungsweise Anwendungsbeispiele 297-301: Man wiederholte Anwendungsbeispiele 287-291, setzte aber anstelle des Isoalkangemischs aus Beispiel 2 das Isoalkangemisch aus Beispiel 3 beziehungsweise aus Beispiel 4 ein.

Anwendungsbeispiele 302-304: W/O/W-Emulsion

Anwendungsbeispiel 302:

| Zusatz | Gew.-% |
|---|---|
| Glycerylstearat | 3,00 |
| PEG-100-Stearat | 0,75 |
| Behenylalkohol | 2,00 |
| Caprylic-/Capric-Triglycerid | 8,0 |
| Octyldodecanol | 5,00 |
| $C_{12}$-$_{15}$-Alkylbenzoat | 3,00 |
| 2-(4'-(Diethylamino)-2'-hydroxybenzoyl)-benzoesäurehexylester | 1,30 |
| Isoalkangemisch nach Beispiel 2 | 1,00 |
| Ethylhexylmethoxycinnamat | 5,00 |
| 2,4-Bis-(4-(2-ethylhexyloxy)-2-hydroxyl)-phenyl)-6-(4-methoxyphenyl)-(1,3,5)-triazin | 1,80 |
| Ethylhexyltriazon | 1,50 |
| Magnesiumsulfat ($MgSO_4$) | 0,80 |
| Ethylendiamintetraessigsäure | 0,10 |
| Konservierungsmittel | q.s. |
| Parfüm | q.s. |
| Wasser | ad 100,0 |
| pH-Wert eingestellt auf 6,0 | |

Anwendungsbeispiel 303 und Anwendungsbeispiele 304: Man wiederholte Anwendungsbeispiele 302, setzte aber anstelle des Isoalkangemischs aus Beispiel 2 das Isoalkangemisch aus Beispiel 3 beziehungsweise aus Beispiel 4 ein.

Anwendungsbeispiele 305-334: Feststoffstabilisierte Emulsionen

Anwendungsbeispiele 305-309:

| Zusatz | Anbsp. 305 | Anbsp. 306 | Anbsp. 307 | Anbsp. 308 | Anbsp. 309 |
|---|---|---|---|---|---|
| Mineralöl | | | 16,0 | 16,0 | |
| Octyldodecanol | 9,0 | 9,0 | 5,0 | | |
| Caprylic/Capric Triglycerid | 9,0 | 9,0 | 6,0 | | |
| $C_{12}$-$_{15}$-Alkylbenzoat | | | | 5,0 | 8,0 |
| Butylenglycoldicaprylat/Dicaprat | | | | | 8,0 |
| Dicaprylylether | 9,0 | | | 4,0 | |
| Dicaprylylcarbonat | | 9,0 | | | |
| Hydroxyoctacosanyl-Hydroxystearat | 2,0 | 2,0 | 2,0 | 2,0 | 1,5 |

(fortgesetzt)

| Zusatz | Anbsp. 305 | Anbsp. 306 | Anbsp. 307 | Anbsp. 308 | Anbsp. 309 |
|---|---|---|---|---|---|
| Disteardimoniumhectorit | 1,0 | 0,75 | 0,5 | 0,5 | 0,25 |
| Cera Microcristallina + Paraffinum Liquidum | | | | | 5,0 |
| Hydroxypropylmethylcellulose | | | | | 0,05 |
| Dimethicon | | | | | 3,0 |
| Ethylhexylmethoxycinnamat | | | | | 3,0 |
| 4-Methylbenzylidencampher | | | | | 4,0 |
| Diethylhexylbutamidotriazon | | | | | 4,0 |
| Methylen-bis-benzotriazolyl Tetramethylbutylphenol | | | | | 4,0 |
| Bis-Ethylhexyloxyphenolmethoxyphenyltriazin | | 0,5 | | 2,00 | 1,00 |
| Drometrizoltrisiloxan | | 0,50 | | 1,00 | |
| Terephthalidendicamphersulfonsäure | | 1,00 | 0,50 | | 1,50 |
| Phenyldibenzimidazoltetrasulfonsäure | | | | 1,50 | 0,5 |
| Titandioxid + Alumina + Simethicon + Aqua | | 2,0 | 4,0 | 2,0 | 4,0 |
| Titandioxid + Trimethoxycaprylylsilan | | | | | 3,0 |
| Zinkoxid | | | | 6,0 | |
| Silicadimethylsilylat | | | 1,0 | | |
| Bornitrid | 2,0 | | | | |
| Stärke/-Natriummetaphosphat-Polymer | | 0,5 | | | |
| Tapioca-Stärke | | | | 1,0 | |
| Isoalkangemisch nach Beispiel 2 | 0,80 | 0,10 | 0,40 | 0,50 | 1,00 |
| 2-(4’-(Diethylamino)-2’-hydroxybenzoyl)-benzoesäurehexylester | 0,40 | 1,80 | 5,00 | 3,50 | 4,00 |
| Natriumchlorid | 1,0 | 1,0 | 1,0 | 1,0 | 1,0 |
| Glycerin | 5,0 | 10,0 | 3,0 | 6,0 | 10,0 |
| Trinatrium EDTA | | 1,0 | | 1,0 | |
| Methylparaben | 0,21 | | | | 0,2 |
| Propylparaben | 0,07 | | | | |
| Phenoxyethanol | 0,5 | | 0,4 | 0,4 | 0,5 |
| Hexamidindiisethionat | | | | | 0,08 |
| Diazolidinyl-Harnstoff | | | 0,28 | 0,28 | |
| Alkohol | | | | 2,5 | |
| Parfüm | q.s. | q.s. | q.s. | q.s. | q.s. |
| Wasser | ad 100 | ad 100 | ad 100 | ad 100 | ad 100 |
| Anbsp. Anwendungsbeispiel | | | | | |

Anwendungsbeispiele 310-314 beziehungsweise Anwendungsbeispiele 315-319: Man wiederholte Anwendungsbeispiele 305-309, setzte aber anstelle des Isoalkangemischs aus Beispiel 2 das Isoalkangemisch aus Beispiel 3 beziehungsweise aus Beispiel 4 ein.

Anwendungsbeispiele 320-324:

| Zusatz | Anbsp. 320 | Anbsp. 321 | Anbsp. 322 | Anbsp. 323 | Anbsp. 324 |
|---|---|---|---|---|---|
| Mineralöl | | | | | 16,0 |
| Octyldodecanol | 6,0 | | 7,5 | 7,5 | 5,0 |
| Caprylic/Caprictriglycerid | | | | | 6,0 |
| $C_{12}$-$_{15}$-Alkylbenzoat | 7,0 | 8,0 | 7,5 | 7,5 | |
| Butylenglycoldicaprylat/Dicaprat | 4,0 | 8,0 | | | |
| Dicaprylylether | | 8,0 | 7,5 | 7,5 | |
| Dicaprylylcarbonat | 4,0 | | | | |
| Hydroxyoctacosanyl-Hydroxystearat | 2,0 | 2,0 | 2,0 | 2,0 | 1,5 |
| PVP/Hexadecencopolymer | | | | 1,0 | 0,7 |
| Disteardimoniumhectorit | 1,0 | 1,0 | 1,0 | 0,5 | 1,0 |
| Dimethicon | | 2,0 | | | |
| Cyclomethicon | | | | 2,0 | |
| Ethylhexylmethoxycinnamat | 5,0 | | 5,0 | | |
| Butylmethoxydibenzoylmethan | | 2,0 | | | 1,0 |
| 4-Methylbenzylidencampher | | 4,0 | | | 2,0 |
| Ethylhexyltriazon | 2,0 | 2,0 | | | 1,0 |
| Methylen-bis-benzotriazolyltetramethylbuthylphenol | | | 1,00 | | 0,50 |
| Dimethicodiethylbenzalmalonat | | 3,80 | | | |
| Bis-Ethylhexyloxyphenolmethoxyphenyltriazin | 2,5 | | 1,5 | 3,00 | |
| Titandioxid + Alumina + Simethicon + Aqua | 1,5 | 2,0 | 4,0 | 0,5 | 1,5 |
| Titandioxid + Trimethoxycaprylylsilan | | | 2,0 | | |
| Zinkoxid | | | 2,0 | | |
| Phenyldibenzimidazoltetrasulfonsäure | 1,00 | | | 2,00 | |
| Phenylbenzimidazolsulfonsäure | 2,0 | | | | |
| Diethylhexyl-2,6-naphthalat | | | | | 3,50 |
| Bornitrid | | | | | 0,5 |
| Stärke-/Natriummetaphosphat-Polymer | 0,5 | | 1,5 | | |
| Kornstärke modifiziert | | 1,0 | | | |
| Acrylatcopolymer | | | | 0,25 | |
| Talk | | | | 2,0 | |
| Natriumchlorid | 1,0 | 1,0 | 1,0 | | |
| Isoalkangemisch nach Beispiel 2 | 0,10 | 0,80 | 0,60 | 1,50 | 0,40 |
| 2-(4'-(Diethylamino)-2'-hydroxybenzoyl)-benzoesäurehexylester | 4,80 | 1,00 | 0,50 | 2,50 | 3,50 |
| Magnesiumsulfat | | | | | 0,7 |
| Natronlauge 45 % | 0,5 | 0,5 | | | |

(fortgesetzt)

| Zusatz | Anbsp. 320 | Anbsp. 321 | Anbsp. 322 | Anbsp. 323 | Anbsp. 324 |
|---|---|---|---|---|---|
| Glycerin | 5,0 | 7,5 | 5,0 | 10,0 | 3,0 |
| Trinatrium EDTA | | 1,0 | 1,0 | | 1,0 |
| Propylencarbonat | 0,33 | 0,33 | 0,33 | | 0,33 |
| Methylparaben | 0,21 | 0,21 | 0,2 | 0,2 | 0,21 |
| Propylparaben | 0,07 | 0,07 | | | 0,07 |
| Phenoxyethanol | 0,5 | 0,5 | 0,5 | 0,5 | 0,5 |
| Hexamidindiisethionat | | | 0,08 | 0,08 | |
| Alkohol | | 5,0 | | | |
| Parfüm | q.s. | q.s. | q.s. | q.s. | q.s. |
| Wasser | ad 100 | ad 100 | ad 100 | ad 100 | ad 100 |
| Anbsp. Anwendungsbeispiel | | | | | |

Anwendungsbeispiele 325-329 beziehungsweise Anwendungsbeispiele 330-334: Man wiederholte Anwendungsbeispiele 320-324, setzte aber anstelle des Isoalkangemischs aus Beispiel 2 das Isoalkangemisch aus Beispiel 3 beziehungsweise aus Beispiel 4 ein.

Anwendungsbeispiele 335-349: Antiperspirant Roll-On

Anwendungsbeispiele 335-339:

| Phase A | Zusatz | INCI | Anbsp. 335 | Anbsp. 336 | Anbsp. 337 | Anbsp. 338 | Anbsp. 339 |
|---|---|---|---|---|---|---|---|
| | Natrosol 250 HR | Hydroxymethyl-cellulose | 0,4 | 0,2 | 0,3 | 0,4 | 0,3 |
| | Wasser dem. | Water | 30 | 30 | 30 | 30 | 30 |
| Phase B | Cremophor CO 40 | PEG-40 Hydrogenated Castor Oil | 2 | 2,5 | 3 | 3,5 | 3 |
| | Bisabolol rac | Bisabolol | 0,1 | 0,1 | 0,1 | 0,1 | 0,1 |
| | Farnesol | Farnesol | 0,3 | 0,2 | 0,3 | 0,1 | 0,3 |
| | Parfüm | Parfume | 0,1 | 0,2 | 0,2 | 0,1 | 0,3 |
| | Wasser dem. | Water | ad 100 | ad 100 | ad 100 | ad 100 | ad 100 |
| | Ethanol 96% | Alcohol | 25 | 30 | 35 | 30 | 32 |
| | Isoalkangemisch aus Beispiel 2 | | 2 | 5 | 7 | 5 | 6 |
| | | | | | | | |
| Phase C | 1,2 Propylene Glycol Care | Propylene Glycol | 3 | 2 | 2 | 3 | 2,5 |
| | Luviquat FC 370 | Polyquaternium-16 | 3 | 2,5 | 2 | 3,5 | 4 |
| | Allantoin | Allantoin | 0,1 | 0,1 | 0,1 | 0,1 | 0,1 |

(fortgesetzt)

| Phase A | Zusatz | INCI | Anbsp. 335 | Anbsp. 336 | Anbsp. 337 | Anbsp. 338 | Anbsp. 339 |
|---|---|---|---|---|---|---|---|
| | Locron L | Aluminum Chlorohydrate | 5 | 5,5 | 7,5 | 6 | 5,5 |
| Anbsp. Anwendungsbeispiel | | | | | | | |

Zur Herstellung des Antiperspirant Roll-On ließ man Phase A quellen; danach löste man Phase B und Phase C getrennt. Man rührte die Lösungen der Phasen B und C in Phase A ein.

Anwendungsbeispiele 340-344 beziehungsweise Anwendungsbeispiele 345-349: Man wiederholte Anwendungsbeispiele 335-339, setzte aber anstelle des Isoalkangemischs aus Beispiel 2 das Isoalkangemisch aus Beispiel 3 beziehungsweise aus Beispiel 4 ein.

Anwendungsbeispiele 350-364: Emulsion

Anwendungsbeispiele 350-354:

| Phase A | Zusatz | INCI | Anbsp. 350 | Anbsp. 351 | Anbsp. 352 | Anbsp. 353 | Anbsp. 354 |
|---|---|---|---|---|---|---|---|
| | Abil EM 97 | Sodium Laureth Sulfate | 3 | 3,5 | 4 | 3 | 3,5 |
| | Isoalkangemisch aus Beispiel 2 | Hydrogenated Polyisobutene | 15 | 10 | 20 | 5 | 17 |
| | Luvitol EHO | Cetearyl Ethylhexanoat e | 1 | 1 | 0,5 | 5 | 3 |
| | | | | | | | |
| Phase B | Wasser dem. | Water | ad 100 | ad 100 | ad 100 | ad 100 | ad 100 |
| | Ethanol 96% | Alcohol | 5 | 7 | 5 | 7,5 | 6 |
| | 1,2 Propylene | Propylene | 15 | 17 | 17 | 20 | 15 |
| | Glycol Care | Glycol | | | | | |
| | Locron L | Aluminum Chlorohydrate | 50 | 50 | 50 | 50 | 50 |
| Anbsp. Anwendungsbeispiel | | | | | | | |

Zur Herstellung der Antiperspirant Emulsion vermischte man die Phasen A und B gründlich. Danach rührte man die Phase B in die Phase A und homogenisierte.

Anwendungsbeispiele 355-359 beziehungsweise Anwendungsbeispiele 360-364: Man wiederholte Anwendungsbeispiele 350-354, setzte aber anstelle des Isoalkangemischs aus Beispiel 2 das Isoalkangemisch aus Beispiel 3 beziehungsweise aus Beispiel 4 ein.

Anwendungsbeispiele 365-379: Deo Stick

Anwendungsbeispiele 365-369:

| Phase A | Zusatz | INCI | Anbsp. 365 | Anbsp. 366 | Anbsp. 367 | Anbsp. 368 | Anbsp. 369 |
|---|---|---|---|---|---|---|---|
| | Stearylalkohol | Stearyl Alcohol | 26 | 30 | 28 | 27 | 26 |
| | Isoalkangemisch aus Beispiel 2 | | 60 | 51 | 58 | 43,5 | 56 |
| | Cremophor CO 40 | PEG-40 Hydrogenated Castor Oil | 5 | 10 | 2,5 | 5 | 5 |
| | Isopropylpalmitat | Isopropyl Palmitate | 2,5 | 3 | 1 | 4 | 2,5 |
| | | | | | | | |
| Phase B | | | | | | | |
| | Locron L | Aluminum Chlorohydrate | 5 | 5 | 10 | 20 | 15 |
| | Parfüm | Fragnance | 1,45 | 1 | 0,5 | 0,5 | 0,5 |
| | BHT | BHT | 0,05 | | | | |
| Anbsp. Anwendungsbeispiel | | | | | | | |

Zur Herstellung des Deosticks wog man die Komponenten der Phase A ein und shmolz sie; unter Rühren ließ man die Phase A auf 50 °C abkühlen; danach gab man die Komponenten der Phase B nacheinander zu und homogenisierte. Man goss das geschmolzene Gemisch in geeignete Formen.

Anwendungsbeispiele 370-374 beziehungsweise Anwendungsbeispiele 375-379: Man wiederholte Anwendungsbeispiele 365-369, setzte aber anstelle des Isoalkangemischs aus Beispiel 2 das Isoalkangemisch aus Beispiel 3 beziehungsweise aus Beispiel 4 ein.

Anwendungsbeispiele 380-391: Sonnenschutzgelcreme:

Anwendungsbeispiele 380-383:

| Zusatz | Anbsp. 380 | Anbsp. 381 | Anbsp. 382 | Anbsp. 383 |
|---|---|---|---|---|
| Acrylat/C10-30 Alkylacrylat Crosspolymer | 0,40 | 0,35 | 0,40 | 0,35 |
| Polyacrylsäure | 0,20 | 0,22 | 0,20 | 0,22 |
| Xanthan Gummi | 0,10 | 0,13 | 0,10 | 0,13 |
| Cetearylalkohol | 3,00 | 2,50 | 3,00 | 2,50 |
| C12-15 Alkylbenzoat | 4,00 | 4,50 | 4,00 | 4,50 |
| Caprylic/Capric Triglycerid | 3,00 | 3,50 | 3,00 | 3,50 |
| Uvinul A Plus | 2,00 | 1,50 | 0,75 | 1,00 |
| UVASorb K2A | | 3,00 | | |
| Ethylhexylmethoxycinnamat | 3,00 | | 1,00 | |
| Bis-Ethylhexyloxyphenol methoxyphenyl Triazin | | 1,50 | | 2,00 |
| Butylmethoxydibenzoylmethan | | | 2,00 | |
| Dinatrium Phenyl Dibenzimidazol Tetrasulfonat | 2,50 | | 0,50 | 2,00 |
| Ethyhexyl Triazon | 4,00 | | 3,00 | 4,00 |
| Octocrylen | | 4,00 | | |
| Diethylhexyl Butamido Triazon | 1,00 | | | 2,00 |

(fortgesetzt)

| Zusatz | Anbsp. 380 | Anbsp. 381 | Anbsp. 382 | Anbsp. 383 |
|---|---|---|---|---|
| Phenylbenzimidazol Sulfonsäure | 0,50 | | 3,00 | |
| Methylen Bis-Benzotriazolyl Tetramethylbutylphenol | 2,00 | | 0,50 | 1,50 |
| Ethylhexylsalicylat | | | 3,00 | |
| Drometrizol Trisiloxan | | | 0,50 | |
| Terephthaliden Dicamphor Sulfonsäure | | 1,50 | | 1,00 |
| Diethylhexyl-2,6-naphthalat | | | 7,00 | |
| mikrofeines Titandioxid | 6,00 | | 3,00 | |
| mikrofeines Zinkoxid | | 9,00 | | 5,25 |
| Isoalkangemisch aus Beispiel 2 | 10,30 | 5,00 | 4,00 | 8,00 |
| Cyclisches Dimethylpolysiloxan | 5,00 | 5,50 | 5,00 | 5,50 |
| Dimethicon Polydimethylsiloxan | 1,00 | 0,60 | 1,00 | 0,60 |
| Glycerin | 1,00 | 1,20 | 1,00 | 1,20 |
| Natriumhydoxid | q.s. | q.s. | q.s. | q.s. |
| Konservierungsmittel | 0,30 | 0,23 | 0,30 | 0,23 |
| Parfüm | 0,20 | | 0,20 | |
| Wasser | ad 100 | ad 100 | ad 100 | ad 100 |
| pH-Wert eingestellt auf 6,0 | | | | |
| Anbsp. Anwendungsbeispiel | | | | |

Anwendungsbeispiele 384-387 beziehungsweise Anwendungsbeispiele 388-391: Man wiederholte Anwendungsbeispiele 380-383, setzte aber anstelle des Isoalkangemischs aus Beispiel 2 das Isoalkangemisch aus Beispiel 3 beziehungsweise aus Beispiel 4 ein.

Anwendungsbeispiele 392-412: O/W-Sonnenschutzformulierung

Anwendungsbeispiele 392-398:

| Zusatz | Anbsp. 392 | Anbsp. 393 | Anbsp. 394 | Anbsp. 395 | Anbsp. 396 | Anbsp. 397 | Anbsp. 398 |
|---|---|---|---|---|---|---|---|
| Glycerinmonostearat SE | 0,50 | 1,00 | 3,00 | | | 1,50 | |
| Glycerl Stearat Citrat | 2,00 | | 1,00 | 2,00 | 4,00 | | |
| Stearinsäure | | 3,00 | | 2,00 | | | |
| PEG-40 Stearat | 0,50 | | | | | 2,00 | |
| Cetyl Phosphat | | | | | | 1,00 | |
| Cetearyl Sulfat | | | | | | | 0,75 |
| Stearyl Alkohol | | | 3,00 | | | 2,00 | 0,60 |
| Cetyl Alkohol | 2,50 | 1,10 | | 1,50 | 0,60 | | 2,00 |
| Isoalkangemisch aus Beispiel 2 | 2,00 | 5,00 | 7,00 | 10,00 | 8,00 | 5,50 | 1,00 |
| Uvinul A Plus | 2,00 | 1,50 | 0,75 | 1,00 | 2,10 | 4,50 | 5,00 |

(fortgesetzt)

| Zusatz | Anbsp. 392 | Anbsp. 393 | Anbsp. 394 | Anbsp. 395 | Anbsp. 396 | Anbsp. 397 | Anbsp. 398 |
|---|---|---|---|---|---|---|---|
| UVASorb K2A | | | | | | | |
| Ethylhexylmethoxycinnamat | | | | | 5,00 | 6,00 | 8,00 |
| Bis-Ethylhexyloxyphenol methoxyphenyl Triazin | | 1,50 | | 2,00 | 2,50 | | 2,50 |
| Butyl Methoxydibenzoylmethan | | | 2,00 | | 2,00 | 1,50 | |
| Dinatrium Phenyl Dibenzimidazol Tetrasulfonat | 2,50 | | 0,50 | 2,00 | | 0,30 | |
| Ethyhexyl Triazon | 4,00 | | 3,00 | 4,00 | | 2,00 | |
| Octocrylen | | 4,00 | | | | | 7,50 |
| Diethylhexyl Butamido Triazon | 1,00 | | | 2,00 | 1,00 | | 1,00 |
| Phenylbenzimidazol Sulfonsäure | 0,50 | | 3,00 | | | | |
| Methylen Bis-Benzotriazolyl Tetramethylbutylphenol | 2,00 | | 0,50 | 1,50 | 2,50 | | |
| Ethylhexysalicylat | | | 3,00 | | | | 5,00 |
| Drometrizol Trisiloxan | | | 0,50 | | | 1,00 | |
| Terephthaliden Dicamphor Sulfonsäure | | 1,50 | | 1,00 | 1,00 | | 0,50 |
| Diethylhexyl-2,6-naphthalat | 3,50 | | 7,00 | | 3,50 | 4,00 | |
| mikrofeines Titandioxid | 1,00 | | 3,00 | | 3,50 | | 1,50 |
| mikrofeines Zinkoxid | | | | 0,25 | | 2,00 | |
| $C_{12-15}$-Alkyl Benzoat | | 0,25 | | | 4,00 | 7,00 | |
| Dicapryl Ether | | | 3,50 | | 2,00 | | |
| Butylenglycol Dicaprylat/Dicaprat | 5,00 | | 6,00 | | | | |
| Cocoglyceride | | | 6,00 | | 2,00 | | |
| Dimethicon | 0,50 | | 1,00 | | 2,00 | | |
| Cyclomethicon | 2,00 | | 0,50 | | 0,50 | | |
| Shea Butter | | 2,00 | | | | | |
| PVP Hexadecen Copolymer | 0,20 | | | 0,50 | | 1,00 | |
| Glycerin | 3,00 | 7,50 | | 7,50 | 5,00 | | 2,50 |
| Xanthan Gummi | 0,15 | | 0,05 | | | 0,30 | |
| Sodium Carbomer | | 0,20 | | 0,15 | 0,25 | | |
| Vitamin E Acetat | 0,60 | | 0,23 | | 0,70 | 1,00 | |
| Glycin Soja | | | | 0,50 | | 1,50 | 1,00 |
| Ethylhexyloxyglycin | 0,30 | | | | | | |
| DMDM Hydantoin | | 0,60 | 0,40 | 0,20 | | | |

(fortgesetzt)

| Zusatz | Anbsp. 392 | Anbsp. 393 | Anbsp. 394 | Anbsp. 395 | Anbsp. 396 | Anbsp. 397 | Anbsp. 398 |
|---|---|---|---|---|---|---|---|
| Glyacil-L | | | | 0,18 | 0,20 | | |
| Methylparaben | 0,15 | | 0,25 | | 0,50 | | |
| Phenoxyethanol | 1,00 | 0,40 | | | 0,40 | 0,50 | 0,40 |
| Trinatrium EDTA | 0,02 | | 0,05 | | | | |
| Iminobernsteinsäure | | | | 0,25 | 1,00 | | |
| Ethanol | 2,00 | 1,50 | | 3,00 | | 1,20 | 5,00 |
| Parfüm | 0,10 | 0,25 | 0,30 | | 0,40 | 0,20 | |
| Wasser | ad 100 | ad 100 | ad 100 | ad 100 | ad 100 | ad100 | ad 100 |
| Anbsp. Anwendungsbeispiel | | | | | | | |

Anwendungsbeispiele 399-405 beziehungsweise Anwendungsbeispiele 406-412: Man wiederholte Anwendungsbeispiele 392-398, setzte aber anstelle des Isoalkangemischs aus Beispiel 2 das Isoalkangemisch aus Beispiel 3 beziehungsweise aus Beispiel 4 ein.

Anwendungsbeispiele 414-428: Sonnenschutzhydrodispersionen

Anwendungsbeispiele 414-418:

| Zusatz | Anbsp. 414 | Anbsp. 415 | Anbsp. 416 | Anbsp. 417 | Anbsp. 418 |
|---|---|---|---|---|---|
| Ceteareth-20 | 1,00 | | | 0,50 | |
| Cetyl Alkohol | | | 1,00 | | |
| Sodium Carbomer | | 0,20 | | 0,30 | |
| Acrylat/C10-30 Alkyl Acrylat Crosspolymer | 0,50 | | 0,40 | 0,10 | 0,50 |
| Xanthan Gummi | | 0,30 | 0,15 | | |
| Isoalkangemisch aus Beispiel 2 | 3,00 | 6,00 | 2,00 | 6,50 | 8,90 |
| Uvinul A Plus | 2,00 | 1,50 | 0,75 | 1,00 | 2,10 |
| UVASorb K2A | | 2,00 | | | |
| Ethylhexylmethoxycinnamat | | | | | 5,00 |
| Bis-Ethylhexyloxyphenol methoxyphenyl Triazin | | 1,50 | | 2,00 | 2,50 |
| Butyl Methoxydibenzoylmethan | | | 2,00 | | 2,00 |
| Dinatrium Phenyl Dibenzimidazol Tetrasulfonat | 2,50 | | 0,50 | 2,00 | |
| Ethyhexyl Triazon | 4,00 | | 3,00 | 4,00 | |
| Octocrylen | | 4,00 | | | |
| Diethylhexyl Butamido Triazon | 1,00 | | | 2,00 | 1,00 |
| Phenylbenzimidazol Sulfonsäure | 0,50 | | 3,00 | | |
| Methylen Bis-Benzotriazolyl Tetramethylbutylphenol | 2,00 | | 0,50 | 1,50 | 2,50 |

(fortgesetzt)

| Zusatz | Anbsp. 414 | Anbsp. 415 | Anbsp. 416 | Anbsp. 417 | Anbsp. 418 |
|---|---|---|---|---|---|
| Ethylhexysalicylat | | | 3,00 | | |
| Drometrizol Trisiloxan | | | 0,50 | | |
| Terephthaliden Dicamphor Sulfonsäure | | 1,50 | | 1,00 | 1,00 |
| Diethylhexyl-2,6-naphthalat | | | 7,00 | | 9,50 |
| mikrofeines Titandioxid | 1,00 | | 3,00 | | 3,50 |
| mikrofeines Zinkoxid | | | | 0,25 | |
| $C_{12-15}$-Alkyl Benzoat | 2,00 | 2,50 | | | |
| Dicapryl Ether | | 4,00 | | | |
| Butylenglycol Dicaprylat/Dicaprat | 4,00 | | 2,00 | 6,00 | |
| Dicapryl Carbonat | | 2,00 | 6,00 | | |
| Dimethicon | | 0,50 | 1,00 | | |
| Phenyltrimethicon | 2,00 | | 0,50 | | |
| Shea Butter | | 2,00 | | 5,00 | |
| PVP Hexadecen Copolymer | 0,50 | | | 0,50 | 1,00 |
| Tricontanyl PVP | 0,50 | | 1,00 | | |
| Ethylhexylglycerin | | | 1,00 | | 0,80 |
| Glycerin | 3,00 | 7,50 | | 7,50 | 8,50 |
| Glycin Soja | | | 1,50 | | 1,00 |
| Vitamin E Acetat | 0,50 | | 0,25 | | 1,00 |
| Alpha-Glucosilrutin | 0,60 | | | 0,25 | |
| DMDM Hydantoin | | 0,60 | 0,45 | 0,25 | |
| Glyacil-S | 0,20 | | | | |
| Methylparaben | 0,50 | | 0,25 | 0,15 | |
| Phenoxyethanol | 0,50 | 0,40 | | 1,00 | |
| Trinatrium EDTA | | 0,01 | 0,05 | | 0,10 |
| Ethanol | 3,00 | 2,00 | 1,50 | | 7,00 |
| Parfüm | 0,20 | | 0,05 | 0,40 | |
| Wasser | ad 100 | ad 100 | ad 100 | ad 100 | ad 100 |
| Anbsp. Anwendungsbeispiel | | | | | |

Anwendungsbeispiele 419-423 beziehungsweise Anwendungsbeispiele 424-428: Man wiederholte Anwendungsbeispiele 414-418, setzte aber anstelle des Isoalkangemischs aus Beispiel 2 das Isoalkangemisch aus Beispiel 3 beziehungsweise aus Beispiel 4 ein.

Anwendungsbeispiele 429-443: W/O-Sonnenschutzformulierungen

Anwendungsbeispiele 429-433:

| Zusatz | Anbsp. 429 | Anbsp. 430 | Anbsp. 431 | Anbsp. 432 | Anbsp. 433 |
|---|---|---|---|---|---|
| Cetyldimethicon Copolyol | | 2,50 | | 4,00 | |
| Polyglyceryl-2-dipolyhydroxystearat | 5,00 | | | | 4,50 |
| PEG-30-dipolyhydroxystearat | | | 5,00 | | |
| Isoalkangemisch aus Beispiel 2 | 1,00 | 8,00 | 9,50 | 3,00 | 9,00 |
| Uvinul A Plus | 2,00 | 1,50 | 0,75 | 1,00 | 2,10 |
| UVASorb K2A | | | 1,50 | | |
| Ethylhexylmethoxycinnamat | | | | | 5,00 |
| Bis-Ethylhexyloxyphenol methoxyphenyl Triazin | | 1,50 | | 2,00 | 2,50 |
| Butyl Methoxydibenzoylmethan | | | 2,00 | | 2,00 |
| Dinatrium Phenyl Dibenzimidazol Tetrasulfonat | 2,50 | | 0,50 | 2,00 | |
| Ethyhexyl Triazon | 4,00 | | 3,00 | 4,00 | |
| Octocrylen | | 4,00 | | | |
| Diethylhexyl Butamido Triazon | 1,00 | | | 2,00 | 1,00 |
| Phenylbenzimidazol Sulfonsäure | 0,50 | | 3,00 | | |
| Methylen Bis-Benzotriazolyl Tetramethylbutylphenol | 2,00 | | 0,50 | 1,50 | 2,50 |
| Ethylhexysalicylat | | | 3,00 | | |
| Drometrizol Trisiloxan | | | 0,50 | | |
| Terephthaliden Dicamphor Sulfonsäure | | 1,50 | | 1,00 | 1,00 |
| Diethylhexyl-2,6-naphthalat | | | 7,00 | | 3,50 |
| mikrofeines Titandioxid | 1,00 | | 3,00 | | 3,50 |
| mikrofeines Zinkoxid | | | | 0,25 | |
| Mineralöl | | 12,00 | 10,00 | | 8,00 |
| C12-15 Alkyl Benzoat | | | | 9,00 | |
| Dicaprylyl Ether | 10,00 | | | | 7,00 |
| Butylenglycol Dicaprylat/Dicaprat | | | 2,00 | 8,00 | 4,00 |
| Dicaprylyl Carbonat | 5,00 | | 6,00 | | |
| Dimethicon | | 4,00 | 1,00 | 5,00 | |
| Cyclomethicon | 2,00 | 25,00 | | | 2,00 |
| Shea Butter | | | 3,00 | | |
| Vaseline | | 4,50 | | | |
| PVP Hexadecen Copolymer | 0,50 | | | 0,50 | 1,00 |
| Ethylhexylglycerin | | 0,30 | 1,00 | | 0,50 |
| Glycerin | 3,00 | 7,50 | | 7,50 | 8,50 |
| Glycin Soja | | 1,00 | 1,50 | | 1,00 |
| $MgSO_4$ | 1,00 | 0,50 | | 0,50 | |
| $MgCl_2$ | | | 1,00 | | 0,70 |

(fortgesetzt)

| Zusatz | Anbsp. 429 | Anbsp. 430 | Anbsp. 431 | Anbsp. 432 | Anbsp. 433 |
|---|---|---|---|---|---|
| Vitamin E Acetat | 0,50 | | 0,25 | | 1,00 |
| Ascorbyl Palmitat | 0,50 | | | 2,00 | |
| DMDM Hydantoin | | 0,60 | 0,40 | 0,20 | |
| Methylparaben | 0,50 | | 0,25 | 0,15 | |
| Phenoxyethanol | 0,50 | 0,40 | | 1,00 | |
| Trinatrium EDTA | 0,12 | 0,05 | | 0,30 | |
| Ethanol | 3,00 | | 1,50 | | 5,00 |
| Parfüm | 0,20 | | 0,40 | 0,35 | |
| Wasser | ad 100 | ad 100 | ad 100 | ad 100 | ad 100 |
| Anbsp. Anwendungsbeispiel | | | | | |

Anwendungsbeispiele 434-438 beziehungsweise Anwendungsbeispiele 439-443: Man wiederholte Anwendungsbeispiele 429-433, setzte aber anstelle des Isoalkangemischs aus Beispiel 2 das Isoalkangemisch aus Beispiel 3 beziehungsweise aus Beispiel 4 ein.

Anwendungsbeispiele 444-458: Feststoffstabilisierte Emulsionen

Anwendungsbeispiele 444-448:

| Zusatz | Anbsp. 444 | Anbsp. 445 | Anbsp. 446 | Anbsp. 447 | Anbsp. 448 |
|---|---|---|---|---|---|
| Mineralöl | | | 16,00 | 16,00 | |
| Octyldodecanol | 9,00 | 9,00 | 5,00 | | |
| Caprylic/Capric Triglycerid | 9,00 | 9,00 | 6,00 | | |
| C12-15 Alkyl Benzoat | | | | 5,00 | 8,00 |
| Butylen Glycol Dicaprylat/Dicaprat | | | | | 8,00 |
| Dicaprylyl Ether | 9,00 | | | 4,00 | |
| Dicaprylyl Carbonat | | 9,00 | | | |
| Hydroxyoctacosanyl Hydroxystearat | 2,00 | 2,00 | 2,20 | 2,50 | 1,50 |
| Disteardimonium Hectorit | 1,00 | 0,75 | | 0,50 | 0,25 |
| Cera Microcristallina + Paraffinum Liquidum | | 0,35 | | | 5,00 |
| Hydroxypropyl Methylcellulose | | | 0,10 | | 0,05 |
| Dimethicon | | | | | 3,00 |
| Isoalkangemisch aus Beispiel 2 | 3,00 | 5,00 | 5,50 | 8,50 | 2,00 |
| Uvinul A Plus | 2,00 | 1,50 | 0,75 | 1,00 | 2,10 |
| UVASorb K2A | | | | | |
| Ethylhexylmethoxycinnamat | | | | | 5,00 |
| Bis-Ethylhexyloxyphenol methoxyphenyl Triazin | | 1,50 | | 2,00 | 2,50 |
| Butyl Methoxydibenzoylmethan | | | 2,00 | | 2,00 |

(fortgesetzt)

| Zusatz | Anbsp. 444 | Anbsp. 445 | Anbsp. 446 | Anbsp. 447 | Anbsp. 448 |
|---|---|---|---|---|---|
| Dinatrium Phenyl Dibenzimidazol Tetrasulfonat | 2,50 | | 0,50 | 2,00 | |
| Ethyhexyl Triazon | 4,00 | | 3,00 | 4,00 | |
| Octocrylen | | 4,00 | | | |
| Diethylhexyl Butamido Triazon | 1,00 | | | 2,00 | 1,00 |
| Phenylbenzimidazol Sulfonsäure | 0,50 | | 3,00 | | |
| Methylen Bis-Benzotriazolyl Tetramethylbutylphenol | 2,00 | | 0,50 | 1,50 | 2,50 |
| Ethylhexysalicylat | | | 3,00 | | |
| Drometrizol Trisiloxan | | | 0,50 | | |
| Terephthaliden Dicamphor Sulfonsäure | | 1,50 | | 1,00 | 1,00 |
| Diethylhexyl-2,6-naphthalat | | | 7,00 | | 10,00 |
| mikrofeines Titandioxid | 1,00 | | 3,00 | | 3,50 |
| mikrofeines Zinkoxid | | | | 0,25 | |
| Titandioxid + Alumina + Simethicon + Aqua | | 3,0 | | 4,0 | |
| Titandioxid + Trimethoxycaprylylsilan | | 2,00 | 4,00 | 2,00 | 4,00 |
| Silica Dimethyl Silylat | 2,50 | | | 6,00 | 2,50 |
| Bornitrid | | | 1,00 | | |
| Stärke/-Natriummetaphosphat-Polymer | 2,00 | | | | |
| Tapioca Stärke | | 0,50 | | | |
| Natriumchlorid | 5,00 | 7,00 | 8,50 | 3,00 | 4,50 |
| Glycerin | | | | 1,00 | |
| Trinatrium EDTA | 1,00 | 1,00 | 1,00 | 1,00 | 1,00 |
| Vitamin E Acetat | 5,00 | 10,00 | 3,00 | 6,00 | 10,00 |
| Ascorbyl Palmitat | 1,00 | 1,00 | | 1,00 | |
| Methylparaben | | 0,60 | | | 0,20 |
| Propylparaben | | | | | 0,20 |
| Phenoxyethanol | | | 0,20 | | |
| Hexamidin Diisethionat | | | 0,40 | 0,50 | 0,40 |
| Diazolidinyl Harnstoff | | | | | 0,08 |
| Ethanol | | | 0,23 | 0,20 | |
| Parfüm | 5,00 | | 3,00 | 4,00 | |
| Wasser | ad 100 | ad 100 | ad 100 | ad 100 | ad 100 |
| Anbsp. Anwendungsbeispiel | | | | | |

Anwendungsbeispiele 449-453 beziehungsweise Anwendungsbeispiele 454-458: Man wiederholte Anwendungsbeispiele 444-448, setzte aber anstelle des Isoalkangemischs aus Beispiel 2 das Isoalkangemisch aus Beispiel 3 beziehungsweise aus Beispiel 4 ein.

Anwendungsbeispiele 459-470: Sonnenschutzstifte

Anwendungsbeispiele 459-462:

| Zusatz | Anbsp. 459 | Anbsp. 460 | Anbsp. 461 | Anbsp. 462 |
|---|---|---|---|---|
| Caprylic/Capric Triglycerid | 12,00 | 10,00 | 6,00 | |
| Octyldodecanol | 7,00 | 14,00 | 8,00 | 3,00 |
| Butylene Glycol Dicaprylat/Dicaprat | | | | 12,00 |
| Pentaerythrityl Tetraisostearat | 10,00 | 6,00 | 8,00 | 7,00 |
| Polyglyceryl-3 Diisostearat | 2,50 | | | |
| Bis-Diglyceryl Polyacyladipate-2 | 9,00 | 8,00 | 10,00 | 8,00 |
| Cetearyl Alcohol | 8,00 | 11,00 | 9,00 | 7,00 |
| Myristyl Myristate | 3,50 | 3,00 | 4,00 | 3,00 |
| Beeswax | 5,00 | 5,00 | 6,00 | 6,00 |
| Cera Carnauba | 1,50 | 2,00 | 2,00 | 1,50 |
| Cera Alba | 0,50 | 0,50 | 0,50 | 0,40 |
| C16-40 Alkyl Stearat | | 1,50 | 1,50 | 1,50 |
| Isoalkangemisch aus Beispiel 2 | 2,00 | 3,00 | 6,50 | 12,00 |
| Uvinul A Plus | 2,00 | 1,50 | 0,75 | 9,00 |
| UVASorb K2A | | 2,00 | | 4,00 |
| Ethylhexylmethoxycinnamat | | 3,00 | | |
| Bis-Ethylhexyloxyphenol methoxyphenyl Triazin | | 1,50 | | 2,00 |
| Butyl Methoxydibenzoylmethan | | | 2,00 | |
| Dinatrium Phenyl Dibenzimidazol | 2,50 | | 0,50 | 2,00 |
| Tetrasulfonat | | | | |
| Ethyhexyl Triazon | 4,00 | | 3,00 | 4,00 |
| Octocrylen | | 4,00 | | |
| Diethylhexyl Butamido Triazon | 1,00 | | | 2,00 |
| Phenylbenzimidazol Sulfonsäure | 0,50 | | 3,00 | |
| Methylen Bis-Benzotriazolyl Tetramethylbutylphenol | 2,00 | | 0,50 | 1,50 |
| Ethylhexysalicylat | | | 3,00 | |
| Drometrizol Trisiloxan | | | 0,50 | |
| Terephthaliden Dicamphor Sulfonsäure | | 1,50 | | 1,00 |
| Diethylhexyl-2,6-naphthalat | | | 7,00 | |
| mikrofeines Titandioxid | 1,00 | | 3,00 | |
| mikrofeines Zinkoxid | | | | 0,25 |
| Vitamin E Acetat | 0,50 | 1,00 | | |
| Ascorbyl Palmitat | 0,05 | | 0,05 | |
| Buxux Chinensis | 2,00 | 1,00 | | 1,00 |
| Parfüm, BHT | 0,10 | 0,25 | | 0,35 |

(fortgesetzt)

| Zusatz | Anbsp. 459 | Anbsp. 460 | Anbsp. 461 | Anbsp. 462 |
|---|---|---|---|---|
| Ricinus Communis | ad 100 | ad 100 | ad 100 | ad 100 |
| Anbsp. Anwendungsbeispiel | | | | |

Anwendungsbeispiele 463-466 beziehungsweise Anwendungsbeispiele 467-470: Man wiederholte Anwendungsbeispiele 459-462, setzte aber anstelle des Isoalkangemischs aus Beispiel 2 das Isoalkangemisch aus Beispiel 3 beziehungsweise aus Beispiel 4 ein.

Anwendungsbeispiele 471-494: PIT-Sonnenschutzemulsionen

Anwendungsbeispiele 471-478:

| Zusatz | Anbsp. 471 | Anbsp. 472 | Anbsp. 473 | Anbsp. 474 | Anbsp. 475 | Anbsp. 476 | Anbsp. 477 | Anbsp. 478 |
|---|---|---|---|---|---|---|---|---|
| Glycerinmonostearat SE | 0,50 | 2,00 | 3,00 | 5,00 | | 0,50 | 4,00 | |
| Glyceryl Isostearat | | | | | 3,50 | 4,00 | 2,00 | |
| Isoceteth-20 | | 0,50 | | | 2,00 | | | |
| Ceteareth-12 | | 5,00 | | 1,00 | | | 3,50 | 5,00 |
| Ceteareth-20 | | 5,00 | | 1,00 | | | | 3,50 |
| PEG-100 Stearat | | | | 2,80 | | 2,30 | 3,30 | |
| Cetyl Alkohol | 5,20 | | 1,20 | 1,00 | 1,30 | | 0,50 | 0,30 |
| Cetyl Palmitat | 2,50 | 1,20 | | 1,50 | | 0,50 | | 1,50 |
| Cetyl Dimethicon Copolyol | | | | 0,50 | | 1,00 | | |
| Polyglyceryl-2 | | | | 0,75 | 0,30 | | | |
| Isoalkangemisch aus Beispiel 2 | 2,00 | 5,00 | 7,90 | 15,00 | 1,00 | 5,50 | 12,5 | 5,00 |
| Uvinul A Plus | 2,00 | 1,50 | 0,75 | 1,00 | 2,10 | 4,50 | | 2,10 |
| UVASorb K2A | | | 3,50 | | | | | 1,20 |
| Ethylhexylmethoxycinnamat | | | | | 5,00 | 6,00 | 8,00 | 5,00 |
| Bis-Ethylhexyloxyphenol methoxyphenyl Triazin | | 1,50 | | 2,00 | 2,50 | | 2,50 | 2,50 |
| Butyl Methoxydibenzoylmethan | | | 2,00 | | 2,00 | 1,50 | | 2,00 |
| Dinatrium Phenyl Dibenzimidazol Tetrasulfonat | 2,50 | | 0,50 | 2,00 | | 0,30 | | |
| Ethyhexyl Triazon | 4,00 | | 3,00 | 4,00 | | 2,00 | | |
| Octocrylen | | 4,00 | | | | | 7,50 | |
| Diethylhexyl Butamido Triazon | 1,00 | | | 2,00 | 1,00 | | 1,00 | 1,00 |
| Phenylbenzimidazol Sulfonsäure | 0,50 | | 3,00 | | | | | |
| Methylen Bis-Benzotriazolyl Tetramethylbutylphenol | 2,00 | | 0,50 | 1,50 | 2,50 | | | 2,50 |
| Ethylhexysalicylat | | | 3,00 | | | | 5,00 | |
| Drometrizol Trisiloxan | | | 0,50 | | | 1,00 | | |
| Terephthaliden Dicamphor Sulfonsäure | | 1,50 | | 1,00 | 1,00 | | 0,50 | 1,00 |

(fortgesetzt)

| Zusatz | Anbsp. 471 | Anbsp. 472 | Anbsp. 473 | Anbsp. 474 | Anbsp. 475 | Anbsp. 476 | Anbsp. 477 | Anbsp. 478 |
|---|---|---|---|---|---|---|---|---|
| Diethylhexyl-2,6-naphthalat | | | 7,00 | | 9,50 | 8,00 | | 8,00 |
| mikrofeines Titandioxid | 1,00 | | 3,00 | | 3,50 | | 1,50 | 3,50 |
| mikrofeines Zinkoxid | | | | 0,25 | | 2,00 | | |
| C12-15 Alkyl Benzoat | 3,50 | | | 6,35 | | | | 0,10 |
| Cocoglyceride | | 3,00 | | 3,00 | | | | 1,00 |
| Dicapryl Ether | 4,50 | | | | | | | |
| Dicaprylyl Carbonat | | 4,30 | | 3,00 | | | | 7,00 |
| Dibutyl Adipate | | | | 0,50 | | | | 0,30 |
| Phenyltrimethicone | 2,00 | | | 3,50 | | 2,00 | | |
| Cyclomethicon | | 3,00 | | | | | | |
| Ethyl Galaktomannan | | 0,50 | | | 2,00 | | | |
| Hydrierte Coco-Glyceride | | | | | 3,00 | 4,00 | | |
| Abil Wax 2440 | | | | | | 1,50 | 2,00 | |
| PVP Hexadecen Copolymer | | | | 1,00 | 1,20 | | | |
| Glycerin | 4,00 | 6,00 | 5,00 | | 8,00 | 10,00 | | |
| Vitamin E Acetat | 0,20 | 0,30 | 0,40 | | 0,30 | | | |
| Shea Butter | | 2,00 | | 3,60 | | 2,00 | | |
| Iodopropyl Butylcarbamat | 0,12 | | | | 0,20 | | | |
| DMDM Hydantoin | 0,10 | | | | 0,12 | | 0,13 | |
| Methylparaben | | 0,50 | 0,30 | | 0,35 | | | |
| Phenoxyethanol | 0,50 | 0,40 | | 1,00 | | | | |
| Octoxyglycerin | | 0,30 | | | 1,00 | | 0,35 | |
| Ethanol | 2,00 | | 2,00 | | | 5,00 | | |
| Trinatrium EDTA | 0,40 | | 0,15 | | | 0,20 | | |
| Parfüm | 0,20 | | 0,20 | | 0,24 | 0,16 | 0,10 | 0,10 |

(fortgesetzt)

| Zusatz | Anbsp. 471 | Anbsp. 472 | Anbsp. 473 | Anbsp. 474 | Anbsp. 475 | Anbsp. 476 | Anbsp. 477 | Anbsp. 478 |
|---|---|---|---|---|---|---|---|---|
| Wasser | ad 100 | ad 100 | ad 100 | ad 100 | ad 100 | ad100 | ad 100 | ad 100 |
| Anbsp. Anwendungsbeispiel | | | | | | | | |

Anwendungsbeispiele 479-486 beziehungsweise Anwendungsbeispiele 487-494: Man wiederholte Anwendungsbeispiele 471-478, setzte aber anstelle des Isoalkangemischs aus Beispiel 2 das Isoalkangemisch aus Beispiel 3 beziehungsweise aus Beispiel 4 ein.

Anwendungsbeispiele 495-506: Gelcreme

Anwendungsbeispiele 495-498:

| Zusatz | Anbsp. 495 | Anbsp. 496 | Anbsp. 497 | Anbsp. 498 |
|---|---|---|---|---|
| Acrylat/C10-30 Alkylacrylat Crosspolymer | 0,40 | 0,35 | 0,40 | 0,35 |
| Polyacrylsäure | 0,20 | 0,22 | 0,20 | 0,22 |
| Luvigel EM | 1,50 | 2,50 | 2,80 | 3,50 |
| Xanthan Gummi | 0,10 | 0,13 | 0,10 | 0,13 |
| Cetearylalkohol | 3,00 | 2,50 | 3,00 | 2,50 |
| C12-15 Alkylbenzoat | 4,00 | 4,50 | 4,00 | 4,50 |
| Caprylic/Capric Triglycerid | 3,00 | 3,50 | 3,00 | 3,50 |
| mikrofeines Titandioxid | 1,00 | | 1,50 | |
| mikrofeines Zinkoxid | | 2,00 | | 0,25 |
| Isoalkangemisch aus Beispiel 2 | 2,00 | 5,00 | 8,00 | 7,50 |
| Dihydroxyaceton | | | 3,00 | 5,00 |
| Cyclisches Dimethylpolysiloxan | 5,00 | 5,50 | 5,00 | 5,50 |
| Dimethicon Polydimethylsiloxan | 1,00 | 0,60 | 1,00 | 0,60 |
| Glycerin | 1,00 | 1,20 | 1,00 | 1,20 |
| Natriumhydoxid | q.s. | q.s. | q.s. | q.s. |
| Konservierungsmittel | 0,30 | 0,23 | 0,30 | 0,23 |
| Parfüm | 0,20 | | 0,20 | |
| Wasser | ad 100 | ad 100 | ad 100 | ad 100 |
| pH-Wert eingestellt auf 6,0 | | | | |
| Anbsp. Anwendungsbeispiel | | | | |

Anwendungsbeispiele 499-502 beziehungsweise Anwendungsbeispiele 503-506: Man wiederholte Anwendungsbeispiele 495-498, setzte aber anstelle des Isoalkangemischs aus Beispiel 2 das Isoalkangemisch aus Beispiel 3 beziehungsweise aus Beispiel 4 ein.

Anwendungsbeispiele 507-527: Kosmetische O/W-Formulierungen

Anwendungsbeispiele 507-513:

| Zusatz | Anbsp. 507 | Anbsp. 508 | Anbsp. 509 | Anbsp. 510 | Anbsp. 511 | Anbsp. 512 | Anbsp. 513 |
|---|---|---|---|---|---|---|---|
| Glycerinmonostearat SE | 0,50 | 1,00 | 3,00 | | | 1,50 | |
| Glycerl Stearat Citrat | 2,00 | | 1,00 | 2,00 | 4,00 | | |

(fortgesetzt)

| Zusatz | Anbsp. 507 | Anbsp. 508 | Anbsp. 509 | Anbsp. 510 | Anbsp. 511 | Anbsp. 512 | Anbsp. 513 |
|---|---|---|---|---|---|---|---|
| Stearinsäure | | 3,00 | | 2,00 | | | |
| PEG-40 Stearat | 0,50 | | | | | 2,00 | |
| Cetyl Phosphat | | | | | | 1,00 | |
| Cetearyl Sulfat | | | | | | | 0,75 |
| Stearyl Alkohol | | | 3,00 | | | 2,00 | 0,60 |
| Cetyl Alkohol | 2,50 | 1,10 | | 1,50 | 0,60 | | 2,00 |
| Isoalkangemisch aus Beispiel 2 | 2,00 | 5,00 | 7,00 | 10,0 | 8,0 | 5,00 | 1,00 |
| Dihydroxyaceton | | | 3,00 | 5,00 | | 4 | |
| mikrofeines Titandioxid | 1,00 | | | | 1,50 | | 1,50 |
| mikrofeines Zinkoxid | | | | 0,25 | | 2,00 | |
| C12-15 Alkyl Benzoat | | 0,25 | | | 4,00 | 7,00 | |
| Dicapryl Ether | | | 3,50 | | 2,00 | | |
| Butylenglycol Dicaprylat/Dicaprat | 5,00 | | 6,00 | | | | |
| Cocoglyceride | | | 6,00 | | 2,00 | | |
| Dimethicon | 0,50 | | 1,00 | | 2,00 | | |
| Cyclomethicon | 2,00 | | 0,50 | | 0,50 | | |
| Shea Butter | | 2,00 | | | | | |
| PVP Hexadecen Copolymer | 0,20 | | | 0,50 | | 1,00 | |
| Glycerin | 3,00 | 7,50 | | 7,50 | 5,00 | | 2,50 |
| Xanthan Gummi | 0,15 | | 0,05 | | | 0,30 | |
| Sodium Carbomer | | 0,20 | | 0,15 | 0,25 | | |
| Vitamin E Acetat | 0,60 | | 0,23 | | 0,70 | 1,00 | |
| Fucogel 1000 | | 3,00 | 10,00 | | | | |
| Glycin Soja | | | | 0,50 | | 1,50 | 1,00 |
| Ethylhexyloxyglycin | 0,30 | | | | | | |
| DMDM Hydantoin | | 0,60 | 0,40 | 0,20 | | | |
| Glyacil-L | | | | 0,18 | 0,20 | | |
| Methylparaben | 0,15 | | 0,25 | | 0,50 | | |
| Phenoxyethanol | 1,00 | 0,40 | | | 0,40 | 0,50 | 0,40 |
| Trinatrium EDTA | 0,02 | | 0,05 | | | | |
| Iminobernsteinsäure | | | | 0,25 | 1,00 | | |
| Ethanol | 2,00 | 1,50 | | 3,00 | | 1,20 | 5,00 |
| Parfüm | 0,10 | 0,25 | 0,30 | | 0,40 | 0,20 | |

(fortgesetzt)

| Zusatz | Anbsp. 507 | Anbsp. 508 | Anbsp. 509 | Anbsp. 510 | Anbsp. 511 | Anbsp. 512 | Anbsp. 513 |
|---|---|---|---|---|---|---|---|
| Wasser | ad 100 | ad 100 | ad 100 | ad 100 | ad 100 | ad100 | ad 100 |
| Anbsp. Anwendungsbeispiel | | | | | | | |

Anwendungsbeispiele 514-520 beziehungsweise Anwendungsbeispiele 521-527: Man wiederholte Anwendungsbeispiele 507-513, setzte aber anstelle des Isoalkangemischs aus Beispiel 2 das Isoalkangemisch aus Beispiel 3 beziehungsweise aus Beispiel 4 ein.

Anwendungsbeispiele 528-542: Kosmetische Hydrodispersionsformulierungen

Anwendungsbeispiele 528-532:

| Zusatz | Anbsp. 528 | Anbsp. 529 | Anbsp. 530 | Anbsp. 531 | Anbsp. 532 |
|---|---|---|---|---|---|
| Ceteaereth-20 | 1,00 | | | 0,50 | |
| Cetyl Alkohol | | | 1,00 | | |
| Luvigel EM | | 2,00 | | 2,50 | 2,00 |
| Acrylat/C10-30 Alkyl Acrylat Crosspolymer | 0,50 | | 0,40 | 0,10 | 0,50 |
| Xanthan Gummi | | 0,30 | 0,15 | | |
| Isoalkangemisch aus Beispiel 2 | 3,00 | 6,00 | 2,00 | 4,00 | 7,00 |
| Dihydroxyaceton | | | 3,00 | 5,00 | |
| Uvinul A Plus | 2,00 | 1,50 | 0,75 | 1,00 | 2,10 |
| mikrofeines Titandioxid | 1,00 | | 1,00 | | 1,00 |
| mikrofeines Zinkoxid | | 1,90 | | 0,25 | |
| C12-15 Alkyl Benzoat | 2,00 | 2,50 | | | |
| Dicapryl Ether | | 4,00 | | | |
| Butylenglycol Dicaprylat/Dicaprat | 4,00 | | 2,00 | 6,00 | |
| Dicapryl Carbonat | | 2,00 | 6,00 | | |
| Dimethicon | | 0,50 | 1,00 | | |
| Phenyltrimethicon | 2,00 | | 0,50 | | |
| Shea Butter | | 2,00 | | 5,00 | |
| PVP Hexadecen Copolymer | 0,50 | | | 0,50 | 1,00 |
| Tricontanyl PVP | 0,50 | | 1,00 | | |
| Ethylhexylglycerin | | | 1,00 | | 0,80 |
| Glycerin | 3,00 | 7,50 | | 7,50 | 8,50 |
| Gylcin Soja | | | 1,50 | | 1,00 |
| Vitamin E Acetat | 0,50 | | 0,25 | | 1,00 |
| Alpha-Glucosilrutin | 0,60 | | | 0,25 | |
| DMDM Hydantoin | | 0,60 | 0,45 | 0,25 | |
| Glyacil-S | 0,20 | | | | |

(fortgesetzt)

| Zusatz | Anbsp. 528 | Anbsp. 529 | Anbsp. 530 | Anbsp. 531 | Anbsp. 532 |
|---|---|---|---|---|---|
| Methylparaben | 0,50 | | 0,25 | 0,15 | |
| Phenoxyethanol | 0,50 | 0,40 | | 1,00 | |
| Trinatrium EDTA | | 0,01 | 0,05 | | 0,10 |
| Ethanol | 3,00 | 2,00 | 1,50 | | 7,00 |
| Parfüm | 0,20 | | 0,05 | 0,40 | |
| Wasser | ad 100 | ad 100 | ad 100 | ad 100 | ad 100 |
| Anbsp. Anwendungsbeispiel | | | | | |

Anwendungsbeispiele 533-537 beziehungsweise Anwendungsbeispiele 538-542: Man wiederholte Anwendungsbeispiele 528-532, setzte aber anstelle des Isoalkangemischs aus Beispiel 2 das Isoalkangemisch aus Beispiel 3 beziehungsweise aus Beispiel 4 ein.

Anwendungsbeispiele 543-557: Kosmetische W/O-Formulierungen

Anwendungsbeispiele 543-547:

| Zusatz | Anbsp. 543 | Anbsp. 544 | Anbsp. 545 | Anbsp. 546 | Anbsp. 547 |
|---|---|---|---|---|---|
| Cetyldimethicon Copolyol | | 2,50 | | 4,00 | |
| Polyglyceryl-2-dipolyhydroxystearat | 5,00 | | | | 4,50 |
| PEG-30-dipolyhydroxystearat | | | 5,00 | | |
| Isoalkangemisch aus Beispiel 2 | 2,00 | 3,00 | 5,00 | 8,50 | 9,00 |
| Uvinul A Plus | 2,00 | 1,50 | 0,75 | 1,00 | 2,10 |
| mikrofeines Titandioxid | 1,00 | | 3,00 | | 3,50 |
| mikrofeines Zinkoxid | | 0,90 | | 0,25 | |
| Mineralöl | | 12,00 | 10,00 | | 8,00 |
| C12-15 Alkyl Benzoat | | | | 9,00 | |
| Dicaprylyl Ether | 10,00 | | | | 7,00 |
| Butylenglycol Dicaprylat/Dicaprat | | | 2,00 | 8,00 | 4,00 |
| Dicaprylyl Carbonat | 5,00 | | 6,00 | | |
| Dimethicon | | 4,00 | 1,00 | 5,00 | |
| Cyclomethicon | 2,00 | 25,00 | | | 2,00 |
| Shea Butter | | | 3,00 | | |
| Vaseline | | 4,50 | | | |
| PVP Hexadecen Copolymer | 0,50 | | | 0,50 | 1,00 |
| Ethylhexylglycerin | | 0,30 | 1,00 | | 0,50 |
| Glycerin | 3,00 | 7,50 | | 7,50 | 8,50 |
| Glycin Soja | | 1,00 | 1,50 | | 1,00 |
| MgSO4 | 1,00 | 0,50 | | 0,50 | |
| MgCl2 | | | 1,00 | | 0,70 |

(fortgesetzt)

| Zusatz | Anbsp. 543 | Anbsp. 544 | Anbsp. 545 | Anbsp. 546 | Anbsp. 547 |
|---|---|---|---|---|---|
| Vitamin E Acetat | 0,50 | | 0,25 | | 1,00 |
| Ascorbyl Palmitat | 0,50 | | | 2,00 | |
| DMDM Hydantoin | | 0,60 | 0,40 | 0,20 | |
| Methylparaben | 0,50 | | 0,25 | 0,15 | |
| Phenoxyethanol | 0,50 | 0,40 | | 1,00 | |
| Trinatrium EDTA | 0,12 | 0,05 | | 0,30 | |
| Ethanol | 3,00 | | 1,50 | | 5,00 |
| Parfüm | 0,20 | | 0,40 | 0,35 | |
| Wasser | ad 100 | ad 100 | ad 100 | ad 100 | ad 100 |
| Anbsp. Anwendungsbeispiel | | | | | |

Anwendungsbeispiele 548-552 beziehungsweise Anwendungsbeispiele 553-557: Man wiederholte Anwendungsbeispiele 543-547, setzte aber anstelle des Isoalkangemischs aus Beispiel 2 das Isoalkangemisch aus Beispiel 3 beziehungsweise aus Beispiel 4 ein.

Anwendungsbeispiele 558-572: Feststoffstablilisierte Formulierungen

Anwendungsbeispiele 558-562:

| Zusatz | Anbsp. 558 | Anbsp. 559 | Anbsp. 560 | Anbsp. 561 | Anbsp. 562 |
|---|---|---|---|---|---|
| Mineralöl | | | 16,00 | 16,00 | |
| Octyldodecanol | 9,00 | 9,00 | 5,00 | | |
| Caprylic/Capric Triglycerid | 9,00 | 9,00 | 6,00 | | |
| C12-15 Alkyl Benzoat | | | | 5,00 | 8,00 |
| Butylen Glycol Dicaprylat/Dicaprat | | | | | 8,00 |
| Dicaprylyl Ether | 9,00 | | | 4,00 | |
| Dicaprylyl Carbonat | | 9,00 | | | |
| Hydroxyoctacosanyl Hydroxystearat | 2,00 | 2,00 | 2,20 | 2,50 | 1,50 |
| Disteardimonium Hectorit | 1,00 | 0,75 | | 0,50 | 0,25 |
| Cera Microcristallina + Paraffinum Liquidum | | 0,35 | | | 5,00 |
| Hydroxypropyl Methylcellulose | | | 0,10 | | 0,05 |
| Dimethicon | | | | | 3,00 |
| Isoalkangemisch aus Beispiel 2 | 3,00 | 5,00 | 5,00 | 6,50 | 2,00 |
| Titandioxid + Alumina + Simethicon + Aqua | | 3,00 | | | |
| Titandioxid + Trimethoxycaprylylsilan | | 2,00 | 4,00 | 2,00 | 4,00 |
| Silica Dimethyl Silylat | 2,50 | | | 6,00 | 2,50 |
| Bornitrid | | | 1,00 | | |
| Stärke/-Natriummetaphosphat-Polymer | 2,00 | | | | |
| Tapioca Stärke | | 0,50 | | | |

(fortgesetzt)

| Zusatz | Anbsp. 558 | Anbsp. 559 | Anbsp. 560 | Anbsp. 561 | Anbsp. 562 |
|---|---|---|---|---|---|
| Natriumchlorid | 5,00 | 7,00 | 8,50 | 3,00 | 4,50 |
| Glycerin | | | | 1,00 | |
| Trinatrium EDTA | 1,00 | 1,00 | 1,00 | 1,00 | 1,00 |
| Vitamin E Acetat | 5,00 | 10,00 | 3,00 | 6,00 | 10,00 |
| Ascorbyl Palmitat | 1,00 | 1,00 | | 1,00 | |
| Methylparaben | | 0,60 | | | 0,20 |
| Propylparaben | | | | | 0,20 |
| Phenoxyethanol | | | 0,20 | | |
| Hexamidin Diisethionat | | | 0,40 | 0,50 | 0,40 |
| Diazolidinyl Harnstoff | | | | | 0,08 |
| Ethanol | | | 0,23 | 0,20 | |
| Parfüm | 5,00 | | 3,00 | 4,00 | |
| Wasser | 0,20 | | 0,30 | 0,10 | |
| Anbsp. Anwendungsbeispiel | | | | | |

Anwendungsbeispiele 563-567 beziehungsweise Anwendungsbeispiele 568-572: Man wiederholte Anwendungsbeispiele 558-562, setzte aber anstelle des Isoalkangemischs aus Beispiel 2 das Isoalkangemisch aus Beispiel 3 beziehungsweise aus Beispiel 4 ein.

Anwendungsbeispiele 574-585: Kosmetische Stiftformulierungen

Anwendungsbeispiele 574-577:

| Zusatz | Anbsp. 574 | Anbsp. 575 | Anbsp. 576 | Anbsp. 577 |
|---|---|---|---|---|
| Caprylic/Capric Triglycerid | 12,00 | 10,00 | 6,00 | |
| Octyldodecanol | 7,00 | 14,00 | 8,00 | 3,00 |
| Butylene Glycol Dicaprylat/Dicaprat | | | | 12,00 |
| Pentaerythrityl Tetraisostearat | 10,00 | 6,00 | 8,00 | 7,00 |
| Polyglyceryl-3 Diisostearat | 2,50 | | | |
| Bis-Diglyceryl Polyacyladipate-2 | 9,00 | 8,00 | 10,00 | 8,00 |
| Cetearyl Alcohol | 8,00 | 11,00 | 9,00 | 7,00 |
| Myristyl Myristate | 3,50 | 3,00 | 4,00 | 3,00 |
| Beeswax | 5,00 | 5,00 | 6,00 | 6,00 |
| Cera Carnauba | 1,50 | 2,00 | 2,00 | 1,50 |
| Cera Alba | 0,50 | 0,50 | 0,50 | 0,40 |
| C16-40 Alkyl Stearat | | 1,50 | 1,50 | 1,50 |
| Isoalkangemisch aus Beispiel 2 | 2,00 | 3,00 | 3,00 | 12,00 |
| mikrofeines Titandioxid | 1,00 | | 3,00 | |
| mikrofeines Zinkoxid | | 1,00 | | 0,25 |

(fortgesetzt)

| Zusatz | Anbsp. 574 | Anbsp. 575 | Anbsp. 576 | Anbsp. 577 |
|---|---|---|---|---|
| Vitamin E Acetat | 0,50 | 1,00 | | |
| Ascorbyl Palmitat | 0,05 | | 0,05 | |
| Buxux Chinensis | 2,00 | 1,00 | | 1,00 |
| Parfüm, BHT | 0,10 | 0,25 | | 0,35 |
| Ricinus Communis | ad 100 | ad 100 | ad 100 | ad 100 |
| Anbsp. Anwendungsbeispiel | | | | |

Anwendungsbeispiele 578-581 beziehungsweise Anwendungsbeispiele 582-585: Man wiederholte Anwendungsbeispiele 574-577, setzte aber anstelle des Isoalkangemischs aus Beispiel 2 das Isoalkangemisch aus Beispiel 3 beziehungsweise aus Beispiel 4 ein.

Anwendungsbeispiele 586-609: Kosmetische PIT-Formulierungen

Anwendungsbeispiele 586-593:

| Zusatz | Anbsp. 586 | Anbsp. 587 | Anbsp. 588 | Anbsp. 589 | Anbsp. 590 | Anbsp. 591 | Anbsp. 592 | Anbsp. 593 |
|---|---|---|---|---|---|---|---|---|
| Glycerinmonostearat SE | 0,50 | 2,00 | 3,00 | 5,00 | | 0,50 | 4,00 | |
| Glyceryl Isostearat | | | | | 3,50 | 4,00 | 2,00 | |
| Isoceteth-20 | | 0,50 | | | 2,00 | | | |
| Ceteareth-12 | | 5,00 | | 1,00 | | | 3,50 | 5,00 |
| Ceteareth-20 | | 5,00 | | 1,00 | | | | 3,50 |
| PEG-100 Stearat | | | | 2,80 | | 2,30 | 3,30 | |
| Cetyl Alkohol | 5,20 | | 1,20 | 1,00 | 1,30 | | 0,50 | 0,30 |
| Cetyl Palmitat | 2,50 | 1,20 | | 1,50 | | 0,50 | | 1,50 |
| Cetyl Dimethicon Copolyol | | | | 0,50 | | 1,00 | | |
| Polyglyceryl-2 | | | | 0,75 | 0,30 | | | |
| Isoalkangemisch aus Beispiel 2 | 2,00 | 5,00 | 7,90 | 15,00 | 1,00 | 6,50 | 5,50 | 9,50 |
| Dihydroxyaceton | | | 3,00 | 5,00 | | | 4,00 | |
| Uvinul A Plus | 2,00 | 1,50 | 0,75 | 1,00 | 2,10 | 4,50 | 5,00 | 2,10 |
| mikrofeines Titandioxid | 1,00 | | 1,50 | | 3,50 | | 1,50 | 1,00 |
| mikrofeines Zinkoxid | | 1,00 | | 0,25 | | 2,00 | | 1,50 |
| C12-15 Alkyl Benzoat | 3,50 | | | 6,35 | | | | 0,10 |
| Cocoglyceride | | 3,00 | | 3,00 | | | | 1,00 |
| Dicapryl Ether | 4,50 | | | | | | | |
| Dicaprylyl Carbonat | | 4,30 | | 3,00 | | | | 7,00 |
| Dibutyl Adipate | | | | 0,50 | | | | 0,30 |

(fortgesetzt)

| Zusatz | Anbsp. 586 | Anbsp. 587 | Anbsp. 588 | Anbsp. 589 | Anbsp. 590 | Anbsp. 591 | Anbsp. 592 | Anbsp. 593 |
|---|---|---|---|---|---|---|---|---|
| Phenyltrimethicone | 2,00 | | | 3,50 | | 2,00 | | |
| Cyclomethicon | | 3,00 | | | | | | |
| Ethyl Galaktomannan | | 0,50 | | | 2,00 | | | |
| Hydrierte Coco-Glyceride | | | | | 3,00 | 4,00 | | |
| Abil Wax 2440 | | | | | | 1,50 | 2,00 | |
| PVP Hexadecen Copolymer | | | | 1,00 | 1,20 | | | |
| Glycerin | 4,00 | 6,00 | 5,00 | | 8,00 | 10,00 | | |
| Vitamin E Acetat | 0,20 | 0,30 | 0,40 | | 0,30 | | | |
| Shea Butter | | 2,00 | | 3,60 | | 2,00 | | |
| Iodopropyl Butylcarbamat | 0,12 | | | | 0,20 | | | |
| DMDM Hydantoin | 0,10 | | | | 0,12 | | 0,13 | |
| Methylparaben | | 0,50 | 0,30 | | 0,35 | | | |
| Phenoxyethanol | 0,50 | 0,40 | | 1,00 | | | | |
| Octoxyglycerin | | 0,30 | | | 1,00 | | 0,35 | |
| Ethanol | 2,00 | | 2,00 | | | 5,00 | | |
| Trinatrium EDTA | 0,40 | | 0,15 | | | 0,20 | | |
| Parfüm | 0,20 | | 0,20 | | 0,24 | 0,16 | 0,10 | 0,10 |
| Wasser | ad 100 | ad 100 | ad 100 | ad 100 | ad 100 | ad100 | ad 100 | ad 100 |
| Anbsp. Anwendungsbeispiel | | | | | | | | |

Anwendungsbeispiele 594-601 beziehungsweise Anwendungsbeispiele 602-609: Man wiederholte Anwendungsbeispiele 586-593, setzte aber anstelle des Isoalkangemischs aus Beispiel 2 das Isoalkangemisch aus Beispiel 3 beziehungsweise aus Beispiel 4 ein.

Anwendungsbeispiele 610-621: Kosmetische Öle und Ölgele

Anwendungsbeispiele 610-613:

| Zusatz | Anbsp. 610 | Anbsp. 611 | Anbsp. 612 | Anbsp. 613 |
|---|---|---|---|---|
| Caprylic/Capric Triglycerid | 12,00 | 10,00 | 6,00 | |
| Octyldodecanol | 7,00 | 14,00 | 8,00 | 3,00 |
| Butylene Glycol Dicaprylat/Dicaprat | | | | 12,00 |
| Pentaerythrityl Tetraisostearat | 10,00 | 6,00 | 8,00 | 7,00 |
| Polyglyceryl-3 Diisostearat | 2,50 | | | |
| Bis-Diglyceryl Polyacyladipate-2 | 9,00 | 8,00 | 10,00 | 8,00 |
| Myristyl Myristate | 3,50 | 3,00 | 4,00 | 3,00 |

(fortgesetzt)

| Zusatz | Anbsp. 610 | Anbsp. 611 | Anbsp. 612 | Anbsp. 613 |
|---|---|---|---|---|
| Bentone -34 | 5,00 | 5,00 | 6,00 | 6,00 |
| Propylencarbonat | 15,00 | 20,00 | 18,00 | 19,50 |
| Isoalkangemisch aus Beispiel 2 | 2,00 | 3,00 | 9,50 | 12,00 |
| Vitamin E Acetat | 0,50 | 1,00 | | |
| Ascorbyl Palmitat | 0,05 | | 0,05 | |
| Buxux Chinensis | 2,00 | 1,00 | | 1,00 |
| Parfüm, BHT | 0,10 | 0,25 | | 0,35 |
| Ricinus Communis | ad 100 | ad 100 | ad 100 | ad 100 |
| Anbsp. Anwendungsbeispiel | | | | |

Anwendungsbeispiele 614-617 beziehungsweise Anwendungsbeispiele 618-621: Man wiederholte Anwendungsbeispiele 610-613, setzte aber anstelle des Isoalkangemischs aus Beispiel 2 das Isoalkangemisch aus Beispiel 3 beziehungsweise aus Beispiel 4 ein.

Anwendungsbeispiele 622-636: Kosmetische after-sun Formulierungen

Anwendungsbeispiele 622-626:

| Zusatz | Anbsp. 622 | Anbsp. 623 | Anbsp. 624 | Anbsp. 625 | Anbsp. 626 |
|---|---|---|---|---|---|
| Ceteareth-20 | 1,00 | | | 0,50 | |
| Cetyl Alkohol | | | 1,00 | | |
| Luvigel EM | | 2,00 | | 2,50 | 2,00 |
| Acrylat/C10-30 Alkyl Acrylat Crosspolymer | 0,50 | 0,30 | 0,40 | 0,10 | 0,50 |
| Xanthan Gummi | | 0,30 | 0,15 | | |
| Isoalkangemisch aus Beispiel 2 | 3,00 | 6,00 | 2,00 | 6,50 | 8,50 |
| C12-15 Alkyl Benzoat | 2,00 | 2,50 | | | |
| Dicapryl Ether | | 4,00 | | | |
| Butylenglycol Dicaprylat/Dicaprat | 4,00 | | 2,00 | 6,00 | |
| Dicapryl Carbonat | | 2,00 | 6,00 | | |
| Dimethicon | | 0,50 | 1,00 | | |
| Phenyltrimethicon | 2,00 | | 0,50 | | |
| Tricontanyl PVP | 0,50 | | 1,00 | | |
| Ethylhexylglycerin | | | 1,00 | | 0,80 |
| Glycerin | 3,00 | 7,50 | | 7,50 | 8,50 |
| Gylcin Soja | | | 1,50 | | 1,00 |
| Vitamin E Acetat | 0,50 | | 0,25 | | 1,00 |
| Alpha-Glucosilrutin | 0,60 | | | 0,25 | |
| Trinatrium EDTA | | 0,01 | 0,05 | | 0,10 |
| Ethanol | 15,00 | 10,00 | 8,00 | 12,00 | 9,00 |

(fortgesetzt)

| Zusatz | Anbsp. 622 | Anbsp. 623 | Anbsp. 624 | Anbsp. 625 | Anbsp. 626 |
|---|---|---|---|---|---|
| Parfüm | 0,20 | | 0,05 | 0,40 | |
| Wasser | ad 100 | ad 100 | ad 100 | ad 100 | ad 100 |
| Anbsp. Anwendungsbeispiel | | | | | |

Anwendungsbeispiele 627-631 beziehungsweise Anwendungsbeispiele 632-636: Man wiederholte Anwendungsbeispiele 622-626, setzte aber anstelle des Isoalkangemischs aus Beispiel 2 das Isoalkangemisch aus Beispiel 3 beziehungsweise aus Beispiel 4 ein.

Anwendungsbeispiele 637-657: Kosmetische Formulierungen für die dekorative Kosmetik

Anwendungsbeispiele 637-643:

| Zusatz | Anbsp. 637 | Anbsp. 638 | Anbsp. 639 | Anbsp. 640 | Anbsp. 641 | Ânbsp. 642 | Anbsp. 643 |
|---|---|---|---|---|---|---|---|
| Glycerinmonostearat SE | 0,50 | 1,00 | 3,00 | | | 1,50 | |
| Glycerl Stearat Citrat | 2,00 | | 1,00 | 2,00 | 4,00 | | |
| Stearinsäure | | 3,00 | | 2,00 | | | |
| PEG-40 Stearat | 0,50 | | | | | 2,00 | |
| Cetyl Phosphat | | | | | | 1,00 | |
| Cetearyl Sulfat | | | | | | | 0,75 |
| Stearyl Alkohol | | | 3,00 | | | 2,00 | 0,60 |
| Cetyl Alkohol | 2,50 | 1,10 | | 1,50 | 0,60 | | 2,00 |
| Isoalkangemisch aus Beispiel 2 | 2,00 | 5,00 | 7,00 | 5,50 | 7,50 | 10,00 | 1,00 |
| Titandioxid | 10,00 | 12,00 | 9,00 | 8,50 | 11,00 | 9,50 | 10,00 |
| Eisenoxide | 2,00 | 4,00 | 3,00 | 5,00 | 3,40 | 6,00 | 4,40 |
| Zinkoxid | | 4,00 | | 2,00 | | 3,00 | |
| C12-15 Alkyl Benzoat | | 0,25 | | | 4,00 | 7,00 | |
| Dicapryl Ether | | | 3,50 | | 2,00 | | |
| Butylenglycol Dicaprylat/Dicaprat | 5,00 | | 6,00 | | | | |
| Cocoglyceride | | | 6,00 | | 2,00 | | |
| Dimethicon | 0,50 | | 1,00 | | 2,00 | | |
| Cyclomethicon | 2,00 | | 0,50 | | 0,50 | | |
| Shea Butter | | 2,00 | | | | | |
| PVP Hexadecen Copolymer | 0,20 | | | 0,50 | | 1,00 | |
| Glycerin | 3,00 | 7,50 | | 7,50 | 5,00 | | 2,50 |
| Xanthan Gummi | 0,15 | | 0,05 | | | 0,30 | |

(fortgesetzt)

| Zusatz | Anbsp. 637 | Anbsp. 638 | Anbsp. 639 | Anbsp. 640 | Anbsp. 641 | Ânbsp. 642 | Anbsp. 643 |
|---|---|---|---|---|---|---|---|
| Sodium Carbomer | | 0,20 | | 0,15 | 0,25 | | |
| Vitamin E Acetat | 0,60 | | 0,23 | | 0,70 | 1,00 | |
| Glycin Soja | | | | 0,50 | | 1,50 | 1,00 |
| Ethylhexyloxyglycin | 0,30 | | | | | | |
| DMDM Hydantoin | | 0,60 | 0,40 | 0,20 | | | |
| Glyacil-L | | | | 0,18 | 0,20 | | |
| Methylparaben | 0,15 | | 0,25 | | 0,50 | | |
| Phenoxyethanol | 1,00 | 0,40 | | | 0,40 | 0,50 | 0,40 |
| Trinatrium EDTA | 0,02 | | 0,05 | | | | |
| Iminobernsteinsäure | | | | 0,25 | 1,00 | | |
| Ethanol | 2,00 | 1,50 | | 3,00 | | 1,20 | 5,00 |
| Parfüm | 0,10 | 0,25 | 0,30 | | 0,40 | 0,20 | |
| Wasser | ad 100 | ad 100 | ad 100 | ad 100 | ad 100 | ad100 | ad 100 |
| Anbsp. Anwendungsbeispiel | | | | | | | |

Anwendungsbeispiele 644-650 beziehungsweise Anwendungsbeispiele 651-657: Man wiederholte Anwendungsbeispiele 637-643, setzte aber anstelle des Isoalkangemischs aus Beispiel 2 das Isoalkangemisch aus Beispiel 3 beziehungsweise aus Beispiel 4 ein.

Anwendungsbeispiele 658-713: Reinigungsformulierungen zum Duschen /Baden / Waschen

Anwendungsbeispiele 658-662:

| Zusatz | Anbsp.658 | Anbsp. 659 | Anbsp. 660 | Anbsp. 661 | Anbsp. 662 |
|---|---|---|---|---|---|
| Texapon N 70 | 13,00 | 15,00 | 10,50 | 12,50 | 10,00 |
| Dehyton PK 45 | 7,50 | 7,00 | 5,00 | 5,50 | 10,00 |
| Cetiol HE | 2,00 | 2,50 | 3,50 | 5,00 | 2,30 |
| Parfüm | 0,10 | 0,10 | 0,10 | 0,10 | 0,10 |
| Isoalkangemisch aus Beispiel 2 | 1,00 | 4,50 | 7,00 | 1,40 | 3,00 |
| D-Panthenol USP | 1,00 | 1,50 | 1,80 | 1,70 | 1,40 |
| Konservierungsmittel | 0,10 | 0,10 | 0,10 | 0,10 | 0,10 |
| Zitronensäure | 0,10 | 0,10 | 0,10 | 0,10 | 0,10 |
| Luviquat Ultra Care | 1,50 | 1,00 | 1,50 | 1,20 | 1,10 |
| Natriumchlorid | 1,50 | 1,40 | 1,40 | 1,30 | 1,50 |
| Wasser dem. | ad 100 | ad 100 | ad 100 | ad 100 | ad 100 |
| Anbsp. Anwendungsbeispiel | | | | | |

Anwendungsbeispiele 663-667 beziehungsweise Anwendungsbeispiele 668-672: Man wiederholte Anwendungsbeispiele 658-662, setzte aber anstelle des Isoalkangemischs aus Beispiel 2 das Isoalkangemisch aus Beispiel

3 beziehungsweise aus Beispiel 4 ein.

Anwendungsbeispiele 673-677:

| Zusatz | Anbsp. 673 | Anbsp. 674 | Anbsp. 675 | Anbsp. 676 | Anbsp. 677 |
|---|---|---|---|---|---|
| Amphotensid GB 2009 | 10,00 | 15,00 | 20,00 | 12,00 | 17,00 |
| Plantacare 2000 | 5,00 | 6,00 | 7,00 | 8,00 | 4,00 |
| Tego Betain L7 | 15,00 | 12,00 | 10,00 | 18,00 | 20,00 |
| Luviquat FC 550 | 0,30 | 0,20 | 0,20 | 0,20 | 0,30 |
| Parfüm | 0,10 | 0,10 | 0,10 | 0,10 | 0,10 |
| Isoalkangemisch aus Beispiel 2 | 3,00 | 6,00 | 5,50 | 4,00 | 1,50 |
| Cremophor PS 20 | 5,00 | 1,00 | 1,00 | 7,00 | 5,00 |
| Konservierungsmittel | 0,10 | 0,10 | 0,10 | 0,10 | 0,10 |
| Rewopal LA 3 | 2,00 | 1,00 | 0,50 | 2,00 | 2,00 |
| Zitronensäure | 0,20 | 0,20 | 0,20 | 0,20 | 0,20 |
| Stepan PEG 600 DS | 3,00 | 2,00 | 2,00 | 3,00 | 2,50 |
| Wasser dem. | ad 100 | ad 100 | ad 100 | ad 100 | ad 100 |
| Anbsp. Anwendungsbeispiel | | | | | |

Anwendungsbeispiele 678-682 beziehungsweise Anwendungsbeispiele 683-687: Man wiederholte Anwendungsbeispiele 673-677, setzte aber anstelle des Isoalkangemischs aus Beispiel 2 das Isoalkangemisch aus Beispiel 3 beziehungsweise aus Beispiel 4 ein.

Anwendungsbeispiele 693-699:

| Zusatz | Anbsp. 693 | Anbsp. 694 | Anbsp. 695 | Anbsp. 696 | Anbsp. 697 | Anbsp. 698 | Anbsp. 699 |
|---|---|---|---|---|---|---|---|
| Texapon NSO | 35,00 | 40,00 | 30,00 | 45,00 | 27,00 | | |
| Plantacare 2000 | 5,00 | 5,50 | 4,90 | 3,50 | 7,00 | | |
| Tego Betain L7 | 10,00 | 5,00 | 12,50 | 7,50 | 15,00 | | |
| Parfüm | 0,10 | 0,10 | 0,10 | 0,10 | 0,10 | 0,10 | 0,10 |
| Isoalkangemisch aus Beispiel 2 | 3,00 | 5,50 | 12,00 | 8,00 | 4,00 | 9,50 | 20,00 |
| D-Panthenol USP | 0,50 | 1,00 | 0,80 | 1,50 | 0,50 | | |
| Konservierungsmittel | 0,10 | 0,10 | 0,10 | 0,10 | 0,10 | 0,10 | 0,10 |
| Zitronensäure | 0,10 | 0,10 | 0,10 | 0,10 | 0,10 | | |
| Rewopal LA 3 | 0,50 | 2,00 | 0,50 | 0,50 | 2,00 | | |
| Natriumchlorid | 1,50 | 1,50 | 1,50 | 1,50 | 1,50 | | |
| Wasser dem. | ad 100 | ad 100 | ad 100 | ad 100 | ad 100 | ad 100 | ad 100 |
| Anbsp. Anwendungsbeispiel | | | | | | | |

Anwendungsbeispiele 700-706 beziehungsweise Anwendungsbeispiele 707-713: Man wiederholte Anwendungsbeispiele 693-699, setzte aber anstelle des Isoalkangemischs aus Beispiel 2 das Isoalkangemisch aus Beispiel 3 beziehungsweise aus Beispiel 4 ein.

Anwendungsbeispiele 714-728: Aerosol-Haarspay

Anwendungsbeispiel 714: VOC 80-Aerosol-Haarspray

| Zusatz | % |
|---|---|
| Isoalkangemisch aus Beispiel 2 | 2,00 |
| Wasser | 18,00 |
| Dimethylether | 40,00 |
| Ethanol | 40,00 |
| weiterer Zusatz: Silikon, Parfüm, Entschäumer usw. | |

Anwendungsbeispiel 715 und Anwendungsbeispiel 716: Man wiederholte Anwendungsbeispiel 714, setzte aber anstelle des Isoalkangemischs aus Beispiel 2 das Isoalkangemisch aus Beispiel 3 beziehungsweise aus Beispiel 4 ein.

Anwendungsbeispiel 717: VOC 55-Aerosol-Haarspray

| Zusatz | % |
|---|---|
| Isoalkangemisch aus Beispiel 2 | 2,00 |
| Wasser | 33,00 |
| Dimethylether | 40,00 |
| Ethanol | 25,00 |
| weiterer Zusatz: Silikon, Parfüm, Entschäumer | |

Anwendungsbeispiel 718 und Anwendungsbeispiel 719: Man wiederholte Anwendungsbeispiel 717, setzte aber anstelle des Isoalkangemischs aus Beispiel 2 das Isoalkangemisch aus Beispiel 3 beziehungsweise aus Beispiel 4 ein.

Anwendungsbeispiel 720: VOC 55-Aerosol-Haarspray

| Zusatz | % |
|---|---|
| Isoalkangemisch aus Beispiel 2 | 6,00 |
| Wasser | 39,00 |
| HFC 152A | 40,00 |
| Ethanol | 15,00 |
| weiterer Zusatz: Silikon, Parfüm, Entschäumer, usw. | |

Anwendungsbeispiel 721 und Anwendungsbeispiel 722: Man wiederholte Anwendungsbeispiel 720, setzte aber anstelle des Isoalkangemischs aus Beispiel 2 das Isoalkangemisch aus Beispiel 3 beziehungsweise aus Beispiel 4 ein.

Anwendungsbeispiel 723: VOC 55-Aerosol-Haarspray

| Zusatz | % |
|---|---|
| Isoalkangemisch aus Beispiel 2 | 5,00 |
| Ultrahold Strong (Fa. BASF) | 1,00 |
| Wasser | 39,00 |
| Dimethylether | 40,00 |
| Ethanol | 15,00 |
| + AMP | auf pH 8,3 |
| weiterer Zusatz: Silikon, Parfüm, Entschäumer, usw. | |

Anwendungsbeispiel 724 und Anwendungsbeispiel 725: Man wiederholte Anwendungsbeispiel 723, setzte aber anstelle des Isoalkangemischs aus Beispiel 2 das Isoalkangemisch aus Beispiel 3 beziehungsweise aus Beispiel 4 ein.

Anwendungsbeispiel 726: VOC 55-Aerosol-Haarspray

| Zusatz | % |
|---|---|
| Isoalkangemisch aus Beispiel 2 | 4,00 |
| Stepanhold R-1 *) (Fa. Stepan Chemical Co.) | 1,00 |
| Wasser | 40,00 |
| Dimethylether | 40,00 |
| Ethanol | 15,00 |
| + AMP | auf pH 8,3 |
| weiterer Zusatz: Silikon, Parfüm, Entschäumer, usw. | |
| *) Stepanhold R-1 = Poly(Vinylpyrrolidon/Ethylmethacrylat/Methacrylsäure) | |

Anwendungsbeispiel 727 und Anwendungsbeispiel 728: Man wiederholte Anwendungsbeispiel 726, setzte aber anstelle des Isoalkangemischs aus Beispiel 2 das Isoalkangemisch aus Beispiel 3 beziehungsweise aus Beispiel 4 ein.

Anwendungsbeispiel 729: VOC 55-Handpumpenspray

| Zusatz | % |
|---|---|
| Isoalkangemisch aus Beispiel 2 | 4,00 |
| Wasser | 41,00 |
| Ethanol | 55,00 |

(fortgesetzt)

| Zusatz | % |
|---|---|
| weiterer Zusatz: Silikon, Parfüm, Entschäumer, usw. | |

Anwendungsbeispiel 730 und Anwendungsbeispiel 731: Man wiederholte Anwendungsbeispiel 729, setzte aber anstelle des Isoalkangemischs aus Beispiel 2 das Isoalkangemisch aus Beispiel 3 beziehungsweise aus Beispiel 4 ein.

Anwendungsbeispiel 732: Wässriges Handpumpspray

| Zusatz | % |
|---|---|
| Isoalkangemisch aus Beispiel 2 | 5,00 |
| Luviset Clear *) (20 %ige Lösung) | 5,00 |
| Wasser | 90,00 |
| weiterer Zusatz: wasserlösliches Silikon, Parfüm, Entschäumer, usw. | |
| *) Luviset Clear: Poly(Vinylpyrrolidon/Methacrylsäureamid/Vinylimidazol), Fa. BASF | |

Anwendungsbeispiel 733 und Anwendungsbeispiel 734: Man wiederholte Anwendungsbeispiel 732, setzte aber anstelle des Isoalkangemischs aus Beispiel 2 das Isoalkangemisch aus Beispiel 3 beziehungsweise aus Beispiel 4 ein.

Anwendungsbeispiel 735: Haargel mit Aculyn 28

| Zusatz | % |
|---|---|
| Phase 1: | |
| Isoalkangemisch aus Beispiel 2 | 6,00 |
| Wasser, dest. | 43,00 |
| Aminomethylpropanol (38 %ige Lösung) | 1,0 |
| weiterer Zusatz: Konservierungsmittel, lösliches ethoxiliertes Silikon, Parfüm, usw. | |
| Phase 2: | % |
| Aculyn 28 (1 %ige wässrige Suspension) | 50,00 |

Herstellung: Die Phasen 1 und 2 wurden getrennt eingewogen und homogenisiert. Dann wurde Phase 2 langsam in Phase 1 eingerührt. Es bildet sich ein im Wesentlichen klares, stabiles Gel.

Anwendungsbeispiel 736 und Anwendungsbeispiel 737: Man wiederholte Anwendungsbeispiel 735, setzte aber anstelle des Isoalkangemischs aus Beispiel 2 das Isoalkangemisch aus Beispiel 3 beziehungsweise aus Beispiel 4 ein.

Anwendungsbeispiele 738-740: Haargel mit Hydroxyethylcellulose Anwendungsbeispiel 738:

| Zusatz | % |
|---|---|
| Phase 1: | |
| Isoalkangemisch aus Beispiel 2 | 6,00 |

(fortgesetzt)

| Zusatz | % |
|---|---|
| Phase 1: | |
| Wasser, dest. | 36,00 |
| weiterer Zusatz: Konservierungsmittel, lösliches ethoxiliertes Silikon, Parfüm, usw. | |
| | |
| Phase 2: | % |
| Natrosol HR 250 (5 %ige Lösung) | 50,00 |
| Hydroxyethylcellulose (Fa. Hercules) | |

Herstellung: Die Phasen 1 und 2 wurden getrennt eingewogen und homogenisiert.
Dann wurde Phase 2 langsam in Phase 1 eingerührt. Es bildete sich ein im Wesentlichen klares, stabiles Gel.

Anwendungsbeispiel 739 und Anwendungsbeispiel 740: Man wiederholte Anwendungsbeispiel 738, setzte aber anstelle des Isoalkangemischs aus Beispiel 2 das Isoalkangemisch aus Beispiel 3 beziehungsweise aus Beispiel 4 ein.

Anwendungsbeispiele 741-743: Flüssiges Makeup

Anwendungsbeispiel: 741

| Zusatz | |
|---|---|
| Phase A | |
| Glycerylstearat | 1,70 |
| Cetylalkohol | 1,70 |
| Ceteareth-6 | 1,70 |
| Ceteareth-25 | 1,70 |
| Capryl/Caprin-Triglycerid | 5,20 |
| Mineralöl | 5,20 |
| | |
| Phase B | |
| Konservierungsmittel | q.s. |
| Propylenglykol | 4,30 |
| Isoalkangemisch aus Beispiel 2 | 2,50 |
| dest. Wasser | 59,50 |
| | |
| Phase C | |
| Parfumöl | q.s. |
| | |
| Phase D | |
| Eisenoxid | 2,00 |
| Titandioxid | 12,00 |

Herstellung: Phase A und Phase B wurden getrennt voneinander auf 80 °C erwärmen. Dann vermischte man Phase B in Phase A mit einem Rührer. Man ließ alles auf 40 °C abkühlen und gab danach Phase C und Phase D zu. Man homogenisierte mehrmals.

Anwendungsbeispiel 742 und Anwendungsbeispiel 743: Man wiederholte Anwendungsbeispiel 741, setzte aber anstelle des Isoalkangemischs aus Beispiel 2 das Isoalkangemisch aus Beispiel 3 beziehungsweise aus Beispiel 4 ein.

Anwendungsbeispiele 744-746: Ölfreies Makeup

Anwendungsbeispiel 744:

| Phase A | |
|---|---|
| Veegum | 0,35 |
| Butylenglykol | 5,00 |
| Xanthangummi | 0,15 |

| Phase B | |
|---|---|
| dest. Wasser | 44,0 |
| Konservierungsmittel | q.s. |
| Polysorbate-20 | 0,2 |
| Tetrahydroxypropylethylenediamin | 1,6 |

| Phase C | |
|---|---|
| Siliciumdioxid | 1,0 |
| Nylon-12 | 2,0 |
| Glimmer (mica) | 4,15 |
| Titandioxid | 6,0 |
| Eisenoxid | 1,85 |

| Phase D | |
|---|---|
| Stearinsäure | 4,0 |
| Glycerylstearat | 1,5 |
| Benzyllaurat | 7,0 |
| Isoeicosan | 5,0 |
| Konservierungsmittel | q.s. |

| Phase E | |
|---|---|
| Panthenol | 0,5 |
| Imidazolidinyl-Harnstoff | 0,1 |

(fortgesetzt)

| Phase E | |
|---|---|
| Isoalkangemisch aus Beispiel 2 | 15,0 |

Herstellung: Man benetzte Phase A mit Butylenglykol, gab sie in Phase B und vermischte gründlich. Danach erwärmte man das Phasengemisch aus Phase A und Phase B auf 75 °C. Die Einsatzstoffe der Phase C wurden pulverisiert und zu dem Gemisch aus Phase A und B gegeben und sorgfältig homogenisiert. Man mischte die Einsatzstoffe von Phase D, erwärmte sie auf 80 °C und gab sie zu dem Gemisch aus Phase A, Phase B und Phase C. Man mischte solange, bis das Gemisch homogen war. Danach überführte man das Gemisch in ein Gefäß mit Propellermischer und vermischte die Einsatzstoffe von Phase E, die man danach in das Gemisch aus den Phasen A, B, C und D gab und gründlich mischte.

Anwendungsbeispiel 745 und Anwendungsbeispiel 746: Man wiederholte Anwendungsbeispiel 744, setzte aber anstelle des Isoalkangemischs aus Beispiel 2 das Isoalkangemisch aus Beispiel 3 beziehungsweise aus Beispiel 4 ein.

Anwendungsbeispiele 747-749: Gesichtsmaske

Anwendungsbeispiel 747:

| Phase A | |
|---|---|
| Ceteareth-6 | 3,00 |
| Ceteareth-25 | 1,50 |
| Cetearylalkohol | 5,00 |
| Cetearyloctanoat | 6,00 |
| Mineralöl | 6,00 |
| Bisabolol | 0,20 |
| Glycerylstearat | 3,00 |

| Phase B | |
|---|---|
| Propylenglykol | 2,00 |
| Panthenol | 5,00 |
| Isoalkangemisch aus Beispiel 2 | 4,00 |
| Konservierungsmittel | q.s. |
| dest. Wasser | 63,80 |

| Phase C | |
|---|---|
| Parfüm | q.s. |
| Tocopherylacetat | 0,50 |

Herstellung: Man erwärmte die Phasen A und B getrennt auf ca. 80 °C. Danach rührte man die Phase B in Phase A unter Homogenisieren ein; nach kurzem Nachhomogenisieren ließ man auf ca. 40 °C abkühlen, gab die Phase C hinzu und homogenisierte nochmals.

Anwendungsbeispiel 748 und Anwendungsbeispiel 749: Man wiederholte Anwendungsbeispiel 747, setzte

aber anstelle des Isoalkangemischs aus Beispiel 2 das Isoalkangemisch aus Beispiel 3 beziehungsweise aus Beispiel 4 ein.

Anwendungsbeispiele 750-752: Gesichtswasser für trockene und empfindliche Haut

Anwendungsbeispiel 750:

| Phase A | |
|---|---|
| hydriertes Rizinusöl-PEG-40 | 2,50 |
| Parfüm | q.s. |
| Bisabolol | 0,40 |

| Phase B | |
|---|---|
| Glycerin | 3,00 |
| Hydroxyethylcetyldimoniumphosphat | 1,00 |
| Zaubernuss (Hamamelis Virginiana) Destillat | 5,00 |
| Panthenol | 0,50 |
| Isoalkangemisch aus Beispiel 2 | 0,50 |
| Konservierungsmittel | q.s. |
| dest. Wasser | 87,60 |

Herstellung: Phase A klar lösen. Phase B in Phase A einrühren.

Anwendungsbeispiel 751 und Anwendungsbeispiel 752: Man wiederholte Anwendungsbeispiel 750, setzte aber anstelle des Isoalkangemischs aus Beispiel 2 das Isoalkangemisch aus Beispiel 3 beziehungsweise aus Beispiel 4 ein.

Anwendungsbeispiele 753-755: Gesichtswaschpaste mit Peelingeffekt

Anwendungsbeispiel 753:

| Phase A | |
|---|---|
| dest. Wasser | 58,00 |
| Isoalkangemisch aus Beispiel 2 | 15,00 |
| Carbomer | 1,50 |
| Konservierungsmittel | q.s. |

| Phase B | |
|---|---|
| Parfüm | q.s. |
| Potassium Cocoyl Hydrolyzed Protein | 7,00 |
| Cocamidpropylbetain | 4,00 |

| Phase C | |
|---|---|
| Triethanolamin | 1,50 |

| Phase D | |
|---|---|
| Polyethylen (Luwax ATM von BASF) | 13,00 |

Herstellung: Man ließ Phase A quellen. Danach löste man Phase B, die eine klare Lösung bildete. Man rührte Phase B in Phase A und neutralisierte mit Phase C. Danach Phase rührte man D einrühren.

Anwendungsbeispiel 754 und Anwendungsbeispiel 755: Man wiederholte Anwendungsbeispiel 753, setzte aber anstelle des Isoalkangemischs aus Beispiel 2 das Isoalkangemisch aus Beispiel 3 beziehungsweise aus Beispiel 4 ein.

Anwendungsbeispiele 756-758: Peeling-Creme, Typ O/W

Anwendungsbeispiel 756:

| Phase A | |
|---|---|
| Ceteareth-6 | 3,00 |
| Ceteareth-25 | 1,50 |
| Glycerylstearat | 3,00 |
| Cetearylalkohol, Sodium Cetearyl Sulfate | 5,00 |
| Cetearyloctanoat | 6,00 |
| Mineralöl | 6,00 |
| Bisabolol | 0,20 |

| Phase B | |
|---|---|
| Propylenglykol | 2,00 |
| Dinatrium-EDTA | 0,10 |
| Isoalkangemisch aus Beispiel 2 | 3,00 |
| Konservierungsmittel | q.s. |
| dest. Wasser | 59,70 |

| Phase C | |
|---|---|
| Tocopherylacetat | 0,50 |
| Parfüm | q.s. |

| Phase D | |
|---|---|
| Polyethylen | 10,00 |

Herstellung: Man erwärmte die Phasen A und B getrennt auf ca. 80 °C. Danach rührte man Phase B in Phase A ein und homogenisierte. Man ließ auf ca. 40 °C abkühlen, gab Phase C hinzu und homogenisierte nochmals kurz. Anschließend rührte man Phase D unter.

Anwendungsbeispiel 757 und Anwendungsbeispiel 758: Man wiederholte Anwendungsbeispiel 756, setzte aber anstelle des Isoalkangemischs aus Beispiel 2 das Isoalkangemisch aus Beispiel 3 beziehungsweise aus Beispiel 4 ein.

Anwendungsbeispiele 759-761: Rasierschaum

Anwendungsbeispiel 759:

| | |
|---|---|
| Ceteareth-25 | 6,00 |
| Poloxamer 407 | 5,00 |
| dest. Wasser | 52,00 |
| Triethanolamin | 1,00 |
| Propylenglykol | 5,00 |
| Lanolinöl-PEG-75 | 1,00 |
| Isoalkangemisch aus Beispiel 2 | 5,00 |
| Konservierungsmittel | q.s. |
| Parfüm | q.s. |
| Sodium Laureth Sulfate | 25,00 |

Herstellung: Alle Komponenten wurden zusammen gewogen und man rührte, bis sich alles gelöst hatte. Abfüllung: 90 Teile Wirksubstanz und 10 Teile Propan/Butan-Mischung 25:75.

Anwendungsbeispiel 760 und Anwendungsbeispiel 761: Man wiederholte Anwendungsbeispiel 759, setzte aber anstelle des Isoalkangemischs aus Beispiel 2 das Isoalkangemisch aus Beispiel 3 beziehungsweise aus Beispiel 4 ein.

Anwendungsbeispiel 762-764: After Shave Balsam

Anwendungsbeispiel 762:

| Phase A | |
|---|---|
| Acrylat/$C_{10-30}$ Alkylacrylat-Copolymer | 0,25 |
| Tocopherylacetat | 1,50 |
| Bisabolol | 0,20 |
| Capryl/Caprin-Triglycerid | 10,00 |
| Parfüm | q.s. |
| hydriertes Rizinusöl-PEG-40 | 1,00 |

| Phase B | |
|---|---|
| Panthenol | 1,00 |
| Alkohol | 15,00 |

(fortgesetzt)

| Phase B | |
|---|---|
| Glycerin | 5,00 |
| Hydroxyethylcellulose | 0,05 |
| Isoalkangemisch aus Beispiel 2 | 1,92 |
| dest. Wasser | 64,00 |

| Phase C | |
|---|---|
| Natriumhydroxid | 0,08 |

Herstellung: Die Komponenten der Phase A vermischt man. Danach rührte man Phase B unter Homogenisieren in Phase A ein und homogenisierte kurz nach. Mit Phase C neutralisierte man und homogenisierte erneut.

Anwendungsbeispiel 763 und Anwendungsbeispiel 764: Man wiederholte Anwendungsbeispiel 762, setzte aber anstelle des Isoalkangemischs aus Beispiel 2 das Isoalkangemisch aus Beispiel 3 beziehungsweise aus Beispiel 4 ein.

Anwendungsbeispiele 765-767: Zahnpasta

Anwendungsbeispiel 765:

| Phase A | |
|---|---|
| dest. Wasser | 34,79 |
| Isoalkangemisch aus Beispiel 2 | 3,00 |
| Konservierungsmittel | 0,30 |
| Glycerin | 20,00 |
| Natriummonofluorphosphat | 0,76 |

| Phase B | |
|---|---|
| Natriumcarboxymethylcellulose | 1,20 |

| Phase C | |
|---|---|
| Aromaöl | 0,80 |
| Saccharin | 0,06 |
| Konservierungsmittel | 0,10 |
| Bisabolol | 0,05 |
| Panthenol | 1,00 |
| Tocopherylacetat | 0,50 |
| Siliciumdioxid | 2,80 |
| Natriumlaurylsulfat | 1,00 |
| Dicalciumphosphat, wasserfrei | 7,90 |

(fortgesetzt)

| Phase C | |
|---|---|
| Dicalciumphosphat-Dihydrat | 25,29 |
| Titandioxid | 0,45 |

Herstellung: Man löste Phase A. Danach streute man Phase B in Phase A ein und löste. Man gab Phase C zu und ließ unter vermindertem Druck bei Raumtemperatur etwa 45 Minuten.

Anwendungsbeispiel 766 und Anwendungsbeispiel 767: Man wiederholte Anwendungsbeispiel 765, setzte aber anstelle des Isoalkangemischs aus Beispiel 2 das Isoalkangemisch aus Beispiel 3 beziehungsweise aus Beispiel 4 ein.

Anwendungsbeispiele 768-770: Mundwasser

Anwendungsbeispiel 768:

| Phase A | |
|---|---|
| Aromaöl | 2,00 |
| hydriertes Rizinusöl-PEG-40 | 4,00 |
| Bisabolol | 1,00 |
| Alkohol | 30,00 |

| Phase B | |
|---|---|
| Saccharin | 0,20 |
| Glycerin | 5,00 |
| Konservierungsmittel | q.s. |
| Poloxamer 407 | 5,00 |
| Isoalkangemisch aus Beispiel 2 | 2,5 |
| dest. Wasser | 50,30 |

Herstellung: Die Phasen A und Phase B wurden getrennt klar gelöst. Man rührte Phase B in Phase A ein.

Anwendungsbeispiel 769 und Anwendungsbeispiel 770: Man wiederholte Anwendungsbeispiel 768, setzte aber anstelle des Isoalkangemischs aus Beispiel 2 das Isoalkangemisch aus Beispiel 3 beziehungsweise aus Beispiel 4 ein.

Anwendungsbeispiele 771-773: Prothesenhaftmittel

Anwendungsbeispiel 771:

| Phase A | |
|---|---|
| Bisabolol | 0,20 |
| Betacarotin | 1,00 |
| Aromaöl | q.s. |

(fortgesetzt)

| Phase A | |
|---|---|
| Cetearyloctanoat | 20,00 |
| Siliciumdioxid | 5,00 |
| Mineralöl | 33,80 |

| Phase B | |
|---|---|
| Isoalkangemisch aus Beispiel 2 | 5,00 |
| PVP (20 %ige Lösung in Wasser) | 35,00 |

Herstellung: Man mischte Phase A gründlich. Danach rührte man Phase B in Phase A ein.

Anwendungsbeispiel 772 und Anwendungsbeispiel 773: Man wiederholte Anwendungsbeispiel 771, setzte aber anstelle des Isoalkangemischs aus Beispiel 2 das Isoalkangemisch aus Beispiel 3 beziehungsweise aus Beispiel 4 ein.

Anwendungsbeispiele 774-776: Lippenpflegecreme

Anwendungsbeispiel 774:

| Phase A | |
|---|---|
| Cetearyloctanoat | 10,00 |
| Polybuten | 5,00 |

| Phase B | |
|---|---|
| Carbomer | 0,10 |

| Phase C | |
|---|---|
| Ceteareth-6 | 2,00 |
| Ceteareth-25 | 2,00 |
| Glycerylstearat | 2,00 |
| Cetylalkohol | 2,00 |
| Dimethicon | 1,00 |
| Benzophenone-3 | 1,00 |
| Bisabolol | 0,20 |
| Mineralöl | 6,00 |

| Phase D | |
|---|---|
| Isoalkangemisch aus Beispiel 2 | 8,00 |
| Panthenol | 3,00 |

(fortgesetzt)

| Phase D | |
|---|---|
| Propylenglykol | 3,00 |
| Konservierungsmittel | q.s. |
| dest. Wasser | 54,00 |

| Phase E | |
|---|---|
| Triethanolamin | 0,10 |

| Phase F | |
|---|---|
| Tocopherylacetat | 0,50 |
| Tocopherol | 0,10 |
| Parfüm | q.s. |

Herstellung: Phase A wurde klar gelöst. Man gab Phase B dazu und homogenisierte. Man gab die Komponenten der Phase C zu und schmolz bei 80 °C. Die Phase D erwärmte man auf 80 °C. Man gab Phase D zu dem Gemisch aus den Phasen A, B und C und homogenisierte. Man ließ auf ca. 40 °C abkühlen, gab die Phase E und Phase F zu und homogenisierte nochmals.

Anwendungsbeispiel 775 und Anwendungsbeispiel 776: Man wiederholte Anwendungsbeispiel 774, setzte aber anstelle des Isoalkangemischs aus Beispiel 2 das Isoalkangemisch aus Beispiel 3 beziehungsweise aus Beispiel 4 ein.

Anwendungsbeispiele 777-779: Erfrischungsgel

Anwendungsbeispiel 777:

| Phase A | |
|---|---|
| Carbomer | 0,60 |
| dest. Wasser | 45,40 |

| Phase B | |
|---|---|
| Bisabolol | 0,50 |
| Farnesol | 0,50 |
| Parfüm | q.s. |
| PEG-40 Hydrogenated Castor Oil | 5,00 |
| Isoalkangemisch aus Beispiel 2 | 2,50 |
| Tetrahydroxypropylethylendiamin | 1,00 |
| Menthol | 1,50 |
| Alkohol | 43,00 |
| C. I. 74 180, Direct Blue 86 | q.s. |

Herstellung: Man ließ Phase A quellen, löste Phase B und rührte Phase B in Phase A ein.

Anwendungsbeispiel 778 und Anwendungsbeispiel 779: Man wiederholte Anwendungsbeispiel 777, setzte aber anstelle des Isoalkangemischs aus Beispiel 2 das Isoalkangemisch aus Beispiel 3 beziehungsweise aus Beispiel 4 ein.

Anwendungsbeispiele 780-782: Roll-on Antiperspirant

Anwendungsbeispiel 780:

| Phase A | |
|---|---|
| Hydroxyethylcellulose | 0,40 |
| dest. Wasser | 50,00 |

| Phase B | |
|---|---|
| Alkohol | 25,00 |
| Bisabolol | 0,10 |
| Farnesol | 0,30 |
| PEG-40 Hydrogenated Castor Oil | 2,00 |
| Parfüm | q.s. |

| Phase C | |
|---|---|
| Aluminumchlorhydrat | 5,00 |
| Propylenglykol | 3,00 |
| Dimethicon-Copolyol | 3,00 |
| Polyquaternium-16 | 3,00 |
| Isoalkangemisch aus Beispiel 2 | 6,00 |
| dest. Wasser | 2,20 |

Herstellung: Man ließ Phase A quellen; danach löste man getrennt jeweils die Phasen B und C. Man rührte Phase A und B in Phase C ein.

Anwendungsbeispiel 781 und Anwendungsbeispiel 782: Man wiederholte Anwendungsbeispiel 780, setzte aber anstelle des Isoalkangemischs aus Beispiel 2 das Isoalkangemisch aus Beispiel 3 beziehungsweise aus Beispiel 4 ein.

Anwendungsbeispiele 783-785: Transparenter Deostift

Anwendungsbeispiel 783:

| | |
|---|---|
| Natriumstearat | 5,00 |
| Triclosan | 0,50 |
| Ceteareth-25 | 3,00 |
| Glycerin | 20,00 |

(fortgesetzt)

| | |
|---|---|
| Isoalkangemisch aus Beispiel 2 | 2,50 |
| Parfüm | q.s. |
| Propylenglykol | 60,00 |
| Bisabolol | 0,20 |
| dest. Wasser | 10,80 |

Herstellung: Phase A wurde zusammen eingewogen, geschmolzen und homogenisiert. Danach goss man in eine Form.

Anwendungsbeispiel 784 und Anwendungsbeispiel 785: Man wiederholte Anwendungsbeispiel 783, setzte aber anstelle des Isoalkangemischs aus Beispiel 2 das Isoalkangemisch aus Beispiel 3 beziehungsweise aus Beispiel 4 ein.

Anwendungsbeispiele 786-787: Tagespflege-Aerosol

Anwendungsbeispiel 786:

| Phase A | |
|---|---|
| Ethylhexylmethoxycinnamate | 4,00 |
| Octocrylen | 1,50 |
| Capryl/Caprin-Triglycerid | 9,00 |
| Simmondsia Chinensis (Jojoba) Seed Oil | 5,00 |
| Cyclomethicon | 1,50 |
| Hydrogenated Coco-Glycerides | 3,00 |
| PVP/Hexadecen-Copolymer | 1,00 |
| Ceteareth-6, Stearylalkohol | 1,00 |

| Phase B | |
|---|---|
| Zinkoxid | 5,00 |

| Phase C | |
|---|---|
| Ceteareth-25 | 2,00 |
| Panthenol | 1,20 |
| Sodium Ascorbyl Phosphate | 0,20 |
| Imidazolidinyl-Harnstoff | 0,30 |
| Disodium EDTA | 0,10 |
| Isoalkangemisch aus Beispiel 2 | 7,50 |
| dest. Wasser | 56,67 |

| Phase D | |
|---|---|
| Tocopherylacetat | 0,50 |
| Bisabolol | 0,20 |
| Capryl/Caprin-Triglycerid, Retinol | 0,33 |
| Parfüm | q.s. |

Herstellung: Man erwärmte Phase A auf 80 °C. Die Phase A löste sich klar. Man arbeitete Phase B ein und homogenisierte. Danach gab man Phase C zu, erhitzte auf 80 °C, schmolz auf und homogenisierte. Unter Rühren ließ man auf ca. 40 °C abkühlen, gab Phase D hinzu und homogenisierte kurz. 90 % Wirkstofflösung: 10 % Propan/Butan mit 3,5 bar (20 °C) abfüllen.

Anwendungsbeispiel 787 und Anwendungsbeispiel 788: Man wiederholte Anwendungsbeispiel 786, setzte aber anstelle des Isoalkangemischs aus Beispiel 2 das Isoalkangemisch aus Beispiel 3 beziehungsweise aus Beispiel 4 ein.

Anwendungsbeispiele 789-791: Pumpmousse

Anwendungsbeispiel 789:

| Phase A | |
|---|---|
| Cocotrimoniummethosulfat | 2,00 |
| Parfüm | q.s. |

| Phase B | |
|---|---|
| dest. Wasser | 84,30 |
| Polyquaternium-46 (10 %ige wässrige Lösung) | 7,00 |
| Isoalkangemisch aus Beispiel 2 | 5,00 |
| PEG-8 | 0,50 |
| Panthenol | 1,00 |
| Konservierungsmittel | q.s. |
| PEG-25 PABA (ethoxilierte p-Aminobenzoesäure) | 0,20 |

Herstellung: Die Komponenten der Phase A vermischte man. Die Komponenten der Phase B gab man nacheinander zu und es bildete sich eine klare Lösung.

Anwendungsbeispiel 790 und Anwendungsbeispiel 791: Man wiederholte Anwendungsbeispiel 789, setzte aber anstelle des Isoalkangemischs aus Beispiel 2 das Isoalkangemisch aus Beispiel 3 beziehungsweise aus Beispiel 4 ein.

Anwendungsbeispiele 792-794: Aerosolschaum

Anwendungsbeispiel 792:

| | |
|---|---|
| Isoalkangemisch aus Beispiel 2 | 15,00 |
| PVP/VA-Copolymer (40 %ige wässrige Lösung) | 5,00 |

(fortgesetzt)

| | |
|---|---|
| Hydroxyethylcetyldimoniumphosphat | 0,50 |
| Ceteareth-25 | 0,20 |
| Parfüm PC 910.781/Cremophor | 0,40 |
| dest. Wasser | 68,90 |
| Konservierungsmittel | q.s. |
| Propan/Butan 3,5 bar (20 °C) | 10,00 |

Herstellung: Man wog alles zusammen ein, rührte, bis sich alles gelöst hatte und füllte ab.

Anwendungsbeispiel 793 und Anwendungsbeispiel 794: Man wiederholte Anwendungsbeispiel 792, setzte aber anstelle des Isoalkangemischs aus Beispiel 2 das Isoalkangemisch aus Beispiel 3 beziehungsweise aus Beispiel 4 ein.

Anwendungsbeispiele 795-797: Farbstyling-Mousse

Anwendungsbeispiel 795:

| Phase A | |
|---|---|
| Cocotrimoniummethosulfat | 2,00 |
| Parfüm | q.s. |

| Phase B | |
|---|---|
| Isoalkangemisch aus Beispiel 2 | 23,50 |
| Acrylatcopolymer (Luvimer 100 P®, Fa. BASF) | 0,50 |
| Aminomethylpropanol | 0,10 |
| Ceteareth-25 | 0,20 |
| Panthenol | 0,20 |
| Hydroxyethylcellulose | 0,20 |
| Alkohol | 10,00 |
| dest. Wasser | 53,17 |
| C.I. 12245, Basic Red 76 | 0,08 |
| C.I. 42510, Basic Violet 14 | 0,05 |

| Phase C | |
|---|---|
| Propan/Butan 3,5 bar (20 °C) | 10,00 |

Herstellung: Man wog alles zusammen ein, rührte bis sich alles gelöst hatte und füllt danach ab. Diese Farbstyling-Mousse ist für dunkelblondes und braunes Haar geeignet.

Anwendungsbeispiel 796 und Anwendungsbeispiel 797: Man wiederholte Anwendungsbeispiel 795, setzte aber anstelle des Isoalkangemischs aus Beispiel 2 das Isoalkangemisch aus Beispiel 3 beziehungsweise aus Beispiel 4 ein.

Anwendungsbeispiele 798-800: dunkelbraune Permanenthaarfarbe (Oxidationshaarfarbe)

Anwendungsbeispiel 798:

| Phase A | |
|---|---|
| dest. Wasser | 46,90 |
| Natriumsulfit | 0,20 |
| Dinatrium-EDTA | 0,05 |
| p-Phenylendiamin | 0,20 |
| Resorcinol | 0,30 |
| 4-Amino-2-hydroxytoluol | 0,20 |
| m-Aminophenol | 0,10 |
| Oleylalkohol | 1,50 |
| Propylenglykol | 4,50 |
| Sodium $C_{12-15}$ Pareth-15 Sulfonate | 2,30 |
| Ölsäure | 20,00 |

| Phase B | |
|---|---|
| Isoalkangemisch aus Beispiel 2 | 5,00 |
| Ammoniumhydroxid | 13,70 |
| i-Propanol | 6,00 |
| Parfum | q.s. |

Herstellung: Man solubiliserte Phase A. Die Komponenten der Phase B gab man nacheinander zu und vermischte.

Anwendungsbeispiel 799 und Anwendungsbeispiel 800: Man wiederholte Anwendungsbeispiel 798, setzte aber anstelle des Isoalkangemischs aus Beispiel 2 das Isoalkangemisch aus Beispiel 3 beziehungsweise aus Beispiel 4 ein.

Anwendungsbeispiel 801-803: hellbraune Semi-Permanenthaarfarbe

Anwendungsbeispiel 801:

| | |
|---|---|
| Cocodiethanolamid | 10,00 |
| Natriumdodecylbenzylsulfonat, 50 %ig | 4,00 |
| Isoalkangemisch aus Beispiel 2 | 5,00 |
| $C_{9-11}$ Pareth-3 | 6,00 |
| Natriumlaurylsulfat | 2,50 |
| 2-Nitro-p-phenylendiamin | 0,40 |
| HC Red No.3 | 0,20 |
| HC Yellow No.2 | 0,20 |

(fortgesetzt)

| | |
|---|---|
| dest. Wasser | 71,70 |

Herstellung: Man gab die Komponenten nacheinander zu und mischte.

Anwendungsbeispiel 802 und Anwendungsbeispiel 803: Man wiederholte Anwendungsbeispiel 801, setzte aber anstelle des Isoalkangemischs aus Beispiel 2 das Isoalkangemisch aus Beispiel 3 beziehungsweise aus Beispiel 4 ein.

Anwendungsbeispiele 804-806: Festiger-Lösung

Anwendungsbeispiel 804:

| Zusatz | Gew.-% |
|---|---|
| Isoalkangemisch aus Beispiel 2 | 18,00 |
| Ethanol | 82,00 |
| weiterer Zusatz: Silikon, Parfüm, Entschäumer, usw. | |

Anwendungsbeispiel 805 und Anwendungsbeispiel 806: Man wiederholte Anwendungsbeispiel 804, setzte aber anstelle des Isoalkangemischs aus Beispiel 2 das Isoalkangemisch aus Beispiel 3 beziehungsweise aus Beispiel 4 ein.

## Patentansprüche

**1.** Kosmetisches oder pharmazeutisches Mittel, enthaltend

A) wenigstens ein Isoalkangemisch, dessen $^1$H-NMR-Spektrum im Bereich einer chemischen Verschiebung $\delta$ von 0,6 bis 1,0 ppm, bezogen auf Tetramethylsilan, ein Flächenintegral von 25 bis 70 %, bezogen auf die Gesamtintegralfläche, aufweist, das wenigstens 70 Gew.-% Alkane mit 8 bis 20 Kohlenstoffatomen enthält, erhalten durch ein Verfahren, bei dem man

a) ein $C_4$-Kohlenwasserstoffgemisch bereitstellt, das weniger als 3 Gew.-% Isobuten enthält,
b) das Kohlenwasserstoffeinsatzmaterial einer Oligomerisierung an einem Übergangsmetall-haltigen Katalysator unterwirft,
c) das in Schritt b) erhaltene Oligomerisierungsprodukt vollständig hydriert,

B) wenigstens einen kosmetisch oder pharmazeutisch akzeptablen Wirk- oder Effektstoff,
C) gegebenenfalls wenigstens einen weiteren, von B) verschiedenen kosmetisch oder pharmazeutisch akzeptablen Hilfsstoff.

**2.** Kosmetisches oder pharmazeutisches Mittel nach Anspruch 1, wobei das $^1$H-NMR-Spektrum des Isoalkangemischs im Bereich einer chemischen Verschiebung $\delta$ von 0,6 bis 1,0 ppm ein Flächenintegral von 30 bis 60 %, bezogen auf die Gesamtintegralfläche, aufweist.

**3.** Kosmetisches oder pharmazeutisches Mittel nach einem der Ansprüche 1 oder 2, wobei das Isoalkangemisch einen Verzweigungsgrad V im Bereich von 0,1 bis 0,35 aufweist.

**4.** Kosmetisches oder pharmazeutisches Mittel nach einem der vorhergehenden Ansprüche, wobei das Isoalkangemisch einen Anteil an endständigen tert.-Butylgruppen von höchstens 20% aufweist.

**5.** Kosmetisches oder pharmazeutisches Mittel nach einem der vorhergehenden Ansprüche, wobei das Isoalkangemisch wenigstens 85 Gew.-%, Alkane mit 8 bis 20 Kohlenstoffatomen enthält.

6. Kosmetisches oder pharmazeutisches Mittel nach einem der vorhergehenden Ansprüche, wobei das Isoalkangemisch wenigstens 70 Gew.-% Alkane mit 12 bis 20 Kohlenstoffatomen enthält.

7. Kosmetisches oder pharmazeutisches Mittel nach einem der vorhergehenden Ansprüche, wobei das Isoalkangemisch wenigstens 70 Gew.-% Alkane mit einer geraden Kohlenstoffatomanzahl enthält.

8. Kosmetisches oder pharmazeutisches Mittel nach einem der vorhergehenden Ansprüche, wobei das Isoalkangemisch, bezogen auf sein Gesamtgewicht, weniger als 95 Gew.-% Alkane desselben Molekulargewichts enthält.

9. Kosmetisches oder pharmazeutisches Mittel nach einem der vorhergehenden Ansprüche, wobei das Isoalkangemisch einen Spreitwert von wenigstens 130 %,bezogen auf Paraffinum perliquidum, aufweist.

10. Kosmetisches oder pharmazeutisches Mittel nach einem der vorhergehenden Ansprüche, wobei das Isoalkangemisch eine Viskosität, bestimmt nach Brookfield, im Bereich von 2 bis 10 mPas aufweist.

11. Kosmetisches oder pharmazeutisches Mittel nach einem der vorhergehenden Ansprüche, wobei man das in Schritt b) erhaltene Oligomerisierungsprodukt und/oder das in Schritt c) erhaltene Hydrierungsprodukt einer Auftrennung unterzieht.

12. Kosmetisches oder pharmazeutisches Mittel nach einem der vorhergehenden Ansprüche, wobei in Schritt a) ein Kohlenwasserstoffgemisch bereitgestellt wird, das einen Gehalt an linearen Olefinen von mindestens 80 Gew.-%, bezogen auf den Gesamtolefingehalt, aufweist.

13. Kosmetisches oder pharmazeutisches Mittel nach einem der vorhergehenden Ansprüche, wobei der in Schritt b) eingesetzte Katalysator ein heterogener, Nickel enthaltender Katalysator ist.

14. Kosmetisches oder pharmazeutisches Mittel nach einem der vorhergehenden Ansprüche, wobei das Isoalkangemisch durch ein Verfahren erhältlich ist, bei dem man das in Schritt c) erhaltene hydrierte Oligomerisierungsprodukt einer Aufarbeitung durch Inkontaktbringen mit wenigstens einem Adsorptionsmittel unterzieht.

15. Kosmetisches oder pharmazeutisches Mittel nach einem der vorhergehenden Ansprüche in Form einer Salbe, Creme, Emulsion, Suspension, Lotion, Milch, Paste, eines Gels, Schaums oder Sprays.

16. Kosmetisches oder pharmazeutisches Mittel nach einem der vorhergehenden Ansprüche, das die Komponente A) in einem Anteil von 0,01 bis 99,9 Gew.-%, bezogen auf das Gesamtgewicht des Mittels, enthält.

17. Kosmetisches oder pharmazeutisches Mittel nach einem der vorhergehenden Ansprüche, wobei die Komponenten B) und C) ausgewählt sind unter Trägern, Exzipienten, Emulgatoren, Tensiden, Konservierungsmitteln, Duftstoffen, Parfümölen, Verdickern, Polymeren, Gelbildnern, Farbstoffen, Pigmenten, Lichtschutzmitteln, Konsistenzgebern, Antioxidantien, Entschäumern, Antistatika, Harzen, Lösemitteln, Lösungsvermittlern, Neutralisierungsmitteln, Stabilisatoren, Sterilantien, Treibmitteln, Trocknungsmitteln, Trübungsmitteln, kosmetisch aktiven Wirkstoffen, Haarpolymeren, Haar- und Hautconditionern, Pfropfpolymeren, wasserlöslichen oder dispergierbaren silikonhaltigen Polymeren, Bleichmitteln, Pflegemitteln, Färbemitteln, Tönungsmitteln, Bräunungsmitteln, Feuchthaltemitteln, Rückfettern, Collagen, Eiweisshydrolysaten, Lipiden, Emollienzien und Weichmachern und Mischungen davon.

18. Haarkosmetisches Mittel, enthaltend

a) 0,05 bis 20 Gew.-% wenigstens eines Haarpolymers,
b) 20 bis 99,95 Gew.-% eines Trägers, enthaltend wenigstens ein Isoalkangemisch, wie in einem der Ansprüche 1 bis 14 definiert,
c) 0 bis 50 Gew.-% wenigstens eines Treibgases,
d) 0 bis 5 Gew.-% wenigstens eines Emulgators,
e) 0 bis 3 Gew.-% wenigstens eines Verdickers, sowie
f) bis zu 25 Gew.-% weitere Bestandteile.

19. Haarkosmetisches Mittel nach Anspruch 18, enthaltend als Wirk- oder Effektstoff B) oder Hilfsstoff C) wenigstens

ein Copolymerisat Ca), das erhältlich ist durch Copolymerisation von

(A) mindestens einem ethylenisch ungesättigten Dicarbonsäureanhydrid, abgeleitet von mindestens einer Dicarbonsäure mit 4 bis 8 C-Atomen,
(B) mindestens einem Oligomeren von verzweigtem oder unverzweigtem $C_3$-$C_{10}$-Alken, wobei mindestens ein Oligomer ein mittleres Molekulargewicht $M_n$ im Bereich von 300 bis 5000 g/mol aufweist oder durch Oligomerisierung von mindestens 3 Äquivalenten $C_3$-$C_{10}$-Alken erhältlich ist,
(C) optional mindestens einem $\alpha$-Olefin mit bis zu 24, bevorzugt mit bis zu 16 C-Atomen,
(D) optional mindestens einem weiteren von (A), (B) und (C) verschiedenen ethylenisch ungesättigten Comonomer, gegebenenfalls Umsetzung mit
(E) mindestens einer Verbindung der allgemeinen Formel Ia, Ib, Ic oder Id

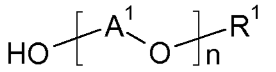
Ia

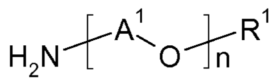
Ib

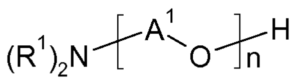
Ic

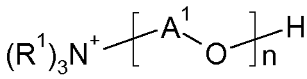
Id

wobei

$A^1$ $C_2$-$C_{20}$-Alkylen, gleich oder verschieden,
$R^1$ $C_1$-$C_{30}$-Alkyl, linear oder verzweigt, Phenyl oder Wasserstoff,
n eine ganze Zahl von 1 bis 200 und
gegebenenfalls anschließendes Kontaktieren mit Wasser,

wobei das Copolymerisat Ca) gegebenenfalls gemischt ist mit mindestens einem zusätzlichen Oligomer Z) von verzweigtem oder unverzweigtem $C_3$-$C_{10}$-Alken, wobei mindestens ein Oligomer Z) ein mittleres Molekulargewicht $M_n$ im Bereich von 300 bis 5000 g/mol aufweist oder durch Oligomerisierung von mindestens 3 Äquivalenten $C_3$-$C_{10}$-Alken erhältlich ist,
wobei die Copolymerkomponente B) und/oder das Oligomer Z) ein Isoalkangemisch, wie in einem der Ansprüche 1 bis 14 definiert, umfasst oder aus einem solchen Isoalkangemisch besteht.

**20.** Verwendung eines kosmetischen oder pharmazeutischen Mittels wie in einem der Ansprüche 1 bis 14 definiert, als oder in der hydrophoben Komponente einer homogenphasigen oder heterogenphasigen kosmetischen oder pharmazeutischen Zusammensetzung.

**21.** Verwendung eines kosmetischen oder pharmazeutischen Mittels wie in einem der Ansprüche 1 bis 14 definiert, in Mitteln zur Pflege und zum Schutz der Haut, Hautreinigungsmitteln, Zubereitungen für die dekorative Kosmetik, in Haarbehandlungsmitteln, zur Feuchthaltung der Haut, zur Konditionierung von Haut oder Haaren, zur Modifizierung der rheologischen Eigenschaften, zur Verbesserung der Hautverträglichkeit und/oder zur Erzeugung einer besseren Benetzung.

## Claims

**1.** A cosmetic or pharmaceutical composition comprising

A) at least one isoalkane mixture, which has a chemical shift $\delta$ in the $^1$H-NMR spectrum in the range of 0.6 to 1.0 ppm, based on tetramethylsilane, and has an area integral of 25 to 70%, based on the total area integral, which comprises at least 70% by weight alkanes having 8 to 20 carbon atoms, obtained by a method in which

a) a $C_4$-hydrocarbon mixture is provided comprising less than 3% by weight isobutene,

b) the hydrocarbon feed material is subjected to an oligomerization over a transition metal-containing catalyst,
c) the oligomerization product obtained in step b) is completely hydrogenated,

B) at least one cosmetically or pharmaceutically acceptable active ingredient or effect ingredient,
C) optionally at least one further cosmetically or pharmaceutically acceptable auxiliary different from B).

**2.** The cosmetic or pharmaceutical composition according to claim 1, wherein the isoalkane mixture has a chemical shift $\delta$ in the $^1$H-NMR spectrum in the range of 0.6 to 1.0 ppm and an area integral of 30 to 60%, based on the total area integral.

**3.** The cosmetic or pharmaceutical composition according to either of claims 1 and 2, wherein the isoalkane mixture has a degree of branching V in the range from 0.1 to 0.35.

**4.** The cosmetic or pharmaceutical composition according to any of the preceding claims, wherein the isoalkane mixture has a proportion of terminal tert-butyl groups of at most 20%.

**5.** The cosmetic or pharmaceutical composition according to any of the preceding claims, wherein the isoalkane mixture comprises at least 85% by weight alkanes having 8 to 20 carbon atoms.

**6.** The cosmetic or pharmaceutical composition according to any of the preceding claims, wherein the isoalkane mixture comprises at least 70% by weight alkanes having 12 to 20 carbon atoms.

**7.** The cosmetic or pharmaceutical composition according to any of the preceding claims, wherein the isoalkane mixture comprises at least 70% by weight alkanes having an even number of carbon atoms.

**8.** The cosmetic or pharmaceutical composition according to any of the preceding claims, wherein the isoalkane mixture, based on its total weight, comprises less than 95% by weight alkanes of the same molecular weight.

**9.** The cosmetic or pharmaceutical composition according to any of the preceding claims, wherein the isoalkane mixture has a spreading value of at least 130%, based on Paraffinum perliquidum.

**10.** The cosmetic or pharmaceutical composition according to any of the preceding claims, wherein the isoalkane mixture has a viscosity, determined according to Brookfield, in the range of 2 to 10 mPas.

**11.** The cosmetic or pharmaceutical composition according to any of the preceding claims, wherein the oligomerization product obtained in step b) and/or the hydrogenation product obtained in step c) is subjected to a fractionation.

**12.** The cosmetic or pharmaceutical composition according to any of the preceding claims, wherein a hydrocarbon mixture is provided in step a) having a content of linear olefins of at least 80% by weight, based on the total olefin content.

**13.** The cosmetic or pharmaceutical composition according to any of the preceding claims, wherein the catalyst used in step b) is a heterogeneous catalyst comprising nickel.

**14.** The cosmetic or pharmaceutical composition according to any of the preceding claims, wherein the isoalkane mixture is obtainable by a method in which the hydrogenated oligomerization product obtained in step c) is subjected to a work-up by bringing into contact with at least one adsorbent.

**15.** The cosmetic or pharmaceutical composition according to any of the preceding claims in the form of an ointment, cream, emulsion, suspension, lotion, milk, paste, a gel, foam or spray.

**16.** The cosmetic or pharmaceutical composition according to any of the preceding claims, which comprises component A) in a proportion of 0.01 to 99.9% by weight, based on the total weight of the composition.

**17.** The cosmetic or pharmaceutical composition according to any of the preceding claims, wherein components B) and C) are selected from carriers, excipients, emulsifiers, surfactants, preservatives, fragrances, perfume oils, thickeners, polymers, gel formers, dyes, pigments, photoprotective agents, consistency regulators, antioxidants, antifoams,

antistats, resins, solvents, solubilizers, neutralizing agents, stabilizers, sterilizing agents, propellants, drying agents, opacifiers, cosmetically active ingredients, hair polymers, hair and skin conditioners, graft polymers, water-soluble or dispersible silicone-containing polymers, bleaches, care agents, colorants, tinting agents, tanning agents, humectants, refatting agents, collagen, protein hydrolyzates, lipids, emollients and softeners and mixtures thereof.

**18.** A hair cosmetic composition comprising

a) 0.05 to 20% by weight of at least one hair polymer,
b) 20 to 99.95% by weight of a carrier comprising at least one isoalkane mixture as defined in any of claims 1 to 14,
c) 0 to 50% by weight of at least one propellant gas,
d) 0 to 5% by weight of at least one emulsifier,
e) 0 to 3% of at least one thickener, and
f) up to 25% by weight of further constituents.

**19.** The hair cosmetic composition according to claim 18 comprising as active ingredient or effect ingredient B) or auxiliary C), at least one copolymer Ca) obtainable by copolymerization of

(A) at least one ethylenically unsaturated dicarboxylic anhydride derived from at least one dicarboxylic acid having 4 to 8 carbon atoms,
(B) at least one oligomer of branched or unbranched $C_3$-$C_{10}$-alkene, wherein at least one oligomer has an average molecular weight $M_n$ in the range from 300 to 5000 g/mol or is obtainable by oligomerization of at least 3 equivalent $C_3$-$C_{10}$-alkenes,
(C) optionally at least one $\alpha$-olefin having up to 24, preferably having up to 16 carbon atoms,
(D) optionally at least one further ethylenically unsaturated comonomer different from (A), (B) and (C), optionally reaction with
(E) at least one compound of the general formula Ia, Ib, Ic or Id

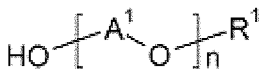

Ia

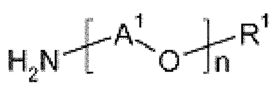

Ib

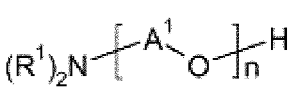

Ic

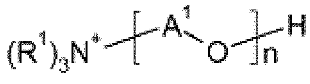

Id

where

$A^1$ is $C_2$-$C_{20}$-alkylene, identical or different,
$R^1$ is $C_1$-$C_{30}$-alkyl, linear or branched, phenyl or hydrogen,
n is an integer from 1 to 200 and
optionally subsequent contact with water,

wherein the copolymer Ca) is optionally mixed with at least one additional oligomer Z) of branched or unbranched $C_3$-$C_{10}$-alkene, wherein at least one oligomer Z) has an average molecular weight $M_n$ in the range from 300 to 5000 g/mol or is obtainable by oligomerization of at least 3 equivalent $C_3$-$C_{10}$-alkenes,
wherein the copolymer component B) and/or the oligomer Z) comprises an isoalkane mixture as defined in any of claims 1 to 14 or consists of such an isoalkane mixture.

**20.** The use of a cosmetic or pharmaceutical composition as defined in any of claims 1 to 14, as or in the hydrophobic component of a homogeneous phase or heterogeneous phase cosmetic or pharmaceutical composition.

**21.** The use of a cosmetic or pharmaceutical composition as defined in any of claims 1 to 14, in compositions for the care and protection of the skin, hair cleansing compositions, preparations for decorative cosmetics, in hair treatment compositions, for moisturizing skin, for conditioning skin or hair, for modifying the rheological properties, for improving

skin tolerability and/or generating better wetting.

## Revendications

1. Agent cosmétique ou pharmaceutique, contenant :

A) au moins un mélange d'isoalcanes, dont le spectre RMN-$^1$H présente, dans la plage d'un déplacement chimique $\delta$ de 0,6 à 1,0 ppm, par rapport au tétraméthylsilane, une intégrale de surface de 25 à 70 %, par rapport à la surface d'intégrale totale, qui contient au moins 70 % en poids d'alcanes de 8 à 20 atomes de carbone, obtenu par un procédé selon lequel

a) un mélange d'hydrocarbures en $C_4$ est préparé, qui contient moins de 3 % en poids d'isobutène,
b) la matière première hydrocarbonée est soumise à une oligomérisation sur un catalyseur contenant un métal de transition,
c) le produit d'oligomérisation obtenu à l'étape b) est entièrement hydrogéné,

B) au moins un agent actif ou à effet cosmétiquement ou pharmaceutiquement acceptable,
C) éventuellement au moins un autre adjuvant cosmétiquement ou pharmaceutiquement acceptable différent de B).

2. Agent cosmétique ou pharmaceutique selon la revendication 1, dans lequel le spectre RMN-$^1$H du mélange d'isoalcanes présente, dans la plage d'un déplacement chimique $\delta$ de 0,6 à 1,0 ppm, une intégrale de surface de 30 à 60 %, par rapport la surface d'intégrale totale.

3. Agent cosmétique ou pharmaceutique selon l'une quelconque des revendications 1 ou 2, dans lequel le mélange d'isoalcanes présente un degré de ramification V dans la plage allant de 0,1 à 0,35.

4. Agent cosmétique ou pharmaceutique selon l'une quelconque des revendications précédentes, dans lequel le mélange d'isoalcanes présente une proportion de groupes tert.-butyle terminaux d'au plus 20 %.

5. Agent cosmétique ou pharmaceutique selon l'une quelconque des revendications précédentes, dans lequel le mélange d'isoalcanes contient au moins 85 % en poids d'alcanes de 8 à 20 atomes de carbone.

6. Agent cosmétique ou pharmaceutique selon l'une quelconque des revendications précédentes, dans lequel le mélange d'isoalcanes contient au moins 70 % en poids d'alcanes de 12 à 20 atomes de carbone.

7. Agent cosmétique ou pharmaceutique selon l'une quelconque des revendications précédentes, dans lequel le mélange d'isoalcanes contient au moins 70 % en poids d'alcanes ayant un nombre pair d'atomes de carbone.

8. Agent cosmétique ou pharmaceutique selon l'une quelconque des revendications précédentes, dans lequel le mélange d'isoalcanes contient, par rapport à son poids total, moins de 95 % en poids d'alcanes du même poids moléculaire.

9. Agent cosmétique ou pharmaceutique selon l'une quelconque des revendications précédentes, dans lequel le mélange d'isoalcanes présente une valeur d'étalement d'au moins 130 %, par rapport au Paraffinum perliquidum.

10. Agent cosmétique ou pharmaceutique selon l'une quelconque des revendications précédentes, dans lequel le mélange d'isoalcanes présente une viscosité, déterminée selon Brookfied, dans la plage allant de 2 à 10 mPas.

11. Agent cosmétique ou pharmaceutique selon l'une quelconque des revendications précédentes, dans lequel le produit d'oligomérisation obtenu à l'étape b) et/ou le produit d'hydrogénation obtenu à l'étape c) sont soumis à une séparation.

12. Agent cosmétique ou pharmaceutique selon l'une quelconque des revendications précédentes, dans lequel un mélange d'hydrocarbures qui présente une teneur en oléfines linéaires d'au moins 80 % en poids, par rapport à la teneur totale en oléfines, est préparé à l'étape a).

13. Agent cosmétique ou pharmaceutique selon l'une quelconque des revendications précédentes, dans lequel le catalyseur utilisé à l'étape b) est un catalyseur hétérogène contenant du nickel.

**14.** Agent cosmétique ou pharmaceutique selon l'une quelconque des revendications précédentes, dans lequel le mélange d'isoalcanes peut être obtenu par un procédé selon lequel le produit d'oligomérisation hydrogéné obtenu à l'étape c) est soumis à un traitement par mise en contact avec au moins un agent d'adsorption.

**15.** Agent cosmétique ou pharmaceutique selon l'une quelconque des revendications précédentes, sous la forme d'un onguent, d'une crème, d'une émulsion, d'une suspension, d'une lotion, d'un lait, d'une pâte, d'un gel, d'une mousse ou d'une pulvérisation.

**16.** Agent cosmétique ou pharmaceutique selon l'une quelconque des revendications précédentes, qui contient le composant A) en une proportion de 0,01 à 99,9 % en poids, par rapport au poids total de l'agent.

**17.** Agent cosmétique ou pharmaceutique selon l'une quelconque des revendications précédentes, dans lequel les composants B) et C) sont choisis parmi les véhicules, les excipients, les émulsifiants, les tensioactifs, les conservateurs, les parfums, les huiles parfumées, les épaississants, les polymères, les gélifiants, les colorants, les pigments, les agents photoprotecteurs, les agents texturants, les antioxydants, les antimousses, les antistatiques, les résines, les solvants, les solubilisants, les neutralisants, les stabilisateurs, les stérilisants, les agents gonflants, les agents siccatifs, les opacifiants, les agents cosmétiquement actifs, les polymères capillaires, les conditionneurs de cheveux et de peau, les polymères greffés, les polymères siliconés solubles ou dispersibles dans l'eau, les agents blanchissants, les agents de soin, les agents colorants, les agents de teinte, les agents bronzants, les agents de rétention de l'humidité, les surgraissants, le collagène, les hydrolysats de blanc d'oeuf, les lipides, les émollients et les plastifiants et leurs mélanges.

**18.** Agent cosmétique capillaire, contenant :

a) 0,05 à 20 % en poids d'au moins un polymère capillaire,
b) 20 à 99,95 % en poids d'un véhicule, contenant au moins un mélange d'isoalcanes, tel que défini dans l'une quelconque des revendications 1 à 14,
c) 0 à 50 % en poids d'au moins un gaz propulseur,
d) 0 à 5 % en poids d'au moins un émulsifiant,
e) 0 à 3 % en poids d'au moins un épaississant, ainsi que
f) jusqu'à 25 % en poids d'autres constituants.

**19.** Agent cosmétique capillaire selon la revendication 18, contenant en tant qu'agent actif ou à effet B) ou adjuvant C) au moins un copolymère Ca), qui peut être obtenu par copolymérisation de

(A) au moins un anhydride d'acide dicarboxylique éthyléniquement insaturé, dérivé d'au moins un acide dicarboxylique de 4 à 8 atomes C,
(B) au moins un oligomère d'un alcène en $C_3$-$C_{10}$ ramifié ou non ramifié, au moins un oligomère présentant un poids moléculaire moyen $M_n$ dans la plage allant de 300 à 5 000 g/mol ou pouvant être obtenu par oligomérisation d'au moins 3 équivalents d'alcène en $C_3$-$C_{10}$,
(C) éventuellement au moins une $\alpha$-oléfine contenant jusqu'à 24, de préférence jusqu'à 16 atomes C,
(D) éventuellement au moins un autre comonomère éthyléniquement insaturé différent de (A), (B) et (C), éventuellement mise en réaction avec
(E) au moins un composé de formule générale Ia, Ib, Ic ou Id

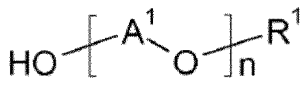

Ia

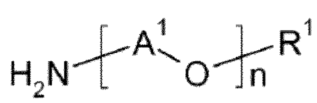

Ib

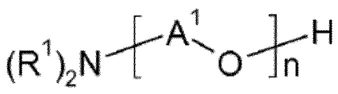

Ic

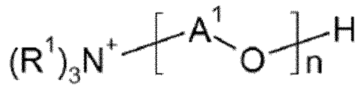

Id

dans lesquelles

les $A^1$ représentent alkylène en $C_2$-$C_{20}$, identiques ou différents,
les $R^1$ représentent alkyle en $C_1$-$C_{30}$, linéaire ou ramifié, phényle ou hydrogène,
n représente un nombre entier de 1 à 200,
puis éventuellement mise en contact avec de l'eau,
le copolymère Ca) étant éventuellement mélangé avec au moins un oligomère Z) supplémentaire d'un alcène en $C_3$-$C_{10}$ ramifié ou non ramifié, au moins un oligomère Z) présentant un poids moléculaire moyen $M_n$ dans la plage allant de 300 à 5 000 g/mol ou pouvant être obtenu par oligomérisation d'au moins 3 équivalents d'un alcène en $C_3$-$C_{10}$,
le composant copolymère B) et/ou l'oligomère Z) comprenant un mélange d'isoalcanes tel que défini dans l'une quelconque des revendications 1 à 14, ou étant constitué d'un tel mélange d'isoalcanes.

**20.** Utilisation d'un agent cosmétique ou pharmaceutique tel que défini dans l'une quelconque des revendications 1 à 14 en tant que ou dans le composant hydrophobe d'une composition cosmétique ou pharmaceutique homogène ou hétérogène.

**21.** Utilisation d'un agent cosmétique ou pharmaceutique tel que défini dans l'une quelconque des revendications 1 à 14 dans des agents pour le soin et pour la protection de la peau, des agents de nettoyage de la peau, des préparations pour la cosmétique décorative, dans des agents de traitement des cheveux, pour l'hydratation de la peau, pour le conditionnement de la peau ou des cheveux, pour la modification des propriétés rhéologiques, pour l'amélioration de la compatibilité avec la peau et/ou pour l'obtention d'un meilleur mouillage.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 4339713 A
- WO 9514647 A
- WO 9925668 A
- WO 0059849 A
- WO 0053546 A
- WO 0172670 A
- EP 1457475 A
- DE 102004018753 A1
- DE OS2360306 A
- WO 2004091555 A
- US 20010051686 A1
- US 4334113 A
- US 4225743 A
- DE 102004018753
- EP 81041 A
- DE 1568542
- DE 19957173 A
- DE 4339713
- US 3597489 A
- US 2898387 A
- GB 799396 A
- GB 1155539 A
- US 3202723 A
- SU 319582
- US 3917540 A
- US 3244644 A
- DE 19642770 A
- DE 10128242 A
- EP 0668257 A
- DE 4333238 A
- DE 3929973
- DE 2150557
- DE 2817369
- DE 3708451
- EP 570838 A1
- EP 775698 A
- EP 0916335 A
- EP 0895776 A
- DE 10353557
- DE 10355402
- DE 10345094
- US 20030157170 A


### In der Beschreibung aufgeführte Nicht-Patentliteratur


- **K. WEISSERMEL ; H.-J. ARPE.** Industrielle organische Chemie. VCH-Verlagsgesellschaft, 1994, vol. 76-81
- *Catalysis Today,* 1990, vol. 6, 329
- Organikum. VEB Deutscher Verlag, 1988, 288
- Ullmann’s Encyclopedia of Industrial Chemistry. 2000
- Römpp, Chemie Lexikon. vol. 6, S.4629f, , S.5156f
- Römpp, Chemie Lexikon. vol. 4, 3376f
- **KARL-HEINZ SCHRADER.** Grundlagen und Rezepturen der Kosmetika. Verlag Hüthig, 319-355
- **SCHRADER.** Grundlagen und Rezepturen der Kosmetika. Hüthig Buch Verlag, 1989
- H. P. Lexikon der Hilfsstoffe für Pharmazie. **FIEDLER.** Kosmetik und angrenzende Gebiete. ECV-Editio-Kantor-Verlag, 1996